# EUROPEAN PATENT APPLICATION

(11) **EP 1 466 977 A1**
(43) Date of publication of application: **13.10.2004**
(21) Application number: 04007618.4
(22) Date of filing: 02.12.1999
(51) Int. Cl.: C12N 15/12, C07K 14/47, C07K 16/18, C12N 15/62, A61K 38/17

(54) **Methods and compositions for inhibiting neoplastic cell growth**

(30) Priority: 08.03.1999 WO PCT/US99/05028
(62) Divisional of application: 99960644.5
(71) Applicant: Genentech, Inc., South San Francisco, CA 94080-4990 (US)
(72) Inventor: Ashkenazi, Avi J., San Mateo, CA 914402 (US); Goddard, Audrey, San Francisco, CA 94131 (US); Godowski, Paul J., Burlingame, CA 94010 (US); Gurney, Austin L., Belmont, CA 94002 (US); Marsters, Scot A., San Carlos, CA 94070 (US); Napier, Mary A., Hillsborough, CA 94010 (US); Pitti, Robert M., El Cerrito, CA 94530 (US); Wood, William I., Hillsborough, CA 94010 (US)
(74) Representative: Denison, Christopher Marcus

(57) **Abstract**

The present invention concerns methods and compositions for inhibiting neoplastic cell growth. In particular, the present invention concerns antitumor compositions and methods for the treatment of tumors. The invention further concerns screening methods for identifying growth inhibitory, e.g., antitumor compounds. In addition, the present invention is directed to novel polypeptides and to nucleic acid molecules encoding those polypeptides. Also provided herein are vectors and host cells comprising those nucleic acid sequences, chimeric polypeptide molecules comprising the polypeptides of the present invention fused to heterologous polypeptide sequences, antibodies which bind to the polypeptides of the present invention and to methods for producing the polypeptides of the present invention.

## Description

### FIELD OF THE INVENTION

The present invention concerns methods and compositions for inhibiting neoplastic cell growth. In particular, the present invention concerns antitumor compositions and methods for the treatment of tumors. The invention further concerns screening methods for identifying growth inhibitory, eg., antitumor compounds.

### BACKGROUND OF THE INVENTION

Malignant tumors (cancers) are the second leading cause of death in the United States, after heart disease (Boring *et al.,* CA Cancel J. Clin., 43:7 (1993)).

Cancer is characterized by the increase in the number of abnormal, or neoplastic, cells derived from a normal tissue which proliferate to form a tumor mass, the invasion of adjacent tissues by these neoplastic tumor cells, and the generation of malignant cells which eventually spread via the blood or lymphatic system to regional lymph nodes and to distant sites (metastasis). In a cancerous state a cell proliferates under conditions in which normal cells would not grow. Cancer manifests itself in a wide variety of forms, characterized by different degrees of invasiveness and aggressiveness.

Despite recent advances in cancer therapy, there is a great need for new therapeutic agents capable of inhibiting neoplastic cell growth. Accordingly, it is the objective of the present invention to identify compounds capable of inhibiting the growth of neoplastic cells, such as cancer cells.

### SUMMARY OF THE INVENTION

### A. Embodiments

The present invention relates to methods and compositions for inhibiting neoplastic cell growth. More particularly, the invention concerns methods and compositions for the treatment of tumors, including cancers, such as breast, prostate, colon, lung, ovarian, renal and CNS cancers, leukemia, melanoma, etc., in mammalian patients, preferably humans.

In one aspect, the present invention concerns compositions of matter useful for the inhibition of neoplastic cell growth comprising an effective amount of a PRO179, PRO207, PRO320, PRO219, PRO221, PRO224, PRO328, PRO301, PRO526, PRO362, PRO356, PRO509 or PRO866 polypeptide as herein defined, or an agonist thereof, in admixture with a pharmaceutically acceptable carrier. In a preferred embodiment, the composition of matter comprises a growth inhibitory amount of a PRO179, PRO207, PRO320, PRO219, PRO221, PRO224, PRO328, PRO301, PRO526, PRO362, PRO356, PRO509 or PRO866 polypeptide, oran agonist thereof. in another preferred embodiment, the composition comprises a cytotoxic amount of a PRO179, PRO207, PRO320, PRO219, PRO221, PRO224, PRO328, PRO301, PRO526, PRO362, PRO356, PRO509 or PRO866 polypeptide, or an agonist thereof. Optionally, the compositions of matter may contain one or more additional growth inhibitory and/or cytotoxic and/or other chemotherapeutic agents.

In a further aspect, the present invention concerns compositions of matter useful for the treatment of a tumor in a mammal comprising a therapeutically effective amount of a PRO179, PRO207, PRO320, PRO219, PRO221, PRO224, PRO328, PRO301, PRO526, PRO362, PRO356, PRO509 or PRO866 polypeptide as herein defined, or an agonist thereof. The tumor is preferably a cancer.

In another aspect, the invention concerns a method for inhibiting the growth of a tumor cell comprising exposing the cell to an effective amount of a PRO179, PRO207, PRO320, PRO219, PRO221, PRO224, PRO328, PRO301, PRO526, PRO362, PRO356, PRO509 or PRO866 polypeptide as herein defined, or an agonist thereof. In a particular embodiment, the agonist is an anti-PRO179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 or anti-PRO866 agonist antibody. In another embodiment, the agonist is a small molecule that mimics the biological activity of a PRO179, PRO207, PRO320, PRO219, PRO221, PRO224, PRO328, PRO301, PRO526, PRO362, PRO356, PRO509 or PRO866 polypeptide. The method may be performed *in vitro* or *in vivo.*

In a still further embodiment, the present invention provides an article of manufacture comprising:
(a) a container;
(b) a composition comprising an active agent contained within the container; wherein the composition is effective for inhibiting the neoplastic cell growth, e.g., growth of tumor cells, and the active agent in the composition is a PRO179, PRO207, PRO320, PRO219, PRO221, PRO224, PRO328, PRO301, PRO526. PRO362, PRO356, PRO509 or PRO866 polypeptide as herein defined, or an agonist thereof; and
(c) a label affixed to said container, or a package insert included in said container referring to the use of said PRO179, PRO207, PRO320, PRO219, PRO221, PRO224, PRO328, PRO301, PRO526, PRO362, PRO356, PRO509 or PRO866 polypeptide or agonist thereof, for the inhibition of neoplastic cell growth, wherein the agonist may be an antibody which binds to the PRO179, PRO207, PRO320, PRO219, PRO221, PRO224, PRO328, PRO301, PRO526, PRO362, PRO356, PRO509 or PRO866 polypeptide. In a particular embodiment, the agonist is an anti-PRO179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 or anti-PRO866 agonist antibody. In another embodiment, the agonist is a small molecule that mimics the biological activity of a PRO179, PRO207, PRO320, PRO219, PRO221, PRO224, PRO328, PRO301, PRO526, PRO362, PRO356, PRO509 or PRO866 polypeptide. Similar articles of manufacture comprising a PRO179, PRO207, PRO320, PRO219, PRO221, PRO224, PRO328, PRO301, PRO526, PRO362, PRO356, PRO509 or PRO866 polypeptide as herein defined, or an agonist thereof in an amount that is therapeutically effective for the treatment of tumor are also within the scope of the present invention. Also within the scope of the invention are articles of manufacture comprising a PRO179, PRO207, PRO320, PRO219, PRO221, PRO224, PRO328, PRO301, PRO526, PRO362, PRO356, PROS09 or PRO866 polypeptide as herein defined, or an agonist thereof, and a further growth inhibitory agent, cytotoxic agent or chemotherapeutic agent.

### B. Additional Embodiments

In other embodiments of the present invention, the invention provides an isolated nucleic acid molecule comprising a nucleotide sequence that encodes a PRO179, PRO207, PRO320, PRO219, PRO221, PRO224, PRO328, PRO301, PRO526, PRO362, PRO356, PRO509 or PRO866 polypeptide.

In one aspect, the isolated nucleic acid molecule comprises a nucleotide sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91 % sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to (a) a DNA molecule encoding a PRO179, PRO207, PRO320, PRO219, PRO221, PRO224, PRO328, PRO301, PRO526, PRO362, PRO356, PRO509 or PRO866 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yetmore preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to (a) a DNA molecule comprising the coding sequence of full-length PRO179, PRO207, PRO320, PRO219, PRO221, PRO224, PRO328, PRO301, PRO526, PRO362, PRO356, PRO509 or PRO866 polypeptide cDNA as disclosed herein, the coding sequence of a PRO179, PRO207, PRO320, PRO219, PRO221, PRO224, PRO328, PRO301, PRO526, PRO362, PRO356, PRO509 or PRO866 polypeptide lacking the signal peptide as disclosed herein, the coding sequence of an extracellular domain of a transmembrane PRO179, PRO207, PRO320, PRO219, PRO221, PRO224, PRO328, PRO301, PRO526, PRO362, PRO356, PRO509 or PRO866 polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to (a) a DNA molecule that encodes the same mature polypeptide encoded by any of the human protein cDNAs deposited with the ATCC as disclosed herein, or (b) the complement of the DNA molecule of (a).

Another aspect the invention provides an isolated nucleic acid molecule comprising a nucleotide sequence encoding a PRO179, PRO207, PRO320, PRO219, PRO221, PRO224, PRO328, PRO301, PRO526, PRO362, PRO356, PRO509 or PRO866 polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO179, PRO207, PRO320, PRO219, PRO221, PRO224, PRO328, PRO301, PRO526, PRO362, PRO356, PRO509 or PRO866 polypeptides are contemplated.

Another embodiment is directed to fragments of a PRO179, PRO207, PRO320, PRO219, PRO221, PRO224, PRO328, PRO301, PRO526, PRO362, PRO356, PRO509 or PRO866 polypeptide coding sequence, or the complement thereof, that may find use as, for example, hybridization probes, for encoding fragments of a PRO179, PRO207, PRO320, PRO219, PRO221, PRO224, PRO328, PRO301, PRO526, PRO362, PRO356, PRO509 or PRO866 polypeptide that may optionally encode a polypeptide comprising a binding site for an anti-PRO179, anti-PRO207,anti-PRO320,anti-PRO219,anti-PRO221, anti-PRO224, anti-PRO328, anti-PRO301, anti-PRO526, anti-PRO362, anti-PRO356, anti-PRO509 or anti-PRO866 antibody or as antisense oligonucleotide probes. Such nucleic acid fragments are usually at least about 20 nucleotides in length, preferably at least about 30 nucleotides in length, more preferably at least about 40 nucleotides in length, yet more preferably at least about 50 nucleotides in length, yet more preferably at least about 60 nucleotides in length, yet more preferably at least about 70 nucleotides in length, yet more preferably at least about 80 nucleotides in length, yet more preferably at least about 90 nucleotides in length, yet more preferably at least about 100 nucleotides in length, yet more preferably at least about 110 nucleotides in length, yet more preferably at least about 120 nucleotides in length, yet more preferably at least about 130 nucleotides in length, yet more preferably at least about 140 nucleotides in length, yet more preferably at least about 150 nucleotides in length, yet more preferably at least about 160 nucleotides in length, yet more preferably at least about 170 nucleotides in length, yet more preferably at least about 180 nucleotides in length, yet more preferably at least about 190 nucleotides in length, yet more preferably at least about 200 nucleotides in length, yet more preferably at least about 250 nucleotides in length, yet more preferably at least about 300 nucleotides in length, yet more preferably at least about 350 nucleotides in length, yet more preferably at least about 400 nucleotides in length, yet more preferably at least about 450 nucleotides in length, yet more preferably at least about 500 nucleotides in length, yet more preferably at least about 600 nucleotides in length, yet more preferably at least about 700 nucleotides in length, yet more preferably at least about 800 nucleotides in length, yet more preferably at least about 900 nucleotides in length and yet more preferably at least about 1000 nucleotides in length, wherein in this context the term "about" means the referenced nucleotide sequence length plus or minus 10% of that referenced length. It is noted that novel fragments of a PRO179, PRO207, PRO320, PRO219, PRO221, PRO224, PRO328, PRO301, PRO526, PRO362, PRO356, PRO509 or PRO866 polypeptide-encoding nucleotide sequence may be determined in a routine manner by aligning the PRO179, PRO207, PRO320, PRO219, PRO221, PRO224, PRO328, PRO301, PRO526, PRO362, PRO356, PRO509 or PRO866 polypeptide-encoding nucleotide sequence with other known nucleotide sequences using any of a number of well known sequence alignment programs and determining which PRO179, PRO207, PRO320, PRO219, PRO221, PRO224, PRO328, PRO301, PRO526, PRO362, PRO356, PRO509 or PRO866 polypeptide-encoding nucleotide sequence fragment(s) are novel. All of such PRO179, PRO207, PRO320, PRO219, PRO221, PRO224, PRO328, PRO301, PRO526, PRO362, PRO356, PRO509 or PRO866 polypeptide-encoding nucleotide sequences are contemplated herein. Also contemplated are the PRO179, PRO207, PRO320, PRO219, PRO221, PRO224, PRO328, PRO301, PRO526, PRO362, PRO356, PRO509 or PRO866 polypeptide fragments encoded by these nucleotide molecule fragments, preferably those PRO179, PRO207, PRO320, PRO219, PRO221, PRO224, PRO328, PRO301, PRO526, PRO362, PRO356, PRO509 or OPRO866 polypeptide fragments that comprise a binding site for an anti-PRO179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 or anti-PRO866 antibody.

In another embodiment, the invention provides isolated PRO179, PRO207, PRO320, PRO219, PRO221, PRO224, PRO328, PRO301, PRO526, PRO362, PRO356, PRO509 or PRO866 polypeptide encoded by any of the isolated nucleic acid sequences hereinabove identified.

In a certain aspect, the invention concerns an isolated PRO179, PRO207, PRO320, PRO219, PRO221, PRO224, PRO328, PRO301, OPRO526, PRO362, PRO356, PRO509 or PRO866 polypeptide, comprising an amino acid sequence having at least about 80% sequence identity, preferably at least about 81 % sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to a PRO179, PRO207, PRO320, PRO219, PRO221, PRO224, PRO328, PRO301, PRO526, PRO362, PRO356, PRO509 or PRO866 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment ofthe full-length amino acid sequence as disclosed herein.

In a further aspect, the invention concerns an isolated PRO179, PRO207, PRO320, PRO219, PRO221, PRO224, PRO328, PRO301, PRO526, PRO362, PRO356, PRO509 or PRO866 polypeptide comprising an amino acid sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91 % sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to an amino acid sequence encoded by any of the human protein cDNAs deposited with the ATCC as disclosed herein.

In a further aspect, the invention concerns an isolated PRO179, PRO207, PRO320, PRO219, PRO221, PRO224, PRO328, PRO301, PRO526, PRO362, PRO356, PRO509 or PRO866 polypeptide comprising an amino acid sequence scoring at least about 80% positives, preferably at least about 81 % positives, more preferably at least about 82% positives, yet more preferably at least about 83% positives, yet more preferably at least about 84% positives, yet more preferably at least about 85% positives, yet more preferably at least about 86% positives, yet more preferably at least about 87% positives, yet more preferably at least about 88% positives, yet more preferably at least about 89% positives, yet more preferably at least about 90% positives, yet more preferably at least about 91% positives, yet more preferably at least about 92% positives, yet more preferably at least about 93% positives, yet more preferably at least about 94% positives, yet more preferably at least about 95% positives, yet more preferably at least about 96% positives, yet more preferably at least about 97% positives, yet more preferably at least about 98% positives and yet more preferably at least about 99% positives when compared with the amino acid sequence of a PRO179, PRO207, PRO320, PRO219, PRO221, PRO224, PRO328, PRO301, PRO526, PRO362, PRO356, PRO509 or PRO866 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a specific aspect, the invention provides an isolated PRO179, PRO207, PRO320, PRO219, PRO221, PRO224, PRO328, PRO301, PRO526, PRO362, PRO356, PRO509 or PRO866 polypeptide without the N-terminal signal sequence and/or the initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule under conditions suitable for expression of the PRO179, PRO207, PRO320, PRO219, PRO221, PRO224, PRO328, PRO301, PRO526, PRO362, PRO356, PRO509 or PRO866 polypeptide and recovering the PRO179, PRO207, PRO320, PRO219, PRO221, PRO224, PRO328, PRO301, PRO526, PRO362, PRO356, PRO509 or PRO866 polypeptide from the cell culture.

Another aspect of the invention provides an isolated PRO179, PRO207, PRO320, PRO219, PRO221, PRO224, PRO328, PRO301, PRO526, PRO362, PRO356, PRO509 or PRO866 polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO179, PRO207, PRO320, PRO219, PRO221, PRO224, PRO328, PRO301, PRO526, PRO362, PRO356, PRO509 or PRO866 polypeptide and recovering the PRO179, PRO207, PRO320, PRO219, PRO221, PRO224, PRO328, PRO301, PRO526, PRO362, PRO356, PRO509 or PRO866 polypeptide from the cell culture.

In yet another embodiment, the invention concerns agonists of a native PRO179, PRO207, PRO320, PRO219, PRO221, PRO224, PRO328, PRO301, PRO526, PRO362, PRO356, PRO509 or PRO866 polypeptide as defined herein. In a particular embodiment, the agonist is an anti-PRO179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 or anti-PRO866 agonist antibody or a small molecule.

In a further embodiment, the invention concerns a method of identifying agonists to a PRO179, PRO207, PRO320, PRO219, PRO221, PRO224, PRO328, PRO301, PRO526, PRO362, PRO356, PRO509 or PRO866 polypeptide which comprise contacting the PRO179, PRO207, PRO320, PRO219, PRO221, PRO224, PRO328, PRO301, PRO526, PRO362, PRO356, PRO509 or PRO866 polypeptide with a candidate molecule and monitoring a biological activity mediated by said PRO179, PRO207, PRO320, PRO219, PRO221, PRO224, PRO328, PRO301, PRO526, PRO362, PRO356, PRO509 or PRO866 polypeptide. Preferably, the PRO179, PRO207, PRO320, PRO219, PRO221, PRO224, PRO328, PRO301, PRO526, PRO362, PRO356, PRO509 or PRO866 polypeptide is a native PRO179, PRO207, PRO320, PRO219, PRO221, PRO224, PRO328, PRO301, PRO526, PRO362, PRO356, PRO509 or PRO866 polypeptide.

In a still further embodiment, the invention concerns a composition of matter comprising a PRO179, PRO207, PRO320, PRO219, PRO221, PRO224, PRO328, PRO301, PRO526, PRO362, PRO356, PRO509 or PRO866 polypeptide, or an agonist of a PRO179, PRO207, PRO320, PRO219, PRO221, PRO224, PRO328, PRO301, PRO526, PRO362, PRO356, PRO509 or PRO866 polypeptide as herein described, or an anti-PRO179, anti-PRO207, anti-PRO320, witi-PRO219, anti-PRO221,anti-PRO224,anti-PRO328,anti-PRO301, anti-PRO526, anti-PRO362, anti-PRO356, anti-PRO509 or anti-PRO866 agonist antibody, in combination with a carrier. Optionally, the carrier is a pharmaceutically acceptable carrier.

Another embodiment of the present invention is directed to the use of a PRO179, PRO207, PRO320, PRO219, PRO221, PRO224, PRO328, PRO301, PRO526, PRO362, PRO356, PRO509 or PRO866 polypeptide, or an agonist thereof as hereinbefore described, or an anti-PRO179, anti-PRO207, anti-PRO320, anti-PRO219, anti-PRO221, anti-PRO224, anti-PRO328, anti-PRO301,anti-PRO526, anti-PRO362, anti-PRO356, anti-PRO509 or anti-PRO866 agonist antibody, for the preparation of a medicament useful in the treatment of a condition which is responsive to the PRO179, PRO207, PRO320, PRO219, PRO221, PRO224, PRO328, PRO301, PRO526, PRO362, PRO356, PRO509 or PRO866 polypeptide, an agonist thereof or an anti-PRO179, anti-PRO207, anti-PRO320, anti-PRO219, anti-PRO221, anti-PRO224, anti-PRO328, anti-PRO301, anti-PRO526, anti-PRO362,anti-PRO356, anti-PRO509 or anti-PRO866 agonist antibody.

In other embodiments of the present invention, the invention provides vectors comprising DNA encoding any of the herein described polypeptides. Host cell comprising any such vector are also provided. By way of example, the host cells may be CHO cells, *E. coli,* yeast, or Baculovirus-infected insect cells. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

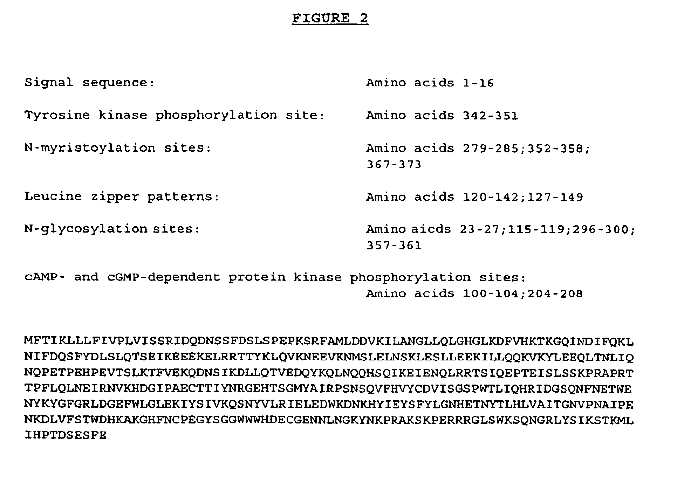

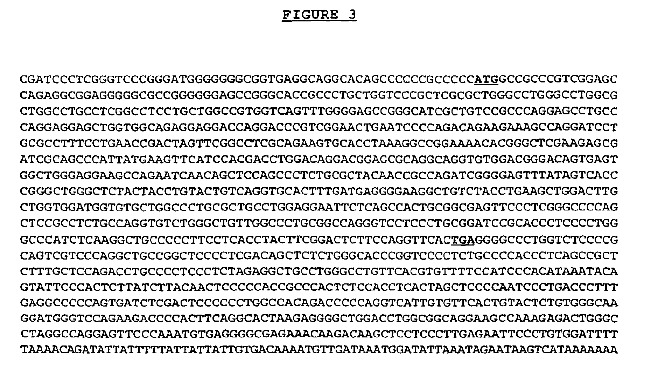

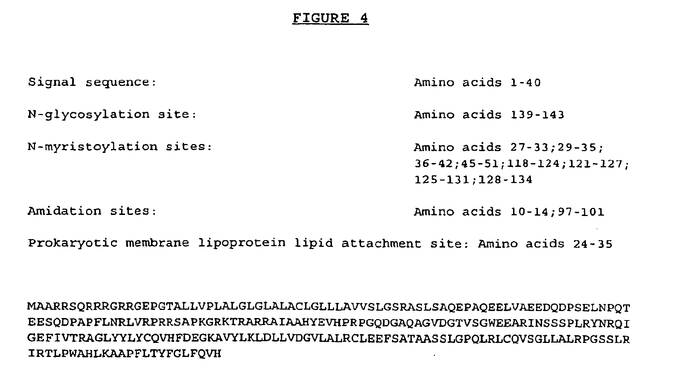

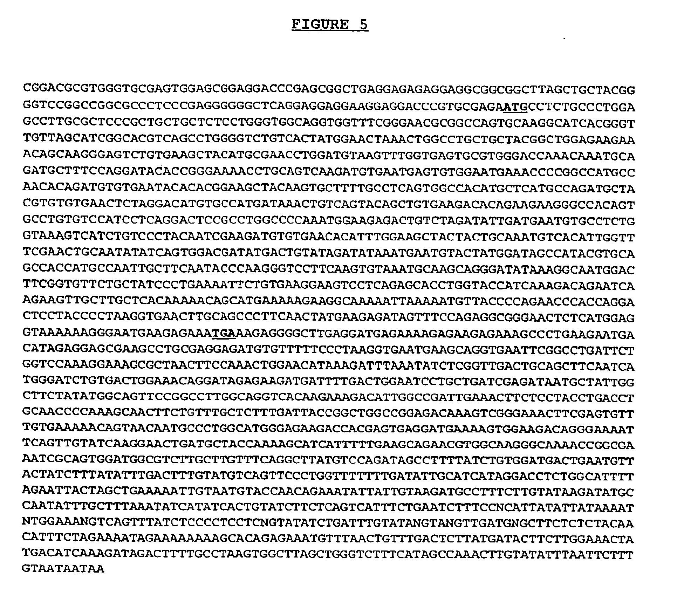

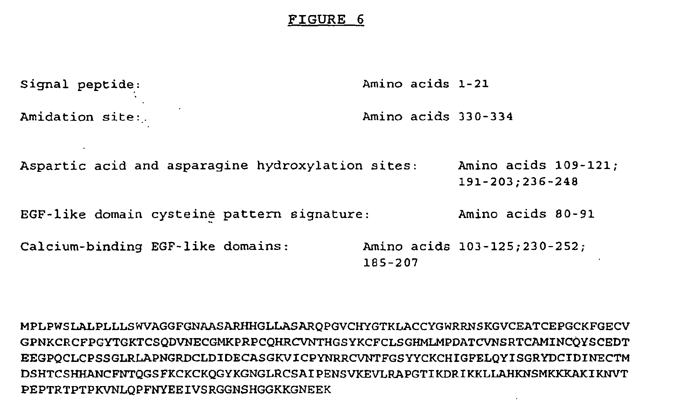

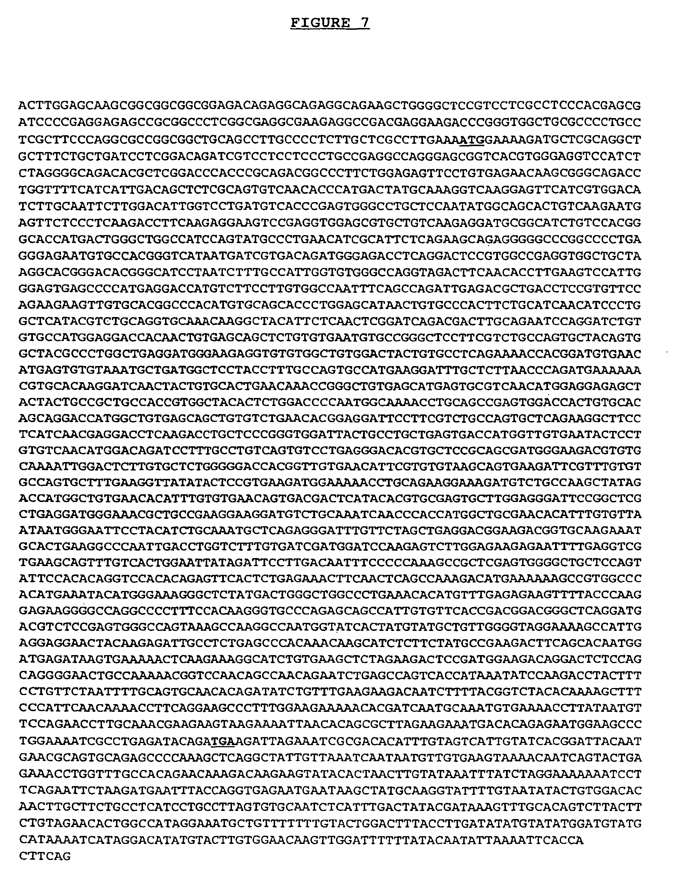

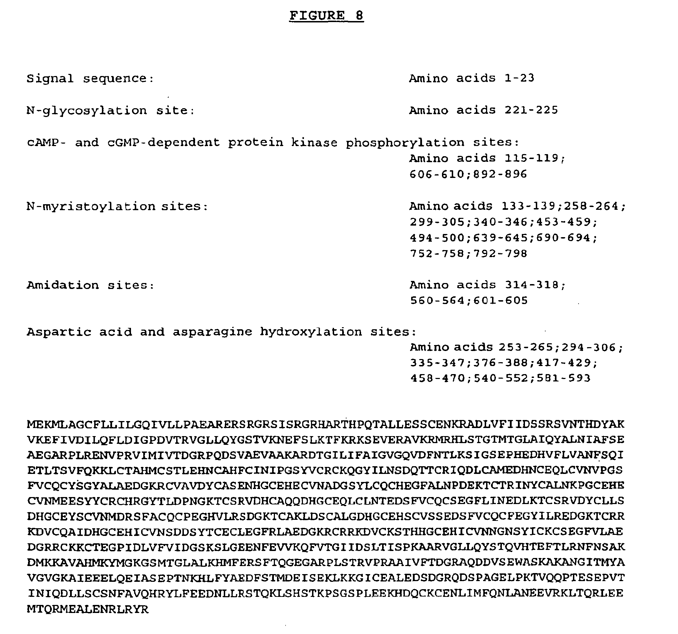

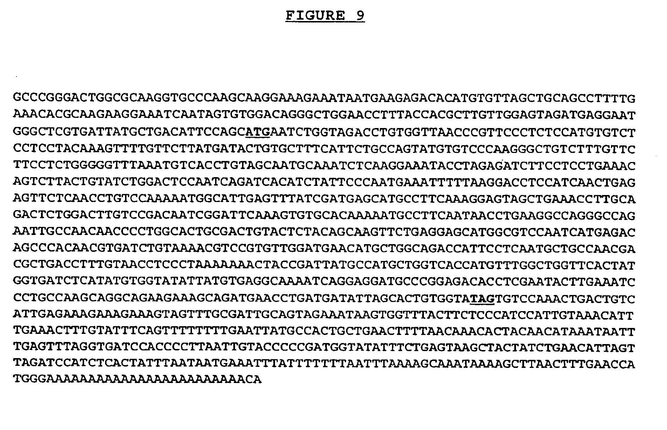

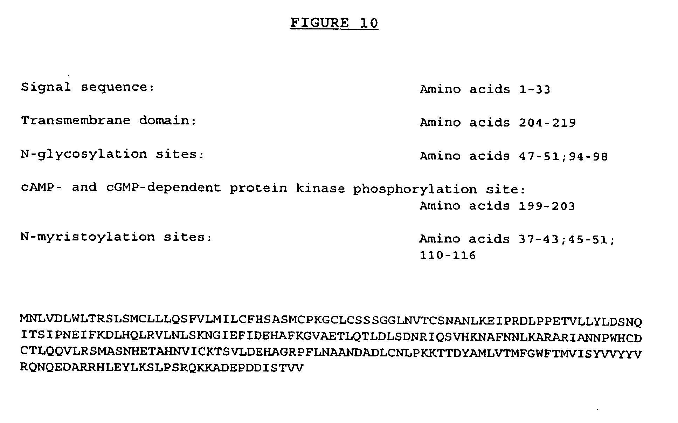

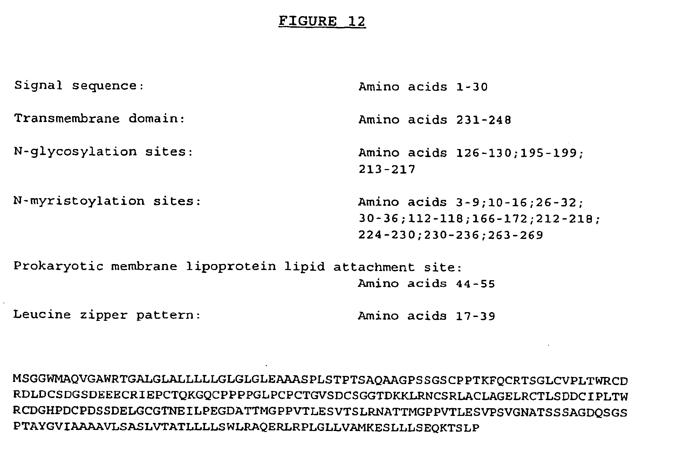

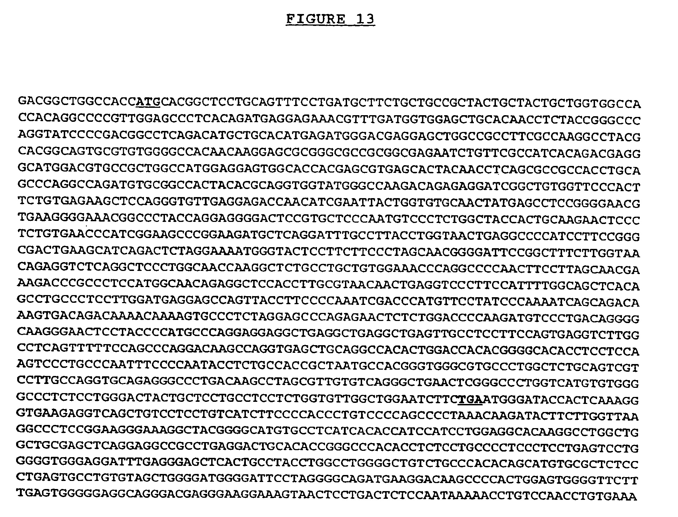

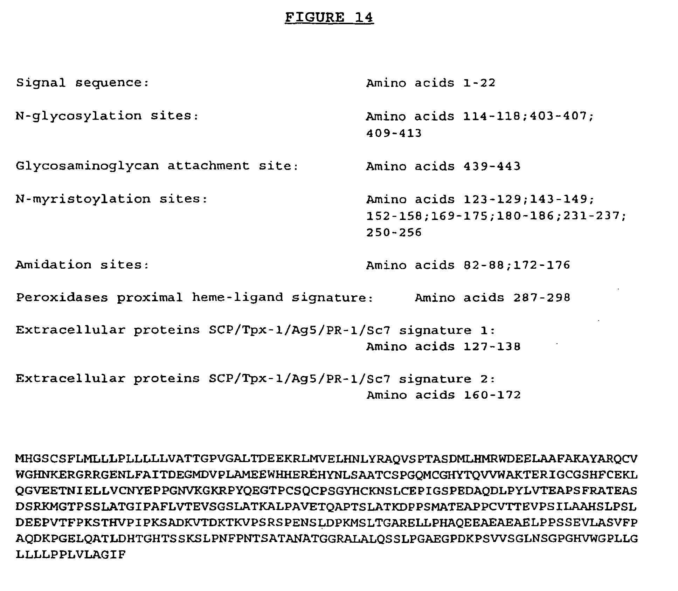

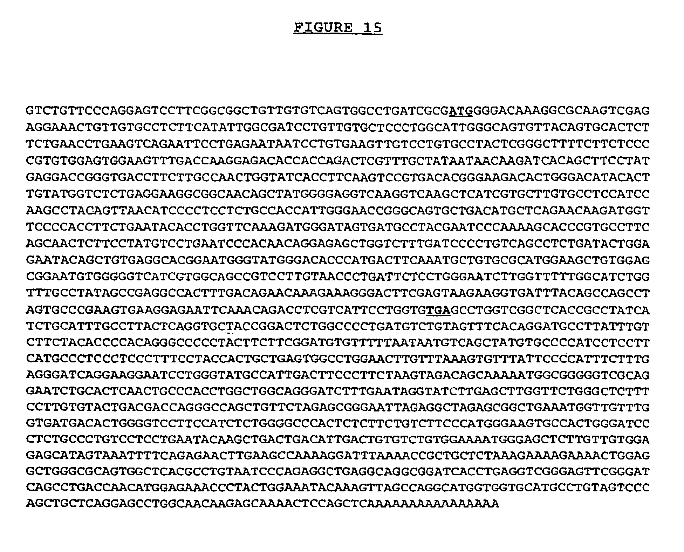

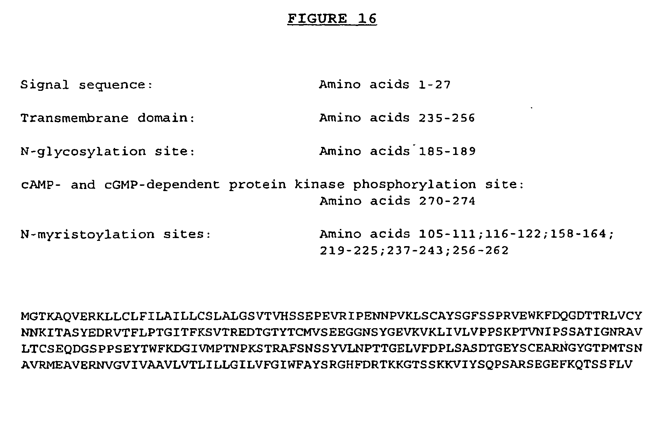

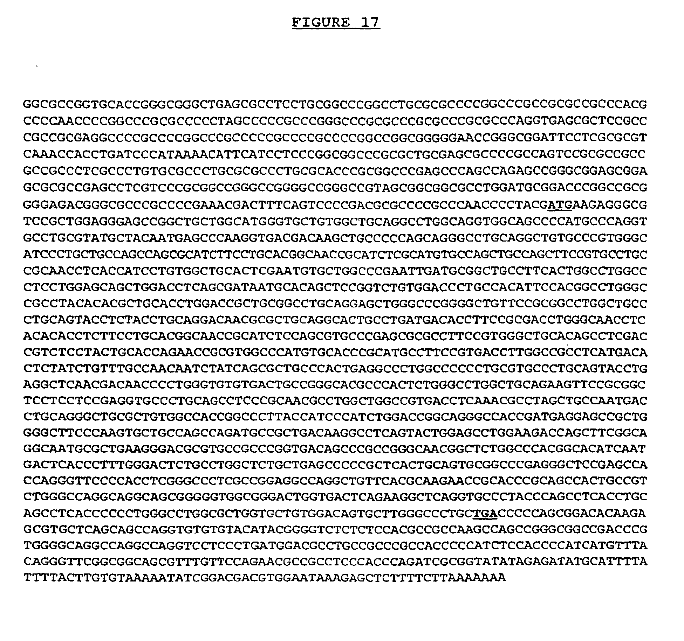

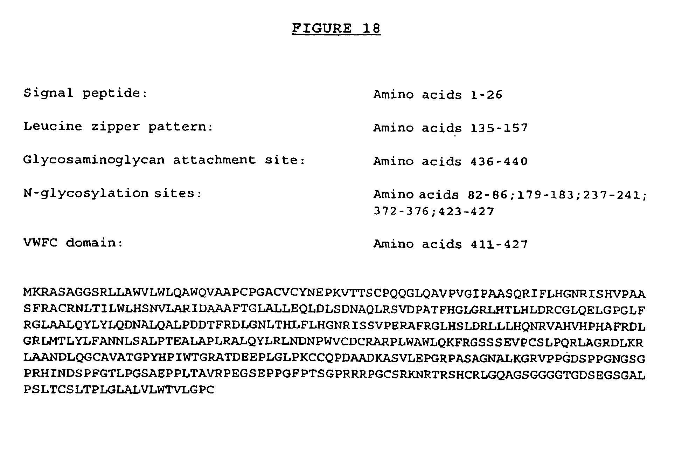

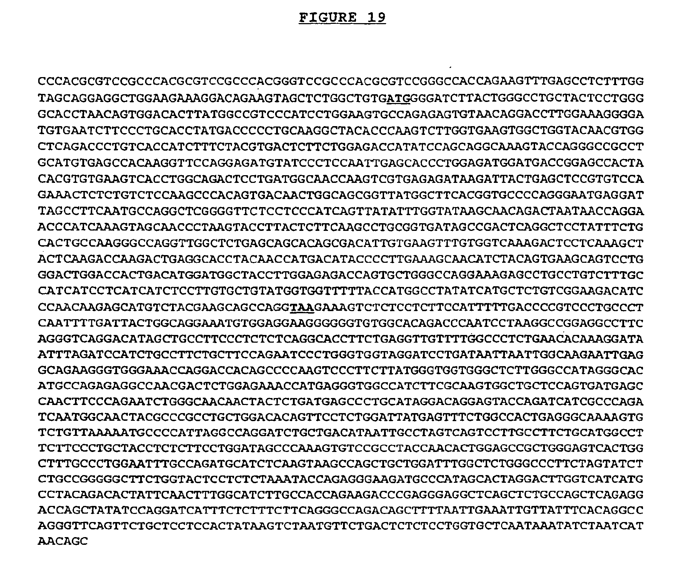

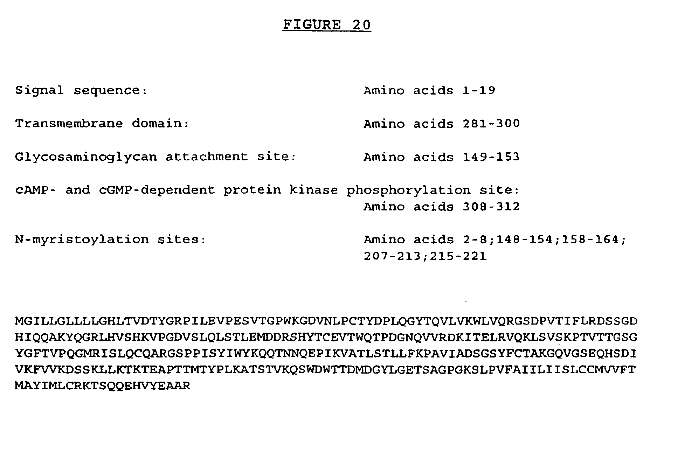

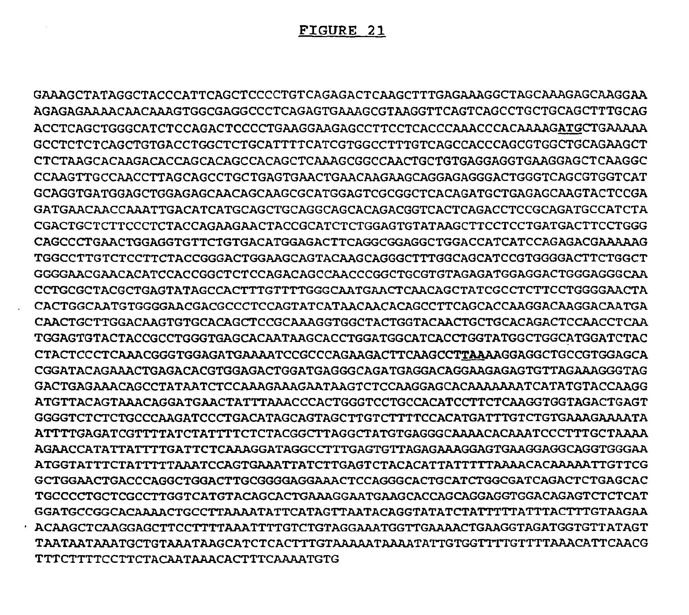

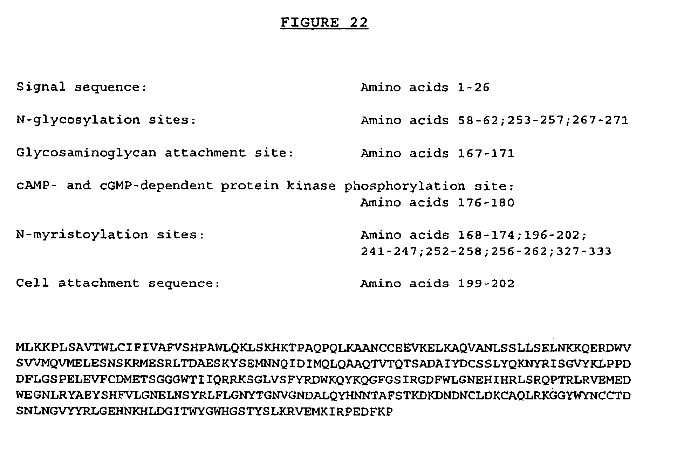

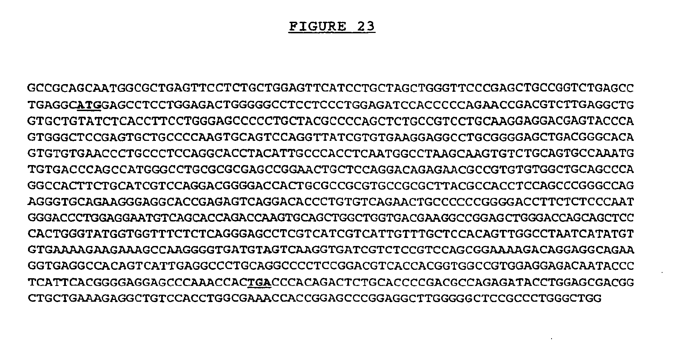

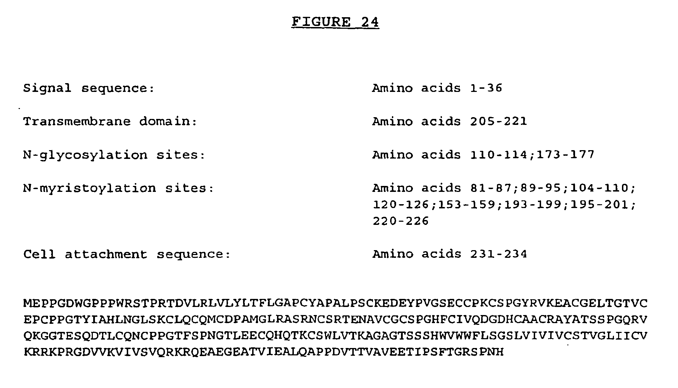

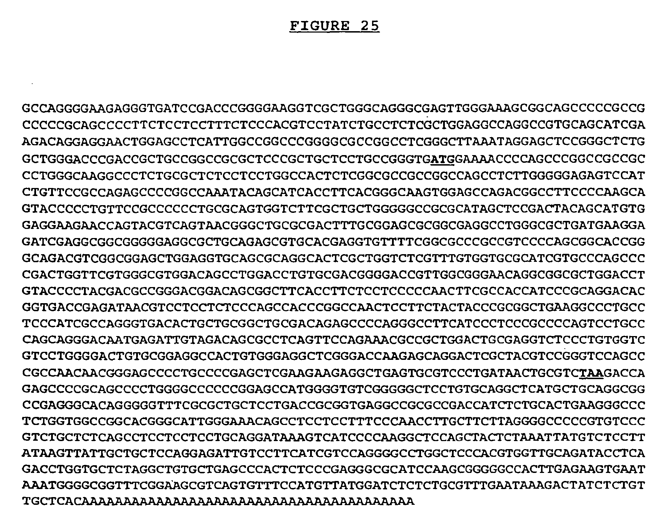

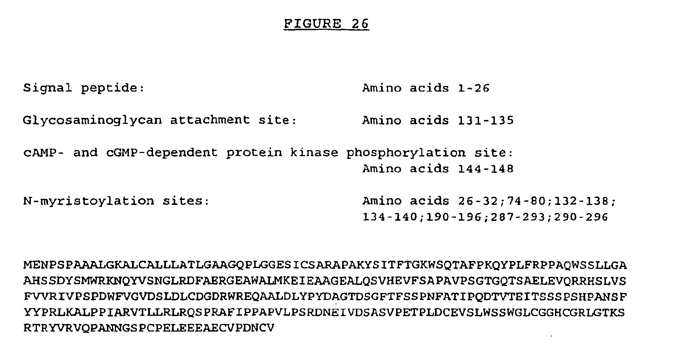

The following clauses, also define aspects and embodiments of the invention.
1. A composition of matter useful for the inhibition of neoplastic cell growth, said composition comprising an effective amount of a PRO179, PRO207, PRO320, PRO219, PRO221, PRO224, PRO328, PRO301, PRO526, PRO362, PRO356, PRO509 or PRO866 polypeptide, or an agonist thereof, in admixture with a pharmaceutically acceptable carrier.
2. The composition of matter of Clause I comprising a growth inhibitory amount of a PRO179, PRO207, PRO320, PRO219, PRO221, PRO224, PRO328, PRO301, PRO526, PRO362, PRO356, PRO509 or PRO866 polypeptide, or an agonist thereof.
3. The composition of matter of Clause 1 comprising a cytotoxic amount of a PRO179, PRO207, PRO320, PRO219, PRO221, PRO224, PRO328, PRO301, PRO526, PRO362, PRO356, PRO509 or PRO866 polypeptide, or an agonist thereof.
4. The composition of matter of Clause 1 additionally comprising a further growth inhibitory agent, cytotoxic agent or chemotherapeutic agent.
5. A composition of matter useful for the treatment of a tumor in a mammal, said composition comprising a therapeutically effective amount of a PRO179, PRO207, PRO320, PRO219, PRO221, PRO224, PRO328, PRO301, PRO526, PRO362, PRO356, PRO509 or PRO866 polypeptide, or an agonist thereof.
6. The composition of matter of Clause 5, wherein said tumor is a cancer.
7. The composition of matter of Clause 6, wherein the cancer is selected from the group consisting of breast cancer, ovarian cancer, renal cancer, colorectal cancer, uterine cancer, prostate cancer, lung cancer, bladder cancer, central nervous system cancer, melanoma and leukemia.
8. A method for inhibiting the growth of a tumor cell comprising exposing said tumor cell to an effective amount of a PRO179, PRO207, PRO320, PRO219, PRO221, PRO224, PRO328, PRO301, PRO526, PRO362, PRO356, PRO509 or PRO866 polypeptide, or an agonist thereof.
9. The method of Clause 8, wherein said agonist is an anti-PRO179, anti-PRO207, anti-PRO320, anti-PRO219, anti-PRO221, anti-PRO224, anti-PRO328, anti-PRO301, anti-PRO526, anti- PRO362, anti-PRO356, anti-PRO509 or anti-PRO866 agonist antibody.
10. The method of Clause 8, wherein said agonist is a small molecule mimicking the biological activity of a PRO179, PRO207, PRO320, PRO219, PRO221, PRO224, PRO328, PRO301, PRO526, PRO362,
11. The method of Clause 8, wherein said step of exposing occurs *in vitro.*
12. The method of Clause 8, wherein said step of exposing occurs *in vivo.*
13. An article of manufacture comprising:
   (a) a container; and
   (b) a composition comprising an active agent contained within the container; wherein said active agent in the composition is a PRO179, PRO207, PRO320, PRO219, PRO221, PRO224, PRO328, PRO301, PRO526, PRO362, PRO356, PRO509 or PRO866 polypeptide, or an agonist thereof.
14. The article of manufacture of Clause 13, further comprising a label affixed to said container, or a package insert included in said container, referring to the use of said composition for the inhibition of neoplastic cell growth.
15. The article of manufacture of Clause 13, wherein said agonist is an anti-PRO179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 or anti-PRO866 agonist antibody.
16. The article of manufacture of Clause 13, wherein said agonist is a small molecule mimicking the biological activity of a PRO179, PRO207, PRO320, PRO219, PRO221, PRO224, PRO328, PRO301, PRO526, PRO362, PRO356, PRO509 or PRO866 polypeptide.
17. The article of manufacture of Clause 13, wherein said active agent is present in an amount that is effective for the treatment of tumor in a mammal.
18. The article of manufacture of Clause 13, wherein said composition additionally comprises a further growth inhibitory agent, cytotoxic agent or chemotherapeutic agent.
19. Isolated nucleic acid having at least 80% nucleic acid sequence identity to a nucleotide sequence that encodes an amino acid sequence selected from the group consisting of the amino acid sequence shown in Figure 2 (SEQ ID NO:2), Figure 4 (SEQ ID NO:7), Figure 6 (SEQ ID NO:10), Figure 8 (SEQ ID NO:15), Figure 10 (SEQ ID NO:20), Figure 12 (SEQ ID NO:25), Figure 14 (SEQ ID NO:30), Figure 16 (SEQ ID NO:35), Figure 18 (SEQ ID NO:43), Figure 20 (SEQ ID NO:48), Figure 22 (SEQ ID NO:55), Figure 24 (SEQ ID NO:60), and Figure 26 (SEQ ID NO:62).
20. Isolated nucleic acid having at least 80% nucleic acid sequence identity to a nucleotide sequence selected from the group consisting of the nucleotide sequence shown in Figure I (SEQ ID NO: 1), Figure 3 (SEQ ID NO:6), Figure 5 (SEQ ID NO:9), Figure 7 (SEQ ID NO: 14), Figure 9 (SEQ ID NO:19), Figure II (SEQ ID NO:24), Figure 13 (SEQ ID NO:29), Figure 15 (SEQ ID NO:34), Figure 17 (SEQ ID NO:42), Figure 19 (SEQ ID NO:47), Figure 21 (SEQ ID NO:54), Figure 23 (SEQ ID NO:59), and Figure 25 (SEQ ID NO:61).
21. Isolated nucleic acid having at least 80% nucleic acid sequence identity to a nucleotide sequence selected from the group consisting of the full-length coding sequence of the nucleotide sequence shown in Figure I (SEQ ID NO: 1), Figure 3 (SEQ ID NO:6), Figure 5 (SEQ ID NO:9), Figure 7 (SEQ ID NO: 14), Figure 9 (SEQ ID NO:19), Figure II (SEQ ID NO:24), Figure 13 (SEQ ID NO:29), Figure 15 (SEQ ID NO:34), Figure 17 (SEQ ID NO:42), Figure 19 (SEQ ID NO:47), Figure 21 (SEQ ID NO:54), Figure 23 (SEQ ID NO:59), and Figure 25 (SEQ ID NO:61).
22. Isolated nucleic acid having at least 80% nucleic acid sequence identity to the full-length coding sequence of the DNA deposited under ATCC accession number 209281, 209358, 209670, 209384, 209262, 209263, 209438, 209432, 209704, 209620, 209422, or 209750.
23. A vector comprising the nucleic acid of any one of Clauses 19 to 22.
24. The vector of Clause 23 operably linked to control sequences recognized by a host cell transformed with the vector.
25. A host cell comprising the vector of Clause 23.
26. The host cell of Clause 25, wherein said cell is a CHO cell.
27. The host cell of Clause 25, wherein said cell is an *E*. *coli.*
28. The host cell of Clause 25, wherein said cell is a yeast cell.
29. The host cell of Clause 25, wherein said cell is a Baculovirus-infected insect cell.
30. A process for producing a PRO179, PRO207, PRO320, PRO219, PRO221, PRO224, PRO328, PRO301, PRO526, PRO362, PRO356, PRO509 or PRO866 polypeptide comprising culturing the host cell of Clause 25 under conditions suitable for expression of said polypeptide and recovering said polypeptide from the cell culture.
31. An isolated polypeptide having at least 80% amino acid sequence identity to an amino acid sequence selected from the group consisting of the amino acid sequence shown in Figure 2 (SEQ )DNO:2), Figure 4 (SEQ ID NO:7), Figure 6 (SEQ ID NO:10), Figure 8 (SEQ ID NO:15), Figure 10 (SEQ ID NO:20), Figure 12 (SEQ ID NO:25), Figure 14 (SEQ ID NO:30), Figure 16 (SEQ ID NO:35), Figure 18 (SEQ ID NO:43), Figure 20 (SEQ ID NO:48), Figure 22 (SEQ ID NO:55), Figure 24 (SEQ ID NO:60), and Figure 26 (SEQ ID NO:62).
32. An isolated polypeptide scoring at least 80% positives when compared to an amino acid sequence selected from the group consisting of the amino acid sequence shown in Figure 2 (SEQ ID NO:2), Figure 4 (SEQ ID NO:7), Figure 6 (SEQ ID NO:10), Figure 8 (SEQ ID NO: 15), Figure 10 (SEQ ID NO:20), Figure 12 (SEQ ID NO:25), Figure 14 (SEQ ID NO:30), Figure 16 (SEQ ID NO:35), Figure 18 (SEQ ID NO:43), Figure 20 (SEQ ID NO:48). Figure 22 (SEQ ID NO:55), Figure 24 (SEQ ID NO:60), and Figure 26 (SEQ ID NO:62).
33. An isolated polypeptide having at least 80% amino acid sequence identity to an amino acid sequence encoded by the full-length coding sequence of the DNA deposited under ATCC accession number 209281, 209358, 209670, 209384, 209262, 209263, 209438, 209432, 209704, 209620, 209422, or 209750.
34. A chimeric molecule comprising a polypeptide according to any one of Clauses 3 to 33 fused to a heterologous amino acid sequence.
35. The chimeric molecule of Clause 34, wherein said heterologous amino acid sequence is an epitope tag sequence.
36. The chimeric molecule of Clause 34, wherein said heterologousamino acid sequence is a Fc region of an immunoglobulin.
37. An antibody which specifically binds to a polypeptide according to any one of Clauses 3 to 33.
38. The antibody of Clause 37, wherein said antibody is a monoclonal antibody, a humanized antibody or a single-chain antibody.
39. Isolated nucleic acid having at least 80% nucleic acid sequence identity to:
   (a) a nucleotide sequence encoding the polypeptide shown in Figure 2 (SEQ ID NO:2), Figure 4 (SEQ ID NO:7), Figure 6 (SEQ ID NO:10), Figure 8 (SEQ ID NO:15), Figure 10 (SEQ ID NO:20), Figure 12 (SEQ ID NO:25), Figure 14 (SEQ ID NO:30), Figure 16 (SEQ ID NO:35). Figure 18 (SEQ ID NO:43), Figure 20 (SEQ ID NO:48), Figure 22 (SEQ ID NO:55), Figure 24 (SEQ )DNO:60), or Figure 26 (SEQ ID NO:62), lacking its associated signal peptide;
   (b) a nucleotide sequence encoding an extracellular domain of the polypeptide shown in Figure 2 (SEQ ID NO:2), Figure 4 (SEQ ID NO:7), Figure 6 (SEQ ID NO:10), Figure 8 (SEQ ID NO:) 15), Figure 10 (SEQ ID NO:20), Figure 12 (SEQ ID NO:25), Figure 14 (SEQ ID NO:30), Figure 16 (SEQ ID NO:35), Figure 18 (SEQ ID NO:43). Figure 20 (SEQ ID NO:48), Figure 22 (SEQ ID NO:55), Figure 24 (SEQ ID NO:60), or Figure 26 (SEQ ID NO:62), with its associated signal peptide; or
   (c) a nucleotide sequence encoding an extracellular domain of the polypeptide shown in Figure 2 (SEQ ID NO:2), Figure 4 (SEQ ID NO:7), Figure 6 (SEQ 10 NO: 1 0), Figure 8 (SEQ ID NO: 15), Figure 10 (SEQ ID NO:20), Figure 12 (SEQ ID NO:25), Figure 14 (SEQ ID NO:30), Figure 16 (SEQ ID NO:35), Figure 18 (SEQ ID NO:43), Figure 20 (SEQ ID NO:48), Figure 22 (SEQ ID NO:55), Figure 24 (SEQ ID NO:60), or Figure 26 (SEQ ID NO:62), lacking its associated signal peptide.
40. An isolated polypeptide having at least 80% amino acid sequence identity to:
   (a) the polypeptide shown in Figure 2 (SEQ ID NO:2), Figure 4 (SEQ ID NO:7), Figure 6 (SEQ ID NO: 10), Figure 8 (SEQ ID NO: 15), Figure 10 (SEQ ID NO:20), Figure 12 (SEQ ID NO:25), Figure 14 (SEQ [D NO:30), Figure 16 (SEQ ID NO:35), Figure 18 (SEQ ID NO:43), Figure 20 (SEQ ID NO:48), Figure 22 (SEQ ID NO:55), Figure 24 (SEQ ID NO:60), or Figure 26 (SEQ ID NO:62), lacking its associated signal peptide;
   (b) an extracellular domain ofthe polypeptide shown in Figure 2 (SEQ ID NO:2), Figure 4 (SEQ ID NO:7), Figure 6 (SEQ ID NO:15), Figure 10 (SEQ ID NO:24), Figure 14 (SEQ ID NO:32), Figure 16 (SEQ ID NO:37), Figure 18 (SEQ ID NO:42), Figure 20 (SEQ ID NO:50), Figure 22 (SEQ ID NO:55), Figure 24 (SEQ ID NO:61 ), Figure 26 (SEQ ID NO:69), Figure 28 (SEQ ID NO:76), Figure 30 (SEQ ID NO:78), Figure 32 (SEQ ID NO:83) or Figure 34 (SEQ ID NO:91), with its associated signal peptide; or
   (c) an extracellular domain of the polypeptide shown in Figure 2 (SEQ ID NO:2), Figure 4 (SEQ ID NO:7), Figure 6 (SEQ ID NO: 15), Figure 10 (SEQ ID NO:24), Figure 14 (SEQ ID NO:32), Figure 16 (SEQ ID NO:37), Figure 18 (SEQ ID NO:42), Figure 20 (SEQ ID NO:50), Figure 22 (SEQ ID NO:5 5), Figure 24 (SEQ ID NO:61), Figure 26 (SEQ ID NO:69), Figure 28 (SEQ ID NO:76), Figure 30 (SEQ ID NO:78), Figure 32 (SEQ ID NO:83) or Figure 34 (SEQ ID NO:91), lacking its associated signal peptide.

### BRIEF DESCRIPTION OF THE DRAWINGS

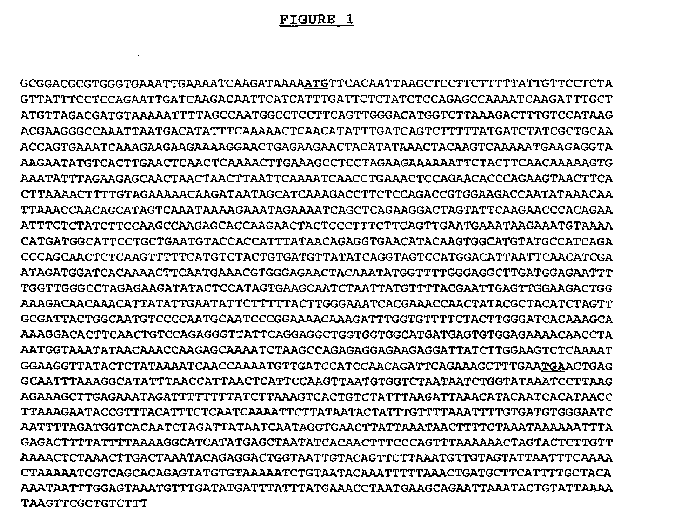

Figure 1 shows the nucleotide sequence (SEQ ID NO:1 ) of a cDNA containing a nucleotide sequence encoding native sequence PRO179, wherein the nucleotide sequence (SEQ ID NO:1) is a clone designated herein as DNA16451-1078. Also presented in bold font and underlined are the positions of the respective start and stop codons.

Figure 2 shows the amino acid sequence (SEQ ID NO:2) of a native sequence PRO179 polypeptide as derived from the coding sequence of SEQ ID NO: 1shown in Figure 1.

Figure 3 shows the nucleotide sequence (SEQ ID NO:6) of a cDNA containing a nucleotide sequence encoding native sequence PRO207, wherein the nucleotide sequence (SEQ ID NO:6) is a clone designated herein as DNA30879-1152. Also presented in bold font and underlined are the positions of the respective start and stop codons.

Figure 4 shows the amino acid sequence (SEQ ID NO:7) of a native sequence PRO207 polypeptide as derived from the coding sequence of SEQ ID NO:6 shown in Figure 3.

Figure 5 shows the nucleotide sequence (SEQ ID NO:9) of a cDNA containing a nucleotide sequence encoding native sequence PRO320, wherein the nucleotide sequence (SEQ ID NO:9) is a clone designated herein as DNA32284-1307. Also presented in bold font and underlined are the positions of the respective start and stop codons.

Figure 6 shows the amino acid sequence (SEQ ID NO:10) of a native sequence PRO320 polypeptide as derived from the coding sequence of SEQ ID NO:9 shown in Figure 5.

Figure 7 shows the nucleotide sequence (SEQ ID NO:14) of a cDNA containing a nucleotide sequence encoding native sequence PRO219, wherein the nucleotide sequence (SEQ ID NO:) 14) is a clone designated herein as DNA32290-1164. Also presented in bold font and underlined are the positions of the respective start and stop codons.

Figure 8 shows the amino acid sequence (SEQ ID NO: 15) of a native sequence PRO219 polypeptide as derived from the coding sequence of SEQ ID NO:14 shown in Figure 7.

Figure 9 shows the nucleotide sequence (SEQ ID NO: 19) of a cDNA containing a nucleotide sequence encoding native sequence PRO221, wherein the nucleotide sequence (SEQ ID NO: 19) is a clone designated herein as DNA33089-1132. Also presented in bold font and underlined are the positions of the respective start and stop codons.

Figure 10 shows the amino acid sequence (SEQ ID NO:20) of a native sequence PRO221 polypeptide as derived from the coding sequence of SEQ ID NO:19 shown in Figure 9.

Figure I I shows the nucleotide sequence (SEQ ID NO:24) of a cDNA containing a nucleotide sequence encoding native sequence PRO224, wherein the nucleotide sequence (SEQ ID NO:24) is a clone designated herein as DNA33221-1133. Also presented in bold font and underlined are the positions of the respective start and stop codons.

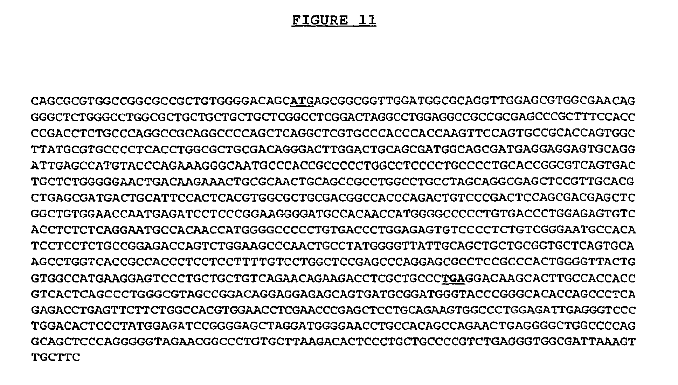

Figure 12 shows the amino acid sequence (SEQ ID NO:25) of a native sequence PRO224 polypeptide as derived from the coding sequence of SEQ ID NO:24 shown in Figure 11.

Figure 13 shows the nucleotide sequence (SEQ ID NO:29) of a cDNA containing a nucleotide sequence encoding native sequence PRO328, wherein the nucleotide sequence (SEQ ID NO:29) is a clone designated herein as DNA40587-1231. Also presented in bold font and underlined are the positions of the respective start and stop codons.

Figure 14 shows the amino acid sequence (SEQ ID NO:30) of a native sequence PRO328 polypeptide as derived from the coding sequence of SEQ ID NO:29 shown in Figure 13.

Figure 15 shows the nucleotide sequence (SEQ ID NO:34) of a cDNA containing a nucleotide sequence encoding native sequence PRO301, wherein the nucleotide sequence (SEQ ID NO:34) is a clone designated herein as DNA40628-1216. Also presented in bold font and underlined are the positions of the respective start and stop codons.

Figure 16 shows the amino acid sequence (SEQ ID NO:35) of a native sequence PRO301 polypeptide as derived from the coding sequence of SEQ ID NO:34 shown in Figure 15.

Figure 17 shows the nucleotide sequence (SEQ ID NO:42) of a cDNA containing a nucleotide sequence encoding native sequence PRO526, wherein the nucleotide sequence (SEQ ID NO:42) is a clone designated herein as DNA44184-1319. Also presented in bold font and underlined are the positions of the respective start and stop codons.

Figure 18 shows the amino acid sequence (SEQ ID NO:43) of a native sequence PRO526 polypeptide as derived from the coding sequence of SEQ ID NO:42 shown in Figure 17.

Figure 19 shows the nucleotide sequence (SEQ ID NO:47) of a cDNA containing a nuclcotide sequence encoding native sequence PRO362, wherein the nucleotide sequence (SEQ ID NO:47) is a clone designated herein as DNA45416-1251. Also presented in bold font and underlined are the positions of the respective start and stop codons.

Figure 20 shows the amino acid sequence (SEQ ID NO:48) of a native sequence PRO362 polypeptide as derived from the coding sequence of SEQ ID NO:47 shown in Figure 19.

Figure 21 shows the nucleotide sequence (SEQ ID NO:54) of a cDNA containing a nucleotide sequence encoding native sequence PRO356, wherein the nucleotide sequence (SEQ ID NO:54) is a clone designated herein as DNA47470-1130-P1. Also presented in bold font and underlined are the positions of the respective start and stop codons.

Figure 22 shows the amino acid sequence (SEQ ID NO:55) of a native sequence PRO356 polypeptide as derived from the coding sequence of SEQ ID NO:54 shown in Figure 21.

Figure 23 shows the nucleotide sequence (SEQ ID NO:59) of a cDNA containing a nucleotide sequence encoding native sequence PRO509, wherein the nucleotide sequence (SEQ ID NO:59) is a clone designated herein as DNA50148-1068. Also presented in bold font and underlined are the positions of the respective start and stop codons.

Figure 24 shows the amino acid sequence (SEQ ID NO:60) of a native sequence PRO509 polypeptide as derived from the coding sequence of SEQ ID NO:59 shown in Figure 23.

Figure 25 shows the nucleotide sequence (SEQ ID NO:61) of a cDNA containing a nucleotide sequence encoding native sequence PRO866, wherein the nucleotide sequence (SEQ ID NO:61) is a clone designated herein as DNA53971-1359. Also presented in bold font and underlined are the positions of the respective start and stop codons.

Figure 26 shows the amino acid sequence (SEQ ID NO:62) of a native sequence PRO866 polypeptide as derived from the coding sequence of SEQ ID NO:61 shown in Figure 25.

### DETAILED DESCRIPTION OF THE INVENTION

The terms "PRO179", "PRO207", "PRO320", "PRO219", "PRO221", "PRO224", "PRO328", "PRO301", "PRO526", "PRO362", "PRO356", "PRO509" or "PRO866" polypeptide or protein when used herein encompass native sequence PRO 179, PRO207. PRO320, PRO219, PRO221, PRO224, PRO328, PRO301, PRO526, PRO362, PRO356, PRO509 and PRO866 polypeptides and PRO179, PRO207, PRO320, PRO219, PRO221, PRO224, PRO328, PRO301, PRO526, PRO362, PRO356, PRO509 and PRO866 variants (which are further defined herein). The PRO179, PRO207, PRO320, PRO219, PRO221, PRO224, PRO328, PRO301, PRO526, PRO362, PRO356, PRO509 or PRO866 polypeptide may be isolated from a variety of sources, such as from human tissue types or from another source, or prepared by recombinant and/or synthetic methods.

A "native sequence PRO179", "native sequence PRO207", "native sequence PRO320", "native sequence PRO219", "native sequence PRO221", "native sequence PRO224", "native sequence PRO328", "native sequence PRO301 ", "native sequence PRO526", "native sequence PRO362", "native sequence PRO356", "native sequence PRO509", or "native sequence PRO866" comprises a polypeptide having the same amino acid sequence as the PRO179, PRO207, PRO320, PRO219, PRO221, PRO224, PRO328, PRO301, PRO526, PRO362, PRO356, PRO509 or PRO866 polypeptide as derived from nature. Such native sequence PRO179, PRO207, PRO320, PRO219, PRO221, PRO224, PRO328, PRO301, PRO526, PRO362, PRO356, PRO509 or PRO866 polypeptide can be isolated from nature or can be produced by recombinant and/or synthetic means. The term "native sequence" PRO179, PRO207, PRO320, PRO219, PRO221, PRO224, PRO328, PRO301, PRO526, PRO362, PRO356, PRO509 or PRO866 specifically encompasses naturally-occurringtruncated or secreted forms (*e.g.,* an extracellular domain sequence), naturally-occurring variant forms (e.g., alternatively spliced forms) and naturally-occurring allelic variants of the PRO179, PRO207, PRO320, PRO219, PRO221, PRO224, PRO328, PRO301, PRO526, PRO362, PRO356, PRO509 and PRO866 polypeptides. In one embodiment of the invention, the native sequence PRO179, PRO207, PRO320, PRO219, PRO221, PRO224, PRO328, PRO301, PRO526, PRO362, PRO356, PRO509 or PRO866 polypeptide is a mature or full-length native sequence PRO179, PRO207, PRO320, PRO219, PRO221, PRO224, PRO328, PRO301, PRO526, PRO362, PRO356, PRO509 or PRO866 polypeptide as shown in Figure 2 (SEQ ID NO:2), Figure 4 (SEQ ID NO:7), Figure 6 (SEQ ID NO:10), Figure 8 (SEQ ID NO: 15), Figure 10 (SEQ ID NO:20), Figure 12 (SEQ ID NO:25), Figure 14 (SEQ ID NO:30), Figure 16 (SEQ ID NO:35), Figure 18 (SEQ ID NO:43), Figure 20 (SEQ ID NO:48), Figure 22 (SEQ ID NO:55), Figure 24 (SEQ ID NO:60) or Figure 26 (SEQ ID NO:62), respectively. Also, while the PRO179, PRO207, PRO320, PRO219, PRO221, PRO224, PRO328, PRO301, PRO526, PRO362, PRO356, PRO509 and PRO866 polypeptides disclosed in Figure 2 (SEQ ID NO:2), Figure 4 (SEQ ID NO:7), Figure 6 (SEQ ID NO:10), Figure 8 (SEQ ID NO:) 15), Figure 10 (SEQ ID NO:20), Figure 12 (SEQ ID NO:25), Figure 14 (SEQ ID NO:30), Figure 16 (SEQ ID NO:35), Figure 18 (SEQ ID NO:43), Figure 20 (SEQ ID NO:48), Figure 22 (SEQ ID NO:55), Figure 24 (SEQ ID NO:60) or Figure 26 (SEQ ID NO:62), respectively, are shown to begin with the methionine residue designated therein as amino acid position 1, it is conceivable and possible that another methionine residue located either upstream or downstream from amino acid position 1 in Figure 2 (SEQ ID NO:2), Figure 4 (SEQ ID NO:7), Figure 6 (SEQ ID NO:10), Figure 8 (SEQ ID NO:15), Figure 10 (SEQ ID NO.20), Figure 12 (SEQ ID NO:25), Figure 14 (SEQ ID NO:30), Figure 16 (SEQ ID NO:35), Figure 18 (SEQ ID NO:43), Figure 20 (SEQ ID NO:48), Figure 22 (SEQ ID NO:55), Figure 24 (SEQ ID NO:60) or Figure 26 (SEQ ID NO:62), respectively, may be employed as the starting amino acid residue for the PRO179, PRO207, PRO320, PRO219, PRO221, PRO224, PRO328, PRO301, PRO526, PRO362, PRO356, PRO509 or PRO866 polypeptide.

The "extracellular domain" or "ECD" of polypeptide disclosed herein refers to a form of the polypeptide which is essentially free ofthe transmembrane and cytoplasmic domains. Ordinarily, a polypeptide ECD will have less than about 1% of such transmembrane and/or cytoplasmic domains and preferably, will have less than about 0.5% of such domains. It will be understood that any transmembrane domain(s) identified for the polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified and as shown in the appended figures. As such, in one embodiment ofthe present invention, the extracellular domain of polypeptide of the present invention comprises amino acids 1 to X of the mature amino acid sequence, wherein X is any amino acid within 5 amino acids on either side of the extracellular domain/transmembrane domain boundary.

The approximate location of the "signal peptides" of the various PRO polypeptides disclosed herein are shown in the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (*e.g.*, Nielsen *et al.,* Prot. Eng., 10:1-6 (1997) and von Heinje *et al.,* Nucl. Acids. Res., 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO179 variant polypeptide" means an active PRO179 polypeptide (other than a native sequence PRO179 polypeptide) as defined below, having at least about 80% amino acid sequence identity with the amino acid sequence of (a) residues 1 or about 17 to 460 of the PRO179 polypeptide shown in Figure 2 (SEQ ID NO:2), (b) X to 460 of the PRO179 polypeptide shown in Figure 2 (SEQ ID NO:2), wherein X is any amino acid residue from 12 to 21 of Figure 2 (SEQ ID NO:2), or (c) another specifically derived fragment of the amino acid sequence shown in Figure 2 (SEQ ID NO:2).

"PRO207 variant polypeptide" means an active PRO207 polypeptide (other than a native sequence PRO207 polypeptide) as defined below, having at least about 80% amino acid sequence identity with the amino acid sequence of (a) residues 1 or about 41 to 249 of the PRO207 polypeptide shown in Figure 4 (SEQ ID NO:7), (b) X to 249 of the PRO207 polypeptide shown in Figure 4 (SEQ ID NO:7), wherein X is any amino acid residue from 36 to 45 of Figure 4 (SEQ ID NO:7), or (c) another specifically derived fragment of the amino acid sequence shown in Figure 4 (SEQ ID NO:7).

"PRO320 variant polypeptide" means an active PRO320 polypeptide (other than a native sequence PRO320 polypeptide) as defined below, having at least about 80% amino acid sequence identity with the amino acid sequence of (a) residues 1 or about 22 to 338 of the PRO320 polypeptide shown in Figure 6 (SEQ ID NO: 10), (b) X to 338 of the PRO320 polypeptide shown in Figure 6 (SEQ ID NO: 10), wherein X is any amino acid residue from 17 to 26 of Figure 6 (SEQ ID NO: 10), or (c) another specifically derived fragment of the amino acid sequence shown in Figure 6 (SEQ ID NO:10).

"PRO219 variant polypeptide" means an active PRO219 polypeptide (other than a native sequence PRO219 polypeptide) as defined below, having at least about 80% amino acid sequence identity with the amino acid sequence of (a) residues 1 or about 24 to 1005 of the PRO219 polypeptide shown in Figure 8 (SEQ ID NO:15), (b) X to 1005 ofthe PRO219 polypeptide shown in Figure 8 (SEQ ID NO: 15), wherein X is any amino acid residue from 19 to 28 of Figure 8 (SEQ ID NO: 15), or (c) another specifically derived fragment of the amino acid sequence shown in Figure 8 (SEQ ID NO: 15).

"PRO221 variant polypeptide" means an active PRO221 polypeptide (other than a native sequence PRO221 polypeptide) as defined below, having at least about 80% amino acid sequence identity with the amino acid sequence of (a) residues 1 or about 34 to 259 of the PRO221 polypeptide shown in Figure 10 (SEQ ID NO:20), (b) X to 259 of the PRO221 polypeptide shown in Figure 10 (SEQ ID NO:20), wherein X is any amino acid residue from 29 to 38 of Figure 10 (SEQ ID NO:20), (c) 1 or about 34 to X of Figure 10 (SEQ ID NO:20), wherein X is any amino acid from amino acid 199 to amino acid 208 of Figure 10 (SEQ ID NO:20), or (d) another specifically derived fragment of the amino acid sequence shown in Figure 10 (SEQ ID NO:20).

"PRO224 variant polypeptide" means an active PRO224 polypeptide (other than a native sequence PRO224 polypeptide) as defined below, having at least about 80% amino acid sequence identity with the amino acid sequence of(a) residues I or about 31 to 282 ofthe PRO224 polypeptide shown in Figure 12 (SEQ ID NO:25), (b) X to 282 of the PRO224 polypeptide shown in Figure 12 (SEQ ID NO:25), wherein X is any amino acid residue from 26 to 35 of Figure 12 (SEQ ID NO:25), (c) I or about 31 to X of Figure 12 (SEQ ID NO:25), wherein X is any amino acid from amino acid 226 to amino acid 235 of Figure 12 (SEQ ID NO:25), or (d) another specifically derived fragment of the amino acid sequence shown in Figure 12 (SEQ ID NO:25).

"PRO328 variant polypeptide" means an active PRO328 polypeptide (other than a native sequence PRO328 polypeptide) as defined below, having at least about 80% amino acid sequence identity with the amino acid sequence of (a) residues 1 or about 23 to 463 of the PRO328 polypeptide shown in Figure 14 (SEQ ID NO:30), (b) X to 463 of the PRO328 polypeptide shown in Figure 14 (SEQ ID NO:30), wherein X is any amino acid residue from 18 to 27 of Figure 14 (SEQ ID NO:30), or (c) another specifically derived fragment of the amino acid sequence shown in Figure 14 (SEQ ID NO:30).

"PRO301 variant polypeptide" means an active PRO301 polypeptide (other than a native sequence PRO301 polypeptide) as defined below, having at least about 80% amino acid sequence identity with the amino acid sequence of (a) residues 1 or about 28 to 299 of the PRO301 polypeptide shown in Figure 16 (SEQ ID NO:35), (b) X to 299 of the PRO301 polypeptide shown in Figure 16 (SEQ ID NO:35), wherein X is any amino acid residue from 23 to 32 of Figure 16 (SEQ ID NO:35), (c) 1 or about 28 to X of Figure 16 (SEQ ID NO:35), wherein X is any amino acid from amino acid 230 to amino acid 239 of Figure 16 (SEQ ID NO:35), or (d) another specifically derived fragment of the amino acid sequence shown in Figure 16 (SEQ ID NO:35).

"PRO526 variant polypeptide" means an active PRO526 polypeptide (other than a native sequence PRO526 polypeptide) as defined below, having at least about 80% amino acid sequence identity with the amino acid sequence of (a) residues 1 or about 27 to 473 of the PRO526 polypeptide shown in Figure 18 (SEQ ID NO:43), (b) X to 473 of the PRO526 polypeptide shown in Figure 18 (SEQ ID NO:43), wherein X is any amino acid residue from 22 to 31 of Figure 18 (SEQ ID NO:43), or (c) another specifically derived fragment of the amino acid sequence shown in Figure 18 (SEQ ID NO:43).

"PRO362 variant polypeptide" means an active PRO362 polypeptide (other than a native sequence PRO362 polypeptide) as defined below, having at least about 80% amino acid sequence identity with the amino acid sequence of(a) residues 1 or about 20 to 321 of the PRO362 polypeptide shown in Figure 20 (SEQ ID NO:48), (b) X to 321 of the PRO362 polypeptide shown in Figure 20 (SEQ ID NO:48), wherein X is any amino acid residue from 15 to 24 of Figure 20 (SEQ ID NO:48), (c) I or about 20 to X of Figure 20 (SEQ ID NO:48), wherein X is any amino acid from amino acid 276 to amino acid 285 of Figure 20 (SEQ ID NO:48), or (d) another specifically derived fragment of the amino acid sequence shown in Figure 20 (SEQ ID NO:48).

"PRO356 variant polypeptide" means an active PRO356 polypeptide (other than a native sequence PRO356 polypeptide) as defined below, having at least about 80% amino acid sequence identity with the amino acid sequence of(a) residues I or about 27 to 346 of the PRO356 polypeptide shown in Figure 22 (SEQ ID NO:55), (b) X to 346 of the PRO356 polypeptide shown in Figure 22 (SEQ ID NO:55), wherein X is any amino acid residue from 22 to 31 of Figure 22 (SEQ ID NO:55), or (c) another specifically derived fragment of the amino acid sequence shown in Figure 22 (SEQ ID NO:55).

"PRO509 variant polypeptide" means an active PRO509 polypeptide (other than a native sequence PRO509 polypeptide) as defined below, having at least about 80% amino acid sequence identity with the amino acid sequence of (a) residues I or about 37 to 283 of the PRO509 polypeptide shown in Figure 24 (SEQ ID NO:60), (b) X to 283 of the PRO509 polypeptide shown in Figure 24 (SEQ ID NO:60), wherein X is any amino acid residue from 32 to 41 of Figure 24 (SEQ ID NO:60), (c) I or about 37 to X of Figure 24 (SEQ ID NO:60), wherein X is any amino acid from amino acid 200 to amino acid 209 of Figure 24 (SEQ ID NO:60), or (d) another specifically derived fragment of the amino acid sequence shown in Figure 24 (SEQ ID NO:60).

"PRO866 variant polypeptide" means an active PRO866 polypeptide (other than a native sequence PRO866 polypeptide) as defined below, having at least about 80% amino acid sequence identity with the amino acid sequence of(a) residues I or about 27 to 331 of the PRO866 polypeptide shown in Figure 26 (SEQ ID NO:62), (b) X to 331 of the PRO866 polypeptide shown in Figure 26 (SEQ ID NO:62), wherein X is any amino acid residue from 22 to 31 of Figure 26 (SEQ ID NO:62), or (c) another specifically derived fragment of the amino acid sequence shown in Figure 26 (SEQ ID NO:62).

Such PRO179, PRO207, PRO320, PRO219, PRO221, PRO224, PRO328, PRO301, PRO526, PRO362, PRO356, PRO509 and PRO866 variants include, for instance, PRO179, PRO207, PRO320, PRO219, PRO221, PRO224, PRO328, PRO301, PRO526, PRO362, PRO356, PRO509 and PRO866 polypeptides wherein one or more amino acid residues are added, or deleted, at the N- or C-terminus, as well as within one or more internal domains of the native sequence.

Ordinarily, a PRO 179 variant will have at least about 80% amino acid sequence identity, more preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with (a) residues 1 or about 17 to 460 of the PRO179 polypeptide shown in Figure 2 (SEQ ID NO:2), (b) X to 460 of the PRO179 polypeptide shown in Figure 2 (SEQ ID NO:2), wherein X is any amino acid residue from 12 to 21 of Figure 2 (SEQ ID NO:2), or (c) another specifically derived fragment of the amino acid sequence shown in Figure 2 (SEQ ID NO:2).

Ordinarily, a PRO207 variant will have at least about 80% amino acid sequence identity, more preferably at least about 81 % amino acid sequence identity, more preferably at least about 82% am ino acid sequence identity, more preferably at least about 83% amino acid sequence identity, more preferably at least about 84% amino acid sequence identity, more preferably at least about 85% amino acid sequence identity, more preferably at (east about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with (a) residues 1 or about 41 to 249 of the PRO207 polypeptide shown in Figure 4 (SEQ ID NO:7), (b) X to 249 of the PRO207 polypeptide shown in Figure 4 (SEQ ID NO:7), wherein X is any amino acid residue from 36 to 45 of Figure 4 (SEQ ID NO:7), or (c) another specifically derived fragment of the amino acid sequence shown in Figure 4 (SEQ ID NO:7).

Ordinarily, a PRO320 variant will have at least about 80% amino acid sequence identity, more preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with (a) residues I or about 22 to 338 of the PRO320 polypeptide shown in Figure 6 (SEQ ID NO:10), (b) X to 338 of the PRO320 polypeptide shown in Figure 6 (SEQ ID NO: 10), wherein X is any amino acid residue from 17 to 26 of Figure 6 (SEQ ID NO:10), or (c) another specifically derived fragment of the amino acid sequence shown in Figure 6 (SEQ ID NO:10).

Ordinarily, a PRO219 variant will have at least about 80% amino acid sequence identity, more preferably at least about 81 % amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with (a) residues I or about 24 to 1005 of the PRO219 polypeptide shown in Figure 8 (SEQ ID NO:15), (b) X to 1005 of the PRO219 polypeptide shown in Figure 8 (SEQ ID NO:15), wherein X is any amino acid residue from 19 to 28 of Figure 8 (SEQ ID NO: 15), or (c) another specifically derived fragment of the amino acid sequence shown in Figure 8 (SEQ ID NO: 15).

Ordinarily, a PRO221 variant will have at least about 80% amino acid sequence identity, more preferably at least about 81 % amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with (a) residues I or about 34 to 259 of the PRO221 polypeptide shown in Figure 10 (SEQ ID NO:20), (b) X to 259 of the PRO221 polypeptide shown in Figure 10 (SEQ ID NO:20), wherein X is any amino acid residue from 29 to 38 of Figure 10 (SEQ ID NO:20), (c) 1 or about 34 to X of Figure 10 (SEQ ID NO:20), wherein X is any amino acid from amino acid 199 to amino acid 208 of Figure 10 (SEQ ID NO:20), or (d) another specifically derived fragment of the amino acid sequence shown in Figure 10 (SEQ ID NO:20).

Ordinarily, a PRO224 variant will have at least about 80% amino acid sequence identity, more preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with (a) residues I or about 31 to 282 of the PRO224 polypeptide shown in Figure 12 (SEQ ID NO:25), (b) X to 282 of the PRO224 polypeptide shown in Figure 12 (SEQ ID NO:25), wherein X is any amino acid residue from 26 to 35 of Figure 12 (SEQ ID NO:25), (c) I or about 31 to X of Figure 12 (SEQ ID NO:25), wherein X is any amino acid from amino acid 226 to amino acid 235 of Figure 12 (SEQ ID NO:25), or(d) another specifically derived fragment of the amino acid sequence shown in Figure 12 (SEQ ID NO:25).

Ordinarily, a PRO328 variant will have at least about 80% amino acid sequence identity, more preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with (a) residues 1 or about 23 to 463 of the PRO328 polypeptide shown in Figure 14 (SEQ ID NO:30), (b) X to 463 of the PRO328 polypeptide shown in Figure 14 (SEQ ID NO:30), wherein X is any amino acid residue from 18 to 27 of Figure 14 (SEQ ID NO:30), or (c) another specifical ly derived fragment of the amino acid sequence shown in Figure 14 (SEQ ID NO:30).

Ordinarily, a PRO301 variant will have at least about 80% amino acid sequence identity, more preferably at least about 81 % amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with (a) residues I or about 28 to 299 of the PRO301 polypeptide shown in Figure 16 (SEQ ID NO:35), (b) X to 299 of the PRO301 polypeptide shown in Figure 16 (SEQ ID NO:35), wherein X is any amino acid residue from 23 to 32 of Figure 16 (SEQ ID NO:35), (c) 1 or about 28 to X of Figure 16 (SEQ ID NO:35), wherein X is any amino acid from amino acid 230 to amino acid 239 of Figure 16 (SEQ ID NO:35), or (d) another specifically derived fragment of the amino acid sequence shown in Figure 16 (SEQ ID NO:35).

Ordinarily, a PRO526 variant will have at least about 80% amino acid sequence identity, more preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% am ino acid sequence identity with (a) residues 1 or about 27 to 473 of the PRO526 polypeptide shown in Figure 18 (SEQ ID NO:43), (b) X to 473 of the PRO526 polypeptide shown in Figure 18 (SEQ ID NO:43), wherein X is any amino acid residue from 22 to 31 of Figure 18 (SEQ ID NO:43), or (c) another specifically derived fragment ofthe amino acid sequence shown in Figure 18 (SEQ ID NO:43).

Ordinarily, a PRO362 variant will have at least about 80% amino acid sequence identity, more preferably at least about 81 % amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with (a) residues 1 or about 20 to 321 of the PRO362 polypeptide shown in Figure 20 (SEQ ID NO:48), (b) X to 321 of the PRO362 polypeptide shown in Figure 20 (SEQ ID NO:48), wherein X is any amino acid residue from 15 to 24 of Figure 20 (SEQ ID NO:48), (c) I or about 20 to X of Figure 20 (SEQ ID NO:48), wherein X is any amino acid from amino acid 276 to amino acid 285 of Figure 20 (SEQ ID NO:48), or (d) another specifically derived fragment of the amino acid sequence shown in Figure 20 (SEQ ID NO:48).

Ordinarily, a PRO356 variant will have at least about 80% amino acid sequence identity, more preferably at least about 81 % amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% am ino acid sequence identity with (a) residues 1 or about 27 to 346 of the PRO356 polypeptide shown in Figure 22 (SEQ ID NO:55), (b) X to 346 of the PRO356 polypeptide shown in Figure 22 (SEQ ID NO:55), wherein X is any amino acid residue from 22 to 31 of Figure 22 (SEQ ID NO:55), or (c) another specifically derived fragment of the amino acid sequence shown in Figure 22 (SEQ ID NO:55).

Ordinarily, a PRO509 variant will have at least about 80% amino acid sequence identity, more preferably at least about 81 % amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with (a) residues 1 or about 37 to 283 of the PRO509 polypeptide shown in Figure 24 (SEQ ID NO:60), (b) X to 283of the PRO509 polypeptide shown in Figure 24 (SEQ ID NO:60), wherein X is any amino acid residue from 32 to 41 of Figure 24 (SEQ ID NO:60), (c) or about 37 to X of Figure 24 (SEQ ID NO:60), wherein X is any amino acid from amino acid 200 to amino acid 209 of Figure 24 (SEQ ID NO:60), or (d) another specifically derived fragment of the amino acid sequence shown in Figure 24 (SEQ ID NO:60).

Ordinarily, a PRO866 variant will have at least about 80% amino acid sequence identity, more preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with (a) residues I or about 27 to 331 of the PRO866 polypeptide shown in Figure 26 (SEQ ID NO:62), (b) X to 331 of the PRO866 polypeptide shown in Figure 26 (SEQ ID NO:62), wherein X is any amino acid residue from 22 to 31 of Figure 26 (SEQ ID NO:62), or (c) another specifically derived fragment of the amino acid sequence shown in Figure 26 (SEQ ID NO:62).

PRO179, PRO207, PRO320, PRO219, PRO221, PRO224, PRO328, PRO301, PRO526, PRO362. PRO356, PRO509 and PRO866 variant polypeptides do not encompass the native PRO179, PRO207, PRO320, PRO219, PRO221, PRO224, PRO328, PRO301, PRO526, PRO362, PRO356, PRO509 and PRO866 polypeptide sequence. Ordinarily, PRO179, PRO207, PRO320, PRO219, PRO221, PRO224, PRO328, PRO301, PRO526, PRO362, PRO356, PRO509 and PRO866 variant polypeptides are at least about 10 amino acids in length, often at least about 20 amino acids in length, more often at least about 30 amino acids in length, more often at least about 40 amino acids in length, more often at least about 50 amino acids in length, more often at least about 60 amino acids in length, more often at least about 70 amino acids in length, more often at least about 80 amino acids in length, more often at least about 90 amino acids in length, more often at least about 100 amino acids in length, more often at least about 150 amino acids in length, more often at least about 200 amino acids in length, more often at least about 250 amino acids in length, more often at least about 300 amino acids in length, or more.

As shown below, Table 1 provides the complete source code for the ALIGN-2 sequence comparison computer program. This source code may be routinely compiled for use on a UNIX operating system to provide the ALIGN-2 sequence comparison computer program.

In addition, Tables 2A-2D show hypothetical exemplifications for using the below described method to determine % amino acid sequence identity (Tables 2A-2B) and % nucleic acid sequence identity (Tables 2C-2D) using the ALIGN-2 sequence comparison computer program, wherein "PRO" represents the amino acid sequence of a hypothetical PRO179, PRO207, PRO320, PRO219, PRO221, PRO224, PRO328, PRO301, PRO526, PRO362, PRO356, PRO509 or PRO866 polypeptide of interest, "Comparison Protein" represents the amino acid sequence of a polypeptide against which the "PRO" polypeptide of interest is being compared, "PRO-DNA" represents a hypothetical PRO179-, PRO207-, PRO320-, PRO219-, PRO221-, PRO224-, PRO328-, PRO301-, PRO526-, PRO362-, PRO356-, PRO509- or PRO866-encoding nucleic acid sequence of interest, "Comparison DNA" represents the nucleotide sequence of nucleic acid molecule against which the "PRO-DNA" nucleic acid molecule of interest is being compared, "X", "Y", and "Z" each represent different hypothetical amino acid residues and "N", "L" and "V" each represent different hypothetical nucleotides.

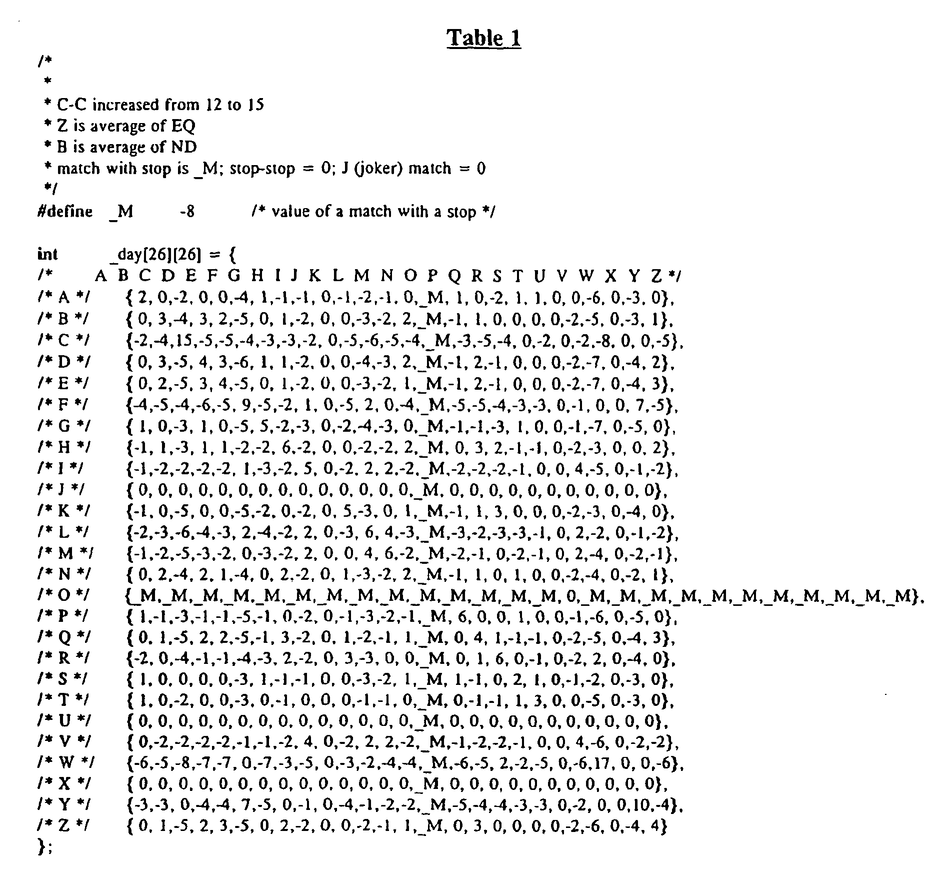

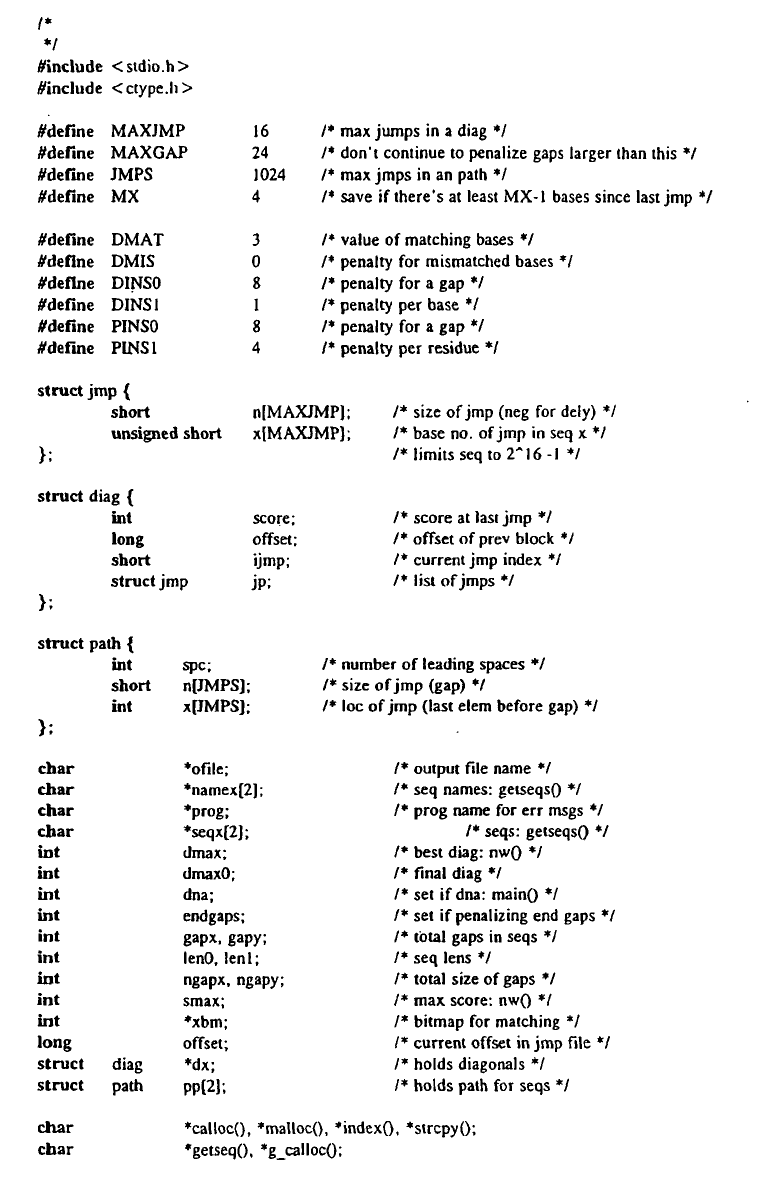

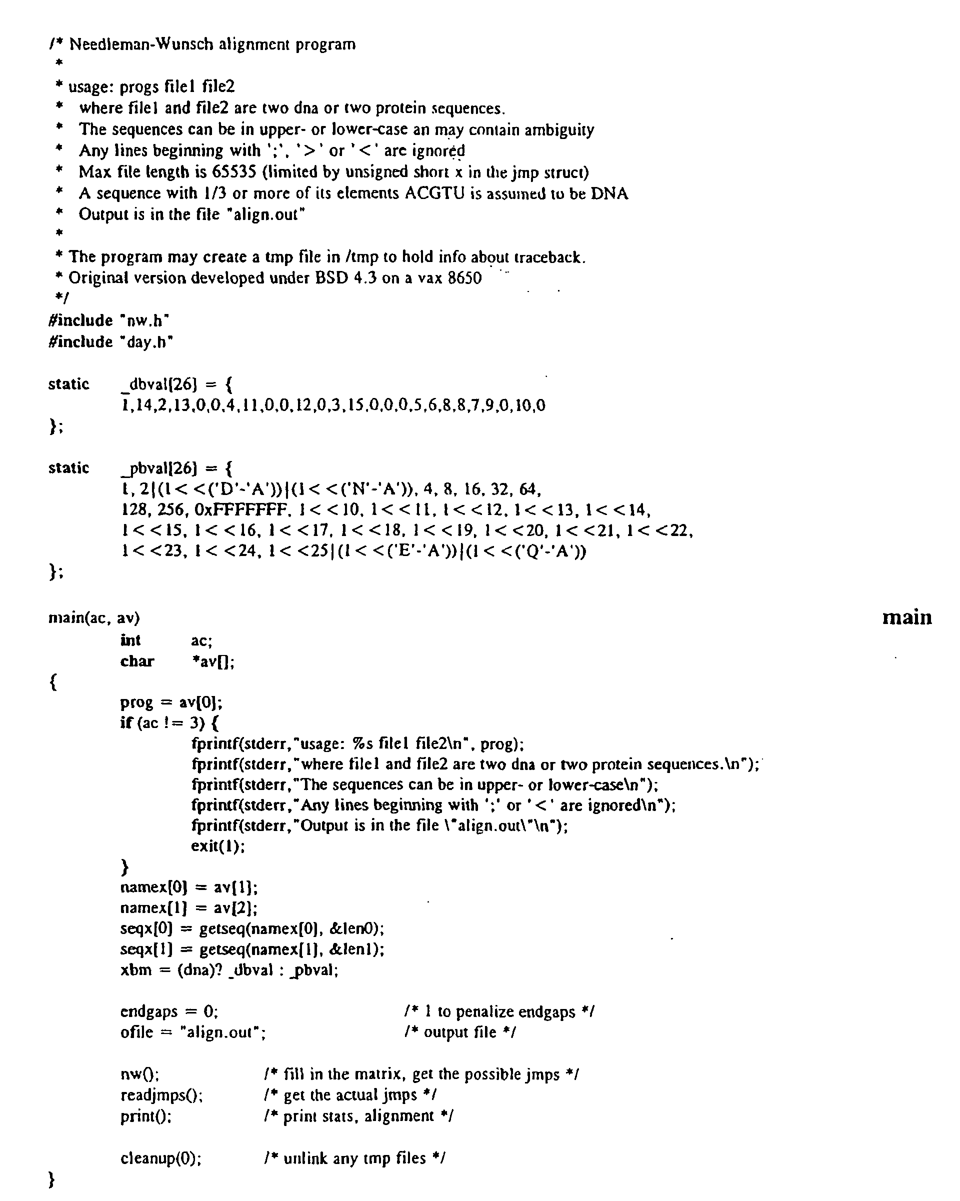

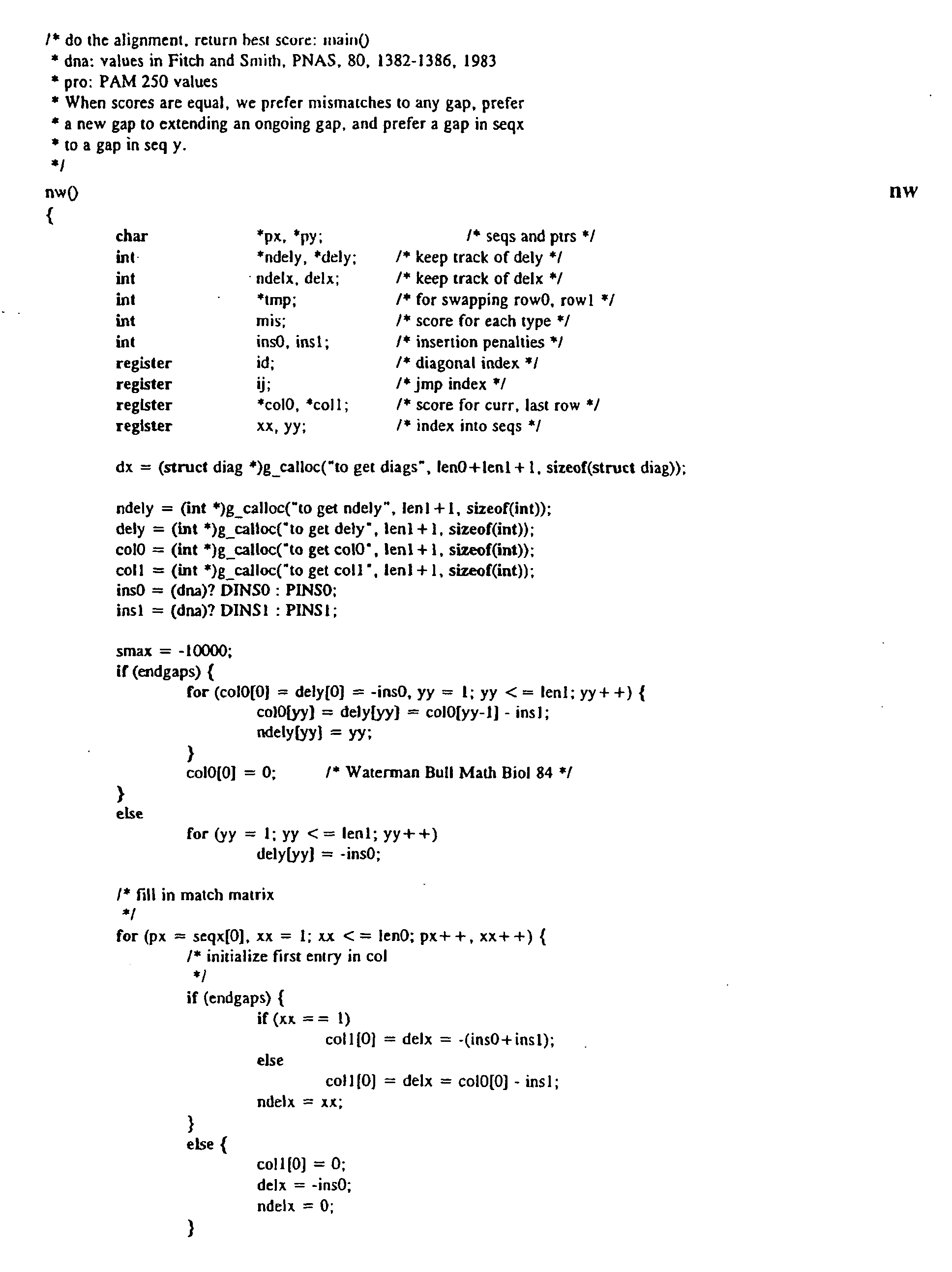

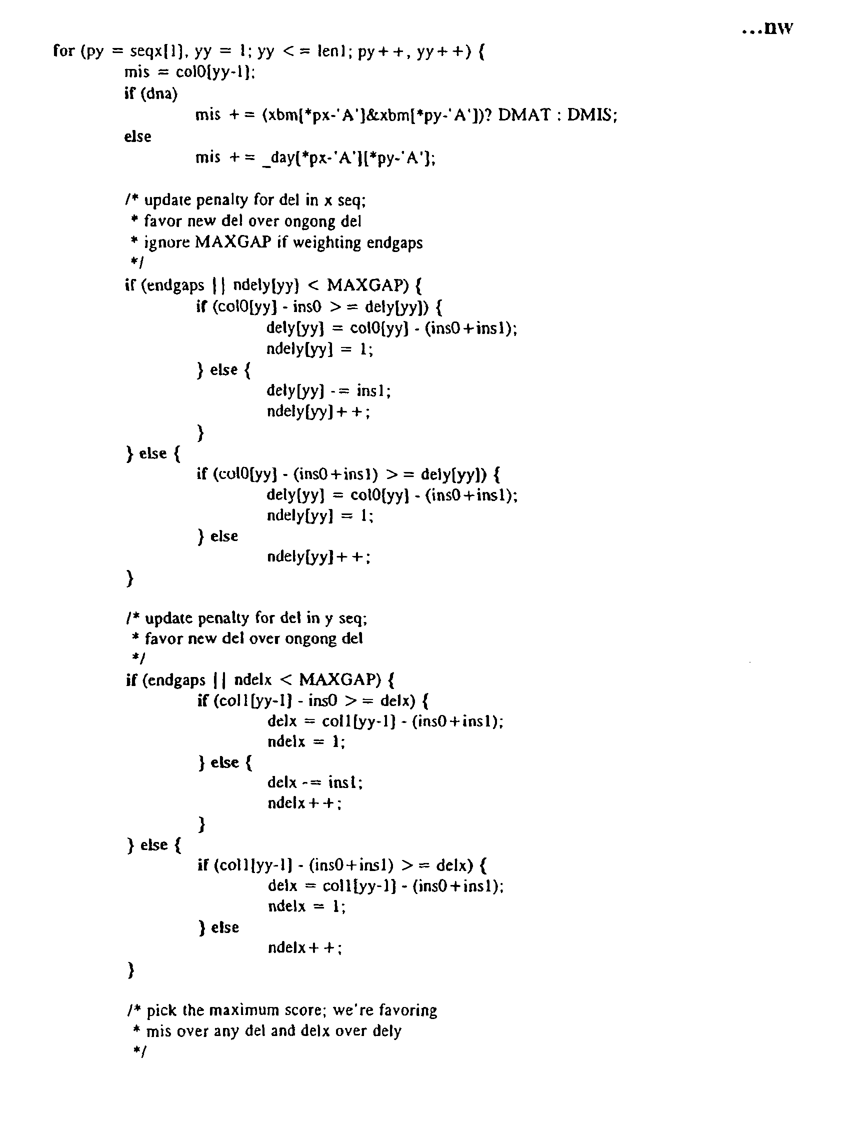

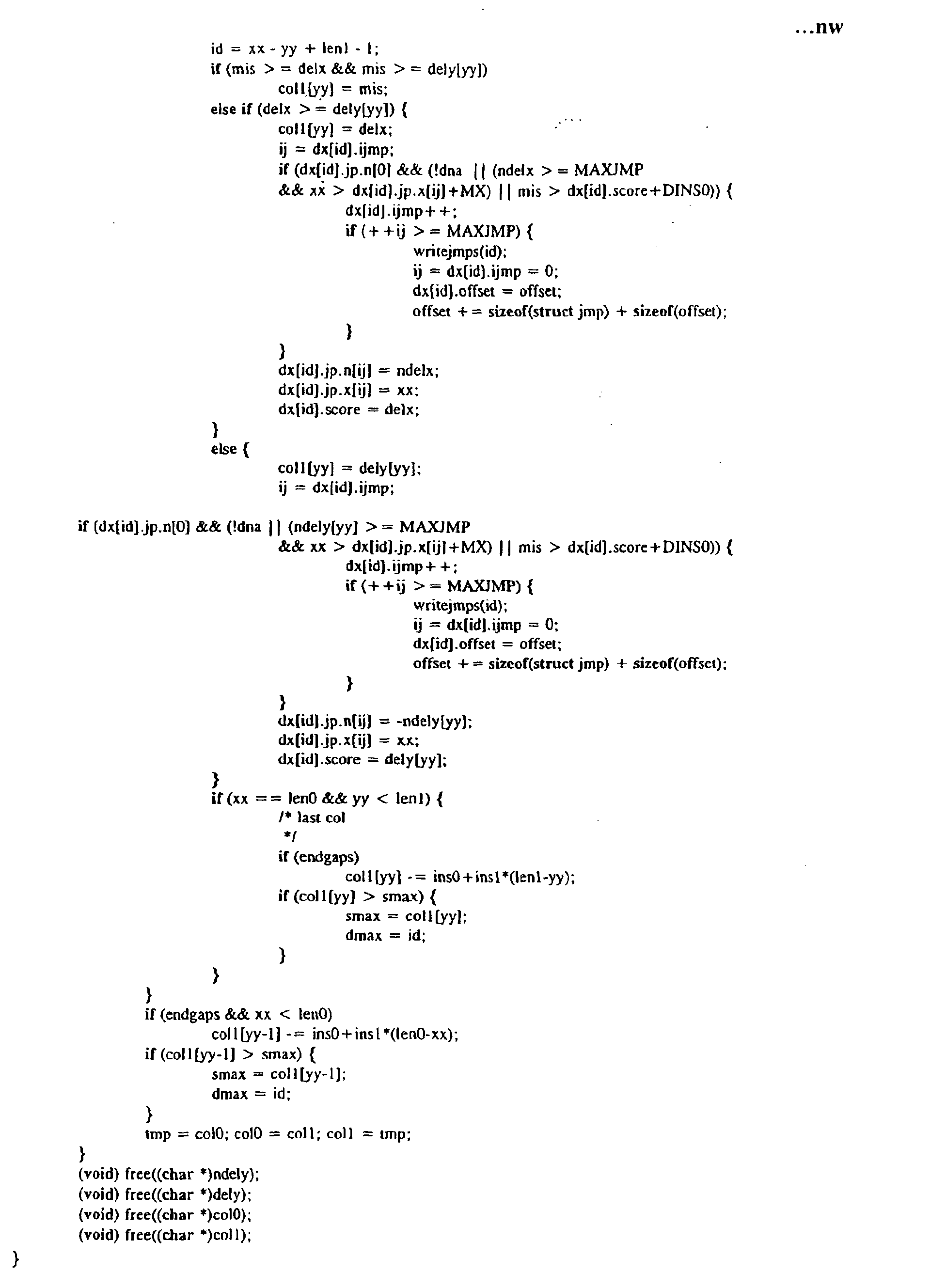

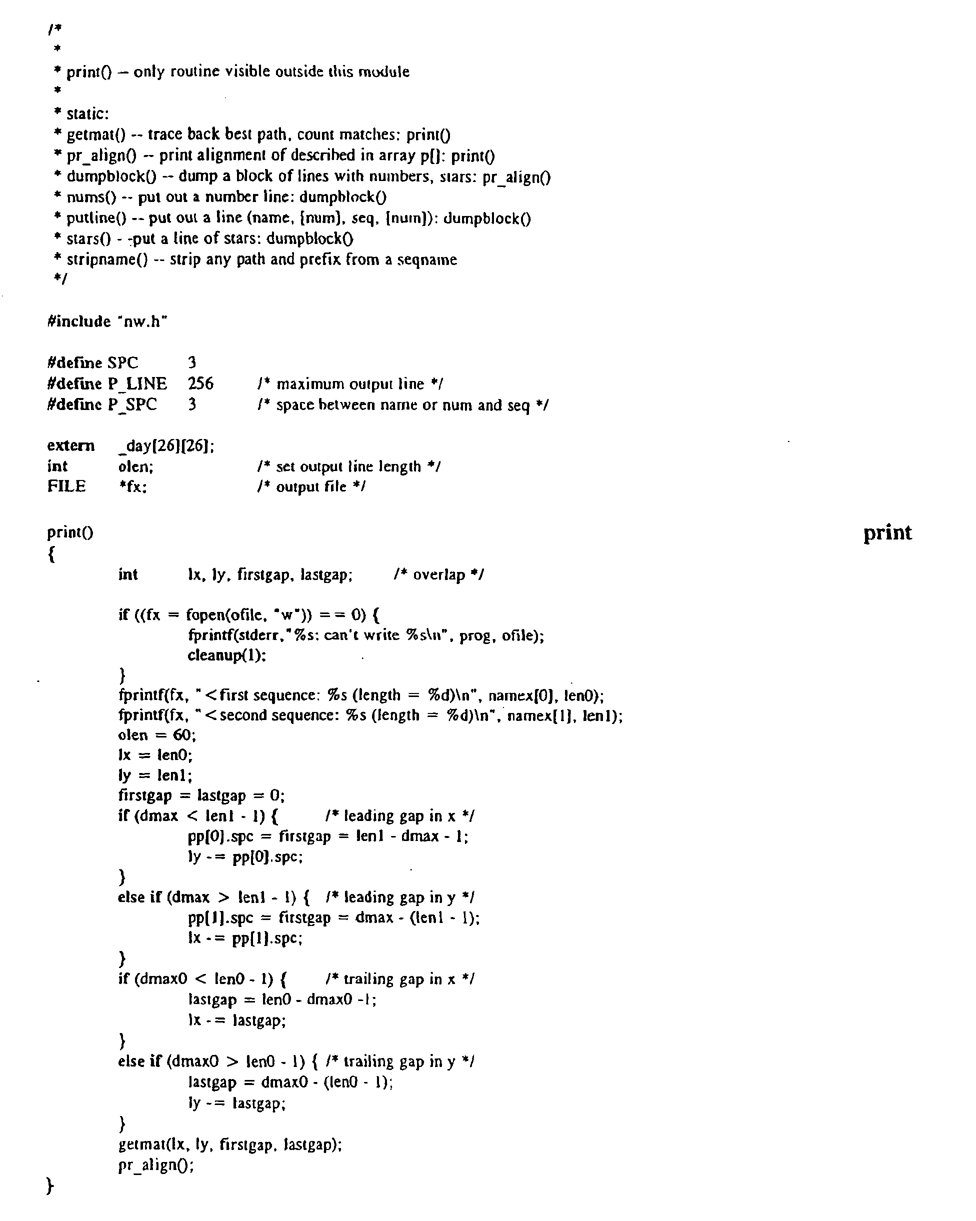

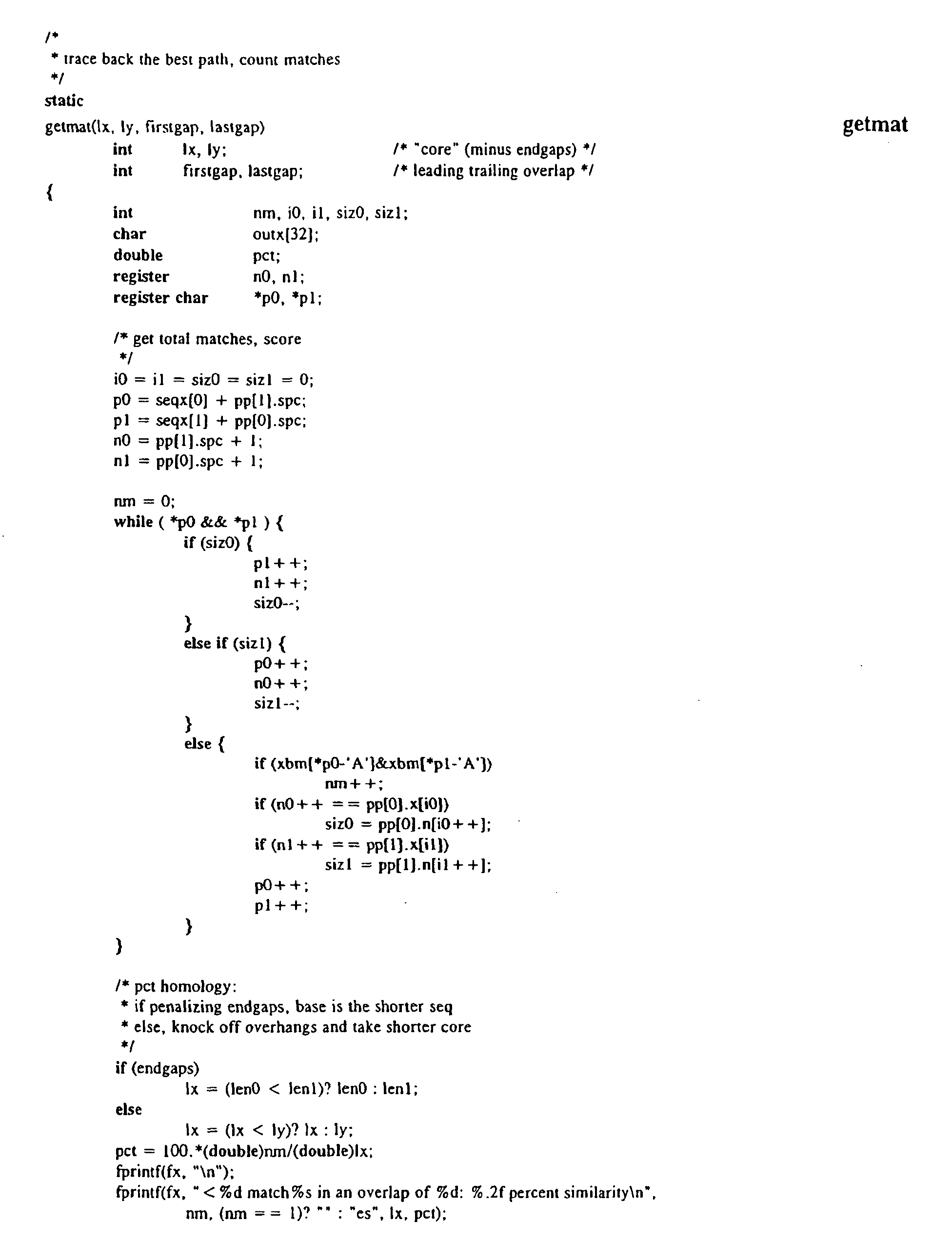

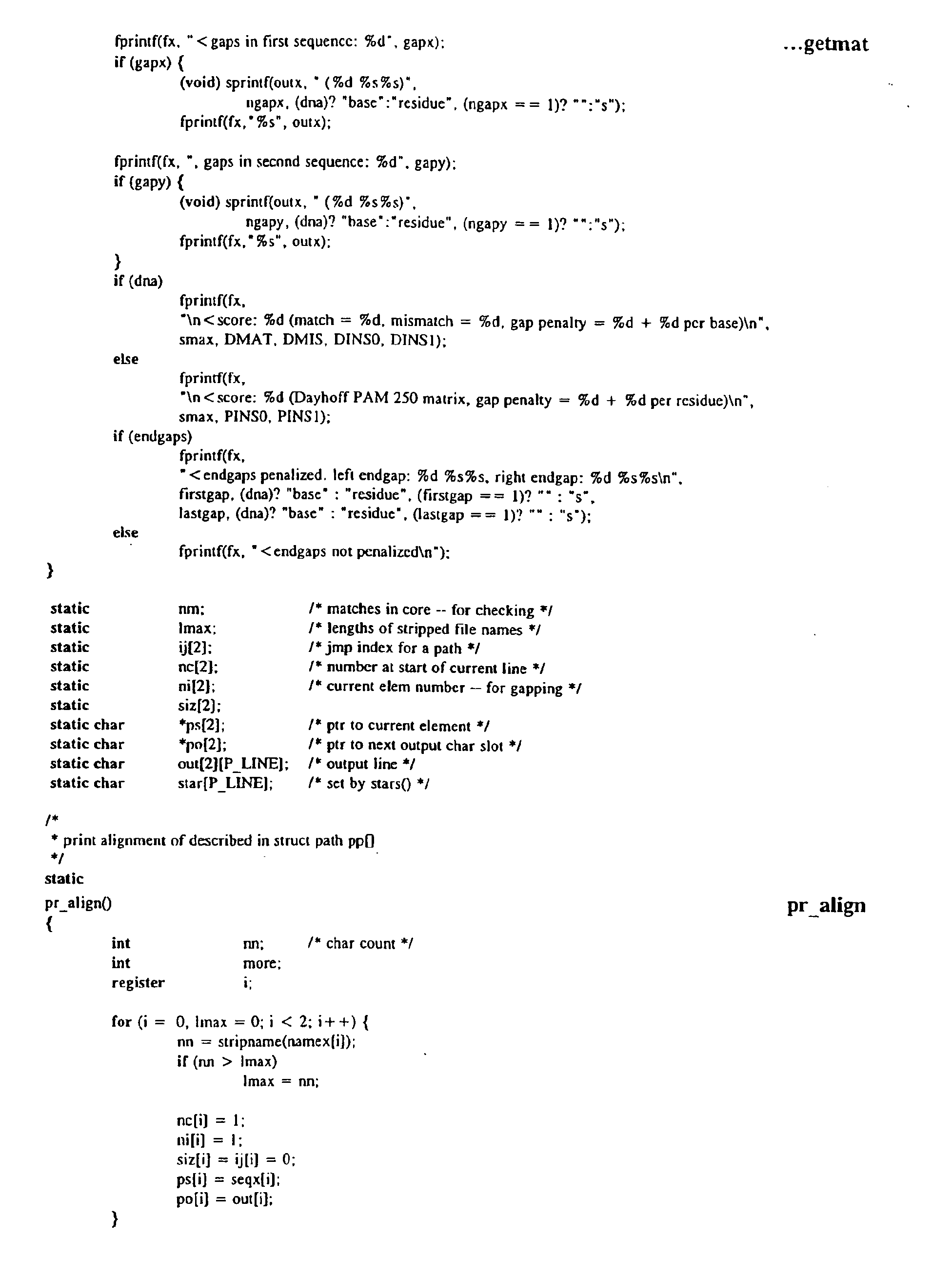

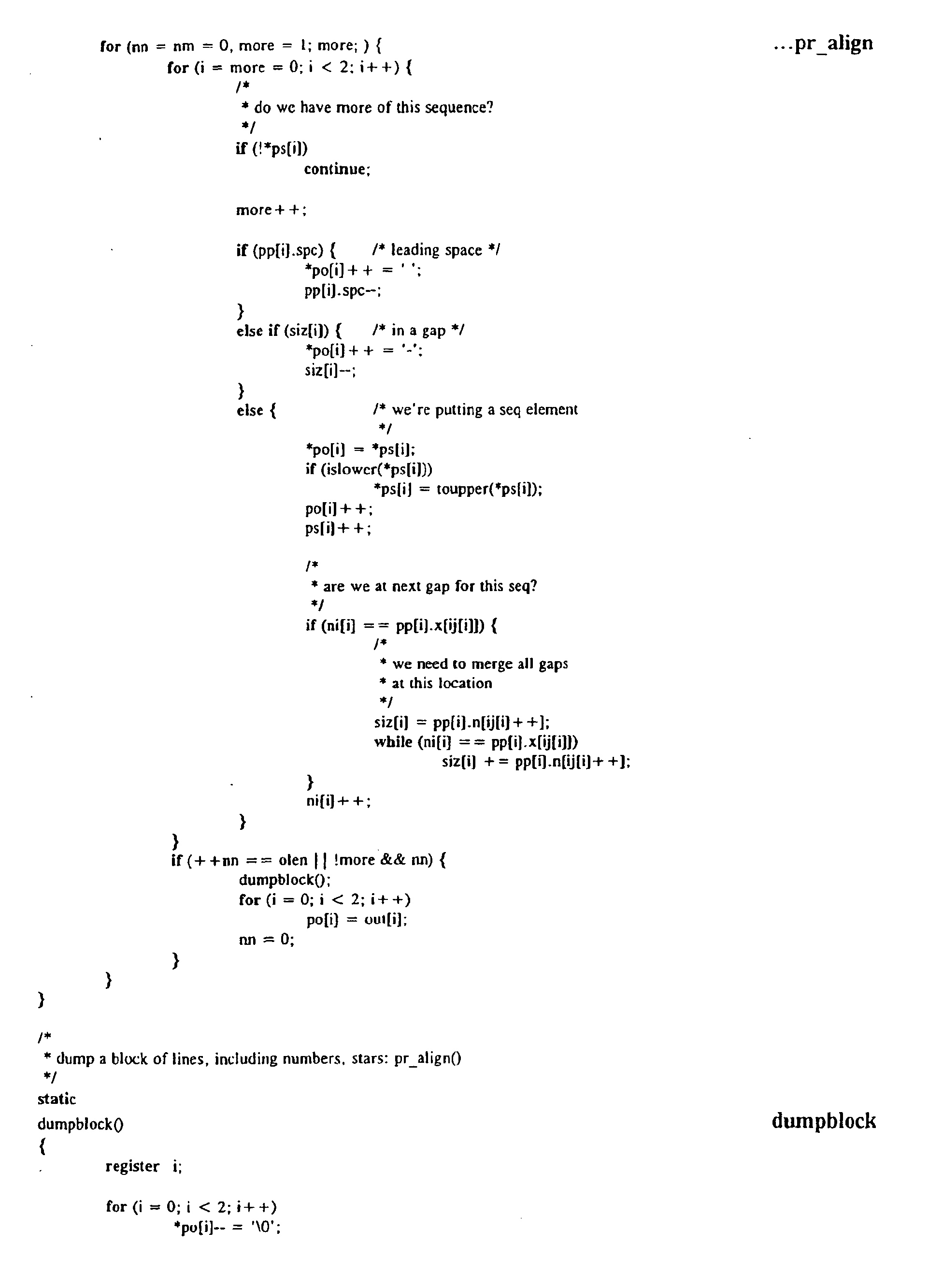

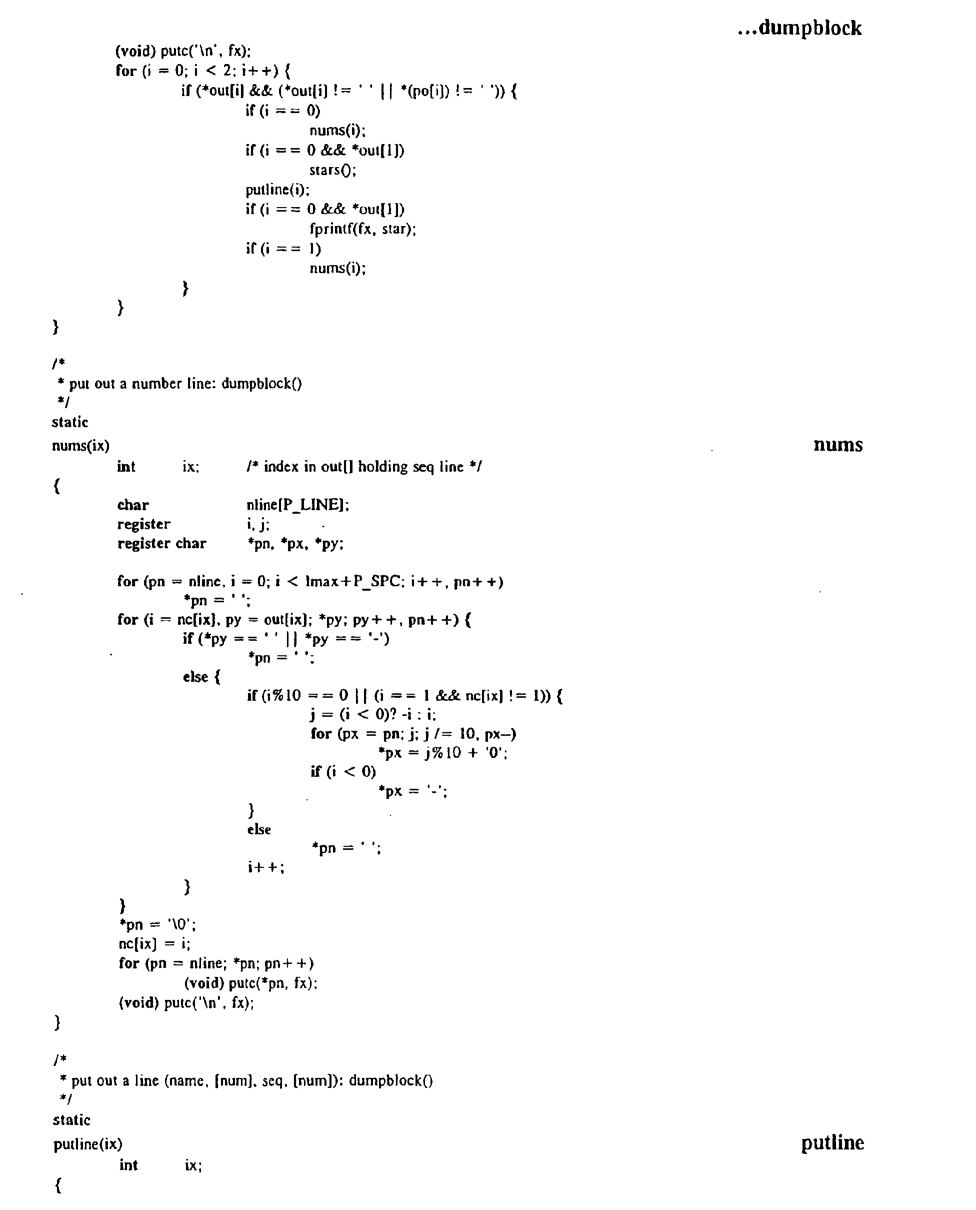

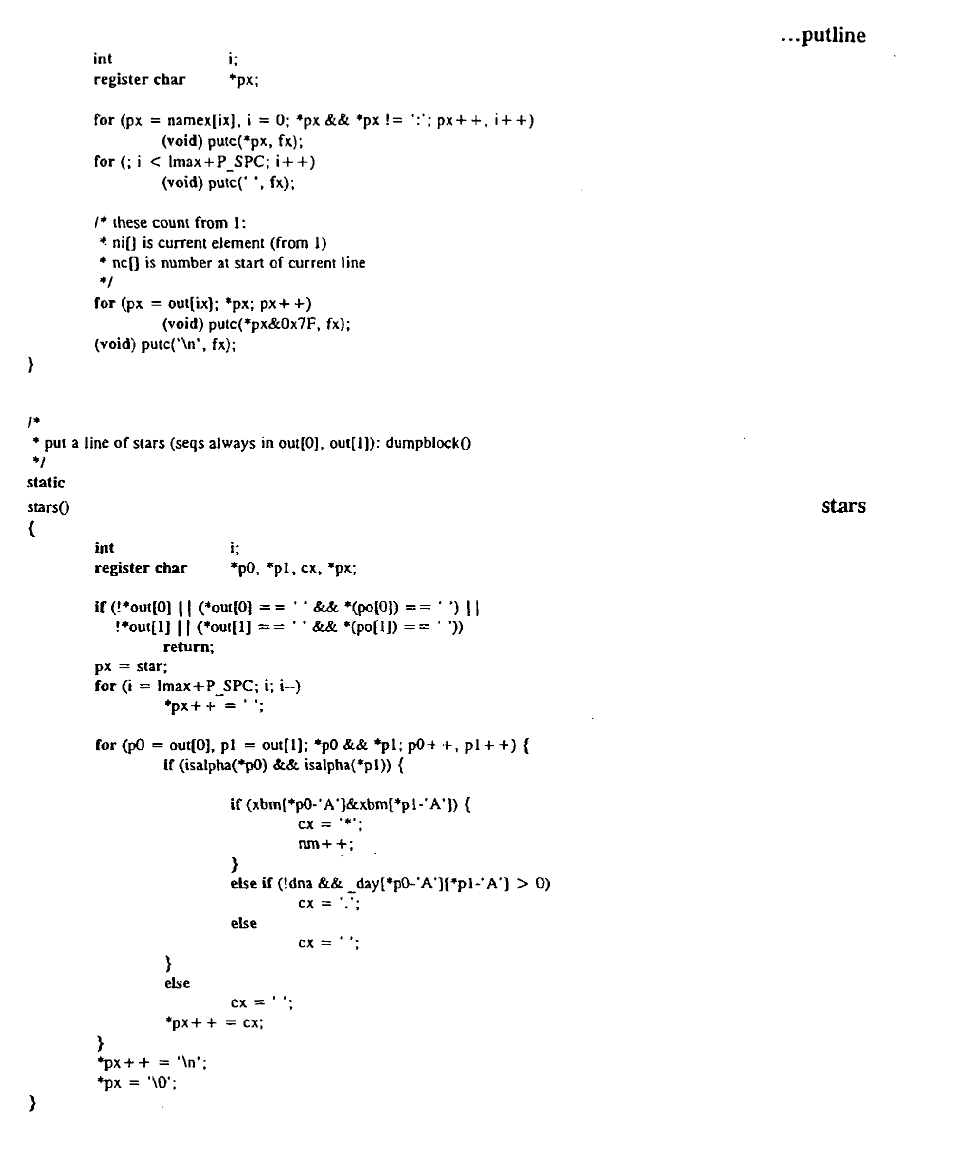

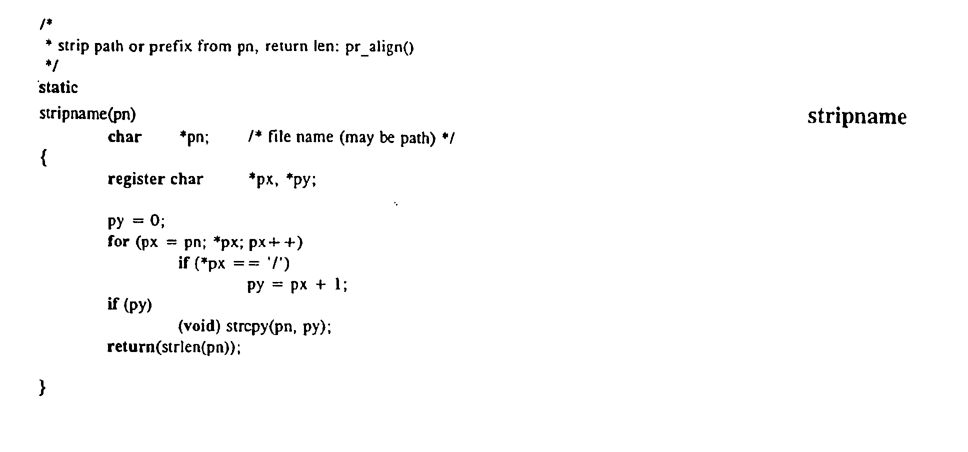

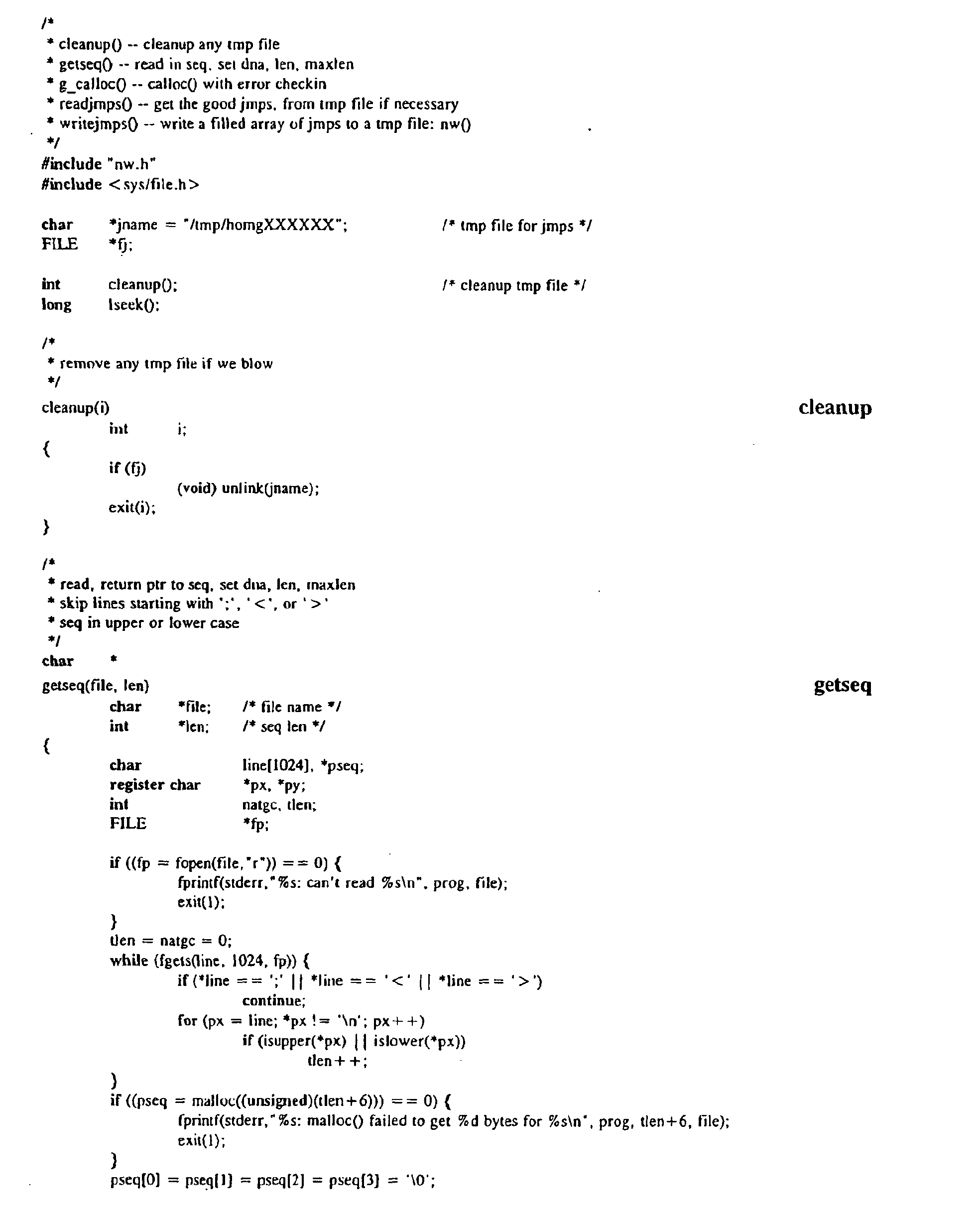

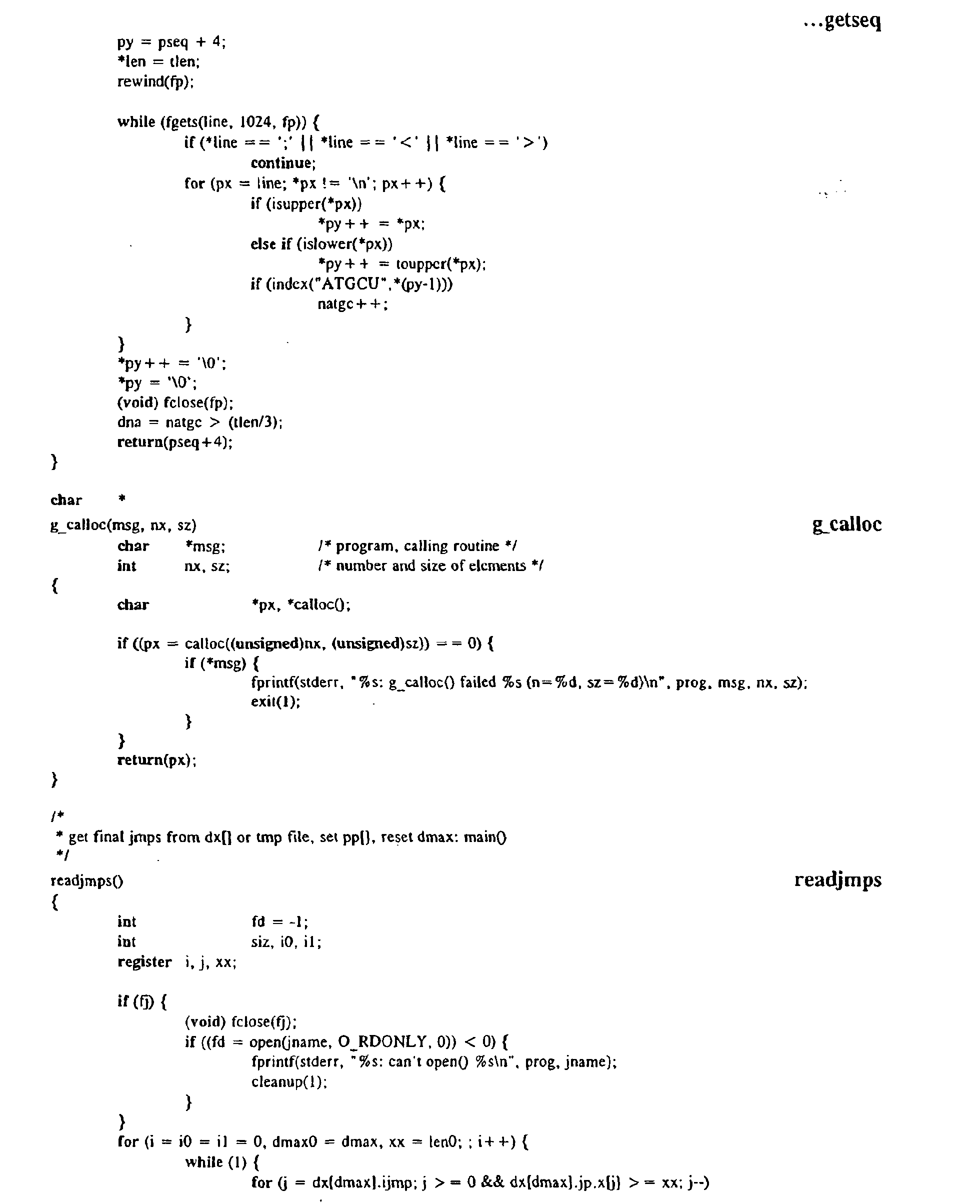

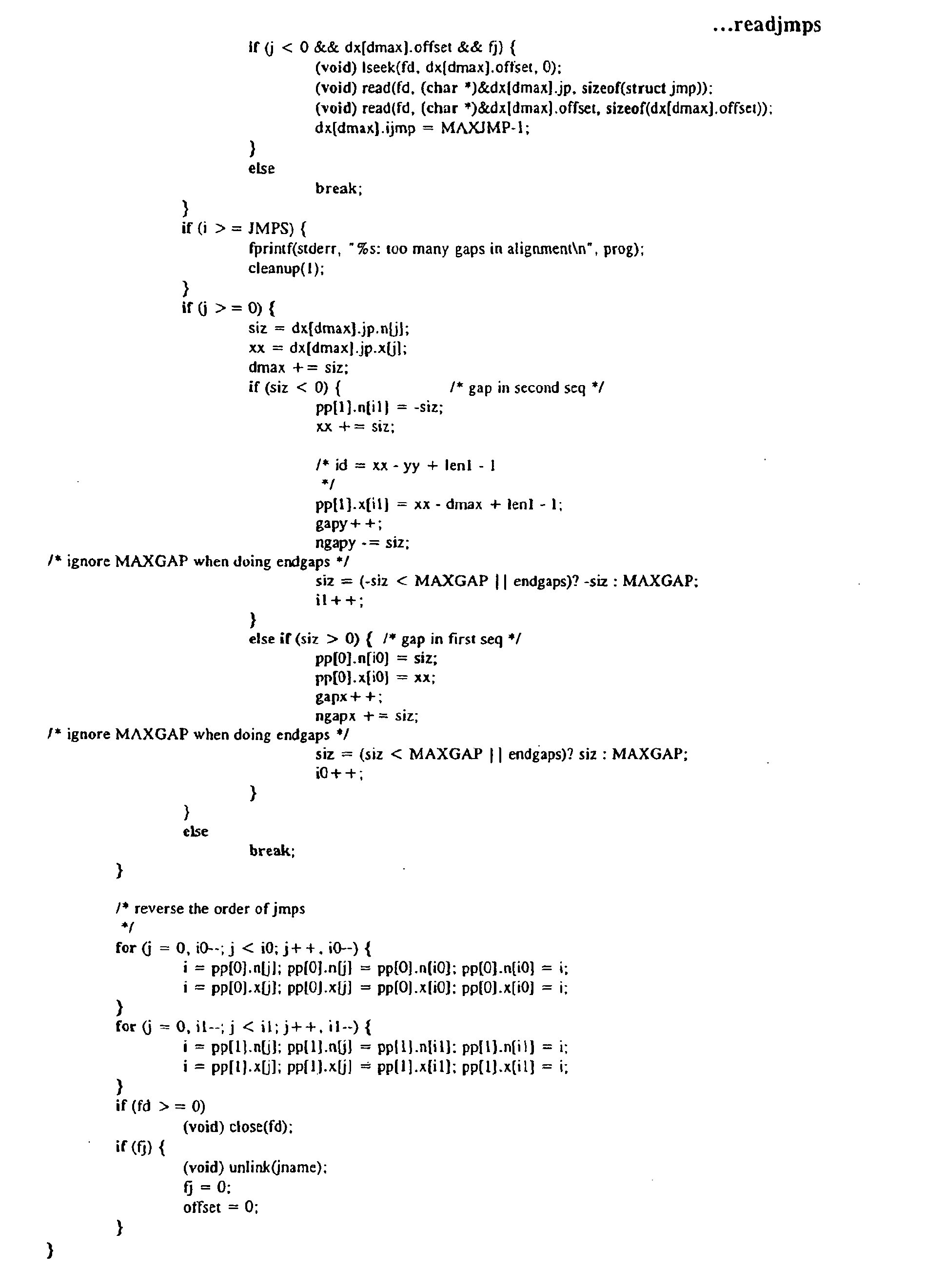

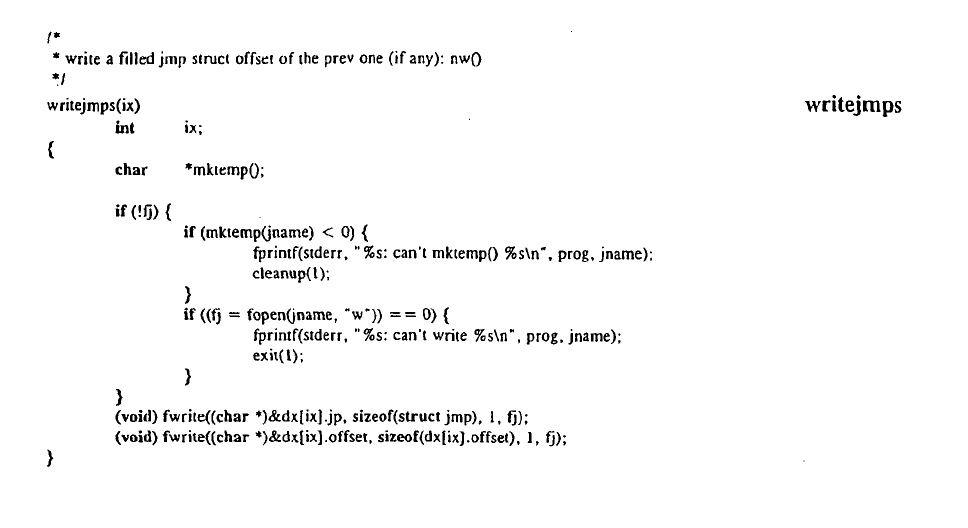

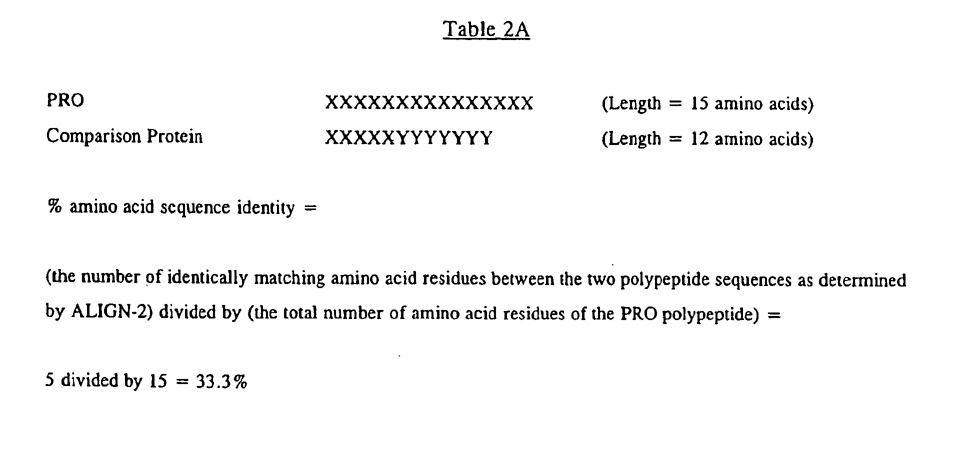

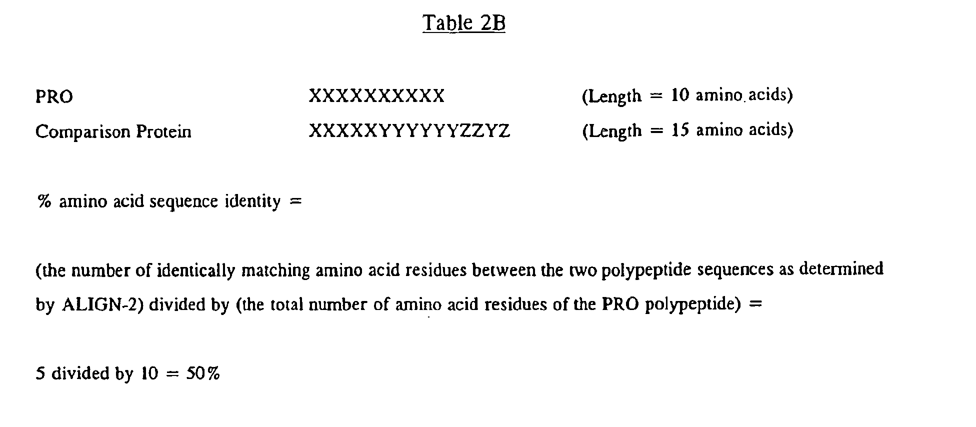

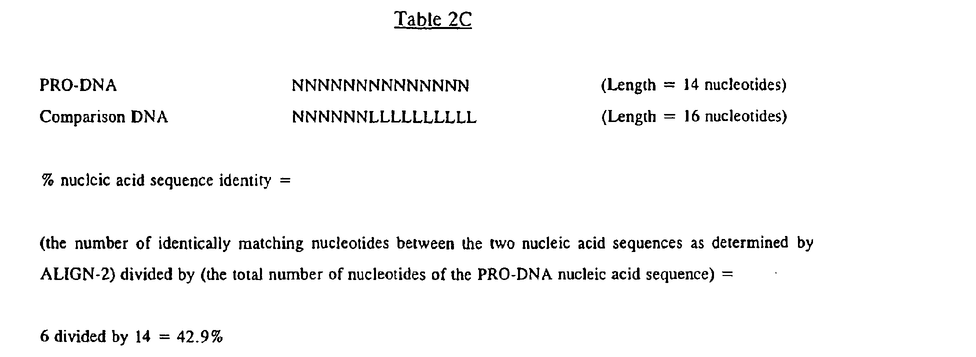

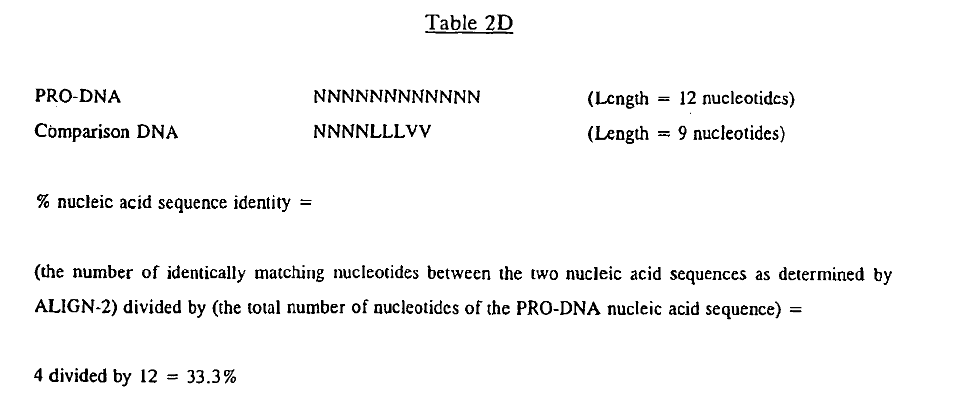

"Percent (%) amino acid sequence identity" with respect to the PRO179, PRO207, PRO320, PRO219, PRO221, PRO224, PRO328, PRO301, PRO526, PRO362, PRO356, PRO509 and PRO866 polypeptide sequences identified herein is defined as the percentage of amino acid residues in a candidate sequence that are identical with the amino acid residues in a PRO179, PRO207, PRO320, PRO219, PRO221, PRO224, PRO328, PRO301, PRO526, PRO362, PRO356, PRO509 or PRO866 sequence, after aligning the sequences and introducing gaps, if necessary, to achieve the maximum percent sequence identity, and not considering any conservative substitutions as part of the sequence identity. Alignment for purposes of determining percent am ino acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are obtained as described below by using the sequence comparison computer program ALIGN-2, wherein the complete source code for the ALIGN-2 program is provided in Table 1. The ALIGN-2 sequence comparison computer program was authored by Genentech, Inc., and the source code shown in Table 1 has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1. The ALIGN-2 program should be compiled for use on a UNIX operating system, preferably digital UNIX V4.0D. All sequence comparison parameters are set by the ALIGN-2 program and do not vary.

For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations, Tables 2A-2B demonstrate how to calculate the % amino acid sequence identity of the amino acid sequence designated "Comparison Protein" to the amino acid sequence designated "PRO".

Unless specifically stated otherwise, all % amino acid sequence identity values used herein are obtained as described above using the ALIGN-2 sequence comparison computer program. However, % amino acid sequence identity may also be determined using the sequence comparison program NCBI-BLAST2 (Altschul *et al.,* Nucleic Acids Res., 25:3389-3402 (1997)). The NCBI-BLAST2 sequence comparison program may be downloaded from http://www.ncbi.nlm.nih.gov. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

In addition, % amino acid sequence identity may also be determined using the WU-BLAST-2 computer program (Altschul *et al.,* Methods in Enzymology, 266:460-480 (1996)). Most of the WU-BLAST-2 search parameters are set to the default values. Those not set to default values, i. e., the adjustable parameters, are set with the following values: overlap span = 1, overlap fraction = 0.125, word threshold (T) = 11, and scoring matrix = BLOSUM62. For purposes herein, a % amino acid sequence identity value is determined by dividing (a) the number of matching identical amino acids residues between the amino acid sequence of the PRO polypeptide of interest having a sequence derived from the native PRO polypeptide and the comparison amino acid sequence of interest (*i.e.,* the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

"PRO179 variant polynucleotide" or "PRO179 variant nucleic acid sequence" means a nucleic acid molecule which encodes an active PRO179 polypeptide as defined below and which has at least about 80% nucleic acid sequence identity with either (a) a nucleic acid sequence which encodes residues 1 or about 17 to 460 of the PRO179 polypeptide shown in Figure 2 (SEQ ID NO:2), (b) a nucleic acid sequence which encodes amino acids X to 460 of the PRO179 polypeptide shown in Figure 2 (SEQ ID NO:2), wherein X is any amino acid residue from 12 to 21 of Figure 2 (SEQ ID NO:2), or (c) a nucleic acid sequence which encodes another specifically derived fragment of the amino acid sequence shown in Figure 2 (SEQ ID NO:2). Ordinarily, a PRO179 variant polynucleotide will have at least about 80% nucleic acid sequence identity, more preferably at least about 81% nucleic acid sequence identity, more preferably at least about 82% nucleic acid sequence identity, more preferably at least about 83%nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues 1 or about 17 to 460 of the PRO179 polypeptide shown in Figure 2 (SEQ ID NO:2), (b) a nucleic acid sequence which encodes amino acids X to 460 of the PRO179 polypeptide shown in Figure 2 (SEQ ID NO:2), wherein X is any amino acid residue from 12 to 21 of Figure 2 (SEQ ID NO:2), or (c) a nucleic acid sequence which encodes another specifically derived fragment of the amino acid sequence shown in Figure 2 (SEQ ID NO:2). PRO 179 polynucleotide variants do not encompass the native PRO179 nucleotide sequence.

"PRO207 variant polynucleotide" or "PRO207 variant nucleic acid sequence" means a nucleic acid molecule which encodes an active PRO207 polypeptide as defined below and which has at least about 80% nucleic acid sequence identity with either (a) a nucleic acid sequence which encodes residues 1 or about 41 to 249 of the PRO207 polypeptide shown in Figure 4 (SEQ ID NO:7), (b) a nucleic acid sequence which encodes amino acids X to 249 of the PRO207 polypeptide shown in Figure 4 (SEQ ID NO:7), wherein X is any amino acid residue from 36 to 45 of Figure 4 (SEQ ID NO:7), or (c) a nucleic acid sequence which encodes another specifically derived fragment of the amino acid sequence shown in Figure 4 (SEQ ID NO:7). Ordinarily, a PRO207 variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues 1 or about 41 to 249 of the PRO207 polypeptide shown in Figure 4 (SEQ ID NO:7), (b) a nucleic acid sequence which encodes amino acids X to 249 of the PRO207 polypeptide shown in Figure 4 (SEQ ID NO:7), wherein X is any amino acid residue from 36 to 45 of Figure 4 (SEQ ID NO:7), or (c) a nucleic acid sequence which encodes another specifically derived fragment of the amino acid sequence shown in Figure 4 (SEQ ID NO:7). PRO207 polynucleotide variants do not encompass the native PRO207 nucleotide sequence.

"PRO320 variant polynucleotide" or "PRO320 variant nucleic acid sequence" means a nucleic acid molecule which encodes an active PRO320 polypeptide as defined below and which has at least about 80% nucleic acid sequence identity with either (a) a nucleic acid sequence which encodes residues 1 or about 22 to 338 of the PRO320 polypeptide shown in Figure 6 (SEQ ID NO:10), (b) a nucleic acid sequence which encodes amino acids X to 338 of the PRO320 polypeptide shown in Figure 6 (SEQ ID NO: 10), wherein X is any amino acid residue from 17 to 26 of Figure 6 (SEQ ID NO:10), or (c) a nucleic acid sequence which encodes another specifically derived fragment of the amino acid sequence shown in Figure 6 (SEQ ID NO:10). Ordinarily, a PRO320 variant polynucleotide will have at least about 80% nucleic acid sequence identity, more preferably at least about 81 % nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at (east about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues 1 or about 22 to 338 of the PRO320 polypeptide shown in Figure 6 (SEQ ID NO: 10), (b) a nucleic acid sequence which encodes amino acids X to 338 of the PRO320 polypeptide shown in Figure 6 (SEQ ID NO:10), wherein X is any amino acid residue from 17 to 26 of Figure 6 (SEQ ID NO: 10), or (c) a nucleic acid sequence which encodes another specifically derived fragment of the amino acid sequence shown in Figure 6 (SEQ ID NO:) 10). PRO320 polynucleotide variants do not encompass the native PRO320 nucleotide sequence.

"PRO219 variant polynucleotide" or "PRO219 variant nucleic acid sequence" means a nucleic acid molecule which encodes an active PRO219 polypeptide as defined below and which has at least about 80% nucleic acid sequence identity with either (a) a nucleic acid sequence which encodes residues I or about 24 to 1005 of the PRO219 polypeptide shown in Figure 8 (SEQ ID NO: 15), (b) a nucleic acid sequence which encodes amino acids X to 1005 of the PRO219 polypeptide shown in Figure 8 (SEQ ID NO: 15), wherein X is any amino acid residue from 19 to 28 of Figure 8 (SEQ ID NO:15), or (c) a nucleic acid sequence which encodes another specifically derived fragment of the amino acid sequence.shown in Figure 8 (SEQ ID NO: 15). Ordinarily, a PRO219 variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues 1 or about 24 to 1005 of the PRO219 polypeptide shown in Figure 8 (SEQ ID NO:15), (b) a nucleic acid sequence which encodes amino acids X to 1005 of the PRO219 polypeptide shown in Figure 8 (SEQ ID NO: 15), wherein X is any amino acid residue from 19 to 28 of Figure 8 (SEQ ID NO:] 15), or (c) a nucleic acid sequence which encodes another specifically derived fragment of the amino acid sequence shown in Figure 8 (SEQ ID NO: 15). PRO219 polynucleotide variants do not encompass the native PRO219 nucleotide sequence.

"PRO221 variant polynucleotide" or "PRO221 variant nucleic acid sequence" means a nucleic acid molecule which encodes an active PRO221 polypeptide as defined below and which has at least about 80% nucleic acid sequence identity with either (a) a nucleic acid sequence which encodes residues 1 or about 34 to 259 of the PRO221 polypeptide shown in Figure 10 (SEQ ID NO:20), (b) a nucleic acid sequence which encodes amino acids X to 259 of the PRO221 polypeptide shown in Figure 10 (SEQ ID NO:20), wherein X is any amino acid residue from 29 to 38 of Figure 10 (SEQ ID NO:20), (c) 1 or about 34 to X of Figure 10 (SEQ ID NO:20), wherein X is any amino acid from amino acid 199 to amino acid 208 of Figure 10 (SEQ ID NO:20), or (d) a nucleic acid sequence which encodes another specifically derived fragment of the amino acid sequence shown in Figure 10 (SEQ ID NO:20). Ordinarily, a PRO221 variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues 1 or about 34 to 259 of the PRO221 polypeptide shown in Figure 10 (SEQ ID NO:20), (b) a nucleic acid sequence which encodes amino acids X to 259 of the PRO221 polypeptide shown in Figure 10 (SEQ ID NO:20), wherein X is any amino acid residue from 29 to 38 of Figure 10 (SEQ ID NO:20), (c) 1 or about 34 to X of Figure 10 (SEQ ID NO:20), wherein X is any amino acid from amino acid 199 to amino acid 208 of Figure 10 (SEQ ID NO:20), or (d) a nucleic acid sequence which encodes another specifically derived fragment ofthe amino acid sequence shown in Figure 10 (SEQ ID NO:20). PRO221 polynucleotide variants do not encompass the native PRO221 nucleotide sequence.

"PRO224 variant polynucleotide" or "PRO224 variant nucleic acid sequence" means a nucleic acid molecule which encodes an active PRO224 polypeptide as defined below and which has at least about 80% nucleic acid sequence identity with either (a) a nucleic acid sequence which encodes residues 1 or about 31 to 282 of the PRO224 polypeptide shown in Figure 12 (SEQ ID NO:25), (b) a nucleic acid sequence which encodes amino acids X to 282 of the PRO224 polypeptide shown in Figure 12 (SEQ ID NO:25), wherein X is any amino acid residue from 26 to 35 of Figure 12 (SEQ ID NO:25), (c) 1 or about 31 to X of Figure 12 (SEQ ID NO:25), wherein X is any amino acid from amino acid 226 to amino acid 235 of Figure 12 (SEQ ID NO:25), or (d) a nucleic acid sequence which encodes another specifically derived fragment of the amino acid sequence shown in Figure 12 (SEQ ID NO:25). Ordinarily, a PRO224 variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues 1 or about 31 to 282 of the PRO224 polypeptide shown in Figure 12 (SEQ ID NO:25), (b) a nucleic acid sequence which encodes amino acids X to 282 of the PRO224 polypeptide shown in Figure 12 (SEQ ID NO:25), wherein X is any amino acid residue from 26 to 35 of Figure 12 (SEQ ID NO:25), (c) 1 or about 31 to X of Figure 12 (SEQ ID NO:25), wherein X is any amino acid from amino acid 226 to amino acid 235 of Figure 12 (SEQ ID NO:25), or (d) a nucleic acid sequence which encodes another specifically derived fragment of the am ino acid sequence shown in Figure 12 (SEQ ID NO:25). PRO224 polynucleotide variants do not encompass the native PRO224 nucleotide sequence.

"PRO328 variant polynucleotide" or "PRO328 variant nucleic acid sequence" means a nucleic acid molecule which encodes an active PRO328 polypeptide as defined below and which has at least about 80% nucleic acid sequence identity with either (a) a nucleic acid sequence which encodes residues 1 or about 23 to 463 of the PRO328 polypeptide shown in Figure 14 (SEQ ID NO:30), (b) a nucleic acid sequence which encodes amino acids X to 463 of the PRO328 polypeptide shown in Figure 14 (SEQ ID NO:30), wherein X is any amino acid residue from 18 to 27 of Figure 14 (SEQ ID NO:30), or (c) a nucleic acid sequence which encodes another specifically derived fragment of the amino acid sequence shown in Figure 14 (SEQ ID NO:30). Ordinarily, a PRO328 variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues 1 or about 23 to 463 of the PRO328 polypeptide shown in Figure 14 (SEQ ID NO:30), (b) a nucleic acid sequence which encodes amino acids X to 463 of the PRO328 polypeptide shown in Figure 14 (SEQ ID NO:30), wherein X is any amino acid residue from 18 to 27 of Figure 14 (SEQ ID NO:30), or (c) a nucleic acid sequence which encodes another specifically derived fragment of the amino acid sequence shown in Figure 14 (SEQ ID NO:30). PRO328 polynucleotide variants do not encompass the native PRO328 nucleotide sequence.

"PRO301 variant polynucleotide" or "PRO301 variant nucleic acid sequence" means a nucleic acid molecule which encodes an active PRO301 polypeptide as defined below and which has at least about 80% nucleic acid sequence identity with either (a) a nucleic acid sequence which encodes residues 1 or about 28 to 299 of the PRO301 polypeptide shown in Figure 16 (SEQ ID NO:35), (b) a nucleic acid sequence which encodes amino acids X to 299 of the PRO301 polypeptide shown in Figure 16 (SEQ ID NO:35), wherein X is any amino acid residue from 23 to 32 of Figure 16 (SEQ 1D NO:35), (c) 1 or about 28 to X of Figure 16 (SEQ ID NO:35), wherein X is any amino acid from amino acid 230 to amino acid 239 of Figure 16 (SEQ ID NO:35), or (d) a nucleic acid sequence which encodes another specifically derived fragment of the amino acid sequence shown in Figure 16 (SEQ ID NO:35). Ordinarily, a PRO301 variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues 1 or about 28 to 299 of the PRO301 polypeptide shown in Figure 16 (SEQ ID NO:35), (b) a nucleic acid sequence which encodes amino acids X to 299 of the PRO301 polypeptide shown in Figure 16 (SEQ ID NO:35), wherein X is any amino acid residue from 23 to 32 of Figure 16 (SEQ ID NO:35), (c) 1 or about 28 to X of Figure 16 (SEQ ID NO:35), wherein X is any amino acid from amino acid 230 to amino acid 239 of Figure 16 (SEQ ID NO:35), or (d) a nucleic acid sequence which encodes another specifically derived fragment ofthe amino acid sequence shown in Figure 16 (SEQ ID NO:35). PRO301 polynucleotide variants do not encompass the native PRO301 nucleotide sequence.

"PRO526 variant polynucleotide" or "PRO526 variant nucleic acid sequence" means a nucleic acid molecule which encodes an active PRO526 polypeptide as defined below and which has at least about 80% nucleic acid sequence identity with either (a) a nucleic acid sequence which encodes residues 1 or about 27 to 473 of the PRO526 polypeptide shown in Figure 18 (SEQ ID NO:43), (b) a nucleic acid sequence which encodes amino acids X to 473 of the PRO526 polypeptide shown in Figure 18 (SEQ ID NO:43), wherein X is any amino acid residue from 22 to 31 of Figure 18 (SEQ ID NO:43), or (c) a nucleic acid sequence which encodes another specifically derived fragment of the amino acid sequence shown in Figure 18 (SEQ ID NO:43). Ordinarily, a PRO526 variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues I or about 27 to 473 of the PRO526 polypeptide shown in Figure 18 (SEQ ID NO:43), (b) a nucleic acid sequence which encodes amino acids X to 473 of the PRO526 polypeptide shown in Figure 18 (SEQ ID NO:43), wherein X is any amino acid residue from 22 to 31 of Figure 18 (SEQ ID NO:43), or (c) a nucleic acid sequence which encodes another specifically derived fragment of the amino acid sequence shown in Figure 18 (SEQ ID NO:43). PRO526 polynucleotide variants do not encompass the native PRO526 nucleotide sequence.

"PRO362 variant polynucleotide" or "PRO362 variant nucleic acid sequence" means a nucleic acid molecule which encodes an active PRO362 polypeptide as defined below and which has at least about 80% nucleic acid sequence identity with either (a) a nucleic acid sequence which encodes residues 1 or about 20 to 321 of the PRO362 polypeptide shown in Figure 20 (SEQ )DNO:48), (b) a nucleic acid sequence which encodes amino acids X to 321 of the PRO362 polypeptide shown in Figure 20 (SEQ ID NO:48), wherein X is any amino acid residue from 15 to 24 of Figure 20 (SEQ ID NO:48), (c) 1 or about 20 to X of Figure 20 (SEQ ID NO:48), wherein X is any amino acid from amino acid 276 to amino acid 285 of Figure 20 (SEQ ID NO:48), or (d) a nucleic acid sequence which encodes another specifically derived fragment of the amino acid sequence shown in Figure 20 (SEQ ID NO:48). Ordinarily, a PRO362 variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues I or about 20 to 321 of the PRO362 polypeptide shown in Figure 20 (SEQ ID NO:48), (b) a nucleic acid sequence which encodes amino acids X to 32 of the PRO362 polypeptide shown in Figure 20 (SEQ lD NO:49), wherein X is any amino acid residue from 15 to 24 of Figure 20 (SEQ ID NO:48), (c) 1 or about 20 to X of Figure 20 (SEQ ID NO.48), wherein X is any amino acid from amino acid 276 to amino acid 285 of Figure 20 (SEQ ID NO:48), or (d) a nucleic acid sequence which encodes another specifically derived fragment of the amino acid sequence shown in Figure 20 (SEQ ID NO:48). PRO362 polynucleotide variants do not encompass the native PRO362 nucleotide sequence.

"PRO356 variant polynucleotide" or "PRO356 variant nucleic acid sequence" means a nucleic acid molecule which encodes an active PRO356 polypeptide as defined below and which has at least about 80% nucleic acid sequence identity with either (a) a nucleic acid sequence which encodes residues 1 or about 27 to 346 of the PRO356 polypeptide shown in Figure 22 (SEQ ID NO:55), (b) a nucleic acid sequence which encodes amino acids X to 346 of the PRO356 polypeptide shown in Figure 22 (SEQ ID NO:55), wherein X is any amino acid residue from 22 to 31 of Figure 22 (SEQ ID NO:55), or (c) a nucleic acid sequence which encodes another specifically derived fragment ofthe amino acid sequence shown in Figure 22 (SEQ ID NO:55). Ordinarily, a PRO356 variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87%nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues or about 27 to 346 of the PRO356 polypeptide shown in Figure 22 (SEQ ID NO:55), (b) a nucleic acid sequence which encodes amino acids X to 346 of the PRO356 polypeptide shown in Figure 22 (SEQ ID NO:55), wherein X is any amino acid residue from 22 to 31 of Figure 22 (SEQ ID NO:55), or (c) a nucleic acid sequence which encodes another specifically derived fragment of the amino acid sequence shown in Figure 22 (SEQ ID NO:55). PRO356 polynucleotide variants do not encompass the native PRO356 nucleotide sequence.

"PRO509 variant polynucleotide" or "PRO509 variant nucleic acid sequence" means a nucleic acid molecule which encodes an active PRO509 polypeptide as defined below and which has at least about 80% nucleic acid sequence identity with either (a) a nucleic acid sequence which encodes residues 1 or about 37 to 283 of the PRO509 polypeptide shown in Figure 24 (SEQ ID NO:60), (b) a nucleic acid sequence which encodes amino acids X to 283 of the PRO509 polypeptide shown in Figure 24 (SEQ ID NO:60), wherein X is any amino acid residue from 32 to 41 of Figure 24 (SEQ ID NO:60), (c) 1 or about 37 to X of Figure 24 (SEQ ID NO:60), wherein X is any amino acid from amino acid 200 to amino acid 209 of Figure 24 (SEQ ID NO:60), or (d) a nucleic acid sequence which encodes another specifically derived fragment of the amino acid sequence shown in Figure 24 (SEQ ID NO:60). Ordinarily, a PRO509 variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues I or about 37 to 283 of the PRO509 polypeptide shown in Figure 24 (SEQ ID NO:60), (b) a nucleic acid sequence which encodes amino acids X to 283 of the PRO509 polypeptide shown in Figure 24 (SEQ ID NO:60), wherein X is any amino acid residue from 32 to 41 of Figure 24 (SEQ ID NO:60), (c) 1 or about 37 to X of Figure 24 (SEQ ID NO:60), wherein X is any amino acid from amino acid 200 to amino acid 209 of Figure 24 (SEQ ID NO:60), or (d) a nucleic acid sequence which encodes another specifically derived fragment of the amino acid sequence shown in Figure 24 (SEQ ID NO:60). PRO509 polynucleotide variants do not encompass the native PRO509 nucleotide sequence.

"PRO866 variant polynucleotide" or "PRO866 variant nucleic acid sequence" means a nucleic acid molecule which encodes an active PRO866 polypeptide as defined below and which has at least about 80% nucleic acid sequence identity with either (a) a nucleic acid sequence which encodes residues 1 or about 27 to 331 of the PRO866 polypeptide shown in Figure 26 (SEQ ID NO:62), (b) a nucleic acid sequence which encodes amino acids X to 331 of the PRO866 polypeptide shown in Figure 26 (SEQ ID NO:62), wherein X is any amino acid residue from 22 to 31 of Figure 26 (SEQ ID NO:62), or (c) a nucleic acid sequence which encodes another specifically derived fragment of the amino acid sequence shown in Figure 26 (SEQ ID NO:62). Ordinarily, a PRO866 variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91 % nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with either (a) a nucleic acid sequence which encodes residues 1 or about 27 to 331 of the PRO866 polypeptide shown in Figure 26 (SEQ ID NO:62), (b) a nucleic acid sequence which encodes amino acids X to 331 of the PRO866 polypeptide shown in Figure 26 (SEQ ID NO:62), wherein X is any amino acid residue from 22 to 31 of Figure 26 (SEQ ID NO:62), or (c) a nucleic acid sequence which encodes another specifically derived fragment of the amino acid sequence shown in Figure 26 (SEQ ID NO:62). PRO866 polynucleotide variants do not encompass the native PRO866 nucleotide sequence.

Ordinarily, PRO179, PRO207, PRO320, PRO219, PRO221, PRO224, PRO328, PRO301, PRO526, PRO362, PRO356, PRO509 and PRO866 variant polynucleotides are at least about 30 nucleotides in length, often at least about 60 nucleotides in length, more often at least about 90 nucleotides in length, more often at least about 120 nucleotides in length, more often at least about 150 nucleotides in length, more often at least about 180 nucleotides in length, more often at least about 210 nucleotides in length, more often at least about 240 nucleotides in length, more often at least about 270 nucleotides in length, more often at least about 300 nucleotides in length, more often at least about 450 nuclcotides in length, more often at least about 600 nucleotides in length, more often at least about 900 nucleotides in length, or more.

"Percent (%) nucleic acid sequence identity" with respect to the PRO 179, PRO207, PRO320, PRO219, PRO221, PRO224, PRO328, PRO301, PRO526, PRO362, PRO356, PRO509 and PRO866 polypeptide-encoding nucleic acid sequences identified herein is defined as the percentage of nucleotides in a candidate sequence that are identical with the nucleotides in a PRO179, PRO207, PRO320, PRO219, PRO221, PRO224, PRO328, PRO301, PRO526, PRO362, PRO356, PRO509 or PRO866 polypeptide-encoding nucleic acid sequence, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % nucleic acid sequence identity values are obtained as described below by using the sequence comparison computer program ALIGN-2, wherein the complete source code for the ALIGN-2 program is provided in Table 1. The ALIGN-2 sequence comparison computer program was authored by Genentech, Inc., and the source code shown in Table I has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1. The ALIGN-2 program should be compiled for use on a UNIX operating system, preferably digital UNIX V4.0D. All sequence comparison parameters are set by the ALIGN-2 program and do not vary.

For purposes herein, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity ofD to C. As examples of % nucleic acid sequence identitycalculations,Tables 2C-2D demonstrate how to calculate the % nucleic acid sequence identity ofthe nucleic acid sequence designated "Comparison DNA" to the nucleic acid sequence designated "PRO-DNA".

Unless specifically stated otherwise, all % nucleic acid sequence identity values used herein are obtained as described above using the ALIGN-2 sequence comparison computer program. However, % nucleic acid sequence identity may also be determined using the sequence comparison program NCBI-BLAST2 (Altschul et al., Nucleic Acids Res., 25:3389-3402 (1997)). The NCBI-BLAST2 sequence comparison program may be downloaded from http://www.ncbi.nlm.nih.gov. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In addition, % nucleic acid sequence identity values may also be generated using the WU-BLAST-2 computer program (Altschul *et al.,* Methods in Enzymoloey, 266:460-480 (1996)). Most of the WU-BLAST-2 search parameters are set to the default values. Those not set to default values, *i.e.,* the adjustable parameters, are set with the following values: overlap span = 1, overlap fraction = 0.125, word threshold (T) = 11, and scoring matrix = BLOSUM62. For purposes herein, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (*i.e.,* the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

In other embodiments, PRO179, PRO207, PRO320, PRO219, PRO221, PRO224, PRO328, PRO301, PRO526, PRO362, PRO356, PRO509 and PRO866 variant polynucleotides are nucleic acid molecules that encode an active PRO179, PRO207, PRO320, PRO219, PRO221, PRO224, PRO328, PRO301, PRO526, PRO362, PRO356, PRO509 or PRO866 polypeptide, respectively, and which are capable of hybridizing, preferably under stringent hybridization and wash conditions, to nucleotide sequences encoding the full-length PRO179 polypeptide shown in Figure 2 (SEQ ID NO:2), to nucleotide sequences encoding the full-length PRO207 polypeptide shown in Figure 4 (SEQ ID NO:7), to nucleotide sequences encoding the full-length PRO320 polypeptide shown in Figure 6 (SEQ ID NO:10), to nucleotide sequences encoding the full-length PRO219 polypeptide shown in Figure 8 (SEQ ID NO:15), to nucleotide sequences encoding the full-length PRO221 polypeptide shown in Figure 10 (SEQ ID NO:20), to nucleotide sequences encoding the full-length PRO224 polypeptide shown in Figure 12 (SEQ ID NO:25), to nucleotide sequences encoding the full-length PRO328 polypeptide shown in Figure 14 (SEQ ID NO:30), to nucleotide sequences encoding the full-length PRO301 polypeptide shown in Figure 16 (SEQ ID NO:35), to nucleotide sequences encoding the full-length PRO526 polypeptide shown in Figure 18 (SEQ ID NO:43), to nucleotide sequences encoding the full-length PRO362 polypeptide shown in Figure 20 (SEQ ID NO:48), to nucleotide sequences encoding the full-length PRO356 polypeptide shown in Figure 22 (SEQ ID NO:55), to nucleotide sequences encoding the full-length PRO509 polypeptide shown in Figure 24 (SEQ ID NO:60), to nucleotide sequences encoding the full-length PRO866 polypeptide shown in Figure 26 (SEQ ID NO:62), respectively. PRO179, PRO207, PRO320, PRO219, PRO221, PRO224, PRO328, PRO301, PRO526, PRO362, PRO356, PRO509 and PRO866 variant polypeptides may be those that are encoded by a PRO179, PRO207, PRO320, PRO219, PRO221, PRO224, PRO328, PRO301, PRO526, PRO362, PRO356, PRO509 or PRO866 variant polynucleotide.

The term "positives", in the context of the amino acid sequence identity comparisons performed as described above, includes amino acid residues in the sequences compared that are not only identical, but also those that have similar properties. Amino acid residues that score a positive value to an amino acid residue of interest are those that are either identical to the amino acid residue of interest or are a preferred substitution (as defined in Table 3 below) of the amino acid residue of interest.

For purposes herein, the % value of positives of a given amino acid sequence A to, with, or against a given amino acid sequence B (which can alternatively be phrased as a given amino acid sequence A that has or comprises a certain % positives to, with, or against a given amino acid sequence B) is calculated as follows:
100 times the fraction X/Y where X is the number of amino acid residues scoring a positive value as defined above by the sequence alignment program ALIGN-2 in that program's alignment of A and B, and where Y is the total number of amino acid residues in B. It will be appreciated that where the length of amino acid sequence A is not equal to the length of amino acid sequence B, the % positives of A to B will not equal the % positives of B to A.

"Isolated," when used to describe the various polypeptides disclosed herein, means polypeptide that has been identified and separated and/or recovered from a component of its natural environment. Preferably, the O isolated polypeptide is free of association with all components with which it is naturally associated. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal am ino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO179, PRO207, PRO320, PRO219, PRO221, PRO224, PRO328, PRO301, PRO526, PRO362, PRO356, PRO509 or PRO866 natural environment will not be present. Ordinarily, however, isolated polypeptide will be prepared by at least one purification step.

An "isolated" nucleic acid molecule encoding a PRO179, PRO207, PRO320, PRO219, PRO221, PRO224, PRO328, PRO301, PRO526, PRO362, PRO356, PRO509 or PRO866 polypeptide or an "isolated" nucleic acid molecule encoding an anti-PRO179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 or anti-PRO866 antibody is a nucleic acid molecule that is identified and separated from at least one contaminant nucleic acid molecule with which it is ordinarily associated in the natural source of the PRO179-, PRO207-, PRO320-, PRO219-, PRO221-, PRO224-, PRO328-, PRO301-, PRO526-, PRO362-, PRO356-, PRO509- or PRO866-encoding nucleic acid or the anti-PRO 179-,anti-PRO207-,anti-PRO320-, anti-PRO219-, anti-PRO221-, anti-PRO224-, anti-PRO328-, anti-PRO301-, anti-PRO526-,anti-PRO362-, anti-PRO356-, anti-PRO509- or anti-PRO866-encoding nucleic acid. Preferably, the isolated nucleic acid is free of association with all components with which it is naturally associated. An isolated PRO179-, PRO207-, PRO320-, PRO219-, PRO221-, PRO224-, PRO328-, PRO301-, PRO526-, PRO362-, PRO356-, PRO509- or PRO866-encoding nucleic acid molecule or an isolated anti-PRO179-, anti-PRO207-, anti-PRO320-, anti-PRO219-, anti-PRO221-, anti-PRO224-, anti-PRO328-, anti-PRO301-, anti-PRO526-,anti- PRO362-, anti-PRO356-, anti-PRO509- or anti-PRO866-encoding nucleic acid molecule is other than in the form or setting in which it is found in nature. Isolated nucleic acid molecules therefore are distinguished from the PRO179-, PRO207-, PRO320-, PRO219-, PRO221-, PRO224-, PRO328-, PRO301 -,PRO526-, PRO362-, PRO356-, PRO509- or PRO866-encoding nucleic acid molecule or from the anti-PRO179-, anti-PRO207-, anti-PRO320-, anti-PRO219-, anti-PRO221-, anti-PRO224-, anti-PRO328-, anti-PRO301-, anti-PRO526-,anti-PRO362-, anti-PRO356-, anti-PRO509-or anti-PRO866-encoding nucleic acid molecule as it exists in natural cells. However, an isolated nucleic acid molecule encoding a PRO179, PRO207, PRO320, PRO219, PRO221, PRO224, PRO328, PRO301, PRO526, PRO362, PRO356, PRO509 or PRO866 polypeptide or an isolated nucleic acid molecule encoding an anti-PRO179, anti-PRO207, anti-PRO320, anti-PRO219, anti-PRO221, anti-PRO224, anti-PRO328, anti-PRO301,anti-PRO526,anti-PRO362, anti-PRO356, anti-PRO509 or anti-PRO866 antibody includes PRO179-, PRO207-, PRO320-, PRO219-, PRO221-, PRO224-, PRO328-, PRO301-, PRO526-, PRO362-, PRO356-, PRO509- or PRO866-nucleic acid molecules or anti-PRO179-, anti-PRO207-, anti-PRO320-, anti-PRO219-, anti-PRO221-, anti-PRO224-, anti-PRO328-, anti-PRO341-, anti-PRO526-,anti- PRO362-, anti-PRO356-, anti-PRO509- or anti-PRO866-nucleicacid molecules contained in cells that ordinarily express PRO179, PRO207, PRO320, PRO219, PRO221, PRO224, PRO328, PRO301, PRO526, PRO362, PRO356, PRO509 or PRO866 polypeptides or anti-PRO179, anti-PRO207, anti-PRO320, anti-PRO219, anti-PRO221, anti-PRO224, anti-PRO328, anti-PRO301, anti-PRO526,anti- PRO362, anti-PRO356, anti-PRO509 or anti-PRO866 antibodies where, for example, the nucleic acid molecule is in a chromosomal location different from that of natural cells.

The term "control sequences" refers to DNA sequences necessary for the expression of an operably linked coding sequence in a particular host organism. The control sequences that are suitable forprokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader,.contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO179, anti-PRO207, anti-PRO320, anti-PRO219, anti-PRO221, anti-PRO224, anti-PRO328, anti-PRO301, anti-PRO526,anti- PRO362, anti-PRO356, anti-PRO509 and anti-PRO866 monoclonal antibodies (including agonist antibodies), anti-PRO179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 and anti-PRO866 antibody compositions with polyepitopic specificity, single chain anti-PRO 179, anti-PRO207, anti-PRO320, anti-PRO219, anti-PRO221, anti-PRO224, anti-PRO328, anti-PRO301, anti-PRO526,anti-PRO362, anti-PRO356, anti-PRO509 and anti-PRO866 antibodies, and fragments of anti-PRO179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 and anti-PRO866 antibodies (see below). The term "monoclonal antibody" as used herein refers to an antibody obtained from a population of substantially homogeneous antibodies, *i.e.,* the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel *et al.,* Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1 % sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCL, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42 °C in 0.2 x SSC (sodium chloride/sodium citrate) and 50% formamide at 55 °C, followed by a high-stringency wash consisting of 0.1 x SSC containing EDTA at 55°C.

"Moderately stringent conditions" may be identified as described by Sambrook *et al.,* Molecular Cloning: A Laboratory Manual, New York: Cold Spring Harbor Press, 1989, and include the use of washing solution and hybridization conditions (*e.g.*, temperature, ionic strength and % SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in I x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO179, PRO207, PRO320, PRO219, PRO221, PRO224, PRO328, PRO301, PRO526, PRO362, PRO356, PRO509 or PRO866 polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i.e.*, is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Active" or "activity" for the purposes herein refers to form(s) of PRO179, PRO207, PRO320, PRO219, PRO221, PRO224, PRO328, PRO301, PRO526, PRO362, PRO356, PRO509 or PRO866 which retain a biological and/or an immunological activity of native or naturally-occurring PRO179, PRO207, PRO320, PRO219, PRO221, PRO224, PRO328, PRO301, PRO526, PRO362, PRO356, PRO509 or PRO866, wherein "biological" activity refers to a biological function (either inhibitory or stimulatory) caused by a native or naturally-occurring PRO179, PRO207, PRO320, PRO219, PRO221, PRO224, PRO328, PRO301, PRO526, PRO362, PRO356, PRO509 or PRO866 other than the ability to induce the production of an antibody against an antigenic epitope possessed by a native or naturally-occurring PRO179, PRO207, PRO320, PRO219, PRO221, PRO224, PRO328, PRO301, PRO526, PRO362, PRO356, PRO509 or PRO866 and an "immunological" activity refers to the ability to induce the production of an antibody against an antigenic epitope possessed by a native or naturally-occurring PRO179, PRO207, PRO320, PRO219, PRO221, PRO224, PRO328, PRO301, PRO526, PRO362, PRO356, PRO509 or PRO866.

"Biological activity" in the context ofan antibody or another agonist that can be identified by the screening assays disclosed herein (*e.g.*, an organic or inorganic small molecule, peptide, etc.) is used to refer to the ability of such molecules to invoke one or more of the effects listed herein in connection with the definition of a "therapeutically effective amount." In specific embodiment, "biological activity" is the ability to inhibit neoplastic cell growth or proliferation. A preferred biological activity is inhibition, including slowing or complete stopping, of the growth of a target tumor (*e.g.,* cancer) cell. Another preferred biological activity is cytotoxic activity resulting in the death of the target tumor (*e.g.*, cancer) cell. Yet another preferred biological activity is the induction of apoptosis of a target tumor (*e.g.,* cancer) cell.

The phrase "immunological activity" means immunological cross-reactivity with at least one epitope of a PRO179, PRO207, PRO320, PRO219, PRO221, PRO224, PRO328, PRO301, PRO526, PRO362, PRO356, PRO509 or PRO866 polypeptide.

"Immunological cross-reactivity" as used herein means that the candidate polypeptide is capable of competitively inhibiting the qualitative biological activity of a PRO179, PRO207, PRO320, PRO219, PRO221, PRO224, PRO328, PRO301, PRO526, PRO362, PRO356, PRO509 or PRO866 polypeptide having this activity with polyclonal antisera raised against the known active PRO179, PRO207, PRO320, PRO219, PRO221, PRO224, PRO328, PRO301, PRO526, PRO362, PRO356, PRO509 or PRO866 polypeptide. Such antisera are prepared in conventional fashion by injecting goats or rabbits, for example, subcutaneously with the known active analogue in complete Freund's adjuvant, followed by booster intraperitoneal orsubcutaneousinjectionin incomplete Freunds. The immunological cross-reactivity preferably is "specific", which means that the binding affinity of the immunologically cross-reactive molecule (*e.g.*, antibody) identified, to the corresponding PRO179, PRO207, PRO320, PRO219, PRO221, PRO224, PRO328, PRO301, PRO526, PRO362, PRO356, PRO509 or PRO866 polypeptide is significantly higher (preferably at least about 2-times, more preferably at least about 4-times, even more preferably at least about 6-times, most preferably at least about 8-times higher) than the binding affinity of that molecule to any other known native polypeptide.

"Tumor", as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include breast cancer, prostate cancer, colon cancer, squamous cell cancer, small-cell lung cancer, non-small cell lung cancer, ovarian cancer, cervical cancer, gastrointestinal cancer, pancreatic cancer, glioblastoma, liver cancer, bladder cancer, hepatoma, colorectal cancer, endometrial carcinoma, salivary gland carcinoma, kidney cancer, vulval cancer, thyroid cancer, hepatic carcinoma and various types of head and neck cancer.

"Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. In tumor (*e.g.*, cancer) treatment, a therapeutic agent may directly decrease the pathology of tumor cells, or render the tumor cells more susceptible to treatment by other therapeutic agents, *e.g.,* radiation and/or chemotherapy.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, etc.

An "effective amount" of a polypeptide disclosed herein or an agonist thereof, in reference to inhibition of neoplastic cell growth, is an amount capable of inhibiting, to some extent, the growth of target cells. The term includes an amount capable of invoking a growth inhibitory, cytostatic and/or cytotoxic effect and/or apoptosis of the target cells. An "effective amount" of a PRO179, PRO207, PRO320, PRO219, PRO221, PRO224, PRO328, PRO301, PRO526, PRO362, PRO356, PRO509 or PRO866 polypeptide or an agonist thereof for purposes of inhibiting neoplastic cell growth may be determined empirically and in a routine manner.

A "therapeutically effective amount", in reference to the treatment of tumor, refers to an amount capable of invoking one or more of the following effects: (1) inhibition, to some extent, of tumor growth, including, slowing down and complete growth arrest; (2) reduction in the number of tumor cells; (3) reduction in tumor size; (4) inhibition (*i.e.*, reduction, slowing down or complete stopping) oftumor cell infiltration into peripheral organs; (5) inhibition (*i.e.,* reduction, slowing down or complete stopping) of metastasis; (6) enhancement of anti-tumor immune response, which may, but does not have to, result in the regression or rejection of the tumor, and/or (7) relief, to some extent, of one or more symptoms associated with the disorder. A "therapeutically effective amount" of a PRO179, PRO207, PRO320, PRO219, PRO221, PRO224, PRO328, PRO301, PRO526, PRO362, PRO356, PRO509 or PRO866 polypeptide or an agonist thereof for purposes of treatment of tumor may be determined empirically and in a routine manner.

A "growth inhibitory amount" of PRO179, PRO207, PRO320, PRO219, PRO221, PRO224, PRO328, PRO301, PRO526, PRO362, PRO356, PRO509 or PRO866 polypeptide or an agonist thereof is an amount capable of inhibiting the growth of a cell, especially tumor, *e.g.*, cancer cell, either *in vitro* or *in vivo.* A "growth inhibitory amount" of a PRO179, PRO207, PRO320, PRO219, PRO221, PRO224, PRO328, PRO301, PRO526, PRO362, PRO356, PRO509 or PRO866 polypeptide or an agonist thereof for purposes of inhibiting neoplastic cell growth may be determined empirically and in a routine manner.

A "cytotoxic amount" of a PRO 179, PRO207, PRO320, PRO219, PRO221, PRO224, PRO328, PRO301, PRO526, PRO362, PRO356, PRO509 or PRO866 polypeptide or an agonist thereof is an amount capable of causing the destruction of a cell, especially tumor, *e.g.*, cancer cell, either *in vitro* or *in* vivo. A "cytotoxic amount" of a PRO179, PRO207, PRO320, PRO219, PRO221, PRO224, PRO328, PRO301, PRO526, PRO362, PRO356, PRO509 or PRO866 polypeptide or an agonist thereof for purposes of inhibiting neoplastic cell growth may be determined empirically and in a routine manner.

The term "cytotoxic agent" as used herein refers to a substance that inhibits or prevents the function of cells and/or causes destruction of cells. The term is intended to include radioactive isotopes (e.g., I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of tumor, *e.g.*, cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, *e.g.*, paclitaxel (Taxol, Bristol-Myers Squibb Oncology, Princeton,NJ), and doxetaxel (Taxotere, Rhone-Poulenc Rorer, Antony, Rnace),toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (*see,* U.S. Patent No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially tumor, *e.g.*, cancer cell, either *in vitro* or *in vivo.* Thus, the growth inhibitory agent is one which significantly reduces the percentage of the target cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest GI also spill over into S-phase arrest, for example, DNA alkylating agents such as tamoxifen, prednisone, dacarbazine, mechtorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami *et al.,* (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and-β; mullerian-inhibiting substance; mouse gonadotropinassociated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-1 and -11; erythropoietin (EPO); osteoinductive factors; interferons such as interferon-α, -β, and -γ; colony stimulating factors (CSFs) such as macrophage-CSF (M-CSF); granulocytemacrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "prodrug" as used in this application refers to a precursor or derivative form of a pharmaceutically active substance that is less cytotoxic to tumor cells compared to the parent drug and is capable of being enzymatically activated or converted into the more active parent form. *See, e.g.*, Wilman, "Prodrugs in Cancer Chemotherapy", Biochemical Society Transactions, 14, pp. 375-382, 615th Meeting Belfast (1986) and Stella *et al.,* "Prodrugs: A Chemical Approach to Targeted Drug Delivery," Directed Drug Delivery, Borchardt *et al.,* (ed.), pp. 247-267, Humana Press (1985). The prodrugs of this invention include, but are not limited to, phosphate-containing prodrugs, thiophosphate-containing prodrugs, glycosylated prodrugs or optionally substituted phenylacetamide-containing prodrugs, 5-fluorocytosine and other 5-fluorouridine prodrugs which can be derivatized into a prodrug form for use in this invention include, but are not limited to, those chemotherapeutic agents described above.

The term "agonist" is used in the broadest sense and includes any molecule that mimics a biological activity of a native PRO179, PRO207, PRO320, PRO219, PRO221, PRO224, PRO328, PRO301, PRO526, PRO362, PRO356. PRO509 or PRO866 polypeptide disclosed herein. Suitable agonist molecules specifically include agonist antibodies or antibody fragments, fragments or amino acid sequence variants of native PRO179, PRO207, PRO320, PRO219, PRO221, PRO224, PRO328, PRO301, PRO526, PRO362, PRO356, PRO509 or PRO866 polypeptides, peptides, small organic molecules, etc. Methods for identifying agonists of a PRO179, PRO207, PRO320, PRO219, PRO221, PRO224, PRO328, PRO301, PRO526, PRO362, PRO356, PRO509 or PRO866 polypeptide may comprise contacting a tumor cell with a candidate agonist molecule and measuring the inhibition of tumor cell growth.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™ , polyethylene glycol (PEG), and PLURONICS™ .

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light-chain variable domain is aligned with the variable domain ofthe heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework regions (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (*see*, Kabat *et al.,* NIH Publ. No.91-3242, Vol. I. pages 647-669 (1991)). The constant domains are not involved directly in binding an antibody to an antigen, but exhibit various effector functions, such as participation of the antibody in antibody-dependent cellular toxicity.

The term "hypervariable region" when used herein refers to the amino acid residues of an antibody which are responsible for antigen-binding. The hypervariable region comprises amino acid residues from a "complementarity determining region" or "CDR" (*i.e.*, residues 24-34 (L1), 50-56 (L2) and 89-97 (L3) in the light chain variable domain and 31-35 (H1), 50-65 (H2) and 95-102 (H3) in the heavy chain variable domain; Kabat *et al.,* Sequences of Proteins of Immunological Interest, 5th Ed. Public Health Service, National Institute of Health, Bethesda, MD. [1991]) and/orthose residues from a "hypervariable loop" (*i.e.*, residues 26-32 (L1), 50-52 (L2) and 91-96 (L3) in the light chain variable domain and 26-32 (H1), 53-55 (H2) and 96-101 (H3) in the heavy chain variable domain; Clothia and Lesk, J. Mol. Biol., 196:901-917 [1987]). "Framework" or "FR" residues are those variable domain residues other than the hypervariable region residues as herein defined.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (Zapata *et al.,* Protein Eng., 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')₂ fragment that has two antigen-combining sites and is still capable of cross-linking antigen.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain ofthe light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), *e.g.*, IgG 1, IgG2, IgG3, IgG4, IgA, and IgA2.

The term "monoclonal antibody" as used herein refers to an antibody obtained from a population of substantially homogeneous antibodies, *i. e.,* the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibodypreparationswhich typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler *et al.,* Nature, 256:495 [1975], or may be made by recombinant DNA methods (see, *e.g.,* U.S. Patent No. 4,816,567). The "monoclonal antibodies" may also be isolated from phage antibody libraries using the techniques described in Clackson *et al.,* Nature. 352:624-628 (1991] and Marks *et al.,* J. Mol. Biol.222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison *et al.,* Proc. Natl. Acad. Sci. USA, 81:6851-6855 [1984)).

"Humanized" forms of non-human *(e.g.,* murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a CDR of the recipient are replaced by residues from a CDR of a non-human species (donor antibody) such as mouse, rat or rabbit having the desired specificity, affinity, and capacity. In some instances, Fv FR residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, *see,* Jones *et al.,* Nature, 321:522-525 (1986); Reichmann *et al.,* Nature, 332:323-329 [1988]; and Presta, Curr. Op. Struct. Biol., 2:593-596 (1992). The humanized antibody includes a PRIMATIZED™antibody wherein the antigen-binding region ofthe antibody is derived from an antibody produced by immunizing macaque monkeys with the antigen of interest.

"Single-chain Fv" or "sFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the sFv to form the desired structure for antigen binding. For a review of sFv, *see,* Pluckthun in The Pharmacology of Monoclonal Antibodies, Vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger *et al.,* Proc. Natl. Acad. Sci. USA,90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in silu* within recombinant cells since at least one component of the antibody's natural environment will not be present. Ordinarily, however, isolated antibody will be prepared by at least one purification step.

The word "label" when used herein refers to a detectable compound or composition which is conjugated directly or indirectly to the antibody so as to generate a "labeled" antibody. The label may be detectable by itself (*e.g.*, radioisotope labels or fluorescent labels) or, in the case of an enzymatic label, may catalyze chemical alteration of a substrate compound or composition which is detectable. The label may also be a non-detectable entity such as a toxin.

By "solid phase" is meant a non-aqueous matrix to which the antibody of the present invention can adhere. Examples of solid phases encompassed herein include those formed partially or entirely of glass (*e.g.*, controlled pore glass), polysaccharides (*e.g.,* agarose), polyacrylamides, polystyrene, polyvinyl alcohol and silicones. In certain embodiments, depending on the context, the solid phase can comprise the well of an assay plate; in others it is a purification column (*e.g.*, an affinity chromatography column). This term also includes a discontinuous solid phase of discrete particles, such as those described in U.S. Patent No. 4,275,149.

A "liposome" is a small vesicle composed of various types oflipids, phospholipids and/or surfactant which is useful for delivery of a drug (such as a PRO179, PRO207, PRO320, PRO219, PRO221, PRO224, PRO328, PRO301, PRO526, PRO362, PRO356, PRO509 or PRO866 polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

### II. Compositions and Methods of the Invention

### A. Full-length PRO179, PRO207, PRO320, PRO219, PRO221, PRO224, PRO328, PRO301, PRO526, PRO362, PRO356, PRO509 and PRO866 Polypeptides

The present invention provides newly identified and isolated nucleotide sequences encoding polypeptides referred to in the present application as PRO179, PRO207, PRO320, PRO219, PRO221, PRO224, PRO328, PRO301, PRO526, PRO362, PRO356, PRO509 and PRO866. In particular, cDNAs encoding PRO179, PRO207, PRO320, PRO219, PRO221, PRO224, PRO328, PRO301, PRO526, PRO362, PRO356, PRO509 and PRO866 polypeptides have been identified and isolated, as disclosed in further detail in the Examples below.

As disclosed in the Examples below, cDNA clones encoding PRO179, PRO207, PRO320, PRO219, PRO221, PRO224, PRO328, PRO301, PRO526, PRO362, PRO356, and PRO866 polypeptides have been deposited with the ATCC [with the exception of clone PRO509 which was not deposited with ATCC]. The actual nucleotide sequences of the clones can readily be determined by the skilled artisan by sequencing of the deposited clones using routine methods in the art. The predicted amino acid sequences can be determined from the nucleotide sequences using routine skill. For the PRO179, PRO207, PRO320, PRO219, PRO221, PRO224, PRO328, PRO301, PRO526, PRO362, PRO356, PRO509 and PRO866 polypeptides and encoding nucleic acids described herein, Applicants have identified what is believed to be the reading frame best identifiable with the sequence information available at the time.

### B. PRO179, PRO207, PRO320, PRO219, PRO221, PRO224, PRO328, PRO301, PRO526, PRO362, PRO356, PRO509 and PRO866 Variants

In addition to the full-length native sequence PRO179, PRO207, PRO320, PRO219, PRO221, PRO224, PRO328, PRO301, PRO526, PRO362, PRO356, PRO509 and PRO866 polypeptides described herein, it is contemplated that PRO179, PRO207, PRO320, PRO219, PRO221, PRO224, PRO328, PRO301, PRO526, PRO362, PRO356, PRO509 and PRO866 variants can be prepared. PRO179, PRO207, PRO320, PRO219, PRO221, PRO224, PRO328, PRO301, PRO526, PRO362, PRO356, PRO509 and PRO866 variants can be prepared by introducing appropriate nucleotide changes into the PRO179, PRO207, PRO320, PRO219, PRO221, PRO224, PRO328, PRO301, PRO526, PRO362, PRO356, PRO509 or PRO866 DNA, and/or by synthesis of the desired PRO179, PRO207,PRO320, PRO219, PRO221, PRO224, PRO328, PRO301, PRO526, PRO362, PRO356, PRO509 or PRO866 polypeptide. Those skilled in the art will appreciate that amino acid changes may alter post-translational processes of the PRO179, PRO207, PRO320, PRO219, PRO221, PRO224, PRO328, PRO301, PRO526, PRO362, PRO356, PRO509 or PRO866 polypeptide, such as changing the number or position of glycosylation sites or altering the membrane anchoring characteristics.

Variations in the native full-length sequence PRO179, PRO207, PRO320, PRO219, PRO221, PRO224, PRO328, PRO301, PRO526, PRO362, PRO356, PRO509 or PRO866 or in various domains of the PRO179, PRO207, PRO320, PRO219, PRO221, PRO224, PRO328, PRO301, PRO526, PRO362, PRO356, PRO509 or PRO866 described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO179, PRO207, PRO320, PRO219, PRO221, PRO224, PRO328, PRO301, PRO526, PRO362, PRO356, PRO509 or PRO866 that results in a change in the amino acid sequence ofthe PRO179, PRO207, PRO320, PRO219, PRO221, PRO224, PRO328, PRO301, PRO526, PRO362, PRO356, PRO509 or PRO866 as compared with the native sequence PRO179, PRO207, PRO320, PRO219, PRO221, PRO224, PRO328, PRO301, PRO526, PRO362, PRO356, PRO509 or PRO866. Optionally the variation is by substitution of at least one amino acid with any other amino acid in one or more of the domains of the PRO179, PRO207, PRO320, PRO219, PRO221, PRO224, PRO328, PRO301, PRO526, PRO362, PRO356, PRO509 or PRO866. Guidance in determining which amino acid residue may be inserted, substituted or deleted without adversely affecting the desired activity may be found by comparing the sequence of the PRO179, PRO207, PRO320, PRO219, PRO221, PRO224, PRO328, PRO301, PRO526, PRO362, PRO356, PRO509 or PRO866 with that of homologous known protein molecules and minimizing the number of amino acid sequence changes made in regions of high homology. Amino acid substitutions can be the result of replacing one amino acid with another am ino acid having similar structural and/or chemical properties, such as the replacement of a leucine with a serine, *i.e.,* conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO179, PRO207, PRO320, PRO219, PRO221, PRO224, PRO328, PRO301, PRO526, PRO362, PRO356, PRO509 and PRO866 polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO179, PRO207, PRO320, PRO219, PRO221, PRO224, PRO328, PRO301, PRO526, PRO362, PRO356, PRO509 or PRO866 polypeptide.

PRO179, PRO207, PRO320, PRO219, PRO221, PRO224, PRO328, PRO301, PRO526, PRO362, PRO356, PRO509 and PRO866 fragments may be prepared by any of a number of conventional techniques. Desired peptide fragments may be chemically synthesized. An alternative approach involves generating PRO179, PRO207, PRO320, PRO219, PRO221, PRO224, PRO328, PRO301, PRO526, PRO362, PRO356, PRO509 and PRO866 fragments by enzymatic digestion, *e.g.*, by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO179, PRO207, PRO320, PRO219, PRO221, PRO224, PRO328, PRO301, PRO526, PRO362, PRO356, PRO509 and PRO866 polypeptide fragments share at least one biological and/or immunological activity with the native PRO179, PRO207. PRO320, PRO219, PRO221, PRO224, PRO328, PRO301, PRO526, PRO362, PRO3S6, PRO509 or PRO866 polypeptides shown in Figure 2 (SEQ ID NO:2), Figure 4 (SEQ ID NO:7), Figure 6 (SEQ ID NO:10), Figure 8 (SEQ ID NO:15), Figure 10 (SEQ ID NO:20), Figure 12 (SEQ ID NO:25), Figure 14 (SEQ ID NO:30), Figure 16 (SEQ ID NO:35), Figure 18 (SEQ ID NO:43), Figure 20 (SEQ ID NO:48), Figure 22 (SEQ ID NO:55), Figure 24 (SEQ ID NO:60), or Figure 26 (SEQ ID NO:62), respectively.

In particular embodiments, conservative substitutions of interest are shown in Table 3 under the heading of preferred substitutions. If such substitutions result in a change in biological activity, then more substantial changes, denominated exemplary substitutions in Table 3, or as further described below in reference to amino acid classes, are introduced and the products screened.

**Table 3**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his: lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr(T) | ser | ser |
| Trp(W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO179, PRO207, PRO320, PRO219, PRO221, PRO224, PRO328, PRO301, PRO526, PRO362, PRO356, PRO509 or PRO866 polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter *et al.,* Nucl. Acids Res.. 13:4331 (1986); Zoller *et al.,* Nucl. Acids Res.. 10:6487 (1987)], cassette mutagenesis [Wells *et al.,* Gene, 34:315 (1985)], restriction selection mutagenesis [Wells *et al.,* Philos. Trans. R. Soc. London SerA, 317:415 (1986)] or other known techniques can be performed on the cloned DNA to produce the PRO179, PRO207, PRO320, PRO219, PRO221, PRO224, PRO328, PRO301, PROS26, PRO362, PRO356, PRO509 or PRO866 variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### C. Modifications of PRO179, PRO207, PRO320, PRO219, PRO221, PRO224, PRO328, PRO301, PRO526. PRO362, PRO356, PRO509 and PRO866

Covalent modifications of PRO 179, PRO207, PRO320, PRO219, PRO221, PRO224, PRO328, PRO301, PRO526, PRO362, PRO356, PRO509 and PRO866 are included within the scope of this invention. One type of covalent modification includes reacting targeted amino acid residues of a PRO179, PRO207, PRO320, PRO219, PRO221, PRO224, PRO328, PRO301, PRO526, PRO362, PRO356, PRO509 or PRO866 polypeptide with an organic derivatizing agent that is capable of reacting with selected side chains or the N- or C- terminal residues of the PRO179, PRO207, PRO320, PRO219, PRO221, PRO224, PRO328, PRO301, PRO526, PRO362, PRO356, PRO509 or PRO866. Derivatization with bifunctional agents is useful, for instance, for crosslinking PRO179, PRO207, PRO320, PRO219, PRO221, PRO224, PRO328, PRO301, PRO526, PRO362, PRO356, PRO509 or PRO866 to a water-insoluble support matrix or surface for use in the method for purifying anti-PRO179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 or anti-PRO866 antibodies, and vice-versa. Commonly used crosslinking agents include, *e.g.*, 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation ofthe α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO179, PRO207, PRO320, PRO219, PRO221, PRO224, PRO328, PRO301, PRO526, PRO362, PRO356, PRO509 or PRO866 polypeptide included within the scope of this invention comprises altering the native glycosylation pattern of the polypeptide. "Altering the native glycosylation pattern" is intended for purposes herein to mean deleting one or more carbohydrate moieties found in native sequence PRO179, PRO207, PRO320, PRO219, PRO221, PRO224, PRO328, PRO301, PRO526, PRO362, PRO356, PRO509 or PRO866 (either by removing the underlying glycosylation site or by deleting the glycosylation by chemical and/or enzymatic means), and/or adding one or more glycosylation sites that are not present in the native sequence PRO179, PRO207, PRO320, PRO219, PRO221, PRO224, PRO328, PRO301, PRO526, PRO362, PRO356, PRO509 or PRO866. In addition, the phrase includes qualitative changes in the glycosylation of the native proteins, involving a change in the nature and proportions of the various carbohydrate moieties present.

Addition of glycosylation sites to the PRO179, PRO207, PRO320, PRO219, PRO221, PRO224, PRO328, PRO301, PRO526, PRO362, PRO356, PRO509 or PRO866 polypeptide may be accomplished by altering the amino acid sequence. The alteration may be made, for example, by the addition of, or substitution by, one or more serine or threonine residues to the native sequence PRO179, PRO207, PRO320, PRO219, PRO221, PRO224, PRO328, PRO301, PRO526, PRO362, PRO356, PRO509 or PRO866 (for O-linked glycosylation sites). The PRO179, PRO207, PRO320, PRO219, PRO221, PRO224, PRO328, PRO301, PRO526, PRO362, PRO356, PRO509 or PRO866 amino acid sequence may optionally be altered through changes at the DNA level, particularly by mutating the DNA encoding the PRO179, PRO207, PRO320, PRO219, PRO221, PRO224, PRO328, PRO301, PRO526, PRO362, PRO356, PRO509 or PRO866 polypeptide at preselected bases such that codons are generated that will translate into the desired amino acids.

Another means of increasing the number of carbohydrate moieties on the PRO 179, PRO207, PRO320, PRO219, PRO221, PRO224, PRO328, PRO301, PRO526, PRO362, PRO356, PRO509 or PRO866 polypeptide is by chemical or enzymatic coupling of glycosides to the polypeptide. Such methods are described in the art, *e.g.*, in WO 87/05330 published 11 September 1987, and in Aplin and Wriston, CRC Crit. Rev. Biochem., pp. 259-306 (1981).

Removal of carbohydrate moieties present on the PRO179, PRO207, PRO320, PRO219, PRO221, PRO224, PRO328, PRO301, PRO526, PRO362, PRO356, PRO509 or PRO866 polypeptide may be accomplished chemically or enzymatically or by mutational substitution of codons encoding for amino acid residues that serve as targets for glycosylation. Chemical deglycosylation techniques are known in the art and described, for instance, by Hakimuddin, *et al.,* Arch. Biochem. Biophys., 259:52 (1987) and by Edge *et al.,* Anal. Biochem., 118: 131 (1981). Enzymatic cleavage of carbohydrate moieties on polypeptides can be achieved by the use of a variety of endo- and exo-glycosidases as described by Thotakura *et al.,* Meth. Enzymol., 138:350 (1987).

Another type of covalent modification of PRO179, PRO207, PRO320, PRO219, PRO221, PRO224, PRO328, PRO301, PRO526, PRO362, PRO356, PRO509 or PRO866 comprises linking the PRO179, PRO207, PRO320, PRO219, PRO221, PRO224, PRO328, PRO301, PRO526, PRO362, PRO356, PRO509 or PRO866 polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO179, PRO207, PRO320, PRO219, PRO221, PRO224, PRO328, PRO301, PRO526, PRO362, PRO356, PRO509 or PRO866 polypeptide of the present invention may also be modified in a way to form a chimeric molecule comprising PRO179, PRO207, PRO320, PRO219, PRO221, PRO224, PRO328, PRO301, PRO526, PRO362, PRO356, PRO509 or PRO866 fused to another, heterologous polypeptide or amino acid sequence.

In one embodiment, such a chimeric molecule comprises a fusion of the PRO179, PRO207, PRO320, PRO219, PRO221, PRO224, PRO328, PRO301, PRO526, PRO362, PRO356, PRO509 or PRO866 polypeptide with a tag polypeptide which provides an epitope to which an anti-tag antibody can selectively bind. The epitope tag is generally placed at the amino- or carboxyl- terminus of the PRO179, PRO207, PRO320, PRO219, PRO221, PRO224, PRO328, PRO301, PRO526, PRO362, PRO356, PRO509 or PRO866 polypeptide. The presence of such epitope-tagged forms of the PRO179, PRO207, PRO320, PRO219, PRO221, PRO224, PRO328, PRO301, PRO526, PRO362, PRO356, PRO509 or PRO866 polypeptide can be detected using an antibody against the tag polypeptide. Also, provision of the epitope tag enables the PRO179, PRO207, PRO320, PRO219, PRO221, PRO224, PRO328, PRO301, PRO526, PRO362, PRO356, PRO509 or PRO866 polypeptide to be readily purified by affinity purification using an anti-tag antibody or another type of affmity matrix that binds to the epitope tag. Various tag polypeptides and their respective antibodies are well known in the art. Examples include poly-histidine (poly-His) or poly-histidine-glycine (poly-His-gly) tags; the flu HA tag polypeptide and its antibody 12CA5 [Field *et al.,* Mol. Cell. Biol., 8:2159-2165 (1988)]; the c-myc tag and the 8F9, 3C7, 6E10, G4, B7 and 9E10 antibodies thereto [Evan *et al.,* Molecular and Cellular Biology, 5:3610-3616 (1985)]; and the Herpes Simplex virus glycoprotein D (gD) tag and its antibody [Paborsky *et al.,* Protein Engineering, 3(6):547-553 (1990)]. Other tag polypeptides include the Flag-peptide [Hopp *et al.,* BioTechnology, 6:1204-1210 (1988)]; the KT3 epitope peptide [Martin *et al.,* Science, 255:192-194 (1992)]; an α-tubulin epitope peptide [Skinner *et al.,* J. Biol. Chem., 266:15163-15166 (1991)]; and the T7 gene 10 protein peptide tag [Lutz-Freyermuth *et al.,* Proc. Natl. Acad. Sci. USA, 87:6393-6397 (1990)].

In an alternative embodiment, the chimeric molecule may comprise a fusion of the PRO179, PRO207, PRO320, PRO219, PRO221, PRO224, PRO328, PRO301, PRO526, PRO362, PRO356, PRO509 or PRO866 polypeptide with an immunoglobulin or a particular region of an immunoglobulin. For a bivalent form of the chimeric molecule (also referred to as an "immunoadhesin"), such a fusion could be to the Fc region of an IgG molecule. The lg fusions preferably include the substitution of a soluble (transmembrane domain deleted or inactivated) form of a PRO 179, PRO207, PRO320, PRO219, PRO221, PRO224, PRO328, PRO301, PRO526, PRO362, PRO356, PRO509 or PRO866 polypeptide in place of at least one variable region within an Ig molecule. In a particularly preferred embodiment, the immunoglobulin fusion includes the hinge, CH2 and CH3, or the hinge, CH1, CH2 and CH3 regions of an IgG 1 molecule. For the production of immunoglobulin fusions *see* also, US Patent No. 5,428,130 issued June 27, 1995.

### D. Preparation of PRO 179, PRO207, PRO320, PRO219, PRO221, PRO224, PRO128, PRO301, PRO526, PRO362, PRO356, PRO509 and PRO866

The description below relates primarily to production of PRO179, PRO207, PRO320, PRO219, PRO221, PRO224, PRO328, PRO301, PRO526, PRO362, PRO356, PRO509 or PRO866 by culturing cells transformed or transfected with a vector containing PRO179, PRO207, PRO320, PRO219, PRO221, PRO224, PRO328, PRO301, PRO526, PRO362, PRO356, PRO509 or PRO866 nucleic acid. It is, of course, contemplated that alternative methods, which are well known in the art, may be employed to prepare PRO179, PRO207, PRO320, PRO219, PRO221, PRO224, PRO328, PRO301, PRO526, PRO362, PRO356, PRO509 or PRO866. For instance, the PRO179, PRO207, PRO320, PRO219, PRO221, PRO224, PRO328, PRO301, PRO526, PRO362, PRO356, PRO509 or PRO866 polypeptide sequence, or portions thereof, may be produced by direct peptide synthesis using solid-phase techniques [see, *e.g*., Stewart *et al.,* Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO179, PRO207, PRO320, PRO219, PRO221, PRO224, PRO328, PRO301, PRO526, PRO362, PRO356, PRO509 or PRO866 polypeptide may be chemically synthesized separately and combined using chemical or enzymatic methods to produce the full-length PRO179, PRO207, PRO320, PRO219, PRO221, PRO224, PRO328, PRO301, PRO526, PRO362, PRO356, PRO509 or PRO866 polypeptide.

### 1. Isolation of DNA Encoding PRO179, PRO207, PRO320, PRO219, PRO221, PRO224, PRO328, PRO301, PRO526, PRO362, PRO356, PRO509 or PRO866

DNA encoding PRO179, PRO207, PRO320, PRO219, PRO221, PRO224, PRO328, PRO301, PRO526, PRO362, PRO356, PRO509 or PRO866 may be obtained from a cDNA library prepared from tissue believed to possess the PRO179, PRO207, PRO320, PRO219, PRO221, PRO224, PRO328, PRO301, PRO526, PRO362, PRO356, PRO509 or PRO866 mRNA and to express it at a detectable level. Accordingly, human PRO179x, PRO207, PRO320, PRO219, PRO221, PRO224, PRO328, PRO301, PRO526, PRO362, PRO356, PRO509 or PRO866 DNA can be conveniently obtained from a cDNA library prepared from human tissue, such as described in the Examples. The PRO179-, PRO207-, PRO320-, PRO219-, PRO221-, PRO224-, PRO328-, PRO301-, PRO526-, PRO362-, PRO356-, PRO509- or PRO866-encoding gene may also be obtained from a genomic library or by known synthetic procedures (*e.g.*, automated nucleic acid synthesis).

Libraries can be screened with probes (such as antibodies to the PRO179, PRO207, PRO320, PRO219, PRO221, PRO224, PRO328, PRO301, PRO526, PRO362, PRO356, PRO509 or PRO866 or oligonucleotides of at least about 20-80 bases) designed to identify the gene of interest or the protein encoded by it. Screening the cDNA or genomic library with the selected probe may be conducted using standard procedures, such as described in Sambrook *et al*., Molecular Cloning: A Laboratory Manual (New York: Cold Spring Harbor Laboratory Press, 1989). An alternative means to isolate the gene encoding PRO179, PRO207, PRO320, PRO219, PRO221, PRO224, PRO328, PRO301, PRO526, PRO362, PRO356, PRO509 or PRO866 is to use PCR methodology [Sambrook *et al., supra;* Dieffenbach *et al.,* PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook *et al., supra.*

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook *et al., supra,* to detect precursors and processing intermediates of mRNA that may not have been reverse-transcribed into cDNA.

### 2. Selection and Transformation of Host Cells

Host cells are transfected or transformed with expression or cloning vectors described herein for PRO 179, PRO207, PRO320, PRO219, PRO221, PRO224, PRO328, PRO301, PRO526, PRO362, PRO356, PRO509 or PRO866 production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (lRL Press, 1991) and Sambrook *et al., supra.*

Methods of eukaryotic cell transfection and prokaryotic cell transformation are known to the ordinarily skilled artisan, for example, CaCl₂, CaPO₄, liposome-mediated and electroporation. Depending on the host cell used, transformation is performed using standard techniques appropriate to such cells. The calcium treatment employing calcium chloride, as described in Sambrook *et al., supra,* or electroporation is generally used for prokaryotes. Infection with *Agrobacterium tumefaciens* is used for transformation of certain plant cells, as described by Shaw *et al.,* Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen *et al.,* J. Bact.*,* 130:946 (1977) and Hsiao *et al.,* Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyomithine, may also be used. For various techniques for transforming mammalian cells, *see,* Keown *et al.,* Methods in Enzymology, 185:527-537(1990) and Mansour *et al.,* Nature, 336:348-352 (1998).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli X* 1776 (ATCC 31,537); *E. coli* strain W3110 (ATCC 27,325) and K5 772 (ATCC 53,635). Other suitable prokaryotic host cells include Enterobacteriaceae such as *Escherichia, e.g., E. coli, Enterobacter, Erwinia, Klebsiella, Proteus, Salmonella, e.g., Salmonella typhimuritim, Serratia, e.g., Serratia marcescans,* and *Shigella,* as well as *Bacilli* such as *B. subtilis* and *B. licheniformis (e.g., B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E*. *coli* W3110 strain IA2, which has the complete genotype *tonA* ; *E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT kan'; E. coli* W3110 strain 37D6, which has the complete genotype *tonA ptr3 phoA E15 (argF-fac)169 degP ompT rbs7 itvG kan'; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, *e.g.,* PCR or other nucleic acid polymerase reactions, are suitable.

In addition to prokaryotes, eukaryotic microbes such as filamentous fungi or yeast are suitable cloning or expression hosts for PRO179-, PRO207-, PRO320-, PRO219-, PRO221-, PRO224-, PRO328-, PRO301-, PRO526-, PRO362-, PRO356-, PRO509- or PRO866-encoding vectors. *Saccharomyces cerevisiae* is a commonly used lower eukaryotic host microorganism. Others include *Schizosaccharomycespombe* (Beach and Nurse, Nature, 290: 140 [1981]; EP 139,383 published 2 May 1985); *Kluyveromyces* hosts (U.S. Patent No. 4,943,529; Fleer *et al.,* Bio/Technology, 9:968-975 (1991)) such as, *e.g., K. lactis* (MW98-8C, CBS683, CBS4574; Louvencourt *et al.,* J. Bacteriol., 737 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg *et al.,* Bio/Technology, 8:135 ( 1990)), *K. thermotolerans,* and *K. matxianus; yarrowia* (EP 402,226); *Pichia pastoris* (EP 183,070; Sreekrishna *et al.,* J. Basic Microbiol., 28:265-278 [1988]); *Candida; Trichoderma reesia* (EP 244,234); *Neurospora crassa* (Case *et al.,* Proc. Natl. Acad. Sci. USA, 76:5259-5263 [1979]); *Schwanniomyces* such as *Schwanniomyces occidentalis* (EP394,538published 31 October 1990); and filamentous fungi such as, *e.g., Neurospora, Penicillium, Tolypocladium* (WO 91/00357 published 10 January 1991), and *Aspergillus* hosts such as *A. nidulans* (Ballance *et al.,* Biochem. Biophys. Res. Commun., 112:294-289 [1983]; Tilburn *et al.,* Gene, 26:205-221 [1983); Yelton *et al.,* Proc. Natl. Acad. Sci. USA, 81: 1470-1474 [ 1984]) and *A. niger* (Kelly and Hynes, EMBO J., 4:475-479 [1985)). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PRO179, PRO207, PRO320, PRO219, PRO221, PRO224, PRO328, PRO301, PRO526, PRO362, PRO356, PRO509 or PRO866 are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham *et al.,* J. Gen. Virol., 36:59(1977)); Chinese hamster ovary cells/-DHFR(CHO,Urlaub and Chasin, Proc.Natl.Acad.Sci.USA, 77:4216 (1980)); mouse sertoli cells (TM4, Mather, Biol. Reprod.,23:243-251 (1980)); human lung cells (W138, ATCC CCL 75); human liver cells (Hep G2, HB 8065); and mouse mammary tumor(MMT 060562, ATCC CCL51). The selection of the appropriate host cell is deemed to be within the skill in the art.

### 3. Selection and Use of a Replicable Vector

The nucleic acid (*e.g.,* cDNA or genomic DNA) encoding PRO179, PRO207, PRO320, PRO219, PRO221, PRO224, PRO328, PRO301, PRO526, PRO362, PRO356, PRO509 or PRO866 may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO179, PRO207, PRO320, PRO219, PRO221, PRO224, PRO328, PRO301, PRO526, PRO362, PRO356, PRO509 or PRO866 may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO179-, PRO207-, PRO320-, PRO219-, PRO221-, PRO224-, PRO328-, PRO301-, PRO526-, PRO362-, PRO356-, PRO509- or PRO866-encoding DNA that is inserted into the vector. The signal sequence may be a prokaryotic signal sequence selected, for example, from the group of the alkaline phosphatase, penicillinase, Ipp, or heat-stable enterotoxin II leaders. For yeast secretion the signal sequence may be, *e.g.*, the yeast invertase leader, alpha factor leader (including *Saccharomyces* and *Kluyveromyces* α-factor leaders, the latter described in U.S. Patent No. 5,010,182), or acid phosphatase leader, the *C*. *albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, *e.g.*, ampicillin, neomycin, methotrexate, or tetracycline, (b) complement auxotrophic deficiencies, or (c) supply critical nutrients not available from complex media, *e.g.*, the gene encoding D-alanine racemase for *Bacilli.*

An example of suitable selectable markers for mammalian cells are those that enable the identification of cells competent to take up the PRO 179-, PRO207-, PRO320-, PRO219-, PRO221 -, PRO224-, PRO328-, PRO301 -, PRO526-, PRO362-, PRO356-, PRO509- or PRO866-encoding nucleic acid, such as DHFR or thymidine kinase. An appropriate host cell when wild-type DHFR is employed is the CHO cell line deficient in DHFR activity, prepared and propagated as described by Urlaub *et al.,* Proc. Natl. Acad. Sci. USA, 77:4216 (1980). A suitable selection gene for use in yeast is the *trp*1 gene present in the yeast plasmid YRp7 [Stinchcomb *et al*., Nature, 282:39 (1979); Kingsman *et al.,* Gene, 7:141 (1979); Tschemper *et al.,* Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO179-, PRO207-, PRO320-, PRO219-, PRO221-, PRO224-, PRO328-, PRO301-, PRO526-, PRO362-, PRO356-, PRO509- or PRO866-encoding nucleic acid sequence to direct mRNA synthesis. Promoters recognized by a variety of potential host cells are well known. Promoters suitable for use with prokaryotic hosts include the β-tactamase and lactose promoter systems [Chang *et al.,* Nature, 275:615 (1978); Goeddel *et al.,* Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer *et al.,* Proc. Natl. Acad. Sci. USA, 80:21-25 (1983)]. Promoters for use in bacterial systems also will contain a Shine-Dalgarno (S.D.) sequence operably linked to the DNA encoding PRO179, PRO207, PRO320, PRO219, PRO221, PRO224, PRO328, PRO301, PRO526, PRO362, PRO356, PRO509 or PRO866,

Examples of suitable promoting sequences for use with yeast hosts include the promoters for 3-phosphoglycerate kinase [Hitzeman *et al.,* J. Biol. Chem., 255:2073 (1980)] or other glycolytic enzymes [Hess *et al.*, J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO179, PRO207, PRO320, PRO219, PRO221, PRO224, PRO328, PRO301, PRO526, PRO362, PRO356, PRO509 or PRO866 transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, *e.g.*, the actin promoter or an immunoglobulin promoter, and from heat-shock promoters, provided such promoters are compatible with the host cell systems.

Transcription of a DNA encoding the PRO 179, PRO207, PRO320, PRO219, PRO221, PRO224, PRO328, PRO301, PRO526, PRO362, PRO356, PRO509 or PRO866 by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO179, PRO207, PRO320, PRO219, PRO221, PRO224, PRO328, PRO301, PRO526, PRO362, PRO356, PRO509 or PRO866 coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regionsof eukaryotic or viral DNAs or cDNAs. These regions contain nucleotide segments transcribed as polyadenylated fragments in the untranslated portion of the mRNA encoding PRO179, PRO207, PRO320, PRO219, PRO221, PRO224, PRO328, PRO301, PRO526, PRO362, PRO356, PRO509 or PRO866.

Still other methods, vectors, and host cells suitable for adaptation to the synthesis of PRO179, PRO207, PRO320, PRO219, PRO221, PRO224, PRO328, PRO301, PRO526, PRO362, PRO356, PRO509 or PRO866 in recombinant vertebrate cell culture are described in Gething *et al.,* Nature, 293 :620-625 (1981); Mantei *et al.,* Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO179, PRO207, PRO320, PRO219, PRO221, PRO224, PRO328, PRO301, PRO526, PRO362, PRO356, PRO509 or PRO866 polypeptide or against a synthetic peptide based on the DNA sequences provided herein or against exogenous sequence fused to PRO179, PRO207, PRO320, PRO219, PRO221, PRO224, PRO328, PRO301, PRO526, PRO362, PRO356, PRO509 or PRO866 DNA and encoding a specific antibody epitope.

### 5. Purification of Polypeptide

Forms of PRO179, PRO207, PRO320, PRO219, PRO221, PRO224, PRO328, PRO301, PRO526, PRO362, PRO356, PRO509 or PRO866 may be recovered from culture medium or from host cell lysates. If membrane-bound, it can be released from the membrane using a suitable detergent solution (e.g., Triton-X 100) or by enzymatic cleavage. Cells employed in expression of PRO179, PRO207, PRO320, PRO219, PRO221, PRO224, PRO328, PRO301, PRO526, PRO362, PRO356, PRO509 or PRO866 can be disrupted by various physical or chemical means, such as freeze-thaw cycling, sonication, mechanical disruption, or cell lysing agents.

It may be desired to purify PRO179, PRO207, PRO320, PRO219, PRO221, PRO224, PRO328, PRO301, PRO526, PRO362, PRO356, PRO509 or PRO866 from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO179, PRO207, PRO320, PRO219, PRO221, PRO224, PRO328, PRO301, PRO526, PRO362, PRO356, PRO509 or PRO866. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO179, PRO207, PRO320, PRO219, PRO221, PRO224, PRO328, PRO301, PRO526, PRO362, PRO356, PRO509 or PRO866 produced.

### E. Antibodies

Some drug candidates for use in the compositions and methods of the present invention are antibodies and antibody fragments which mimic the biological activity of a PRO179, PRO207, PRO320, PRO219, PRO221, PRO224, PRO328, PRO301, PRO526, PRO362, PRO356, PRO509 or PRO866 polypeptide.

### 1. Polyclonal Antibodies

Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO179, PRO207, PRO320, PRO219, PRO221, PRO224, PRO328, PRO301, PRO526, PRO362, PRO356, PRO509 or PRO866 polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 2. Monoclonal Antibodies

The antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO179, PRO207, PRO320, PRO219, PRO221, PRO224, PRO328, PRO301, PRO526, PRO362, PRO356, PRO509 or PRO866 polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, arninopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-humanheteromyetoma cell lines also have been described for the production of human monoclonal antibodies [Kozbor, J. Immunol., 133:3001 (1984); Brodeur *et al.,* Monoclonal Antibody Production Techniques and Applications, Marcel Dekker, Inc., New York, (1987) pp. 51-63].

The culture medium in which the hybridoma cells arc cultured can then be assayed for the presence of monoclonal antibodies directed against PRO179, PRO207, PRO320, PRO219, PRO221, PRO224, PRO328, PRO301, PRO526, PRO362, PRO356, PRO509 or PRO866, Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, *supra*]. Suitable culture media for this purpose include, for example, Dulbecco's Modified Eagle's Medium and RPMI- 1640 medium. Alternatively, the hybridoma cells may be grown *in vivo* as ascites in a mammal.

The monoclonal antibodies secreted by the subclones may be isolated or purified from the culture medium orascites fluid by conventional immunoglobulin purificationproceduressuch as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (*eg.*, by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison *et al., supra*]or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

### 3. Human and Humanized Antibodies

The antibodies of the invention may further comprise humanized antibodies or human antibodies. Humanized forms of non-human (*e.g.*, murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all ofat least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones *et al.,* Nature, 321:522-525 (1986); Riechmann *et al.,* Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones *et al.,* Nature, 321:522-525 (1986); Riechmann *et al.,* Nature, 332:323-327 (1988); Verhoeyen *et al.,* Science. 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol. 227:381 (1991); Marks *et al.,* J. Mol. Biol., 222:581 (1991)]. The techniques of Cole *et al.,* and Boemer *et al.,* are also available for the preparation of human monoclonal antibodies (Cole *et al.,* Monoclonal Antibodies and Cancer Therapy, Alan R. Liss, p. 77 (1985) and Boerner *et al.,* J. Immunol., 147(1):86-95 (1991)]. Similarly, human antibodies can be made by the introducing of human immunoglobulin loci into transgenic animals, *e.g.*, mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5.545.806; 5,569,825; 5,625,126; 5,633,425; 5,661,016, and in the following scientific publications: Marks *et al.*, Bio/Technology, 10: 779-783 (1992); Lonberg *et al.,* Nature, 368: 856-859 (1994); Morrison, Nature, 368: 812-13 (1994); Fishwild *et al.,* Nature Biotechnology, 14:845-51 (1996); Neuberger, Nature Biotechnology, 14: 826 (1996); Lonberg and Huszar, Intern. Rev. Immunol., 13 :65-93 (1995).

### 4. Bispecific Antibodies

Bispecific antibodies are monoclonal, preferably human or humanized, antibodies that have binding specificities for at least two different antigens. In the present case, one of the binding specificities is for the PRO179, PRO207, PRO320, PRO219, PRO221, PRO224, PRO328, PRO301, PRO526, PRO362, PRO356, PRO509 or PRO866, the other one is for any other antigen, and preferably for a cell-surface protein or receptor or receptor subunit.

Methods for making bispecific antibodies are known in the art. Traditional ly, the recombinant production ofbispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker *et al.,* EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh *et al.,* Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (*e.g.*, tyrosine or tryptophan). Compensatory "cavities" of identical or similar size to the large side chain(s) are created on the interface of the second antibody molecule by replacing large amino acid side chains with smaller ones (*e.g.*, alanine or threonine). This provides a mechanism for increasing the yield of the heterodimer over other unwanted end-products such as homodimers.

Bispecific antibodies can be prepared as full length antibodies or antibody fragments *(e.g.,* F(ab')₂ bispecific antibodies). Techniques for generating bispecific antibodies from antibody fragments have been described in the literature. For example, bispecific antibodies can be prepared using chemical linkage. Brennan *et al.,* Science. 229:81 (1985) describe a procedure wherein intact antibodies are proteolytical ly cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby *et al.,* J. Exp. Med., 175:217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny *et al.,* J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger *et al.,* Proc. Natl. Acad. Sci. USA. 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. *See,* Gruber *et al.,* J. Immunol., 152:5368 (1994).

Antibodies with more than two valencies are contemplated. For example, trispecific antibodies can be prepared. Tutt *et al.,* J. Immunol., 147:60 (1991).

Exemplary bispecific antibodies may bind to two different epitopes on a given PRO179, PRO207, PRO320, PRO219, PRO221, PRO224, PRO328, PRO301, PRO526, PRO362, PRO356, PRO509 or PRO866 polypeptide herein. Alternatively, an anti-PRO179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 or anti-PRO866 polypeptide arm may be combined with an arm which binds to a triggering molecule on a leukocyte such as a T-cell receptor molecule (*e.g.*, CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular PRO179, PRO207, PRO320, PRO219, PRO221, PRO224, PRO328, PRO301, PRO526, PRO362, PRO356, PRO509 or PRO866 polypeptide. Bispecific antibodies may also be used to localize cytotoxic agents to cells which express a particular PRO179, PRO207, PRO320, PRO219, PRO221, PRO224, PRO328, PRO301, PRO526, PRO362, PRO356, PRO509 or PRO866 polypeptide. These antibodies possess a PRO179-, PRO207-, PRO320-, PRO219-, PRO221-, PRO224-, PRO328-,PRO301-, PRO526-, PRO362-, PRO356-, PRO509- or PRO866-binding arm and an arm which binds a cytotoxic agent or a radionuclide chelator, such as EOTUBE, DPTA, DOTA, or TETA. Another bispecific antibody of interest binds the PRO 179, PRO207, PRO320, PRO219, PRO221, PRO224, PRO328, PRO301, PRO526, PRO362, PRO356, PRO509 or PRO866 polypeptide and further binds tissue factor (TF).

### 5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 6. Effector Function Engineering

It may be desirable to modify the antibody of the invention with respect to effector function, so as to enhance, *eg.*, the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). *See,* Caron *et al.,* J. Exp. Med., 176: 1191-1 195 (1992) and Shopes, J. Immunol., 148: 2918-2922 (1992). Homodimeric antibodies with enhanced anti-tumor activity may also be prepared using heterobifunctional cross-linkers as described in Wolff *et al.,* Cancer Research, 53: 2560-2565 (1993). Alternatively, an antibody can be engineered that has dual Fc regions and may thereby have enhanced complement lysis and ADCC capabilities. *See,* Stevenson *et al.,* Anti-Cancer Drug Design, 3:219-230 (1989).

### 7. Immunoconjugates

The invention also pertains to immunoconjugates comprising an antibody conjugated to a cytotoxic agent such as a chemotherapeutic agent, toxin (*eg.*, an enzymatically active toxin of bacterial, fungal, plant, or animal origin, or fragments thereof), or a radioactive isotope (*i.e.,* a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa),* ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyidithiol)propionate (SPDP), iminothiolane (IT), bifunctional derivatives ofimidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta *et al*., Science, 238: 1098(1987). Carbon-14-labeled 1-isothiocyanatobenzyl-3-methyldiethylene triaminepentaacetic acid (MX-DTPA) is an exemplary chelating agent for conjugation of radionucleotide to the antibody. *See,* WO94/11026.

In another embodiment, the antibody may be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (*e.g.*, avidin) that is conjugated to a cytotoxic agent (*e.g.,* a radionucleotide).

### 8. Immunoliposomes

The antibodies disclosed herein may also be formulated as immunoliposomes. Liposomes containing the antibody are prepared by methods known in the art, such as described in Epstein *et al.,* Proc. Natl. Acad. Sci. USA, 82: 3688 (1985); Hwang *et al.,* Proc. Natl. Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin *et al.,* J. Biol. Chem., 257: 286-288 (1982) via a disulfide-interchange reaction. A chemotherapeutic agent (such as Doxorubicin) is optionally contained within the liposome. *See*, Gabizon *et al.,* J. National Cancer Inst., 81(19): 1484 (1989).

### F. Identification of Proteins Capable of Inhibitine Neoplastic Cell Growth or Proliferation

The proteins disclosed in the present application have been assayed in a panel of 60 tumor cell lines currently used in the investigational, disease-oriented, *in vitro* drug-discovery screen of the National Cancer Institute (NCI). The purpose of this screen is to identify molecules that have cytotoxic and/or cytostatic activity against different types of tumors. NCI screens more than 10,000 new molecules per year (Monks *et al.,* J. Natl. Cancer Inst., 83:757-766 (1991); Boyd, Cancer: Princ. Pract. Oncol. Update, 3(10): 1-12 ([1989)]. The tumor cell lines employed in this study have been described in Monks *et al., supra.* The cell lines the growth of which has been significantly inhibited by the proteins of the present application are specified in the Examples.

The results have shown that the proteins tested show cytostatic and, in some instances and concentrations, cytotoxic activities in a variety of cancer cell lines, and therefore are useful candidates for tumor therapy.

Other cell-based assays and animal models for tumors (*e.g.*, cancers) can also be used to verify the findings of the NCl cancer screen, and to further understand the relationship between the protein identified herein and the development and pathogenesis of neoplastic cell growth. For example, primary cultures derived from tumors in transgenic animals (as described below) can be used in the cell-based assays herein, although stable cell lines are preferred. Techniques to derive continuous cell lines from transgenic animals are well known in the art *(see, e.g.,* Small *et al.,* Mol. Cell. Biol., 5:642-648 [1985]).

### G. Animal Models

A variety of well known animal models can be used to further understand the role of the molecules identified herein in the development and pathogenesis of tumors, and to test the efficacy of candidate therapeutic agents, including antibodies, and other agonists of the native polypeptides, including small molecule agonists. The *in vivo* nature of such models makes them particularly predictive of responses in human patients. Animal models of tumors and cancers (*e.g.*, breast cancer, colon cancer, prostate cancer, lung cancer, etc.) include both non-recombinant and recombinant (transgenic) animals. Non-recombinant animal models include, for example, rodent, *e.g.*, murine models. Such models can be generated by introducing tumor cells into syngeneic mice using standard techniques, e.g., subcutaneous injection, tail vein injection, spleen implantation, intraperitoneal implantation, implantation under the renal capsule, or orthopin implantation, *e.g.*, colon cancer cells implanted in colonic tissue. (*See*, *e.g.*, PCT publication No. WO 97/33551, published September 18, 1997).

Probably the most often used animal species in oncological studies are immunodeficient mice and, in particular, nude mice. The observation that the nude mouse with hypo/aplasia could successfully act as a host for human tumor xenografts has lead to its widespread use for this purpose. The autosomal recessive *nu* gene has been introduced into a very large number of distinct congenic strains of nude mouse, including, for example, ASW, A/He, AKR, BALB/c, B10.LP, C 17, C3H, C57BL, C57, CBA, DBA, ODD, I/st, NC, NFR, NFS, NFS/N, NZB, NZC, NZW, P, RIII and SJL. In addition, a wide variety of other animals with inherited immunological defects other than the nude mouse have been bred and used as recipients of tumor xenografts. For further details *see, e.g.,* The Nude Mouse in Oncology Research, E. Boven and B. Winograd, eds., CRC Press, Inc., 1991.

The cells introduced into such animals can be derived from known tumor/cancer cell lines, such as, any of the above-listed tumor cell lines, and, for example, the B104-1-1 cell line (stable NIH-3T3 cell line transfected with the *neu* protooncogene); *ras*-transfected NIH-3T3 cells; Caco-2 (ATCC HTB-37); a moderately well-differentiated grade II human colon adenocarcinoma cell line, HT-29 (ATCC HTB-38), or from tumors and cancers. Samples of tumor or cancer cells can be obtained from patients undergoing surgery, using standard conditions, involving freezing and storing in liquid nitrogen (Karmali *et al.,* Br. J. Cancer, 48:689-696 [1983]).

Tumor cells can be introduced into animals, such as nude mice, by a variety of procedures. The subcutaneous (s.c.) space in mice is very suitable for tumor implantation. Tumors can be transplanted s.c. as solid blocks, as needle biopsies by use of a trochar, or as cell suspensions. For solid block or trochar implantation, tumor tissue fragments of suitable size are introduced into the s.c. space. Cell suspensions are freshly prepared from primary tumors or stable tumor cell lines, and injected subcutaneously. Tumor cells can also be injected as subdermal implants. In this location, the inoculum is deposited between the lower part of the dermal connective tissue and the s.c. tissue. Boven and Winograd (1991), *supra.* Animal models of breast cancer can be generated, for example, by implanting rat neuroblastoma cells (from which the *neu* oncogen was initially isolated), or *neu*-transformed NIH-3T3 cells into nude mice, essentially as described by Drebin *et al.,* Proc. Natl. Acad. Sci. USA, 83:9129-9133 (1986).

Similarly, animal models of colon cancer can be generated by passaging colon cancer cells in animals, *e.g.*, nude mice, leading to the appearance of tumors in these animals. An orthotopic transplant model of human colon cancer in nude mice has been described, for example, by Wang *et al.,* Cancer Research, 54:4726-4728 (1994) and Too *et al*., Cancer Research, 55:681-684 (1995). This model is based on the so-called "METAMOUSE" sold by AntiCancer, Inc., (San Diego, California).

Tumors that arise in animals can be removed and cultured *in vitro.* Cells from the *in vitro* cultures can then be passaged to animals. Such tumors can serve as targets for further testing or drug screening. Alternatively, the tumors resulting from the passage can be isolated and RNA from pre-passage cells and cells isolated after one or more rounds of passage analyzed for differential expression of genes of interest. Such passaging techniques can be performed with any known tumor or cancer cell lines.

For example, Meth A, CMS4, CMS5, CMS21, and WEHI-164 are chemically induced fibrosarcomas of BALB/c female mice (DeLeo *et al.,* J. Exp. Med., 146:720 [1977]), which provide a highly controllable model system for studying the anti-tumor activities of various agents (Palladino *et al.,* J. Immunol., 138:4023-4032 [1987]). Briefly, tumor cells are propagated *in vitro* in cell culture. Prior to injection into the animals, the cell lines are washed and suspended in buffer, at a cell density of about 10x10⁶ to 10x10⁷ cells/ml. The animals are then infected subcutaneously with 10 to 100 *µl* of the cell suspension, allowing one to three weeks for a tumor to appear.

In addition, the Lewis lung (3LL) carcinoma of mice, which is one of the most thoroughly studied experimental tumors, can be used as an investigational tumor model. Efficacy in this tumor model has been correlated with beneficial effects in the treatment of human patients diagnosed with small cell carcinoma of the lung (SCCL). This tumor can be introduced in normal mice upon injection of tumor fragments from an affected mouse or of cells maintained in culture (Zupi *et al.,* Br. J. Cancer, 41, suppl. 4:309 [1980]), and evidence indicates that tumors can be started from injection of even a single cell and that a very high proportion of infected tumor cells survive. For further information about this tumor model see, Zacharski, Haemostasis, 16:300-320 [1986]).

One way of evaluating the efficacy of a test compound in an animal model is implanted tumor is to measure the size of the tumor before and after treatment. Traditionally, the size of implanted tumors has been measured with a slide caliper in two or three dimensions. The measure limited to two dimensions does not accurately reflect the size of the tumor, therefore, it is usually converted into the corresponding volume by using a mathematical formula. However, the measurement of tumor size is very inaccurate. The therapeutic effects of a drug candidate can be better described as treatment-induced growth delay and specific growth delay. Another important variable in the description of tumor growth is the tumor volume doubling time. Computer programs for the calculation and description of tumor growth are also available, such as the program reported by Rygaard and Spang-Thomsen, Proc. 6th Int. Workshop on Immune-Deficient Animals ,Wu and Sheng eds., Basel, 1989, 301. It is noted, however, that necrosis and inflammatory responses following treatment may actually result in an increase in tumor size, at least initially. Therefore, these changes need to be carefully monitored, by a combination of a morphometric method and flow cytometric analysis.

Recombinant (transgenic) animal models can be engineered by introducing the coding portion of the genes identified herein into the genome of animals of interest, using standard techniques for producing transgen ic an imals. Animals that can serve as a target for transgenic manipulation include, without limitation, mice, rats, rabbits, guinea pigs, sheep, goats, pigs, and non-human primates, *eg.*, baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (*eg.*, Van der Putten *et al.,* Proc. Natl. Acad. Sci. USA, 82:6148-615 [1985]); gene targeting in embryonic stem cells (Thompson *et al*., Cell, 56:313-321 [1989]); electroporation of embryos (Lo, Mol. Cell. Biol., 3:1803-1814 [1983]); sperm-mediated gene transfer (Lavitrano *et al.,* Cell, 57:717-73 [1989]). For review, *see,* for example, U.S. Patent No. 4,736,866.

For the purpose of the present invention, transgenic animals include those that carry the transgene only in part of their cells ("mosaic animals"). The transgene can be integrated either as a single transgene, or in concatamers, *e.g.*, head-to-head or head-to-tail tandems. Selective introduction of a transgene into a particular cell type is also possible by following, for example, the technique of Lasko *et al.,* Proc. Natl. Acad. Sci. USA, 89:6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry. The animals are further examined for signs of tumor or cancer development.

The efficacy of antibodies specifically binding the polypeptides identified herein and other drug candidates, can be tested also in the treatment of spontaneous animal tumors. A suitable target for such studies is the feline oral squamous cell carcinoma (SCC). Feline oral SCC is a highly invasive, malignant tumor that is the most common oral malignancy of cats, accounting for over 60% of the oral tumors reported in this species. It rarely metastasizes to distant sites, although this low incidence of metastasis may merely be a reflection of the short survival times for cats with this tumor. These tumors are usually not amenable to surgery, primarily because of the anatomy of the feline oral cavity. At present, there is no effective treatment for this tumor. Prior to entry into the study, each cat undergoes complete clinical examination, biopsy, and is scanned by computed tomography (CT). Cats diagnosed with sublingual oral squamous cell tumors are excluded from the study. The tongue can become paralyzed as a result of such tumor, and even if the treatment kills the tumor, the animals may not be able to feed themselves. Each cat is treated repeatedly, over a longer period of time. Photographs of the tumors will be taken daily during the treatment period, and at each subsequent recheck. After treatment, each cat undergoes another CT scan. CT scans and thoracic radiograms are evaluated every 8 weeks thereafter. The data are evaluated for differences in survival, response and toxicity as compared to control groups. Positive response may require evidence of tumor regression, preferably with improvement of quality of life and/or increased life span.

In addition, other spontaneous animal tumors, such as fibrosarcoma, adenocarcinoma, lymphoma, chrondroma, leiomyosarcoma of dogs, cats, and baboons can also be tested. Of these mammary adenocarcinoma in dogs and cats is a preferred model as its appearance and behavior are very similar to those in humans. However, the use of this model is limited by the rare occurrence of this type of tumor in animals.

### H. Screening Assays for Drug Candidates

Screening assays for drug candidates are designed to identify compounds that competitively bind or complex with the receptor(s) of the polypeptides identified herein, or otherwise signal through such receptor(s). Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art.

In binding assays, the interaction is binding and the complex formed can be isolated or detected in the reaction mixture. In a particular embodiment, a receptor of a polypeptide encoded by the gene identified herein or the drug candidate is immobilized on a solid phase, *e.g.,* on a microtiter plate, by covalent or non-covalent attachments. Non-covalent attachment generally is accomplished by coating the solid surface with a solution of the polypeptide and drying. Alternatively, an immobilized antibody, *e.g.*, a monoclonal antibody, specific for the polypeptide to be immobilized can be used to anchor it to a solid surface. The assay is performed by adding the non-immobilized component, which may be labeled by a detectable label, to the immobilized component, *e.g.*, the coated surface containing the anchored component. When the reaction is complete, the non-reacted components are removed, *e.g.*, by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

if the candidate compound interacts with but does not bind to a particular receptor, its interaction with that polypeptide can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature (London), 340:245-246 (1989); Chien *et al.,* Proc. Natl. Acad. Sci. USA, 88:9578-9582 (1991)] as disclosed by Chevray and Nathans [Proc. Natl. Acad. Sci. USA, 89:5789-5793 (1991)]. Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain ofGAL4, and another, in which candidate activating proteins are fused to the activation domain. The expression of a GALI-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™ ) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

### 1. Pharmaceutical Compositions

The polypeptides of the present invention, agonist antibodies specifically binding proteins identified herein, as well as other molecules identified by the screening assays disclosed herein, can be administered for the treatment of tumors, including cancers, in the form of pharmaceutical compositions.

Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (*see, e.g.,* Marasco *et al.,* Proc. Natl. Acad. Sci. USA, 90:7889-7893 [1993]).

The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

Therapeutic formulations of the polypeptides identified herein, or agonists thereof are prepared for storage by mixing the active ingredient having the desired degree of purity with optional pharmaceutically acceptable carriers, excipients or stabilizers (*Remington's Pharmaceutical Sciences,* 16th edition, Osol, A. ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.*, Zn-protein complexes); and/or non-ionic surfactants such as TWEEN™, PLURONICS™ or polyethylene glycol (PEG).

The formulation herein may also contain more than one active compound as necessary for the particular indication being treated, preferably those with complementary activities that do not adversely affect each other. Altemativcly, or in addition, the composition may comprise a cytotoxic agent, cytokine or growth inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. ed. (1980).

The formulations to be used for *in vivo* administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

Sustained-release preparations may be prepared. Suitable examples of sustained-release preparations include semipermeable matrices of solid hydrophobic polymers containing the antibody, which matrices are in the form of shaped articles, *e.g.,* films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### J. Methods of Treatment

It is contemplated that the polypeptides of the present invention and their agonists, including antibodies, peptides, and small molecule agonists, may be used to treat various tumors, *e.g.*, cancers. Exemplary conditions or disorders to be treated include benign or malignant tumors (*e.g.*, renal, liver, kidney, bladder, breast, gastric, ovarian, colorectal, prostate, pancreatic, lung, vulval, thyroid, hepatic carcinomas; sarcomas; glioblastomas; and various head and neck tumors); leukemias and lymphoid malignancies; other disorders such as neuronal, glial, astrocytal, hypothalamic and other glandular, macrophagal, epithelial, stromal and blastocoelic disorders; and inflammatory, angiogenic and immunologic disorders. The anti-tumor agents of the present invention (including the polypeptides disclosed herein and agonists which mimic their activity, *e.g.*, antibodies, peptides and small organic molecules), are administered to a mammal, preferably a human, in accord with known methods, such as intravenous administration as a bolus or by continuous infusion over a period of time, or by intramuscular, intraperitoneal, intracerobrospinal, intraocular, intraarterial, intralesional, subcutaneous, intraarticular, intrasynovial, intrathecal, oral, topical, or inhalation routes.

Other therapeutic regimens may be combined with the administration of the anti-cancer agents of the instant invention. For example, the patient to be treated with such anti-cancer agents may also receive radiation therapy. Alternatively, or in addition, a chemotherapeutic agent may be administered to the patient. Preparation and dosing schedules for such chemotherapeutic agents may be used according to manufacturers' instructions or as determined empirically by the skilled practitioner. Preparation and dosing schedules for such chemotherapy are also described in Chemotherapy Service, ed., M.C. Perry, Williams & Wilkins, Baltimore, MD (1992). The chemotherapeutic agent may precede, or follow administration of the anti-tumor agent of the present invention, or may be given simultaneously therewith. The anti-cancer agents of the present invention may be combined with an anti-oestrogen compound such as tamoxifen or an anti-progesterone such as onapristone (see, EP 616812) in dosages known for such molecules.

It may be desirable to also administer antibodies against tumor associated antigens, such as antibodies which bind to the ErbB2, EGFR, ErbB3, ErbB4, or vascular endothelial factor (VEGF). Alternatively, or in addition, two or more antibodies binding the same or two or more different cancer-associated antigens may be co-administered to the patient. Sometimes, it may be beneficial to also administer one or more cytokines to the patient. In a preferred embodiment, the anti-cancer agents herein are co-administered with a growth inhibitory agent. For example, the growth inhibitory agent may be administered first, followed by the administration of an anti-cancer agent of the present invention. However, simultaneous administration or administration of the anti-cancer agent of the present invention first is also contemplated. Suitable dosages for the growth inhibitory agent are those presently used and may be lowered due to the combined action (synergy) of the growth inhibitory agent and the antibody herein.

For the prevention or treatment of disease, the appropriate dosage of an anti-tumor agent herein will depend on the type of disease to be treated, as defined above, the severity and course of the disease, whether the agent is administered for preventive or therapeutic purposes, previous therapy, the patient's clinical history and response to the agent, and the discretion of the attending physician. The agent is suitably administered to the patient at one time or over a series of treatments. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" in Toxicokinetics and New Drug Development, Yacobi *et al.,* eds., Pergamon Press, New York 1989, pp. 42-96.

For example, depending on the type and severity of the disease, about 1 *µ*g/kg to 15 mg/kg (*e.g.*, 0.1-20 mg/kg) of an antitumor agent is an initial candidate dosage for adm inistration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays. Guidance as to particular dosages and methods of delivery is provided in the literature; *see,* for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

### K. Articles of Manufacture

In another embodiment of the invention, an article of manufacture containing materials useful for the diagnosis or treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is an anti-tumor agent of the present invention. The label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's , instructions unless otherwise indicated. The source of those cells identified in the following examples, and throughout the specification, by ATCC accession numbers is the American Type Culture Collection, Manassas, VA. EXAMPLE I: Extracellular Domain Homology Screening to Identify Novel Polypeptides and cDNA Encoding Therefor

The extracellular domain (ECD) sequences (including the secretion signal sequence, if any) from about 950 known secreted proteins from the Swiss-Prot public database were used to search EST databases. The EST databases included public databases (*e.g.*, Dayhoff, GenBank), and proprietary databases (*e.g.* LIFESEQ® , lncyte Pharmaceuticals, Palo Alto, CA). The search was performed using the computer program BLAST or BLAST-2 (Altschul *et al.,* Methods in Enzymology, 266:460-480 (1996)) as a comparison of the ECD protein sequences to a 6 frame translation of the EST sequences. Those comparisons with a BLAST score of 70 (or in some cases 90) or greater that did not encode known proteins were clustered and assembled into consensus DNA sequences with the program "phrap" (Phil Green, University of Washington, Seattle, Washington).

Using this extracellular domain homology screen, consensus DNA sequences were assembled relative to the other identified EST sequences using phrap. In addition, the consensus DNA sequences obtained were often (but not always) extended using repeated cycles of BLAST or BLAST-2 and phrap to extend the consensus sequence as far as possible using the sources of EST sequences discussed above.

Based upon the consensus sequences obtained as described above, oligonucleotides were then synthesized and used to identify by PCR a cDNA library that contained the sequence of interest and for use as probes to isolate a clone of the full-length coding sequence for a PRO polypeptide. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5 kbp. In order to screen several libraries for a full-length clone, DNA from the libraries was screened by PCR amplification, as per Ausubel *et al.,* Current Protocols in Molecular Biology, with the PCR primer pair. A positive library was then used to isolate clones encoding the gene of interest using the probe oligonucleotide and one of the primer pairs.

The cDNA libraries used to isolate the cDNA clones were constructed by standard methods using commercially available reagents such as those from Invitrogen, San Diego, CA. The cDNA was primed with oligo dT containing a Not[ site, linked with blunt to SalI hemikinased adaptors, cleaved with Not[, sized appropriately by gel electrophoresis, and cloned in a defined orientation into a suitable cloning vector (such as pRKB or pRKD; pRK5B is a precursor of pRKSD that does not contain the Sfil site; *see,* Holmes *et al.,* Science, 253:1278-1280 (1991)) in the unique Xhol and NotI sites.

### EXAMPLE 2

### Isolation of cDNA clones Encoding Human PRO179

A cDNA clone (DNA16451-1078) encoding a native human PRO179 polypeptide was identified using a yeast screen, in a human fetal liver library that preferentially represents the 5' ends of the primary cDNA clones.

The primers used for the identification of DNA 16451-1078 are as follows:

OLI114:
5'-CCACGTTGGCTTGAAATTGA-3'     (SEQ ID NO:3)

OLI115:
5'-CCTTTAGAATTGATCAAGACAATTCATGATTTGATTCTCTATCTCCAGAG-3' (SEQ ID NO:4)

OLI116:
5'-TCGTCTAACATAGCAAATC-3'    (SEQ ID NO:5)

Clone DNA 16451-1078 contains a single open reading frame with an apparent translational initiation site at nucleotide positions 37-39, and an apparent stop codon at nucleotide positions 1417-1419 (Figures 1; SEQ ID NO:1). The predicted polypeptide precursor is 460 amino acids long. The full-length PRO179 protein is shown in Figure 2 (SEQ ID NO:2).

Analysis of the full-length PRO179 sequence shown in Figure 2 (SEQ ID NO:2) evidences the presence of important polypeptide domains as shown in Figure 2, wherein the locations given forthose important polypeptide domains are approximate as described above. Analysis of the full-length PRO179 sequence (Figure 2; SEQ ID NO:2) evidences the presence of the following: a signal peptide from about amino acid 1 to about amino acid 16; N-glycosylation sites from about amino acid 23 to about amino acid 27, from about amino acid 115 to about amino acid 119, from about amino acid 296 to about amino acid 300, and from about amino acid 357 to about amino acid 361; CAMP- and cGMP-dependent protein kinase phosphorylation sites from about amino acid 100 to about amino acid 104 and from about amino acid 204 to about amino acid 208; a tyrosine kinase phosphorylation site from about amino acid 342 to about amino acid 351; N-myristoylation sites from about amino acid 279 to about amino acid 285, from about amino acid 352 to about amino acid 358, and from about amino acid 367 to about amino acid 373; and leucine zipper patterns from about amino acid 120 to about amino acid 142 and from about amino acid 127 to about amino 149.

Clone DNA 16451-1078 has been deposited with ATCC on September 18, 1997 and is assigned ATCC deposit no. 209281. The full-length PRO179 protein shown in Figure 2 has an estimated molecular weight of about 53,637 daltons and a pl of about 6.61.

An analysis of the Dayhoff database (version 35.45 SwissProt 35) of the full-length sequence shown in Figure 2 (SEQ ID NO:2), evidenced the presence of a fibrinogen-like domain exhibiting a high degree of sequence homology with the two known human ligands of the TIE-2 receptor (h-TIE-2L1 and h-TIE-2L2). The abbreviation "TIE" is an acronym which stands for "tyrosine kinase containing Ig and EGF homology domains" and was coined to designate a new family of receptor tyrosine kinases. Accordingly, PRO179 has been identified as a novel member of the TIE ligand family.

### EXAMPLE 3

### Isolation of cDNA clones Encoding Human PRO207

An expressedsequence tag (EST) DNA database (LIFESEQ®, Incyte Pharmaceuticals, Palo Alto, CA) was searched and an EST was identified which showed homology to human Apo-2 ligand. A human fetal kidney cDNA library was then screened. mRNAisolated from human fetal kidney tissue (Clontech) was used to prepare the cDNA library. This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the SalI/Notl linkered cDNA was cloned into XhoI/NotI cleaved vector. pRK5D is a cloning vector that has an sp6 transcription initiation site followed by an Sfil restriction enzyme site preceding the XhoI/NotI cDNA cloning sites. The library was screened by hybridization with a synthetic oligonucleotide probe: 5'-CCAGCCCTCTGCGCTACAACCGCCAGATCGGGGAGTTTATAGTCACCCGG-3' (SEQ ID NO:8) based on the EST.

A cDNA clone was sequenced in entirety. A nucleotide sequence of the full-length DNA30879-1152 is shown in Figure 3 (SEQ ID NO:6). Clone DNA30879-1152 contains a single open reading frame with an apparent translational initiation site at nucleotide positions 58-60 (Figure 3; SEQ ID NO:6) and an apparent stop codon at nucleotide positions 805-807. The predicted polypeptide precursor is 249 amino acids long.

Analysis of the full-length PRO207 sequence shown in Figure 4 (SEQ ID NO:7) evidences the presence of important polypeptide domains as shown in Figure 4, wherein the locations given for those important polypeptide domains are approximate as described above. Analysis of the full-length PRO207 sequence (Figure 4; SEQ ID NO:7) evidences the presence of the following: a signal peptide from about amino acid 1 to about amino acid 40; an N-glycosylation site from about amino acid 139 to about amino acid 143; N-myristoylation sites from about amino acid 27 to about amino acid 33, from about amino acid 29 to about amino acid 35, from about amino acid 36 to about amino acid 42, from about amino acid 45 to about amino acid 51, from about amino acid 118 to about amino acid 124, from about amino acid 121 to about amino acid 127, from about amino acid 125 to about amino acid 131, and from about amino acid 128 to about amino acid 134; amidation sites from about amino acid 10 to about amino acid 14 and from about amino acid 97 to about amino acid 101; and a prokaryotic membrane lipoprotein lipid attachment site from about amino acid 24 to about amino acid 35. Clone DNA30879-1152 has been deposited with ATCC on October 10, 1997 and is assigned ATCC deposit no. 209358. The full-length PRO207 protein shown in Figure 4 has an estimated molecular weight of about 27,216 daltons and a pI of about 9.61.

Based on a BLAST and FastA sequence alignment analysis (using the ALIGN-2 computer program) of the full-length PRO207sequence shown in Figure 4 (SEQ ID NO:7), PRO207 shows amino acid sequence identity to several members of the TNF cytokine family, and particularly, to human lymphotoxin-beta (23.4%) and human CD40 ligand (19.8%).

### EXAMPLE 4

### Isolation of cDNA clones Encoding Human PRO320

A consensus DNA sequence was assembled relative to other EST sequences using phrap as described in Example 1 above. This consensus sequence is designated herein as DNA28739. Based on the DNA28739 consensus sequence, oligonucleotides were synthesized: 1) to identify by PCR a cDNA library that contained the sequence of interest, and 2) for use as probes to isolate a clone of the full-length coding sequence for PRO320.

A pair of PCR primers (forward and reverse) were synthesized: 
forward PCR primer:
5'-CCTCAGTGGCCACATGCTCATG-3'    (SEQ ID NO:11)
 
reverse PCR primer:
5'-GGCTGCACGTATGGCTATCCATAG-3'          (SEQ ID NO:12)
 Additionally, a synthetic oligonucleotide hybridization probe was constructed from the consensus DNA28739 sequence which had the following nucleotide sequence:

hybridization probe:
5'-GATAAACTGTCAGTACAGCTGTGAAGACACAGAAGAAGGGCCACAGTGCC-3' (SEQ ID NO:13)

In order to screen several libraries for a source of full-length clone, DNA from the libraries was screened by PCR amplification with the PCR primer pair identified above. A positive library was then used to isolate clones encoding the PRO320 gene using the probe oligonucleotide and one of the PCR primers. RNA for construction of the cDNA libraries was isolated from human fetal lung tissue (LIB025).

DNA sequencing of the isolated clones isolated as described above gave the full-length DNA sequence for DNA32284-1307 [Figure 5, SEQ ID NO:9]; and the derived protein sequence for PRO320.

The entire coding sequence of DNA32284-1307 is included in Figure 5 (SEQ ID NO:9). Clone DNA32284-1307 contains a single open reading frame with an apparent translational initiation site at nucleotide positions 135-137, and an apparent stop codon at nucleotide positions 1149-1151. The predicted polypeptide precursor is 338 amino acids long. Analysis of the full-length PRO320 sequence shown in Figure 6 (SEQ ID NO: 10) evidences the presence of a variety of important polypeptide domains, wherein the locations given for those important polypeptide domains are approximate as described above. Analysis of the full-length PRO320 polypeptide shown in Figure 6 evidences the presence of the following: a signal peptide from about amino acid 1 to about amino acid 21; an amidation site from about amino acid 330 to about amino acid 334; aspartic acid and asparagine hydroxylation sites from about amino acid 109 to about amino acid 121, from about amino acid 191 to about amino acid 203, and from about ammo acid 236 to about amino acid 248; an EGF-like domain cysteine pattern signature from about amino acid 80 to about amino acid 91; calcium-binding EGF-like domains from about amino acid 103 to about amino acid 125, from about amino acid 230 to about amino acid 252, and from about amino acid 185 to about amino acid 207. Clone DNA32284-1307 has been deposited with the ATCC on March 11, 1998 and is assigned ATCC deposit no. 209670. The full-length PRO320 protein shown in Figure 6 has an estimated molecular weight of about 37,143 daltons and a pl of about 8.92.

### EXAMPLE 5

### Isolation of cDNA clones Encoding Human PRO219

A consensus DNA sequence was assembled relative to other EST sequences using phrap as described in Example I above. This consensus sequence is designated herein as DNA28729. Based on the DNA28729 consensus sequence, oligonucleotides were synthesized: 1) to identify by PCR a cDNA library that contained the sequence of interest, and 2) for use as probes to isolate a clone of the full-length coding sequence for PRO219.

A pair of PCR primers (forward and reverse) were synthesized: 
forward PCR primer:
5'-GTGACCCTGGTTGTGAATACTCC-3' (SEQ ID NO:16)
 
reverse PCR primer:
5'-ACAGCCATGGTCTATAGCTTGG-3' (SEQ ID NO:17)
 Additionally, a synthetic oligonucleotide hybridization probe was constructed from the consensus DNA28729 sequence which had the following nucleotide sequence:

hybridization probe:
5'-GCCTGTCAGTGTCCTGAGGGACACGTGCTCCGCAGCGATGGGAAG-3' (SEQ ID NO:18)

In order to screen several libraries for a source of a full-length clone, DNA from the libraries was screened by PCR amplification with the PCR primer pair identified above. A positive library was then used to isolate clones encoding the PRO219 gene using the probe oligonucleotide and one of the PCR primers. RNA for construction of the cDNA libraries was isolated from human fetal kidney tissue.

DNA sequencing of the isolated clones isolated as described above gave the full-length DNA sequence for DNA32290-1164 [Figure 7, SEQ ID NO: 14]; and the derived protein sequence for PRO219.

The entire coding sequence of DNA32290-1164 is included in Figure 7 (SEQ ID NO:14). Clone DNA32290- 1164 contains a single open reading frame with an apparent translational initiation site at nucleotide positions 204-206, and an apparent stop codon at nucleotide positions 2949-2951. The predicted polypeptide precursor is 1005 amino acids long. Analysis of the full-length PRO219 sequence shown in Figure 8 (SEQ ID NO: 15) evidences the presence of a variety of important polypeptide domains, wherein the locations given for those important polypeptide domains are approximate as described above. Analysis of the full-length PRO219 polypeptide shown in Figure 8 evidences the presence of the following: a signal peptide from about amino acid 1 to about amino acid 23; an N-glycosylation site from about amino acid 221 to about amino acid 225; cAMP- and cGMP-dependent protein kinase phosphorylation sites from about amino acid 115 to about amino acid 119, from about amino acid 606 to about amino acid 610, and from about amino acid 892 to about amino acid 896; N-myristoylation sites from about amino acid 133 to about amino acid 139, from about amino acid 258 to about amino acid 264, from about amino acid 299 to about amino acid 305, from about amino acid 340 to about amino acid 346, from about amino acid 453 to about amino acid 459, from about amino acid 494 to about amino acid 500, from about amino acid 639 to about amino acid 645, from about amino acid 690 to about amino acid 694, from about amino acid 752 to about amino acid 758, and from about amino acid 792 to about amino acid 798; amidation sites from about amino acid 314 to about amino acid 318, from about amino acid 560 to about amino acid 564, and from about amino acid 601 to about amino acid 605; and aspartic acid and asparagine hydroxylation sites from about amino acid 253 to about amino acid 265, from about amino acid 294 to about amino acid 306, from about amino acid 335 to about amino acid 347, from about amino acid 376 to about amino acid 388, from about amino acid 417 to about amino acid 429, from about amino acid 458 to about amino acid 470, from about amino acid 540 to about amino acid 552, and from about amino acid 581 to about amino acid 593. Clone DNA32290-1164 has been deposited with the ATCC on October 17, 1997 and is assigned ATCC deposit no. 209384. The full-length PRO219 protein shown in Figure 8 has an estimated molecular weight of about 102,233 daltons and a pI of about 6.02.

An analysis of the full-length PRO219 sequence shown in Figure 8 (SEQ ID NO: 15), suggests that portions of it possess significant homology to the mouse and human matrilin-2 precursor polypeptides.

### EXAMPLE 6

### Isolation of cDNA clones Encoding Human PRO221

A consensus DNA sequence was assembled relative to other EST sequences using phrap as described in Example 1 above. This consensus sequence is designated herein as DNA28756. Based on the DNA28756 consensus sequence, oligonucleotides were synthesized: 1) to identify by PCR a cDNA library that contained the sequence of interest, and 2) for use as probes to isolate a clone ofthe full-length coding sequence for PRO221.

A pair of PCR primers (forward and reverse) were synthesized: 
forward PCR primer:
5'-CCATGTGTCTCCTCCTACAAAG-3' (SEQ ID NO:21)
 
reverse PCR primer:
5'-GGGAATAGATGTGATCTGATTGG-3' (SEQ ID NO:22)
 Additionally, a synthetic oligonucleotide hybridization probe was constructed from the consensus DNA28756 sequence which had the following nucleotide sequence:

hybridization probe:
5'-CACCTGTAGCAATGCAAATCTCAAGGAAATACCTAGAGATCTTCCTCCTG-3' (SEQ ID NO:23)

In order to screen several libraries for a source of a full-length clone, DNA from the libraries was screened by PCR amplification with the PCR primer pair identified above. A positive library was then used to isolate clones encoding the PRO221 gene using the probe oligonucleotide and one of the PCR primers. RNA for construction of the cDNA libraries was isolated from human fetal lung tissue.

DNA sequencing of the isolated clones isolated as described above gave the full-length DNA sequence for DNA33089-1132 [Figure 9, SEQ ID NO: 19]; and the derived protein sequence for PRO221.

The entire coding sequence of DNA33089-1132 is included in Figure 9 (SEQ ID NO: 19). Clone DNA33089-1132 contains a single open reading frame with an apparent translational initiation site at nucleotide positions 179-181, and an apparent stop codon at nucleotide positions 956-958. The predicted polypeptide precursor is 259 amino acids long. Analysis of the full-length PRO221 sequence shown in Figure 10 (SEQ ID NO:20) evidences the presence of a variety of important polypeptide domains, wherein the locations given for those important polypeptide domains are approximate as described above. Analysis of the full-length PRO221 polypeptide shown in Figure 10 evidences the presence of the following: a signal peptide from about amino acid 1 to about amino acid 33; a transmembrane domain from about amino acid 204 to about amino acid 219; N-glycosylation sites from about amino acid 47 to about amino acid 51 and from about amino acid 94 to about amino acid 98; a cAMP- and cGMP-dependent protein kinase phosphorylation site from about amino acid 199 to about amino acid 203; and N-myristoylation sites from about amino acid 37 to about amino acid 43, from about amino acid 45 to about amino acid 51, and from about amino acid 110 to about amino acid 116. Clone DNA33089-1132 has been deposited with the ATCC on September 16, 1997 and is assigned ATCC deposit no. 209262. The full-length PRO22) protein shown in Figure 10 has an estimated molecular weight of about 29,275 daltons and a pl of about 6.92.

An analysis of the full-length PRO221sequence shown in Figure 10 (SEQ ID NO:20), shows it has homology to members of the leucine rich repeat protein superfamily, including SLIT protein.

### EXAMPLE 7

### Isolation of cDNA clones Encoding Human PRO224

A consensus DNA sequence was assembled relative to other EST sequences using phrap as described in Example I above. This consensus sequence is designated herein as DNA30845. Based on the DNA30845 consensus sequence, oligonucleotides were synthesized: 1) to identify by PCR a cDNA library that contained the sequence of interest, and 2) for use as probes to isolate a clone of the full-length coding sequence for PRO224.

A pair of PCR primers (forward and reverse) were synthesized: 
forward PCR primer:
5'-AAGTTCCAGTGCCGCACCAGTGGC-3' (SEQ ID NO:26)
 
reverse PCR primer:
5'-TTGGTTCCACAGCCGAGCTCGTCG-3' (SEQ ID NO:27)
 Additionally, a synthetic oligonucleotide hybridization probe was constructed from the consensus DNA30845 sequence which had the following nucleotide sequence:

hybridization probe:
5'-GAGGAGGAGTGCAGGATTGAGCCATGTACCCAGAAAGGGCAATGCCCACC-3' (SEQ ID NO:28)

In order to screen several libraries for a source of a full-length clone, DNA from the libraries was screened by PCR amplification with the PCR primer pair identified above. A positive library was then used to isolate clones encoding the PRO224 gene using the probe oligonucleotide and one of the PCR primers. RNA for construction of the cDNA libraries was isolated from human fetal liver tissue.

DNA sequencing of the isolated clones isolated as described above gave the full-length DNA sequence for DNA33221-1133 [Figure II SEQ ID NO:24]; and the derived protein sequence for PRO224.

The entire coding sequence of DNA33221-1133 is included in Figure 11 (SEQ ID NO:24). Clone DNA33221-1133 contains a single open reading frame with an apparent translational initiation site at nucleotide positions 33-35, and an apparent stop codon at nucleotide positions 879-881. The predicted polypeptide precursor is 282 amino acids long. Analysis of the full-length PRO224 sequence shown in Figure 12 (SEQ ID NO:25) evidences the presence of a variety of important polypeptide domains, wherein the locations given for those important polypeptide domains are approximate as described above. Analysis of the full-length PRO224 polypeptide shown in Figure 12 evidences the presence of the following: a signal peptide from about amino acid I to about amino acid 30; a transmembrane domain from about amino acid 231 to about amino acid 248; N-glycosylation sites from about amino acid 126 to about amino acid 130, from about amino acid 195 to about amino acid 199, and from about amino acid 213 to about amino acid 217; N-myristoylation sites from about amino acid 3 to about amino acid 9, from about amino acid 10 to about amino acid 16, from about amino acid 26 to about amino acid 32, from about amino acid 30 to about amino acid 36, from about amino acid 112 to about amino acid 118, from about amino acid 166 to about amino acid 172, from about amino acid 212 to about amino acid 218, from about amino acid 224 to about amino acid 230, from about amino acid230 to about amino acid 236, and from about amino acid 263 to about amino acid 269; a prokaryotic membrane lipoprotein lipid attachment site from about amino acid 44 to about amino acid 55; and a leucine zipper pattern from about amino acid 17 to about amino acid 39. Clone DNA33221-1133 has been deposited with the ATCC on September 16, 1997 and is assigned ATCC deposit no. 209263. The full-length PRO224 protein shown in Figure 12 has an estimated molecular weight of about 28,991 daltons and a pl of about 4.62.

An analysis of the full-length PRO224 sequence shown in Figure 12 (SEQ ID NO:25), suggests that it has homology to very low-density lipoprotein receptors, apolipoprotein E receptor and chicken oocyte receptor P95. Based on a BLAST and FastA sequence alignment analysis of the full-length sequence, PRO224 has amino acid sequence identity to portions of these proteins in the range from 28% to 45%, and overall identity with these proteins in the range from 33% to 39%.

### EXAMPLE 8

### Isolation of cDNA clones Encoding Human PRO328

A consensus DNA sequence was assembled relative to other EST sequences using phrap as described in Example I above. This consensus sequence is designated herein as DNA35615. Based on the DNA35615 consensus sequence, oligonucleotides were synthesized: 1) to identify by PCR a cDNA library that contained the sequence of interest, and 2) for use as probes to isolate a clone of the full-length coding sequence for PRO328.

A pair of PCR primers (forward and reverse) were synthesized: 
[?]

forward PCR primer:

5'-TCCTGCAGTTTCCTGATGC-3' (SEQ ID NO:31)
 
reverse PCR primer:
5'-CTCATATTGCACACCAGTAATTCG-3'          (SEQ ID NO:32)
 Additionally, a synthetic oligonucleotide hybridization probe was constructed from the consensus DNA35615 sequence which had the following nucleotide sequence:

hybridization probe:
5'-ATGAGGAGAAACGTTTGATGGTGGAGCTGCACAACCTCTACCGGG-3' (SEQ ID NO:33)

In order to screen several libraries for a source of full-length clone, DNA from the libraries was screened by PCR amplification with the PCR primer pair identified above. A positive library was then used to isolate clones encoding the PRO328 gene using the probe oligonucleotide and one of the PCR primers. RNA for construction of the cDNA libraries was isolated from human fetal kidney tissue.

DNA sequencing of the isolated clones isolated as described above gave the full-length DNA sequence for DNA40587-1231 [Figure 13, SEQ ID NO:29]; and the derived protein sequence for PRO328.

The entire coding sequence of DNA40587-1231 is included in Figure 13 (SEQ ID NO:29). Clone DNA40587-1231 contains a single open reading frame with an apparent translational initiation site at nucleotide positions 15-17, and an apparent stop codon at nucleotide positions 1404-1406. The predicted polypeptide precursor is 463 amino acids long. Analysis of the full-length PRO328 sequence shown in Figure 14 (SEQ ID NO:30) evidences the presence of a variety of important polypeptide domains, wherein the locations given for those important polypeptide domains are approximate as described above. Analysis of the full-length PRO328 polypeptide shown in Figure 14 evidences the presence of the following: a signal peptide from about amino acid 1 to about amino acid 22; N-glycosylation sites from about amino acid 114 to about amino acid 118, from about amino acid 403 to about amino acid 407, and from about amino acid 409 to about amino acid 413; a glycosaminoglycan attachment site from about amino acid 439 to about amino acid 443; N-myristoylation sites from about amino acid 123 to about amino acid 129, from about amino acid 143 to about amino acid 149, from about amino acid 152 to about amino acid 158, from about amino acid 169 to about amino acid 175, from about amino acid 180 to about amino acid 186, from about amino acid 231 to about amino acid 237, and from about amino acid 250 to about amino acid 256; amidation sites from about amino acid 82 to about amino acid 88 and from about amino acid 172 to about amino acid 176; a peroxidase proximal heme-ligand signature from about amino acid 287 to about amino acid 298; an extracellular protein SCP/Tpx-1/Ag5/PR-1/Sc7 signature 1 domain from about amino acid 127 to about amino acid 138; and an extracellular protein SCP/Tpx-1/Ag5PR-1/Sc7 signature 2 domain from about amino acid 160 to about amino acid 172. Clone DNA40587-1231 has been deposited with the ATCC on November 7, 1997 and is assigned ATCC deposit no. 209438. The full-length PRO328 protein shown in Figure 14 has an estimated molecular weight of about 49,471 daltons and a pl of about 5.36.

An analysis of the full-length PRO328sequence shown in Figure 14 (SEQ ID NO:30), suggests that portions of it possess significant homology to the human glioblastoma protein, and to the cysteine rich secretory protein thereby indicating that PRO328 may be a novel glioblastoma protein or cysteine rich secretory protein.

### EXAMPLE 9

### Isolation of cDNA clones Encoding Human PRO301

A consensus DNA sequence was assembled relative to other EST sequences using phrap as described in Example. above. This consensus sequence is designated herein as DNA35936. Based on the DNA35936 consensus sequence, oligonucleotides were synthesized: 1) to identify by PCR a cDNA library that contained the sequence of interest, and 2) for use as probes to isolate a clone of the full-length coding sequence for PRO301.

The oligonucleotides used in the above procedure were the following:

forward PCR primer 1:
5'-TCGCGGAGCTGTGTTCTGTTTCCC-3'                    (SEQ ID NO:36)

forward PCR primer 2:
5'-ACACCTGGTTCAAAGATGGG-3' (SEQ ID NO:37)

forward PCR primer 3:
5'-TTGCCTTACTCAGGTGCTAC-3' (SEQ ID NO:38)

reverse PCR primer 1:
5'-TAGGAAGAGTTGCTGAAGGCACGG-3' (SEQ ID NO:39)

reverse PCR primer 2:
5'-ACTCAGCAGTGGTAGGAAAG-3'                    (SEQ ID NO:40)

hybridization probe 1:
5'-TGATCGCGATGGGGACAAAGGCGCAAGCTCGAGAGGAAACTGTTGTGCCT-3' (SEQ ID NO:41)

In order to screen several libraries for source of a full-length clone, DNA from the libraries was screened by PCR amplification with the PCR primer pair identified above. A positive library was then used to isolate clones encoding the PRO301 gene using the probe oligonucleotide and one of the PCR primers. RNA for construction of the cDNA libraries was isolated from human fetal kidney tissue.

DNA sequencing of the isolated clones isolated as described above gave the full-length DNA sequence for DNA40628-1216 [Figure 15, SEQ ID NO:34]; and the derived protein sequence for PRO301.

The entire coding sequence of DNA40628-1216 is included in Figure 15 (SEQ ID NO:34). Clone DNA40628-1216 contains a single open reading frame with an apparent translational initiation site at nucleotide positions 52-54, and an apparent stop codon at nucleotide positions 949-951. The predicted polypeptide precursor is 299 amino acids long. Analysis of the full-length PRO301 sequence shown in Figure 16 (SEQ ID NO:35) evidences the presence of a variety of important polypeptide domains, wherein the locations given for those important polypeptide domains are approximate as described above. Analysis of the full-length PRO301 polypeptide shown in Figure 16 evidences the presence of the following: a signal peptide from about amino acid 1 to about amino acid 27; a transmembrane domain from about amino acid 235 to about amino acid 256; an N-glycosylation site from about amino acid 185 to about amino acid 189; a cAMP- and cGMP-dependent protein kinase phosphorylation site from about amino acid 270 to about amino acid 274; and N-myristoylation sites from about amino acid 105 to about amino acid 111, from about amino acid 116 to about amino acid 122, from about amino acid 158 to about amino acid 164, from about amino acid 219 to about amino acid 225, from about amino acid 237 tq about amino acid 243, and from about amino acid 256 to about amino acid 262. Clone DNA40628-1216 has been deposited with the ATCC on November 7, 1997 and is assigned ATCC deposit no. 209432. The full-length PRO301 protein shown in Figure 16 has an estimated molecular weight of about 32,583 daltons and a pl of about 8.29.

Based on a BLAST and FastA sequence alignment of the full-length PRO301 sequence shown in Figure 16 (SEQ ID NO:35), PRO301 shows amino acid sequence identity to the A33 antigen precursor (30%) and the coxsackie and adenovirus receptor protein (29%).

### EXAMPLE 10

### Isolation of cDNA clones Encodine Human PRO526

A consensus DNA sequence was assembled relative to other EST sequences using phrap as described in Example 1 above. An initial consensus sequence was identified designated herein as DNA39626.init. In addition, the initial consensus DNA sequence was extended using repeated cycles of BLAST and phrap to extend the initial consensus sequence as far as possible using the sources of EST sequences discussed above. The assembled consensus sequence is designated herein as <consen01>. Based on the <consen01 > consensus sequence, oligonucleotides were synthesized: 1) to identify by PCR a cDNA library that contained the sequence of interest, and 2) for use as probes to isolate a clone of the full-length coding sequence for PRO526.

A pair of PCR primers (forward and reverse) were synthesized: 
forward PCR primer:
5'-TGGCTGCCCTGCAGTACCTCTACC-3' (SEQ ID NO:44)
 
reverse PCR primer:
5'-CCCTGCAGGTCATTGGCAGCTAGG-3' (SEQ ID NO:45)
 Additionally, a synthetic oligonucleotide hybridization probe was constructed from the <consen01> consensus sequence which had the following nucleotide sequence:

hybridization probe:
5'-AGGCACTGCCTGATGACACCTTCCGCGACCTGGGCAACCTCACAC-3' (SEQ ID NO:46)

In order to screen several libraries for a source of a full-length clone, DNA from the libraries was screened by PCR amplification with the PCR primer pair identified above. A positive library was then used to isolate clones encoding the PRO526 gene using the probe oligonucleotide and one of the PCR primers. RNA for construction of the cDNA libraries was isolated from human fetal liver tissue (LIB228).

DNA sequencing of the isolated clones isolated as described above gave the full-length DNA sequence for DNA44184-1319 [Figure 17, SEQ ID NO:42]; and the derived protein sequence for PRO526.

The entire coding sequence of DNA44184-1319 is included in Figure 17 (SEQ ID NO:42). Clone DNA44184-1319 contains a single open reading frame with an apparent translational initiation site at nucleotide positions 514-516, and an apparent stop codon at nucleotide positions 1933-1935. The predicted polypeptide precursor is 473 amino acids long. Analysis of the full-length PRO526 sequence shown in Figure 18 (SEQ ID NO:43) evidences the presence of a variety of important polypeptide domains, wherein the locations given for those important polypeptide domains are approximate as described above. Analysis of the full-length PRO526 polypeptide shown in Figure 18 evidences the presence of the following: a signal peptide from about amino acid 1 to about amino acid 26; a leucine zipper pattern from about amino acid 135 to about amino acid 157; a glycosaminoglycan attachment site from about amino acid 436 to about amino acid 440; N-glycosylation sites from about amino acid 82 to about amino acid 86, from about amino acid 179 to about amino acid 183, from about amino acid 237 to about amino acid 241, from about amino acid 372 to about amino acid 376, and from about amino acid 423 to about amino acid 427; and a von Willebrand factor (VWF) type C domain from about amino acid 411 to about amino acid 427. Clone DNA44184-1319 has been deposited with the ATCC on March 26, 1998 and is assigned ATCC deposit no. 209704. The full-length PRO526 protein shown in Figure 18 has an estimated molecular weight of about 50,708 daltons and a pl of about 9.28.

An analysis of the full-length PRO526 sequence shown in Figure 18 (SEQ ID NO:43), suggests that portions of it possess significant homology to leucine repeat rich proteins including ALS, SLIT, carboxypeptidase and platelet glycoprotein V thereby indicating that PRO526 is a novel protein which is involved in protein-protein interactions.

### EXAMPLE 1

### Isolation of cDNA clones Encoding Human PRO362

A consensus DNA sequence was assembled relative to other EST sequences using phrap as described in Example 1 above. This consensus sequence is designated herein as DNA42257. Based on the DNA42257 consensus sequence, oligonucleotides were synthesized: 1) to identify by PCR a cDNA library that contained the sequence of interest, and 2) for use as probes to isolate a clone of the full-length coding sequence for PRO362.

A pair of PCR primers (forward and reverse) were synthesized: 
forward PCR primer 1:
5'-TATCCCTCCAATTGAGCACCCTGG-3' (SEQ ID NO:49)
 
forward PCR primer 2:
5'-GTCGGAAGACATCCCAACAAG-3' (SEQ ID NO:50)
 
reverse PCR primer 1:
5'-CTTCACAATGTCGCTGTGCTGCTC-3' (SEQ ID NO:51)
 
reverse PCR primer 2:
5'-AGCCAAATCCAGCAGCTGGCTTAC-3' (SEQ ID NO:52)
 Additionally, a synthetic oligonucleotide hybridization probe was constructed from the DNA42257 consensus sequence which had the following nucleotide sequence:

hybridization probe:
5'-TGGATGACCGGAGCCACTACACGTGTGAAGTCACCTGGCAGACTCCTGAT-3' (SEQ ID NO:53)

In order to screen several libraries for a source of full-length clone, DNA from the libraries was screened by PCR amplification with the PCR primer pair identified above. A positive library was then used to isolate clones encoding the PRO362 gene using the probe oligonucleotide and one of the PCR primers. RNA for construction of the cDNA libraries was isolated from human fetal brain tissue (LIB153).

DNA sequencing of the isolated clones isolated as described above gave the full-length DNA sequence for DNA45416-1251 [Figure 19, SEQ ID NO:47]; and the derived protein sequence for PRO362.

The entire coding sequence of DNA45416-1251 is included in Figure 19 (SEQ ID NO:47). Clone DNA45416-1251 contains a single open reading frame with an apparent translational initiation site at nucleotide positions 119-121, and an apparent stop codon at nucleotide positions 1082-1084. The predicted polypeptide precursor is 321 amino acids long. Analysis of the full-length PRO362 sequence shown in Figure 20 (SEQ ID NO:48) evidences the presence of a variety of important polypeptide domains, wherein the locations given for those important polypeptide domains are approximate as described above. Analysis of the full-length PRO362 polypeptide shown in Figure 20 evidences the presence of the following: a signal peptide from about amino acid I to about amino acid 19; a transmembrane domain from about amino acid 281 to about amino acid 300; a glycosaminoglycan attachment site from about amino acid 149 to about amino acid 153; a cAMP- and eGMP- dependent protein kinase phosphorylation site from about amino acid 308 to about amino acid 312; and N-myristoylation sites from about amino acid 2 to about amino acid 8, from about amino acid 148 to about amino acid 154, from about amino acid 158 to about amino acid 164, from about amino acid 207 to about amino acid 213, and from about amino acid 215 to about amino acid 221. Clone DNA4S416-1251 has been deposited with the ATCC on February 5, 1998 and is assigned ATCC deposit no. 209620. The full-length PRO362 protein shown in Figure 20 has an estimated molecular weight of about 35,544 daltons and a pl of about 8.51.

An analysis of the full-length PRO362 sequence shown in Figure 20 (SEQ ID NO:48), suggests that it possesses significant similarity to the A33 antigen protein and the HCAR protein. More specifically, an analysis ofthe Dayhoff database (version 35.45 SwissProt 35) evidenced significant homology between the PRO362 amino acid sequence and the following Dayhoff sequences: AB002341_1, HSU55258_1, HSC7NRCAM_1, RNU81037_1, A33_HUMAN, P_W14158, NMNCAMRI_1, HSTITINN2_1, S71824_1, and HSU63041_1.

### EXAMPLE 12

### Isolation of cDNA clones Encodine Human PRO356

An expressed sequence tag (EST) DNA database (LIFESEQ®, Incyte Pharmaceuticals, Palo Alto, CA) was searched and an EST (#2939340) was identified that had homology to PRO179 [identified in EXAMPLE 2 above and designated DNA 16451-1078 (Figure 1; SEQ ID NO:1)]. To clone PRO356, a human fetal lung library prepared from mRNA purchased from Clontech, Inc., (Palo Alto, CA), catalog # 6528-I was used, following the manufacturer's instructions.

The cDNA libraries used to isolate the cDNA clones encoding human PRO356 were constructed by standard methods using commercially available reagents such as those from Invitrogen, San Diego, CA. The cDNA was primed with oligo dT containing a Notl site, linked with blunt to SalI hemikinased adaptors, cleaved with Notl, sized appropriately by gel electrophoresis, and cloned in a defined orientation into a suitable cloning vector (such as pRKB or pRKD; pRK5B is a precursor of pRK5D that does not contain the Sfil site; *see,* Holmes *et al.,* Science, 253:1278-1280 (1991)) in the unique Xhol and Notl.

Oligonucleotide probes based upon the above described EST sequence were then synthesized: I) to identify by PCR a cDNA library that contained the sequence of interest, and 2) for use as probes to isolate a clone of the full-length coding sequence for PRO356. Forward and reverse PCR primers generally range from 20-30 nucleotides and are often designed to give a PCR product of about 100-1000 bp in length. The probe sequences are typically 40-55 bp in length. In order to screen several libraries for a full-length clone, DNA from the libraries was screened by PCR amplification, as per Ausubel *et al.,* Current Protocols in Molecular Biology, *supra,* with the PCR primer pair. A positive library was then used to isolate clones encoding the gene of interest using the probe oligonucleotide and one of the primer pairs.
The oligonucleotide sequences used were as follows:

5'-TTCAGCACCAAGGACAAGGACAATGACAACT-3' (SEQ ID NO:56)

5'-TGTGCACACTTGTCCAAGCAGTTGTCATTGTC-3' (SEQ ID NO:57)

5'-GTAGTACACTCCATTGAGGTTGG-3'    (SEQ ID NO:58)

A cDNA clone was identified and sequenced in entirety. The entire nucleotide sequence of DNA47470-1130-Pl is shown in Figure 21 (SEQ ID NO:54). Clone DNA47470-1130-P1 contains a single open reading frame with an apparent translational initiation site at nucleotide positions 215-217, and a stop codon at nucleotide positions 1253-1255 (Figure 21; SEQ ID NO:54). The predicted polypeptide precursor is 346 amino acids long, and has a calculated molecular weight of approximately 40,018 daltons and an estimated pl of about 8.19. The full-length PRO356 protein is shown in Figure 22 (SEQ ID NO:55).

Analysis of the full-length PRO356 sequence shown in Figure 22 (SEQ ID NO:55) evidences the presence of important polypeptide domains as shown in Figure 22, wherein the locations given for those important polypeptide domains are approximate as described above. Analysis of the full-length PRO356 sequence (Figure 22; SEQ ID NO:55) evidences the presence of the following: a signal peptide from about amino acid 1 to about amino acid 26; N-glycosylation sites from about amino acid 58 to about amino acid 62, from about amino acid 253 to about amino acid 257, and from about amino acid 267 to about amino acid 271; a glycosaminoglycan attachment site from about amino acid 167 to about amino acid 171; a cAMP- and cGMP-dependent protein kinase phosphorylation site from about amino acid 176 to about amino acid 180; N-myristoylation sites from about amino acid 168 to about amino acid 174, from about amino acid 196 to about amno acid 202, from about amino acid 241 to about amino acid 247, from about amino acid 252 to about amino acid 258, from about amino acid 256 to about amino acid 262, and from about amino acid 327 to about amino acid 333; and a cell attachment sequence from about amino acid 199 to about amino acid 202.

Clone DNA47470-1130-PI has been deposited with ATCC on October 28, 1997 and is assigned ATCC deposit no. 209422. It is understood that the deposited clone has the actual correct sequence rather than the representations provided herein.

An analysis of the Dayhoffdatabase (version 35.45 SwissProt35), using the ALIGN-2 sequence alignment analysis of the full-length sequence shown in Figure 22 (SEQ ID NO:55), shows amino acid sequence identity between the PRO356 amino acid sequence and both TIE-2L1 (32%) and TIE-2L2 (34%). The abbreviation "TIE" is an acronym which stands for "**t**yrosine kinase containing **I**g and **E**GF homology domains" and was coined to designate a new family of receptor tyrosine kinases.

### EXAMPLE 13

### Isolation of cDNA clones Encoding Human PRO509

To isolate a cDNA for DNA50148- 1068, a bacteriophage library of human retinal cDNA (commercially available from Clontech) was screened by hybridization with a synthetic oligonucleotide probe based on an EST sequence (GenBank locus AA021617), which showed some degree of homology to members of the TNFR family. The oligonucleotide probe employed in the screening was 60 bp long. Five positive clones (containing cDNA inserts of 1.8-1.9 kb) were identified in the cDNA library, and the positive clones were confirmed to be specific by PCR using the above hybridization probe as a PCR primer. Single phage plaques containing each of the five positive clones were isolated by limiting dilution and the DNA was purified using a Wizard Lambda Prep DNA purification kit (commercially available from Promega).

The cDNA inserts from three of the five bacteriophage clones were excised from the vector arms by digestion with EcoRl, gel-purified, and subcloned into pRK5 and sequenced on both strands. The three clones contained an identical open reading frame (with the exception of an intron found in one of the clones).

The entire nucleotide sequence of DNA50148-1068 is shown in Figure 23 (SEQ ID NO: 59). The cDNA contained one open reading frame with a translational initiation site assigned to the ATG codon at nucleotide positions 82-84. The open reading frame ends at the termination codon TGA at nucleotide positions 931-933.

The predicted amino acid sequence of the full length PRO509 polypeptide sequence contains 283 amino acids. The full-length PRO509 protein is shown in Figure 24 (SEQ ID NO:60) and has an estimated molecular weight of approximately 30,420 and a pl of about 7.34.

Analysis ofthe full-length PRO509 sequence shown in Figure 24 (SEQ ID NO:60) evidences the presence of important polypeptide domains as shown in Figure 24, wherein the locations given for those important polypeptide domains are approximate as described above. Analysis of the full-length PRO509 sequence (Figure 24; SEQ ID NO:60) evidences the presence of the following: a signal peptide from about amino acid I to about amino acid 36; a transmembrane domain from about amino acid 205 to about amino acid 221; N-glycosylation sites from about amino acid 110 to about amino acid 114 and from about amino acid 173 to about amino acid 177; N-myristoylation sites from about amino acid 81 to about amino acid 87, from about amino acid 89 to about amino acid 95, from about amino acid 104 to about amino acid 110, from about amino acid 120 to about amino acid 126, from about amino acid 153 to about amino acid 159, from about amino acid 193 to about amino acid 199, from about amino acid 195 to about amino acid 201, and from about amino acid 220 to about amino acid 226; and a cell attachment sequence from about amino acid 231 to about amino acid 234.

An alignment (using the ALIGN™ computer program) of a 58 amino acid long cytoplasmic region of PRO509 with other known members of the human TNF receptor family showed some sequence similarity, and in particular to CD40 (12 identities) and LT-beta receptor (11 identities).

### EXAMPLE 14

### Isolation of cDNA clones Encoding Human PRO866

A consensus DNA sequence was assembled relative to other EST sequences using phrap as described in Example 1 above. This consensus sequence is designated herein as DNA42257. Based on the DNA44708 consensus sequence, oligonucleotides were synthesized: 1) to identify by PCR a cDNA library that contained the sequence of interest, and 2) for use as probes to isolate a clone of the full-length coding sequence for PRO866.

PCR primers (forward and reverse) were synthesized: 
forward PCR primer 1:
5'-CAGCACTGCCAGGGGAAGAGGG-3'          (SEQ ID NO:63)
 
forward PCR primer 2:
5'-CAGGACTCGCTACGTCCG-3' (SEQ ID NO:64)
 
forward PCR primer 3:
5'-CAGCCCCTTCTCCTCCTTTCTCCC-3' (SEQ ID NO:65)
 
reverse PCR primer 1:
5'-GCAGTTATCAGGGACGCACTCAGCC-3'     (SEQ ID NO:66)
 
reverse PCR primer 2:
5'-CCAGCGAGAGGCAGATAG-3'                    (SEQ ID NO:67)
 
reverse PCR primer 3:
5'-CGGTCACCGTGTCCTGCGGGATG-3'          (SEQ ID NO:68)
 Additionally, a synthetic oligonucleotide hybridization probe was constructed from the DNA44708 consensus sequence which had the following nucleotide sequence:

hybridization probe:
5'-CAGCCCCTTCTCCTCCTTTCTCCCACGTCCTATCTGCCTCTC-3' (SEQ ID NO:69)

In order to screen several libraries for a source of a full-length clone, DNA from the libraries was screened by PCR amplification with the PCR primer pair identified above. A positive library was then used to isolate clones encoding the PRO866 gene using the probe oligonucleotide and one of the PCR primers. RNA for construction of the cDNA libraries was isolated from human fetal kidney tissue (LIB228).

DNA sequencing of the isolated clones isolated as described above gave the full-length DNA sequence for DNA53971-1359 [Figure 25. SEQ ID NO:611; and the derived protein sequence for PRO866.

The entire coding sequence of DNA53971-1359 is included in Figure 25 (SEQ ID NO:61). Clone DNAS3971-1359 contains a single open reading frame with an apparent translational initiation site at nucleotide positions 275-277, and an apparent stop codon at nucleotide positions 1268-1270. The predicted polypeptide precursor is 331 amino acids long. Analysis of the full-length PRO866 sequence shown in Figure 26 (SEQ ID NO:62) evidences the presence of variety of important potypeptide domains, wherein the locations given for those important polypeptide domains are approximate as described above. Analysis of the full-length PRO866 polypeptide shown in Figure 26 evidences the presence of the following: a signal peptide from about amino acid I to about amino acid 26; a glycosaminoglycan attachment site from about amino acid 131 to about amino acid 135; a CAMP- and cGMP-dependent protein kinase phosphorylation site from about amino acid 144 to about amino acid 148; and N-myristoylation sites from about amino acid 26 to about amino acid 32, from about amino acid 74 to about amino acid 80, from about amino acid 132 to about amino acid 138, from about amino acid 134 to about amino acid 140, from about amino acid 190 to about amino acid 196, from about amino acid 287 to about amino acid 293, and from about amino acid 290 to about amino acid 296. Clone DNAS3971-1359 has been deposited with the ATCC on April 4, 1998 and is assigned ATCC deposit no. 209750. The full-length PRO866 protein shown in Figure 26 has an estimated molecular weight of about 35,844 daltons and a pl of about 5.45.

An analysis of the full-length PRO866 sequence shown in Figure 26 (SEQ ID NO:62), suggests that it possesses significant similarity to the mindin/spondin family of proteins, thereby indicating that PRO866 may be a novel mindin homolog. More specifically, an analysis of the Dayhoff database (version 35.45 SwissProt 35) evidenced significant homology between the PRO866 amino acid sequence and the following Dayhoff sequences: AB006085_1, AB006084_1, AB006086_1, AF017267_1, CWU42213_1, AC004160_1, CPMICPP_1, S49108, A48569 and 146687.

### EXAMPLE 15

### In situ Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis, and aid in chromosome mapping.

*In situ* hybridization was performed following an optimized version of the protocol by Lu and Gillett, Cell Vision, 1: 169-176 (1994), using PCR-generated ³³P-labeled riboprobes. Briefly, formalin-fixed, paraffin-embedded human tissues were sectioned, deparaffinized, deproteinated in proteinase K (20 g/ml) for 15 minutes at 37°C, and further processed for *in situ* hybridization as described by Lu and Gillett, *supra.* A (³³-P)UTP-labeled antisense riboprobe was generated from a PCR product and hybridized at 55°C overnight. The slides were dipped in Kodak NTB2™ nuclear track emulsion and exposed for 4 weeks.

### ³³P-Riboprobe synthesis

6.0 *µ*l (125 mCi) of ³³P-UTP (Amersham BF 1002, SA<2000 Ci/mmol) were speed-vacuum dried. To each tube containing dried ³³P-UTP, the following ingredients were added:
2.0 µl 5x transcription buffer
1.0 µl DTT (100 mM)
2.0 µl NTP mix (2.5 mM: 10 µl each of 10 mM GTP, CTP & ATP + 10 µl H₂O)
1.0 µl UTP (50 µM)
1.0 µl RNAsin
1.0 µl DNA template (1 µg)
1.0 µl H₂O
1.0 µl RNA polymerase (for PCR products T3 = AS, T7 = S, usually)

The tubes were incubated at 37°C for one hour. A total of 1.0 µl RQ1 DNase was added, followed by incubation at 37°C for 15 minutes. A total of 90 µl TE (10 mM Tris pH 7.6/l mM EDTA pH 8.0) was added, and the mixture was pipetted onto DE81 paper. The remaining solution was loaded in a MICROCON-50™ ultrafiltration unit, and spun using program 10 (6 minutes). The filtration unit was inverted over a second tube and spun using program 2 (3 minutes). After the final recovery spin, a total of 100 µl TE was added, then 1 µl of the final product was pipetted on DE81 paper and counted in 6 ml of BIOFLUOR II™.

The probe was run on a TBE/urea gel. A total of 1-3 µl of the probe or 5 µl of RNA Mrk III was added to 3 µl of loading buffer. After heating on a 95 °C heat block for three minutes, the gel was immediately placed on ice. The wells of gel were flushed, and the sample was loaded and run at 180-250 volts for 45 minutes. The gel was wrapped in plastic wrap (SARAN™ brand) and exposed to XAR film with an intensifying screen in a - 70°C freezer one hour to overnight.

### ³³P-Hybridization

### A. Pretreatment of frozen sections

The slides were removed from the freezer, placed on aluminum trays, and thawed at room temperature for 5 minutes. The trays were placed in a 55°C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ H₂O). After deproteination in 0.5 µg/ml proteinase K for 10 minutes at 37°C (12.5 µl of 10 mg/ml stock in 250 ml prewarmed RNAse-free RNAse buffer), the sections were washed in 0.5 x SSC for 10 minutes at room temperature. The sections were dehydrated in 70%, 95%, and 100% ethanol, 2 minutes each.

### B. Pretreatment of paraffin-embedded sections

The slides were deparaffinized, placed in SQ H₂O, and rinsed twice in 2 x SSC at room temperature, for 5 minutes each time. The sections were deproteinated in 20 µg/ml proteinase K (500 µl of 10 mg/ml in 250 ml RNase-free RNase buffer; 37°C, 15 minutes) for human embryo tissue, or 8 x proteinase K (100 µl in 250 ml Rnase buffer, 37°C, 30 minutes) for formalin tissues. Subsequent rinsing in 0.5 x SSC and dehydration were performed as described above.

### C. Prehybridization

The slides were laid out in a plastic box lined with Box buffer (4 x SSC, 50% formamide) - saturated filter paper. The tissue was covered with 50 µl of hybridization buffer (3.75 g dextran sulfate + 6 ml SQ H₂O), vortexed, and heated in the microwave for 2 minutes with the cap loosened. After cooling on ice, 18.75 ml formamide, 3.75 ml 20 x SSC, and 9 ml SQ H₂O were added, and the tissue was vortexed well and incubated at 42°C for 1-4 hours.

### D. Hybridization

1.0 x 10⁶ cpm probe and 1.0 µl tRNA (50 mg/ml stock) per slide were heated at 95 °C for 3 minutes. The slides were cooled on ice, and 48 µl hybridization buffer was added per slide. After vortexing, 50 µl ³³P mix was added to 50 µl prehybridization on the slide. The slides were incubated overnight at 55°C.

### E. Washes

Washing was done for 2x 10 minutes with 2xSSC, EDTA at room temperature (400 ml 20 x SSC + 16 ml 0.25 M EDTA, Vᵣ=4L), followed by RNAseA treatment at 37°C for 30 minutes (500 µl of 10 mg/ml in 250 ml Rnase buffer = 20 µg/ml), The slides were washed 2 x 10 minutes with 2 x SSC, EDTA at room temperature. The stringency wash conditions were as follows: 2 hours at 55 °C, 0.1 x SSC, EDTA (20 m 120 x SSC + 16 ml EDTA, Vᵣ=4L).

### F. Oligonucleotides

*In situ* analysis was performed on 5 of the DNA sequences disclosed herein. The oligonucleotides employed for these analyses are as follows:

### (1) DNA30879-1152 (PRO207)

p1:
5'-GGA TTC TAA TAC GAC TCA CTA TAG GGC TCC TGC GCC TTT CCT GAA CC-3' (SEQ ID NO:70)

p2:
5'-CTA TGA AAT TAA CCC TCA CTA AAG GGA GAC CCA TCC TTG CCC ACA GAG-3' (SEQ ID NO:71)

### (3) DNA33089-1132 (PRO221)

p1:
5'-GGA TTC TAA TAC GAC TCA CTA TAG GGC TGT GCT TTC ATT CTG CCA GTA-3' (SEQ ID NO:72)

p2:
5'-CTA TGA AAT TAA CCC TCA CTA AAG GGA GGG TAC AAT TAA GGG GTG GAT-3' (SEQ ID NO:73)

### (4) DNA33221-1133 (PRO224)

p1:
5'-GGA TTC TAA TAC GAC TCA CTA TAG GGC GCA GCG ATG GCA GCG ATG AGG-3' (SEQ ID NO:74)

p2:
5'-CTA TGA AAT TAA CCC TCA CTA AAG GGA CAG ACG GGG CAG CAG GGA GTG-3' (SEQ ID NO:75)

### (5) DNA40628-1216 (PRO301)

pl:
5'-GGA TTC TAA TAC GAC TCA CTA TAG GGC GAG TCC TTC GGC GGC TGT T-3' (SEQ ID NO:76)

p2:
5'-CTA TGA AAT TAA CCC TCA CTA AAG GGA CGG GTG CTT TTG GGA TTC GTA-3' (SEQ ID NO:77)

### (6) DNA45416-1251 (PRO362)

p1:
5'-GGA TTC TAA TAC GAC TCA CTA TAG GGC CTC CAA GCC CAC AGT GAC AA-3' (SEQ ID NO:78)

p2:
5'-CTA TGA AAT TAA CCC TCA CTA AAG GGA CCT CCA CAT TTC CTG CCA GTA-3' (SEQ ID NO:79)

### G. Results

*In situ* analysis was performed on the above 5 DNA sequences disclosed herein. The results from these analyses are as follows:

### (1) DNA30879-1152 (PRO207) (Apo2L Homolog)

Low-level expression was observed over a chondrosarcoma, and over one other soft-tissue sarcoma. All other tissues were negative.

Human fetal tissues examined (E 12-E 16 weeks) included: placenta,umbilical cord, liver, kidney, adrenals, thyroid, lungs, heart, great vessels, esophagus, stomach, small intestine, spleen, thymus, pancreas, brain, eye, spinal cord, body wall, pelvis and lower limb.

Adult tissues examined included: kidney (normal and end-stage), adrenal, myocardium, spleen, lymph node, pancreas, lung, skin, eye (including retina), bladder, and liver (normal, cirrhotic, acute failure).

Non-human primate tissues examined included:
Chimp tissues: salivary gland, stomach, thyroid, parathyroid, tongue, thymus, ovary, and lymph node.
Rhesus monkey tissues: cerebral cortex, hippocampus, cerebellum, penis

### (2) DNA33089-1132 (PRO221) (1 TM receptor)

Specific expression was observed over fetal cerebral white and grey matter, as well as over neurones in the spinal cord. The probe appears to cross react with rat. Low level expression was seen over cerebellar neurones in adult rhesus brain. All other tissues were negative.

Fetal tissues examined (E12-E16 weeks) included: placenta, umbilical cord, liver, kidney, adrenals, thyroid, lungs, heart, great vessels, esophagus, stomach, small intestine, spleen, thymus, pancreas, brain, eye, spinal cord, body wall, pelvis and lower limb.

Adult tissues examined included: liver, kidney, adrenal, myocardium, aorta, spleen, lymph node, pancreas, lung, skin, cerebral cortex (rm), hippocampus (rm), cerebellum (rm), penis, eye, bladder, stomach, gastric carcinoma, colon, colonic carcinoma, and chondrosarcoma; also acetominophen induced liver injury and hepatic cirrhosis.

### (3) DNA33221-1133 (PRO224) (LDLR homolog - 1 TM)

Observed expression was limited to vascular endothelium in fetal spleen, adult spleen, fetal liver, adult thyroid and adult lymph node (chimp). Additional site of expression was seen in the developing spinal ganglia. All other tissues were negative.

Human fetal tissues examined (E 12-E 16 weeks) included: placenta, umbilical cord, liver, kidney, adrenals, thyroid, lungs, heart, great vessels, esophagus, stomach, small intestine, spleen, thymus, pancreas, brain, eye, spinal cord, body wall, pelvis and lower limb.

Adult tissues examined included: kidney, (normal and end-stage), adrenal, myocardium, aorta, spleen, lymph node, pancreas, lung, skin, eye (including retina), bladder, and liver (normal, cirrhotic, acute failure).

Non-human tissues examined included:
Chimp tissues: salivary gland, stomach, thyroid, parathyroid, skin, thymus, ovary, lymph node.
Rhesus monkey tissues: cerebral cortex, hippocampus, cerebellum, penis.

### (4) DNA40628-1216 (PRO301) (CD22 homolog (JAM hlog, A33 Ag hlog)

### Expression in inflamed human tissues (psoriasis, IBD, inflamed kidney, inflamed lung, hepatitis, normal tonsil, adult and chimp multiblocks):

Expression was evaluated in predominantly inflamed human tissue with a few normal human and non-human primate tissues. Expression was seen in every epithelial structure evaluated including the mucosal epithelium of the colon, bronchial large airway epithelium, oral mucosa (tongue), tonsillar crypt mucosa, placental mucosa, prostatic mucosa, glandular stomach mucosa, epithelial cells ofthymic Hassall's corpuscles, hepatocytes, biliary epithelium, and placental epithelium. The only evidence of expression outside of an epithelial structure was weak low, inconsistent expression in the germinal centers of follicles in a tonsil with reactive hyperplasia.

In non-human primate tissues the following was observed:

Chimp tissues: weak diffuse expression was observed in the epidermis of the tongue epithelium; in the thymus, weak specific expression was seen in thymic epithelium of Hassall's corpuscles; in the stomach, mild diffuse expression was observed in the epithelium of the glandular mucosa.

In human tissues: In the liver (multiblock including: chronic cholangitis, lobular hyperplasia, acetominophen toxicity): there was diffuse low to moderate expression in hepatocytes and biliary epithelium. Expression was most prominent in perilobular/periportal hepatocytes. It was most prominent in biliary eptithelium in sections of the liver with chronic sclerosing cholangitis. Expression was not present in all samples present; this may reflect sample quality more than expression variability.

In psoriasis: weak expression in the epidermis was seen.

In the lung with chronic interstitial pneumonia or chronic bronchitis: low diffuse expression was seen in the mucosal epithelium of large airways; weak diffuse expression was also seen in alveolar epithelium. There was no expression in the epithelium of the submucosal glands of bronchi/bronchioles.

In placenta: there was moderate diffuse expression in placental epithelium.

In the prostate: there was low diffuse expression in prostatic epithelium.

In the gall bladder: there was moderate diffuse expression in the mucosal epithelium.

in the tonsil with reactive hyperplasia: high diffuse expression was seen in the epithelium of the tonsillar mucosa and crypts; the signal was highest in the mucosal cells which line the tonsillar crypts. There was weak inconsistent diffuse expression in the germinal centers of cortical follicles (B lymphocyte areas); however, in no other tissue evaluated with lymphoid structures or lymphocytic inflammation was there any expression in B lymphocytes.

In the colon with inflammatory bowel disease and polyp/adenomatous changes: low expression was observed in the mucosal epithelium; expression was greatest in the villi tips. In the one specimen with a polyp, there was no evidence of increased expression ofthe dysplastic epithelium ofthe polyp as compared to the adjacent mucosa. There was no apparent expression in reactive mucosal lymphoid tissue that was present in many of the sections.

### (5) DNA45416-1251 (PRO362) (Ig domain homolog)

### Expression in inflamed human tissues (psoriasis, IBD, inflamed kidney, inflamed lung, ireparilis, normal tonsil, adult and chimp multiblocks):

The expression of this novel protein was evaluated in a variety of human and non-human primate tissues and was found to be highly restricted. Expression was present only in alveolar macrophages in the lung and Kupffercells of the hepatic sinusoids. Expression in these cells was significantly increased when these distinct cell populations were activated. Although these two subpopulations of tissue macrophages are located in different organs, they have similar biological functions. Both types of these phagocytes act as biological filters to remove material from the blood stream or airways including pathogens, senescent cells and proteins and both are capable of secreting a wide variety of important proinflammatory cytokines.

In inflamed lung (seven patient samples), expression was prominent in reactive alveolar macrophage cell populations defined as large, pale often vacuolated cells present singly or in aggregates within alveoli and was weak to negative in normal, non-reactive macrophages (single scattered cells of normal size). Expression in alveolar macrophages was increased during inflammation when these cells were both increased in numbers and size (activated). Despite the presence of histocytes in areas of interstitial inflammation and peribronchial lymphoid hyperplasia in these tissues, expression was restricted to alveolar macrophages. Many of the inflamed lungs also had some degree of suppurative inflammation; expression was not present in neutrophilic granulocytes.

In liver, there was strong expression in reactive/activated Kupffer cells in livers with acute centrilobular necrosis (acetominophen toxicity) or fairly marked periportal inflammation. However, there was weak or no expression in Kupffer cells in normal liver or in liver with only mild inflammation or mild to moderate lobular hyperplasia/hypertrophy. Thus, as in the lung, there was increased expression in activated/reactive cells.

There was no expression of this molecule in histiocytes/macrophages present in the inflammed bowel, hyperplastic/reactive tonsil or normal lymph node. The lack of expression in these tissues which all contain histiocytic inflammation or resident macrophage populations strongly supports restricted expression to the unique macrophage subset populations defined as alveolar macrophage and hepatic Kupffer cells.

Human tissues evaluated which had no detectable expression included: infammatory bowel disease (seven patient samples with moderate to severe disease), tonsil with reactive hyperplasia, peripheral lymph node, psoriatic skin (two patient samples with mild to moderate disease), heart, and peripheral nerve.

Chimp tissues evaluated which had no detectable expression included: tongue, stomach, and thymus.

### EXAMPLE 16

### Use of PRO179, PRO207, PRO320, PRO219, PRO221, PRO224, PRO328, PRO301, PRO526, PRO362, PRO356, PRO509 or PRO866 as a Hybridization Probe

The following method describes use of a nucleotide sequence encoding PRO 179, PRO207, PRO320, PRO219, PRO221, PRO224, PRO328, PRO301, PRO526, PRO362, PRO356, PRO509 or PRO866 as a hybridization probe.

DNA comprising the coding sequence of full-length or mature PRO179, PRO207, PRO320, PRO219, PRO221, PRO224, PRO328, PRO301, PRO526, PRO362, PRO356, PRO509 or PRO866 (as shown in Figure 1, 3, 5, 7, 9, 11, 13, 15, 17, 19, 21, 23, and 25, respectively, SEQ ID NOS: 1, 6,9, 14, 19,24,29,34,42,47,54, 59, and 61, respectively) or a fragment thereof is employed as a probe to screen for homologous DNAs (such as those encoding naturally-occurring variants of PRO179, PRO207, PRO320, PRO219, PRO221, PRO224, PRO328, PRO301, PRO526, PRO362, PRO356, PRO509 or PRO866) in human tissue cDNA libraries or human tissue genomic libraries.

Hybridization and washing of filters containing either library DNAs is performed under the following high-stringency conditions. Hybridization of radiolabeled probe derived from the gene encoding a PRO179, PRO207, PRO320, PRO219, PRO221, PRO224, PRO328, PRO301, PRO526, PRO362, PRO356, PRO509 or PRO866 polypeptide to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence can then be identified using standard techniques known in the art.

### EXAMPLE 17

### Expression of PRO179, PRO207, PRO320, PRO219, PRO221, PRO224, PRO328, PRO301, PRO526 PRO362, PRO356,PRO509 or PRO866 in E.coli

This example illustrates preparation of an unglycosylated form of PRO179, PRO207, PRO320, PRO219, PRO221, PRO224, PRO328, PRO301, PRO526, PRO362, PRO356, PRO509 or PRO866 by recombinant expression in *E. coli.*

The DNA sequence encoding PRO179, PRO207, PRO320, PRO219, PRO221, PRO224, PRO328, PRO301, PRO526, PRO362, PRO356, PRO509 or PRO866 is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli; see* Bolivar *et al.,* Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a poly-His leader (including the first six STII codons, poly-His sequence, and enterokinase cleavage site), the PRO179, PRO207, PRO320, PRO219, PRO221, PRO224, PRO328, PRO301, PRO526, PRO362, PRO356, PRO509 or PRO866 coding region, lambda transcriptional terminator, and an argU gene.

The ligation mixture is then used to transform a selected *E. coli* strain using the methods described in Sambrook *et al., supra.* Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO179, PRO207, PRO320, PRO219, PRO221, PRO224, PRO328, PRO301, PRO526, PRO362, PRO356, PRO509 or PRO866 protein can then be purified using a metal chelating column under conditions that allow tight binding of the protein.

PRO179, PRO207, PRO320, PRO219, PRO221, PRO224, PRO328, PRO301, PRO526, PRO362, PRO356, PRO509 or PRO866 may be expressed in *E. coli* in a poly-His tagged form, using the following procedure. The DNA encoding PRO179, PRO207, PRO320, PRO219, PRO221, PRO224, PRO328, PRO301, PRO526, PRO362, PRO356, PRO509 or PRO866 is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) Ion galE rpoHts(htpRts) clpP(laclq). Transformants are first grown in LB containing 50 mg/ml carbenicillin at 30°C with shaking until an OD₆₀₀ of 3-5 is reached. Cultures are then diluted 50-100 fold into CRAP media (prepared by mixing 3.57 g (NH₄)₂SO₄, 0.71 g sodium citrate·2H2O, 1.07 g KCI, 5.36 g Difco yeast extract, 5.36 g Sheffield hycase SF in 500 ml water, as well as 110 mM MPOS, pH 7.3, 0.55% (w/v) glucose and 7 mM MgSO₄) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1 M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni ²⁺-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1% TFA with elution with a gradient of acetonitrile from 10 to 80%. Aliquots of fractions with A₂₈₀ absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO179, PRO207, PRO320, PRO219, PRO221, PRO224, PRO328, PRO301, PRO526, PRO362, PRO356, PRO509 or PRO866 polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

PRO207, PRO224, and PRO301 were successfully expressed in *E. coli* in a poly-His tagged form by the above procedure.

### EXAMPLE 18

### Expression of PRO179, PRO207, PRO320, PRO219, PRO221, PRO224, PRO328, PRO301, PRO526, PRO362, PRO356, PRO509 or PRO866 in mammalian cells

This example illustrates preparation of a potentially glycosylated form of PRO179, PRO207, PRO320, PRO219, PRO221, PRO224, PRO328, PRO301, PRO526, PRO362, PRO356, PRO509 or PRO866 by recombinant expression in mammalian cells.

The vector, pRK5 (see EP 307,247, published March 15, 1989), is employed as the expression vector. Optionally, the PRO179, PRO207, PRO320, PRO219, PRO221, PRO224, PRO328, PRO301, PRO526, PRO362, PRO356, PRO509 or PRO866 DNA is ligated into pRK5 with selected restriction enzymes to allow insertion of the PRO179, PRO207, PRO320, PRO219, PRO221, PRO224, PRO328, PRO301, PRO526, PRO362, PRO356, PRO509 or PRO866 DNA using ligation methods such as described in Sambrook *et al., supra.* The resulting vector is called pRK5-PRO 179,pRK5-PRO207,pRK5-PRO320,pRK5-PRO219,pRK5-PRO221 ,pRK5-PRO224, pRK5-PRO328, pRK5-PRO301, pRK5-PRO526, pRK5-PRO362, pRK5-PRO356, pRK5-PRO509 or pRK5-PRO866, respectively.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-PRO 179, pRK5-PRO207, pRK5-PRO320, pRK5-PRO219, pRK5-PRO221,pRK5-PRO224,pRK5-PRO328,pRK5-PRO301,pRK5-PRO526,pRK5-PRO362, pRK5-PRO356, pRK5-PRO509 or pRK5-PRO866 DNA is mixed with about 1 µg DNA encoding the VA RNA gene [Thimmappaya *et al.,* Cell, 31:543 (1982)] and dissolved in 500 µl of 1 mM Tris-HCl, 0. 1 mM EDTA, 0.227 M CaCl₂. To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of the PRO179, PRO207, PRO320, PRO219, PRO221, PRO224, PRO328, PRO301, PRO526, PRO362, PRO356, PRO509 or PRO866 polypeptide. The cultures containing transfected cells may undergo further incubation (in serum free medium) and the medium is tested in selected bioassays.

In an alternative technique, PRO 179, PRO207, PRO320, PRO219, PRO221, PRO224, PRO328, PRO301, PRO526, PRO362, PRO356, PRO509 or PRO866 may be introduced into 293 cells transiently using the dextran sulfate method described by Somparyrac *et al.,* Proc. Natl. Acad. Sci., 12:7575 (1981). 293 cells are grown to maximal density in a spinner flask and 700 µg pRK5-PRO179, pRK5-PRO207, pRK5-PRO320, pRK5-PRO219, pRK5-PRO221, pRK5-PRO224,pRK5-PRO328,pRK5-PRO301,pRK5-PRO526,pRK5-PRO362,pRK5-PRO356, pRK5-PRO509 or pRK5-PRO866 DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 µg/ml bovine transferrin. After about four days, the conditioned media is centrifuged and filtered to remove cells and debris. The sample containing expressed PRO179, PRO207, PRO320, PRO219, PRO221, PRO224, PRO328, PRO301, PRO526, PRO362, PRO356, PRO509 or PRO866 can then be concentrated and purified by any selected method, such as dialysis and/or column chromatography.

In another embodiment, PRO179, PRO207, PRO320, PRO219, PRO221, PRO224, PRO328, PRO301, PRO526, PRO362, PRO356, PRO509 or PRO866 can be expressed in CHO cells. The pRK5-PRO179, pRK5-PRO207, pRK5-PRO320, pRKS-PRO219, pRKS-PRO221, pRK5-PRO224, pRK5-PRO328, pRK5-PRO301, pRK5-PRO526, pRK5-PRO362, pRK5-PRO356, pRK5-PRO509 or pRK5-PRO866 can be transfected into CHO cells using known reagents such as CAPO₄ or DEAE-dextran. As described above, the cell cultures can be incubated, and the medium replaced with culture medium (alone) or medium containing a radiolabel such as ³⁵S-methionine. After determining the presence of a PRO179, PRO207, PRO320, PRO219, PRO221, PRO224, PRO328, PRO301, PRO526, PRO362, PRO356, PRO509 or PRO866 polypeptide, the culture medium may be replaced with serum free medium. Preferably, the cultures are incubated for about 6 days, and then the conditioned medium is harvested. The medium containing the expressed PRO179, PRO207, PRO320, PRO219, PRO221, PRO224, PRO328, PRO301, PRO526, PRO362, PRO356, PRO509 or PRO866 polypeptide can then be concentrated and purified by any selected method.

Epitope-tagged PRO179, PRO207, PRO320, PRO219, PRO221, PRO224, PRO328, PRO301, PRO526, PRO362, PRO356, PRO509 or PRO866 may also be expressed in host CHO cells. The PRO179, PRO207, PRO320, PRO219, PRO221, PRO224, PRO328, PRO301, PRO526, PRO362, PRO356, PRO509 or PRO866 may be subcloned out of the pRK5 vector. The subclone insert can undergo PCR to fuse in frame with a selected epitope tag such as a poly-His tag into a Baculovirus expression vector. The poly-His tagged PRO179, PRO207, PRO320, PRO219, PRO221, PRO224, PRO328, PRO301, PRO526, PRO362, PRO356, PRO509 or PRO866 insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PRO179, PRO207, PRO320, PRO219, PRO221, PRO224, PRO328, PRO301, PRO526, PRO362, PRO356, PRO509 or PRO866 can then be concentrated and purified by any selected method, such as by Ni²⁺-chelate affinity chromatography.

PRO179, PRO207, PRO320, PRO219, PRO221, PRO224, PRO328, PRO301, PRO526, PRO362, PRO356, PRO509 or PRO866 may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (*e.g.*, extracellular domains) of the respective proteins are fused to an IgGI constant region sequence containing the hinge, CH2 and CH2 domains and/or as a poly-His tagged form.

Following PCR amplification, the respective DNAs are subcloned in a CHO expression vector using standard techniques as described in Ausubel *et al.,* Current Protocols of Molecular Biology Unit 3.16, John Wiley and Sons (1997). CHO expression vectors are constructed to have compatible restriction sites 5' and 3' of the DNA ofinterestto allow the convenient shuttling of cDNA's. The vector used in expression in CHO cells is as described in Lucas *et al.,* Nucl. Acids Res., 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect® (Quiagen), Dosper® or Fugene® (Boehringer Mannheim). The cells are grown as described in Lucas *et al., supra.* Approximately 3 x 10⁻⁷ cells are frozen in an ampule for further growth and production as described below.

The ampules containing the plasmid DNA are thawed by placement into a water bath and mixed by vortexing. The contents are pipetted into a centrifuge tube containing 10 mls of media and centrifuged at 1000 rpm for 5 minutes. The supernatant is aspirated and the cells are resuspended in 10 ml of selective media (0.2 µm filtered PS20 with 5% 0.2 µm diafiltered fetal bovine serum). The cells are then aliquoted into a 100 ml spinner containing 90 ml of selective media. After 1-2 days, the cells are transferred into a 250 ml spinner filled with 150 ml selective growth medium and incubated at 37°C. After another 2-3 days, 250 ml, 500 ml and 2000 ml spinners are seeded with 3 x 10⁵ cells/ml. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x 10⁶ cells/ml. On day 0, the cell number and pH is determined. On day 1, the spinner is sampled and sparging with filtered air is commenced. On day 2, the spinner is sampled, the temperature shifted to 33°C, and 30 ml of 500 g/L glucose and 0.6 ml of 10% antifoam (*e.g.*, 35% polydimethylsiloxane emulsion, Dow Coming 365 Medical Grade Emulsion) taken. Throughout the production, the pH is adjusted as necessary to keep it at around 7.2. After 10 days, or until the viability drops below 70%, the cell culture is harvested by centrifugation and filtering through a 0.22 µm filter. The filtrate is either stored at 4°C or immediately loaded onto columns for purification.

For the poly-His tagged constructs, the proteins are purified using a Ni ²⁺-NTA column (Qiagen). Before purification, imidazole is added to the conditioned media to a concentration of 5 mM. The conditioned media is pumped onto a 6 ml Ni ²⁺-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which has been equilibrated in 20 mM Na phosphate buffer, pH 6.8. After loading, the column is washed extensively with equilibration buffer before elution with 100 mM citric acid, pH 3.5. The eluted protein is immediately neutralized by collecting I ml fractions into tubes containing 275 µl of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

PRO179, PRO320, PRO219, PRO221, PRO224, PRO328, PRO301, PRO356, PRO509, and PRO866 were stably expressed in CHO cells by the above described method. In addition, PRO224, PRO328, PRO301, and PRO356 were expressed in CHO cells by the transient expression procedure.

### EXAMPLE 19

### Expression of PRO119, PRO207, PRO320, PRO219, PRO221, PRO224, PRO328, PRO301, PRO526, PRO362, PRO356, PRO509 or PRO866 in Yeast

The following method describes recombinant expression of PRO179, PRO207, PRO320, PRO219, PRO221, PRO224, PRO328, PRO301, PRO526, PRO362, PRO356, PRO509 or PRO866 in yeast.

First, yeast expression vectors are constructed for intracellular production or secretion of PRO179, PRO207, PRO320, PRO219, PRO221, PRO224, PRO328, PRO301, PRO526, PRO362, PRO356, PRO509 or PRO866 from the ADH2/GAPDH promoter. DNA encoding PRO179, PRO207, PRO320, PRO219, PRO221, PRO224, PRO328, PRO301, PRO526, PRO362, PRO356, PRO509 or PRO866 and the promoter is inserted into suitable restriction enzyme sites in the selected plasmid to direct intracellular expression of PRO179, PRO207, PRO320, PRO219, PRO221, PRO224, PRO328, PRO301, PRO526, PRO362, PRO356, PRO509 or PRO866. For secretion, DNA encoding PRO179, PRO207, PRO320, PRO219, PRO221, PRO224, PRO328, PRO301, PRO526, PRO362, PRO356, PRO509 or PRO866 can be cloned into the selected plasmid, together with DNA encoding the ADH2/GAPDH promoter, a native PRO 179, PRO207, PRO320, PRO219, PRO221, PRO224, PRO328, PRO301, PRO526, PRO362, PRO356, PRO509 or PRO866 signal peptide or other mammalian signal peptide, or, for example, a yeast alpha-factor or invertase secretory signal/leader sequence, and linker sequences (if needed) for expression of PRO179, PRO207, PRO320, PRO219, PRO221, PRO224, PRO328, PRO301, PRO526, PRO362, PRO356, PRO509 or PRO866.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO179, PRO207, PRO320, PRO219, PRO221, PRO224, PRO328, PRO301, PRO526, PRO362, PRO356, PRO509 or PRO866 can subsequently be isolated and purified by removing the yeast cells from the fermentation medium by centrifugation and then concentrating the medium using selected cartridge filters. The concentrate containing PRO179, PRO207, PRO320, PRO219, PRO221, PRO224, PRO328, PRO301, PRO526, PRO362, PRO356, PRO509 or PRO866 may further be purified using selected column chromatography resins.

### EXAMPLE 20

### Expression of PRO179, PRO207, PRO320, PRO219, PRO221, PRO224, PRO328, PRO301, PRO526, PRO362, PRO356, PRO509 or PRO866 in Baculovirus-Infected Insect Cells

The following method describes recombinant expression in Baculovirus-infected insect cells.

The sequence coding for PRO179, PRO207, PRO320, PRO219, PRO221, PRO224, PRO328, PRO301, PRO526, PRO362, PRO356, PRO509 or PRO866 is fused upstream of an epitope tag contained within a baculovirus expression vector. Such epitope tags include poly-His tags and immunoglobulin tags (like Fc regions of IgG). A variety of plasmids may be employed, including plasmids derived from commercially available plasmids such as pVL1393 (Novagen). Briefly, the sequence encoding PRO179, PRO207, PRO320, PRO219, PRO221, PRO224, PRO328, PRO301, PRO526, PRO362, PRO356, PRO509 or PRO866 or the desired portion of the coding sequence of PRO179, PRO207, PRO320, PRO219, PRO221, PRO224, PRO328, PRO301, PRO526, PRO362, PRO356, PRO509 or PRO866 (such as the sequence encoding the extracellular domain of a transmembrane protein or the sequence encoding the mature protein if the protein is extracellular) is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold™ virusDNA (Pharmingen) into *Spodoptera frugiperda("Sf9")* cells (ATCCCRL 171)using lipofectin (commercially available from GIBCO-BRL). After 4 - 5 days of incubation at 28°C, the released viruses are harvested and used for further amplifications. Viral infection and protein expression are performed as described by O'Reilley *et al.,* Baculovirus expression vectors: A Laboratory Manual, Oxford: Oxford University Press (1994).

Expressed poly-His tagged PRO179, PRO207, PRO320, PRO219. PRO221, PRO224, PRO328, PRO301, PRO526, PRO362, PRO356, PRO509 or PRO866 can then be purified, for example, by Ni²⁺-chelate affinity chromatography as follows. Extracts are prepared from recombinant virus-infected Sf9 cells as described by Rupert *el al.,* Nature, 362:175-179 (1993). Briefly, Sf9 cells are washed, resuspended in sonication buffer (25 ml Hepes, pH 7.9; 12.5 mM MgCl_{z}; 0.1 mM EDTA; 10% glycerol; 0.1% NP-40; 0.4 M KCI), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 mm filter. A Ni²⁺.NTA agarose column (commercially available from Qiagen) is prepared with a bed volume of 5 ml, washed with 25 ml of water and equilibrated with 25 ml of loading buffer. The filtered cell extract is loaded onto the column at 0.5 ml per minute. The column is washed to baseline A₂₈₀ with loading buffer, at which point fraction collection is started. Next, the column is washed with a secondary wash buffer (50 mM phosphate; 300 mM NaCI, 10% glycerol, pH 6.0), which elutes nonspecifically bound protein. After reaching A₂₈₀ baseline again, the column is developed with a 0 to 500 mM imidazole gradient in the secondary wash buffer. One ml fractions are col lected and analyzed by SDS-PAGE and silver staining or Western blot with Ni²⁺-NTA-conjugated to alkaline phosphatase (Qiagen). Fractions containing the eluted His₁₀-tagged PRO179, PRO207, PRO320, PRO219, PRO221, PRO224, PRO328, PRO301, PRO526, PRO362, PRO356, PRO509 or FRO866, respectively, are pooled and dialyzed against loading buffer.

Alternatively, purification of the IgG tagged (or Fc tagged) PRO179, PRO207, PRO320, PRO219, PRO221, PRO224, PRO328, PRO301, PRO526, PRO362, PRO356, PRO509 or PRO866 can be performed using known chromatography techniques, including for instance, Protein A or protein G column chromatography.

Following PCR amplification, the respectivecodingsequences are subcloned into a baculovirus expression vector (pb.PH.1gG for 1gG fusions and pb.PH.His.c for poly-His tagged proteins), and the vector and Baculogold® baculovirus DNA (Pharmingen) are co-transfected into 105 *Spodoplera frugiperda* ("Sf9") cells (ATCC CRL 1711), using Lipofectin (Gibco BRL). pb.PH.IgG and pb.PH.His are modifications of the commercially available baculovirus expression vector pVL 1393 (Pharmingen), with modified polylinker regions to include the His or Fc tag sequences. The cells are grown in Hink's TNM-FH medium supplemented with 10% FBS (Hyclone). Cells are incubated for 5 days at 28°C. The supernatant is harvested and subsequently used for the first viral amplification by infecting Sf9 cells in Hink's TNM-FH medium supplemented with 10% FBS at an approximate multiplicity of infection (MOl) of 10. Cells are incubated for 3 days at 28°C. The supernatant is harvested and the expression of the constructs in the baculovirus expression vector is determined by batch binding of 1 ml of supernatant to 25 ml of Ni ²⁺-NTA beads (QlAGEN) for histidine tagged proteins or Protein-A Sepharose CL-4B beads (Pharmacia) for IgG tagged proteins followed by SDS-PAGE analysis comparing to a known concentration of protein standard by Coomassie blue staining.

The first viral amplification supernatant is used to infect a spinner culture (500 ml) of Sf9 cells grown in ESF-921 medium (Expression Systems LLC) at an approximate MOl of 0.1. Cells are incubated for 3 days at 28°C. The supernatant is harvested and filtered. Batch binding and SDS-PAGE analysis is repeated, as necessary, until expression of the spinner culture is confirmed.

The conditioned medium from the transfected cells (0.5 to 3 L) is harvested by centrifugation to remove the cells and filtered through 0.22 micron filters. For the poly-His tagged constructs, the protein construct is purified using a Ni ²⁺-NTA column (Qiagen). Before purification, imidazole is added to the conditioned media to a concentration of 5 mM. The conditioned media is pumped onto a 6 ml Ni ²⁺-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc containing) constructs of proteins are purified from the conditioned media as follows. The conditioned media is pumped onto a 5 ml Protein A column (Pharmacia) which has been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 ml of I M Tris buffer, pH 9. The highly purified protein is subsequently desalted into storage buffer as described above for the poly-His tagged proteins. The homogeneity of the proteins is verified by SDS polyacrylamide gel (PEG) electrophoresis and N-terminal amino acid sequencing by Edman degradation.

PRO301, PRO362, PRO356, PRO509 and PRO866 were expressed in baculovirus infected Sf9 insect cells.

Alternatively, a modified baculovirus procedure may be used incorporating high-5 cells. In this procedure, the DNA encoding the desired sequence is amplified with suitable systems, such as Pfu (Stratagene), or fused upstream (5'-of) of an epitope tag contained with a baculovirus expression vector. Such epitope tags include poly-His tags and immunoglobulin tags (like Fc regions of IgG). A variety of plasmids may be employed, including plasmids derived from commercially available plasmids such as plE1-1 (Novagen). The plE 1 -1 and plE 1-2 vectors are designed for constitutive expression of recombinant proteins from the baculovirus iel promoter in stably-transformed insect cells (1). The plasmids differ only in the orientation of the multiple cloning sites and contain all promoter sequences known to be important for ie I-mediated gene expression in uninfected insect cells as well as the hr5 enhancer element. pIE I-1 and pIE 1-2 include the translation initiation site and can be used to produce fusion proteins. Briefly, the desired sequence or the desired portion of the sequence (such as the sequence encoding the extracellular domain of a transmembrane protein) is amplified by PCR with primers complementary to the 5' and 3' regions. The 5' primer may incorporate flanking (selected) restriction enzyme sites. The product is then digested with those selected restriction enzymes and subcloned into the expression vector. For example, derivatives of pIE 1 -1 can include the Fc region of human IgG (pb.PH.IgG) or an 8 histidine (pb.PH.His) tag downstream (3'-of) the desired sequence. Preferably, the vector construct is sequenced for confirmation.

High-5 cells are grown to a confluency of 50% under the conditions of, 27°C, no CO₂, NO pen/strep. For each 150 mm plate, 30 *µ*g of pIE based vector containing the sequence is mixed with 1 ml Ex-Cell medium (Media: Ex-Cell 401 + 1/100 L-Glu JRH Biosciences # 14401-78P (note: this media is light sensitive)), and in a separate tube, 100 µl of CellFectin (CellFECTIN (GibcoBRL #10362-010) (vortexed to mix)) is mixed with 1 ml ofEx-Cell medium. The two solutions are combined and allowed to incubate at room temperature for 15 minutes. 8 ml of Ex-Cell media is added to the 2ml of DNA/CellFECTIN mix and this is layered on high-5 cells that have been washed once with Ex-Cell media. The plate is then incubated in darkness for I hour at room temperature. The DNA/CellFECTIN mix is then aspirated, and the cells are washed once with Ex-Cell to remove excess CellFECTIN, 30 ml of fresh Ex-Cell media is added and the cells are incubated for 3 days at 28°C. The supernatant is harvested and the expression of the sequence in the baculovirus expression vector is determined by batch binding of 1 ml of supematent to 25 ml ofNi ²⁺ -NTA beads (QlAGEN) for histidine tagged proteins or Protein-A Sepharose CL-4B beads (Pharmacia) for IgG tagged proteins followed by SDS-PAGE analysis comparing to a known concentration of protein standard by Coomassie blue staining.

The conditioned media from the transfected cells (0.5 to 3 L) is harvested by centrifugation to remove the cells and filtered through 0.22 micron filters. For the poly-His tagged constructs, the protein comprising the sequence is purified using aNi ²⁺ -NTA column (Qiagen). Before purification, imidazole is added to the conditioned media to a concentration of 5 mM. The conditioned media is pumped onto a 6 ml Ni ²⁺-NTA column equilibrated in 20 mM Hepes, pH 7.4, buffer containing 0.3 M NaCl and 5 mM imidazole at a flow rate of 4-5 ml/min. at 48°C. After loading, the column is washed with additional equilibration buffer and the protein eluted with equilibration buffer containing 0.25 M imidazole. The highly purified protein is then subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc containing) constructs of proteins are purified from the conditioned media as follows. The conditioned media is pumped onto a 5 ml Protein A column (Pharmacia) which has been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 ml of 1 M Tris buffer, pH 9. The highly purified protein is subsequently desalted into storage buffer as described above for the poly-His tagged proteins. The homogeneity of the sequence is assessed by SDS polyacrylamide gels and by N-terminal amino acid sequencing by Edman degradation and other analytical procedures as desired or necessary.

PRO179, PRO221, PRO224, PRO328, PRO301, PRO526, PRO362, and PRO356 were expressed using the above baculovirus procedure employing high-5 cells.

### EXAMPLE 21

### Preparation of Antibodies that Bind PRO179, PRO207, PRO320, PRO219, PRO221, PRO224, PRO328, PRO301, PRO526, PRO362, PRO356, PRO509 or PRO866

This example illustrates preparation of monoclonal antibodies which can specifically bind PRO179, PRO207, PRO320, PRO219, PRO221, PRO224, PRO328, PRO301, PRO526, PRO362, PRO356, PRO509 or PRO866.

Techniques for producing the monoclonal antibodies are known in the art and are described, for instance, in Goding, *supra.* Immunogens that may be employed include purified PRO179, PRO207, PRO320, PRO219, PRO221, PRO224, PRO328, PRO301, PRO526, PRO362, PRO356, PRO509 or PRO866 fusion proteins containing PRO179, PRO207, PRO320, PRO219, PRO221, PRO224, PRO328, PRO301, PRO526, PRO362, PRO356, PRO509 or PRO866 and cells expressing recombinant PRO 179, PRO207, PRO320, PRO219, PRO221, PRO224, PRO328, PRO301, PRO526, PRO362, PRO356, PRO509 or PRO866 on the cell surface. Selection of the immunogen can be made by the skilled artisan without undue experimentation.

Mice, such as Balb/c, are immunized with the PRO179, PRO207, PRO320, PRO219, PRO221, PRO224, PRO328, PRO301, PRO526, PRO362, PRO356, PRO509 or PRO866 immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 or anti-PRO866 antibodies.

After a suitable antibody titer has been detected, the animals "positive" for antibodies can be injected with a final intravenous injection of PRO179, PRO207, PRO320, PRO219, PRO221, PRO224, PRO328, PRO301, PRO526, PRO362, PRO356, PRO509 or PRO866. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO179, PRO207, PRO320, PRO219, PRO221, PRO224, PRO328, PRO301, PRO526, PRO362, PRO356, PRO509 or PRO866. Determination of "positive" hybridoma cells secreting the desired monoclonal antibodies against PRO179, PRO207, PRO320, PRO219, PRO221, PRO224, PRO328, PRO301, PRO526, PRO362, PRO356, PRO509 or PRO866 is within the skill in the art.

The positive hybridoma cells can be injected intraperitoneally into syngeneic Balb/c mice to produce ascites containing the anti-PRO179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 or anti-PRO866 monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 22

### Purification of PRO 179, PRO207, PRO320, PRO219, PRO221, PRO224, PRO328, PRO301, PRO526, PRO362, PRO356, PRO509 or PRO866 Polypeptides Using Specific Antibodies

Native or recombinant PRO179, PRO207, PRO320, PRO219, PRO221, PRO224, PRO328, PRO301, PRO526, PRO362, PRO356, PRO509 or PRO866 polypeptides may be purified by a variety ofstandard techniques in the art of protein purification. For example, pro-PRO179, pro-PRO207, pro-PRO320, pro-PRO219, pro-PRO221, pro-PRO224, pro-PRO328, pro-PRO301, pro-PRO526, pro-PRO362, pro-PRO356, pro-PRO509 or pro-PRO866 polypeptide, mature PRO179, mature PRO207, mature PRO320, mature PRO219, mature PRO22 1 , mature PRO224, mature PRO328, mature PRO301, mature PRO526, mature PRO362, mature PRO356, mature PRO509 or mature PRO866 polypeptide, or pre-PRO179, pre-PRO207, pre-PRO320, pre-PRO219, pre-PRO221, pre-PRO224, pre-PRO328, pre-PRO301, pre-PRO526, pre-PRO362, pre-PRO356, pre-PRO509 or pre-PRO866 polypeptide is purified by immunoaffinity chromatography using antibodies specific for the PRO179, PRO207, PRO320, PRO219, PRO221, PRO224, PRO328, PRO301, PRO526, PRO362, PRO356, PRO509 or PRO866 polypeptide of interest. In general, an immunoaffinity column is constructed by covalently coupling the anti-PRO 179, anti-PRO207, anti-PRO320, anti-PRO219, anti-PRO221, anti-PRO224, anti-PRO328, anti-PRO301, anti-PRO526, anti-PRO362, anti-PRO356, anti-PRO509 or anti-PRO866 polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonalantibodies are prepared from mouse ascites fluid by ammonium sulfate precipitation or chromatography on immobilized Protein A. Partially purified immunoglobulin is covalently attached to a chromatographic resin such as CnBr-activated SEPHAROSE^{TM} (Pharmacia LKB Biotechnology). The antibody is coupled to the resin, the resin is blocked, and the derivative resin is washed according to the manufacturer's instructions.

Such an immunoaffinity column is utilized in the purification of the PRO179, PRO207, PRO320, PRO219, PRO221, PRO224, PRO328, PRO301, PRO526, PRO362, PRO356, PRO509 or PRO866 polypeptide by preparing a fraction from cells containing the PRO 179, PRO207, PRO320, PRO219, PRO221, PRO224, PRO328, PRO301, PRO526, PRO362, PRO356, PRO509 or PRO866 polypeptide in a soluble form. This preparation is derived by solubilization of the whole cell or of a subcellular fraction obtained via differential centrifugation by the addition of detergent or by other methods well known in the art. Alternatively, soluble PRO179, PRO207, PRO320, PRO219, PRO221, PRO224, PRO328, PRO301, PRO526, PRO362, PRO356, PRO509 or PRO866 polypeptide containing a signal sequence may be secreted in useful quantity into the medium in which the cells are grown.

A soluble PRO179, PRO207, PRO320, PRO219, PRO221, PRO224, PRO328, PRO301, PRO526, PRO362, PRO356, PRO509 or PRO866 polypeptide-containing preparation is passed over the immunoaffinity column, and the column is washed under conditions that allow the preferential absorbance of the PRO 179, PRO207, PRO320, PRO219, PRO221, PRO224, PRO328, PRO301, PRO526, PRO362, PRO356, PRO509 or PRO866 polypeptide (e.g., high ionic strength buffers in the presence of detergent). Then, the column is eluted under conditions that disrupt antibody/PRO179, antibody/PRO207, antibody/PRO320, antibody/PRO219, antibody/PRO221, antibody/PRO224,antibody/PRO328,antibody/PRO301 , antibody/PRO526,antibody/PRO362, antibody/PRO356, antibody/PRO509 or antibody/PRO866 polypeptide binding (*e.g.*, a low pH buffer such as approximately pH 2-3, or a high concentration of a chaotrope such as urea or thiocyanate ion), and the PRO179, PRO207, PRO320, PRO219, PRO221, PRO224, PRO328, PRO301, PRO526, PRO362, PRO356, PRO509 or PRO866 polypeptide is collected.

### EXAMPLE 23

### Drug Screening

This invention is particularly useful for screening compounds by using PRO179, PRO207, PRO320, PRO219, PRO221, PRO224, PRO328, PRO301, PRO526, PRO362, PRO356, PRO509 or PRO866 polypeptides or a binding fragment thereof in any of a variety of drug screening techniques. The PRO 179, PRO207, PRO320, PRO219, PRO221, PRO224, PRO328, PRO301, PRO526, PRO362, PRO356, PRO509 or PRO866 polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressingthe PRO179, PRO207, PRO320, PRO219, PRO221, PRO224, PRO328, PRO301, PRO526, PRO362, PRO356, PRO509 or PRO866 polypeptide or fragment. Drugs are screened against such transformed cells in competitive binding assays. Such cells, either in viable or fixed form, can be used for standard binding assays. One may measure, for example, the formation of complexes between a PRO179, PRO207, PRO320, PRO219, PRO221, PRO224, PRO328, PRO301, PRO526, PRO362, PRO356, PRO509 or PRO866 polypeptide or a fragment and the agent being tested. Alternatively, one can examine the diminution in complex formation between the PRO179, PRO207, PRO320, PRO219, PRO221, PRO224, PRO328, PRO301, PRO526, PRO362, PRO356, PRO509 or PRO866 polypeptide and its target cell or target receptors caused by the agent being tested.

Thus, the present invention provides methods ofscreening for drugs or any other agents which can affect a PRO179, PRO207, PRO320, PRO219, PRO221, PRO224, PRO328, PRO301, PRO526, PRO362, PRO356, PRO509 or PRO866 polypeptide-associated disease or disorder. These methods comprise contacting such an agent with a PRO179, PRO207, PRO320, PRO219, PRO221, PRO224, PRO328, PRO301, PRO526, PRO362, PRO356, PRO509 or PRO866 polypeptide or fragment thereof and assaying (i) for the presence of a complex between the agent and the PRO179, PRO207, PRO320, PRO219, PRO221, PRO224, PRO328, PRO301, PRO526, PRO362, PRO356, PRO509 or PRO866 polypeptide or fragment, or (ii) for the presence of a complex between the PRO179, PRO207, PRO320, PRO219, PRO221, PRO224, PRO328, PRO301, PRO526, PRO362, PRO356, PRO509 or PRO866 polypeptide or fragment and the cell, by methods well known in the art. In such competitive binding assays, the PRO179, PRO207, PRO320, PRO219, PRO221, PRO224, PRO328, PRO301, PRO526, PRO362, PRO356, PRO509 or PRO866 polypeptide or fragment is typically labeled. After suitable incubation, the free PRO179, PRO207, PRO320, PRO219, PRO221, PRO224, PRO328, PRO301, PRO526, PRO362, PRO356, PRO509 or PRO866 polypeptide or fragment is separated from that present in bound form, and the amount of free or uncomplexed label is a measure of the ability of the particular agent to bind to the PRO179, PRO207, PRO320, PRO219, PRO221, PRO224, PRO328, PRO301, PRO526, PRO362, PRO356, PRO509 or PRO866 polypeptide or to interfere with the PRO179, PRO207, PRO320, PRO219, PRO221, PRO224, PRO328, PRO301, PRO526, PRO362, PRO356, PRO509 or PRO866 polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO179, PRO207, PRO320, PRO219, PRO221, PRO224, PRO328, PRO301, PRO526, PRO362, PRO356, PRO509 or PRO866 polypeptide, the peptide test compounds are reacted with the PRO179, PRO207, PRO320, PRO219, PRO221, PRO224, PRO328, PRO301, PRO526, PRO362, PRO356, PRO509 or PRO866 polypeptide and washed. Bound PRO179, PRO207, PRO320, PRO219, PRO221, PRO224, PRO328, PRO301, PRO526, PRO362, PRO356, PRO509 or PRO866 polypeptide is detected by methods well known in the art. Purified PRO179, PRO207, PRO320, PRO219, PRO221, PRO224, PRO328, PRO301, PRO526, PRO362, PRO356, PRO509 or PRO866 polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding a PRO179, PRO207, PRO320, PRO219, PRO221, PRO224, PRO328, PRO301, PRO526, PRO362, PRO356, PRO509 or PRO866 polypeptide specifically compete with a test compound for binding to the PRO179, PRO207, PRO320, PRO219, PRO221, PRO224, PRO328, PRO301, PRO526, PRO362, PRO356, PRO509 or PRO866 polypeptide or fragments thereof. In this manner, the antibodies can be used to detect the presence of any peptide which shares one or more antigenic determinants with a PRO179, PRO207, PRO320, PRO219, PRO221, PRO224, PRO328, PRO301, PRO526, PRO362, PRO356, PRO509 or PRO866 polypeptide.

### EXAMPLE 24

### Rational Drug Design

The goal of rational drug design is to produce structural analogs of a biologically active polypeptide of interest (*i.e.,* a PRO179, PRO207, PRO320, PRO219, PRO221, PRO224, PRO328, PRO301, PRO526, PRO362, PRO356, PRO509 or PRO866 polypeptide) or of small molecules with which they interact, e.g., agonists, antagonists, or inhibitors. Any of these examples can be used to fashion drugs which are more active or stable forms of the PRO179, PRO207, PRO320, PRO219, PRO221, PRO224, PRO328, PRO301, PRO526, PRO362, PRO356, PRO509 or PRO866 polypeptide or which enhance or interfere with the function of the PRO179, PRO207, PRO320, PRO219, PRO221, PRO224, PRO328, PRO301, PRO526, PRO362, PRO356, PRO509 or PRO866 polypeptide *in vivo* (*c.f.,* Hodgson, Bio/Technology, 9: 19-21 (1991)).

In one approach, the three-dimensional structure of the PRO179, PRO207, PRO320, PRO219, PRO221, PRO224, PRO328, PRO301, PRO526, PRO362, PRO356, PRO509 or PRO866 polypeptide, or of a PRO179, PRO207, PRO320, PRO219, PRO221, PRO224, PRO328, PRO301, PRO526, PRO362, PRO356, PRO509 or PRO866 polypeptide-inhibitor complex, is determined by x-ray crystallography, by computer modeling or, most typically, by a combination of the two approaches. Both the shape and charges ofthe PRO 179, PRO207, PRO320, PRO219, PRO221, PRO224, PRO328, PRO301, PRO526, PRO362, PRO356, PRO509 or PRO866 polypeptide must be ascertained to elucidate the structure and to determine active site(s) of the molecule. Less often, useful information regarding the structure of the PRO179, PRO207, PRO320, PRO219, PRO221, PRO224, PRO328, PRO301, PRO526, PRO362, PRO356, PRO509 or PRO866 polypeptide may be gained by modeling based on the structure of homologous proteins. In both cases, relevant structural information is used to design analogous PRO179, PRO207, PRO320, PRO219, PRO221, PRO224, PRO328, PRO301, PRO526, PRO362, PRO356, PRO509 or PRO866 polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists of native peptides as shown by Athauda *et al.,* J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO179, PRO207, PRO320, PRO219, PRO221, PRO224, PRO328, PRO301, PRO526, PRO362, PRO356, PRO509 or PRO866 polypeptide may be made available to perform such analytical studies as X-ray crystallography. In addition, knowledge of the PRO179, PRO207, PRO320, PRO219, PRO221, PRO224, PRO328, PRO301, PRO526, PRO362, PRO356, PRO509 or PRO866 polypeptide amino acid sequence provided herein will provide guidance to those employing computer modeling techniques in place of or in addition to x-ray crystallography.

### EXAMPLE 25

### In Vitro Antitumor Assay

The antiproliferative activity of the PRO 179, PRO207, PRO320, PRO219, PRO221, PRO224, PRO328, PRO301, PRO526, PRO362, PRO356, PRO509 and PRO866 polypeptides was determined in the investigational, disease-oriented *in vitro* anti-cancer drug discovery assay of the National Cancer Institute (NCl), using a sulforhodamine B (SRB) dye binding assay essentially as described by Skehan *et al.,* J. Natl. Cancer Inst., 82:1107-1112 (1990). The 60 tumor cell lines employed in this study ("the NCl panel"), as well as conditions for their maintenance and culture *in vitro* have been described by Monks *et al.,* J. Natl. Cancer Inst. 83:757-766 (1991). The purpose of this screen is to initially evaluate the cytotoxic and/or cytostatic activity of the test compounds against different types of tumors (Monks *et al., supra;* Boyd, Cancer: Princ. Pract. Oncol. Update, 3(10):1-12 [1989]).

Cells from approximately 60 human tumor cell lines were harvested with trypsin/EDTA (Gibco), washed once, resuspended in IMEM and their viability was determined. The cell suspensions were added by pipet (100 *µ*l volume) into separate 96-well microtiter plates. The cell density for the 6-day incubation was less than for the 2-day incubation to prevent overgrowth. Inoculates were allowed a preincubation period of 24 hours at 37°C for stabilization. Dilutions at twice the intended test concentration were added at time zero in 100 *µ*l aliquots to the microtiter plate wells (1:2 dilution). Test compounds were evaluated at five half-log dilutions (1000 to 100,000-fold). incubations took place for two days and six days in a 5% CO₂ atmosphere and 100% humidity.

After incubation, the medium was removed and the cells were fixed in 0.1 ml of 10% trichloroacetic acid at 40°C. The plates were rinsed five times with deionized water, dried, stained for 30 minutes with 0.1 ml of 0.4% sulforhodamine B dye (Sigma) dissolved in 1% acetic acid, rinsed four times with 1% acetic acid to remove unbound dye, dried, and the stain was extracted for five minutes with 0.1 ml of 10 mM Tris base [tris(hydroxymethyl)aminomethane], pH 10.5. The absorbance (OD) of sulforhodamine B at 492 nm was measured using a computer-interfaced, 96-well microtiter plate reader.

A test sample is considered positive if it shows at least 40% growth inhibitory effect at one or more concentrations. The results are shown in the following Table 4, where the tumor cell type abbreviations are as follows: NSCL = non-small cell lung carcinoma; CNS = central nervous system

**Table 4**

| Compound | Tumor Cell Type | Designation |
|---|---|---|
| PRO179 | Leukemia | CCRF-CEM |
| PRO179 | Breast | HS 578T |
| PRO179 | NSCL | SR |
| PRO179 | Breast | NCI/ADR-RES |
| PRO179 | Leukemia | HL-60 (TB); SR |
| PRO179 | NSCL | HOP-62; NCI-H460 |
| PRO179 | Breast | MDA-N |
| PRO179 | NSCL | NCI-H522 |
| PRO179 | Colon | COLO 205; HCC-2998 |
| PRO179 | CNS | SF-295 |
| PRO179 | Breast | MDA-MB-435 |
| PRO179 | Prostate | PC-3 |
| PRO179 | Leukemia | MOLT-4 |
| PRO179 | Melanoma | SK-MEL-5; SK-MEL-2 |
| PRO179 | Breast | MDA-MD-435; T-47D |
| PRO179 | Melanoma | MALME-3M |
| PRO179 | NSCL | NCI-H322 |
| PRO179 | Colon | HCT-15 |
| PRO179 | Ovarian | OVCAR-3 |
| PRO179 | NSCL | NCI-H226 |
| PRO179 | Renal | RXF-393 |
| | | |
| PRO207 | Renal | CAKI-1; RXF-393 |
| PRO207 | Leukemia | MOLT-4; SR |
| PRO207 | NSCL | NCl-H322M; NCI-H522 |
| PRO207 | NSCL | HOP-62 |
| PRO207 | Colon | COLO 205 |
| PRO207 | Melanoma | LOX IMVI |
| PRO207 | Ovarian | IGROVI |
| PRO207 | Renal | ACHN |
| PRO207 | Prostate | PC-3 |
| PRO207 | Breast | MDA-MB-231/ATCC |
| | | |
| PRO320 | Leukemia | CCRF-CEM; RPMI-8226 |
| PRO320 | NSCL | HOP62; NCI 11322M |
| | Colon | HCT-116 |
| PRO320 | Renal | SN12C |
| PRO320 | Breast | MDA-N |
| PRO320 | Ovarian | OVCAR-3 |
| PRO320 | Melanoma | MALME-3M |
| | | |
| PRO219 | Leukemia | SR |
| PRO219 | NSCL | NCI-H5222 |
| PRO219 | Breast | MCF7 |
| PRO219 | Leukemia | K-562; RPMI-8226 |
| PRO219 | NSCL | HOP-62; NCl-H322M |
| | NSCL | NCI -H460 |
| PRO219 | Colon | HT29; KM12; HCT-116 |
| PRO219 | CNS | SF-539; U251 |
| PRO219 | Prostate | DU-145 |
| PRO219 | Breast | MDA-N |
| PRO219 | Ovarian | IGROV1 |
| PRO219 | NSCL | NCl-H226 |
| PRO219 | Leukemia | MOLT-4 |
| PRO219 | NSCL | A549/ATCC; EKVX; NCI-H23 |
| PRO219 | Colon | HCC-2998 |
| PRO219 | CNS | SF-295; SNB-19 |
| PRO219 | Melanoma | SK-MEL-2; SK-MEL-5 |
| PRO219 | Melanoma | UACC-257; UACC-62 |
| PRO219 | Ovarian | OCAR-4; SK-OV-3 |
| PRO219 | Renal | 786-0; ACHN; CAKI-1; SN12C |
| PRO219 | Renal | TK-10; UO-31 |
| PRO219 | Breast | NCI/ADR-RES;BT-549;T-47D |
| PRO219 | Breast | MDA-MB-435 |
| | | |
| PRO221 | Leukemia | CCRF-CEM |
| PRO221 | Leukemia | MOLT-4 |
| PRO221 | NSCL | HOP-62 |
| PRO221 | Breast | MDA-N |
| PRO221 | Leukemia | RPMI-8226; SR |
| PRO221 | NSCL | NCl-H460 |
| PRO221 | Colon | HCC-2998 |
| PRO221 | Ovarian | IGROV1 |
| PRO221 | Renal | TK- 10 |
| PRO221 | Breast | MCF7 |
| PRO221 | Leukemia | K-562 |
| PRO221 | Breast | MDA-MB-435 |
| | | |
| PRO224 | Ovarian | OVCAR-4 |
| PRO224 | Renal | RXF 393 |
| PRO224 | Prostate | DU-145 |
| PRO224 | NSCL | HOP-62; NCI-H322M |
| PRO224 | Melanoma | LOX IMVI |
| PRO224 | Ovarian | OVCAR-8 |
| PRO224 | Leukemia | SR |
| PRO224 | NSCL | NCI-H460 |
| PRO224 | CNS | SF-295 |
| PRO224 | Leukemia | RPMI-8226 |
| PRO224 | Breast | BT-549 |
| PRO224 | Leukemia | CCRF-CEM; LH-60 (TB) |
| PRO224 | Colon | HCT-116 |
| PRO224 | Breast | MDA-MB-435 |
| PRO224 | Leukemia | HL-60 (TB) |
| PRO224 | Colon | HCC-2998 |
| PRO224 | Prostate | PC-3 |
| PRO224 | CNS | U251 |
| PRO224 | Colon | HCT-15 |
| PRO224 | CNS | SF-539 |
| PRO224 | Renal | ACHN |
| | | |
| PRO328 | Leukemia | RPM 1-8226 |
| PRO328 | NSCL | A549/ATCC; EKVX; HOP-62 |
| PRO328 | NSCL | NCI-H23; NCI-H322M |
| PRO328 | Colon | HCT-15; KM12 |
| PRO328 | CNS | SF-295;SF-539; SNB-19; U251 |
| PRO328 | Melanoma | M 14; UACC-257; UCAA-62 |
| PRO328 | Renal | 786-0; ACHN |
| PRO328 | Breast | MCF7 |
| PRO328 | Leukemia | SR |
| PRO328 | Colon | NCl-H23 |
| PRO328 | Melanoma | SK-MEL-5 |
| PRO328 | Prostate | DU-145 |
| PRO328 | Melanoma | LOX IMVI |
| PRO328 | Breast | MDA-MB-435 |
| PRO328 | Ovarian | OVCAR-3 |
| PRO328 | Breast | T-47D |
| | | |
| PRO301 | NSCL | NCI-H322M |
| PRO301 | Leukemia | MOLT-4; SR |
| PRO301 | NSCL | A549/ATCC; EKVX; |
| PRO301 | NSCL | NCl-H23; NCl-460; NCl-H226 |
| PRO301 | Colon | COLO 205; HCC-2998; |
| PRO301 | Colon | HCT-15; KM12; HT29; |
| PRO301 | Colon | HCT-116 |
| PRO301 | CNS | SF-268; SF-295; SNB-19 |
| PRO301 | Melanoma | MALME-3M; SK-MEL-2; |
| PRO301 | Melanoma | SK-MEL-5;UACC-257 |
| PRO301 | Melanoma | UACC-62 |
| PRO301 | Ovarian | IGROV 1; OVCAR-4 |
| PRO301 | Ovarian | OVCAR-5 |
| PRO301 | Ovarian | OVCAR-8; SK0OV-3 |
| PRO301 | Renal | ACHN;CAKI-1 ;TK-10; UO-31 |
| PRO301 | Prostate | PC-3; DU-145 |
| PRO301 | Breast | NCI/ADR-RES; HS 578T |
| PRO301 | Breast | MDA-MB-435;MDA-N;T-47D |
| PRO301 | Melanoma | M14 |
| PRO301 | Leukemia | CCRF-CEM;HL-60(TB);K-562 |
| PRO301 | Leukemia | RPMI-8226 |
| PRO301 | Melanoma | LOX IMVI |
| PRO301 | Renal | 786-0; SN12C |
| PRO301 | Breast | MCF7;MDA-MB-231/ATCC |
| PRO301 | Breast | BT-549 |
| PRO301 | NSCL | HOP-62 |
| PRO301 | CNS | SF-539 |
| PRO301 | Ovarian | OVCAR-3 |
| | | |
| PRO526 | NSCL | HOP-62; NCI-H322M |
| PRO526 | Colon | HCT-116 |
| PRO526 | Melanoma | LOX IMVI; SK-MEL-2 |
| PRO526 | Ovarian | OVCAR-3 |
| PRO526 | Prostate | PC-3 |
| PRO526 | NSCL | NCI-H226 |
| PRO526 | CNS | SF-539 |
| PRO526 | Renal | CAKI-1; RXF 393 |
| | | |
| PRO362 | NSCL | NCI-H322M |
| PRO362 | Colon | HCT-116 |
| PRO362 | CNS | SF-295 |
| PRO362 | Melanoma | LOX IMVI |
| PRO362 | Leukemia | MOLT-4; RPMI-8226; SR |
| PRO362 | Colon | COLO 205 |
| PRO362 | Breast | HS 578T; MDA-N |
| PRO362 | Prostate | PC-3 |
| PRO362 | Leukemia | HL-60 (TB); K-562 |
| PRO362 | NSCL | EKVX; NCI-H23 |
| PRO362 | Colon | HCC-2998 |
| PRO362 | CNS | U251 |
| PRO362 | Melanoma | UACC-257; UACC-62 |
| PRO362 | Ovarian | OVCAR-8 |
| PRO362 | Breast | T-47D |
| PRO362 | NSCL | NCI-H522 |
| PRO362 | Renal | RXF 393; UO-31 |
| PRO362 | Breast | MDA-MB-435 |
| PRO362 | NSCL | HOP-62; NCI-H522 |
| PRO362 | Colon | KM12 |
| PRO362 | Melanoma | MALME-3M; SK-MEL-2 |
| PRO362 | Melanoma | SK-MEL-28; SK-MEL-5 |
| PRO362 | Ovarian | OVCAR-3; OVCAR-4 |
| PRO362 | Breast | MCF7 |
| | | |
| PRO356 | Leukemia | CCRF-CEM; MOLT-4; SR |
| PRO356 | NSCL | NCI-H23; NCI-H322M |
| PRO356 | NSCL | NCI-H460 |
| PRO356 | NSCL | A549/ATCC |
| PRO356 | Colon | HCT-116; HCT-15; H29; KM12 |
| PRO356 | CNS | SF-268; SF-295; SF-539 |
| PRO356 | CNS | SNB-19 |
| PRO356 | Melanoma | LOX IMVI; SK-MEL-5 |
| PRO356 | Melanoma | UACC-257 |
| PRO356 | Melanoma | UACC-62 |
| PRO356 | Ovarian | OVCAR-8; OVCAR-5 |
| PRO356 | Renal | SN12C |
| PRO356 | Prostate | DU-145 |
| PRO356 | Leukemia | K-562 |
| PRO356 | Leukemia | HL-60 (TB) |
| PRO356 | Breast | MDA-N |
| PRO356 | NSCL | EKVX; HOP-92 |
| | Colon | COLO 205; SW-620 |
| PRO356 | CNS | SNB-75; U251 |
| PRO356 | Melanoma | M14 |
| PRO356 | Ovarian | IGROV 1; OVCAR-4; |
| PRO356 | Renal | RXF 393 |
| PRO356 | Breast | BT-549 |
| PRO356 | NSCL | NCI-H226 |
| PRO356 | Breast | MDA-MB-435 |
| PRO356 | NSCL | HOP-62 |
| PRO356 | Renal | UO-31 |
| PRO356 | Leukemia | RPMI-8226 |
| | | |
| PRO509 | Leukemia | K-562; MOLT-4 |
| PRO509 | NSCL | HOP-92 |
| PRO509 | Colon | SW-620 |
| PRO509 | CNS | U251 |
| PRO509 | Melanoma | SK-MEL-28 |
| PRO509 | Renal | A498 |
| PRO509 | Breast | MDA-MB-435 |
| PRO509 | Leukemia | RPMI-8226 |
| PRO509 | Melanoma | SK-MEL-2 |
| PRO509 | Ovarian | OVCAR-3 |
| PRO509 | Renal | CAKI-1 |
| | | |
| PRO866 | Leukemia | HL-60 (TB); MOLT-4; SR |
| PRO866 | NSCL | HOP-62 |
| PRO866 | NSCL | HOP-92 |
| PRO866 | Colon | KM12 |
| PRO866 | CNS | SF-295 |
| PRO866 | Ovarian | IGROV I |
| PRO866 | Breast | MDA-MB-435 |
| PRO866 | Melanoma | LOX IMVI |

### Deposit of Material

The following materials have been deposited with the American Type Culture Collection, 10801 University Blvd., Manassas, VA 20110 -2209, USA (ATCC):

| Material | ATCC Dep. No. | Deposit Date |
|---|---|---|
| DNA16451-1078 | 209281 | September 18, 1997 |
| DNA30879-1152 | 209358 | October 10, 1997 |
| DNA32284-1307 | 209670 | March 11, 1998 |
| DNA32290-1164 | 209384 | October 17, 1997 |
| DNA33089-1132 | 209262 | September 16, 1997 |
| DNA33221-1133 | 209263 | September 16, 1997 |
| DNA40587-1231 | 209438 | November 7, 1997 |
| DNA40628-1216 | 209432 | November 7, 1997 |
| DNA44184-1319 | 209704 | March 26, 1998 |
| DNA45416-1251 | 209620 | February 5, 1998 |
| DNA47470-1130-P1 | 209422 | October 28, 1997 |
| DNA53971-1359 | 209750 | April 7, 1998 |

These deposits were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposits will be made available by ATCC under the terms of the Budapest Treaty, and subject to an agreement between Genentech, Inc., and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 U.S.C. § 122 and the Commissioner's rules pursuant thereto (including 37 CFR § 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

### Annex to the application document - subsequently filed sequences linting

Sequence Listing

<110> Genentech, Inc.

<120> METHODS AND COMPOSITIONS FOR INHIBITING NEOPLASTIC
      CELL GROWTH

<140> EP 04007618.4
<141> 1999-12-02

<150> PCT/US99/05028
<151> 1999-03-08

<160> 79

<210> 1
<211> 2042
<212> DNA
<213> Homo Sapien

<400> 1
gcggacgcgt gggtgaaatt gaaaatcaag ataaaaatgt tcacaattaa 50

gctccttctt tttattgttc ctctagttat ttcctccaga attgatcaag 100

acaattcatc atttgattct ctatctccag agccaaaatc aagatttgct 150

atgttagacg atgtaaaaat tttagccaat ggcctccttc agttgggaca 200

tggtcttaaa gactttgtcc ataagacgaa gggccaaatt aatgacatat 250

ttcaaaaact caacatattt gatcagtctt tttatgatct atcgctgcaa 300

accagtgaaa tcaaagaaga agaaaaggaa ctgagaagaa ctacatataa 350

actacaagtc aaaaatgaag aggtaaagaa tatgtcactt gaactcaact 400

caaaacttga aagcctccta gaagaaaaaa ttctacttca acaaaaagtg 450

aaatatttag aagagcaact aactaactta attcaaaatc aacctgaaac 500

tccagaacac ccagaagtaa cttcacttaa aacttttgta gaaaaacaag 550

ataatagcat caaagacctt ctccagaccg tggaagacca atataaacaa 600

ttaaaccaac agcatagtca aataaaagaa atagaaaatc agctcagaag 650

gactagtatt caagaaccca cagaaatttc tctatcttcc aagccaagag 700

caccaagaac tactcccttt cttcagttga atgaaataag aaatgtaaaa 750

catgatggca ttcctgctga atgtaccacc atttataaca gaggtgaaca 800

tacaagtggc atgtatgcca tcagacccag caactctcaa gtttttcatg 850

tctactgtga tgttatatca ggtagtccat ggacattaat tcaacatcga 900

atagatggat cacaaaactt caatgaaacg tgggagaact acaaatatgg 950

ttttgggagg cttgatggag aattttggtt gggcctagag aagatatact 1000

ccatagtgaa gcaatctaat tatgttttac gaattgagtt ggaagactgg 1050

aaagacaaca aacattatat tgaatattct ttttacttgg gaaatcacga 1100

aaccaactat acgctacatc tagttgcgat tactggcaat gtccccaatg 1150

caatcccgga aaacaaagat ttggtgtttt ctacttggga tcacaaagca 1200

aaaggacact tcaactgtcc agagggttat tcaggaggct ggtggtggca 1250

tgatgagtgt ggagaaaaca acctaaatgg taaatataac aaaccaagag 1300

caaaatctaa gccagagagg agaagaggat tatcttggaa gtctcaaaat 1350

ggaaggttat actctataaa atcaaccaaa atgttgatcc atccaacaga 1400

ttcagaaagc tttgaatgaa ctgaggcaat ttaaaggcat atttaaccat 1450

taactcattc caagttaatg tggtctaata atctggtata aatccttaag 1500

agaaagcttg agaaatagat tttttttatc ttaaagtcac tgtctattta 1550

agattaaaca tacaatcaca taaccttaaa gaataccgtt tacatttctc 1600

aatcaaaatt cttataatac tatttgtttt aaattttgtg atgtgggaat 1650

caattttaga tggtcacaat ctagattata atcaataggt gaacttatta 1700

aataactttt ctaaataaaa aatttagaga cttttatttt aaaaggcatc 1750

atatgagcta atatcacaac tttcccagtt taaaaaacta gtactcttgt 1800
taaaactcta aacttgacta aatacagagg actggtaatt gtacagttct 1850

taaatgttgt agtattaatt tcaaaactaa aaatcgtcag cacagagtat 1900

gtgtaaaaat ctgtaataca aatttttaaa ctgatgcttc attttgctac 1950

aaaataattt ggagtaaatg tttgatatga tttatttatg aaacctaatg 2000

aagcagaatt aaatactgta ttaaaataag ttcgctgtct tt 2042

<210> 2
<211> 460
<212> PRT
<213> Homo Sapien

<400> 2
Met Phe Thr Ile Lys Leu Leu Leu Phe Ile Val Pro Leu Val Ile
1               5                   10                  15

Ser Ser Arg Ile Asp Gln Asp Asn Ser Ser Phe Asp Ser Leu Ser
                20                  25                  30

Pro Glu Pro Lys Ser Arg Phe Ala Met Leu Asp Asp Val Lys Ile
                35                  40                  45

Leu Ala Asn Gly Leu Leu Gln Leu Gly His Gly Leu Lys Asp Phe
                50                  55                  60

Val His Lys Thr Lys Gly Gln Ile Asn Asp Ile Phe Gln Lys Leu
                65                  70                  75

Asn Ile Phe Asp Gln Ser Phe Tyr Asp Leu Ser Leu Gln Thr Ser
                80                  85                  90

Glu Ile Lys Glu Glu Glu Lys Glu Leu Arg Arg Thr Thr Tyr Lys
                95                  100                 105

Leu Gln Val Lys Asn Glu Glu Val Lys Asn Met Ser Leu Glu Leu

                110                 115                 120

Asn Ser Lys Leu Glu Ser Leu Leu Glu Glu Lys Ile Leu Leu Gln
                125                 130                 135

Gln Lys Val Lys Tyr Leu Glu Glu Gln Leu Thr Asn Leu Ile Gln
                140                 145                 150

Asn Gln Pro Glu Thr Pro Glu His Pro Glu Val Thr Ser Leu Lys
                155                 160                 165

Thr Phe Val Glu Lys Gln Asp Asn Ser Ile Lys Asp Leu Leu Gln
                170                 175                 180

Thr Val Glu Asp Gln Tyr Lys Gln Leu Asn Gln Gln His Ser Gln
                185                 190                 195

Ile Lys Glu Ile Glu Asn Gln Leu Arg Arg Thr Ser Ile Gln Glu
                200                 205                 210

Pro Thr Glu Ile Ser Leu Ser Ser Lys Pro Arg Ala Pro Arg Thr
                215                 220                 225

Thr Pro Phe Leu Gln Leu Asn Glu Ile Arg Asn Val Lys His Asp
                230                 235                 240
Gly Ile Pro Ala Glu Cys Thr Thr Ile Tyr Asn Arg Gly Glu His
                245                 250                 255

Thr Ser Gly Met Tyr Ala Ile Arg Pro Ser Asn Ser Gln Val Phe
                260                 265                 270

His Val Tyr Cys Asp Val Ile Ser Gly Ser Pro Trp Thr Leu Ile
                275                 280                 285

Gln His Arg Ile Asp Gly Ser Gln Asn Phe Asn Glu Thr Trp Glu
                290                 295                 300

Asn Tyr Lys Tyr Gly Phe Gly Arg Leu Asp Gly Glu Phe Trp Leu
                305                 310                 315

Gly Leu Glu Lys Ile Tyr Ser Ile Val Lys Gln Ser Asn Tyr Val
                320                 325                 330

Leu Arg Ile Glu Leu Glu Asp Trp Lys Asp Asn Lys His Tyr Ile
                335                 340                 345

Glu Tyr Ser Phe Tyr Leu Gly Asn His Glu Thr Asn Tyr Thr Leu
                350                 355                 360

His Leu Val Ala Ile Thr Gly Asn Val Pro Asn Ala Ile Pro Glu
                365                 370                 375

Asn Lys Asp Leu Val Phe Ser Thr Trp Asp His Lys Ala Lys Gly
                380                 385                 390

His Phe Asn Cys Pro Glu Gly Tyr Ser Gly Gly Trp Trp Trp His
                395                 400                 405

Asp Glu Cys Gly Glu Asn Asn Leu Asn Gly Lys Tyr Asn Lys Pro
                410                 415                 420

Arg Ala Lys Ser Lys Pro Glu Arg Arg Arg Gly Leu Ser Trp Lys
                425                 430                 435

Ser Gln Asn Gly Arg Leu Tyr Ser Ile Lys Ser Thr Lys Met Leu

440                 445                 450

Ile His Pro Thr Asp Ser Glu Ser Phe Glu
                455                 460

<210> 3
<211> 20
<212> DNA
<213> Artificial Sequence

<220>
<223> Synthetic Oligonucleotide Probe

<400> 3
ccacgttggc ttgaaattga 20

<210> 4
<211> 50
<212> DNA
<213> Artificial Sequence

<220>
<223> Synthetic Olgonucleotide Probe
<400> 4
cctttagaat tgatcaagac aattcatgat ttgattctct atctccagag 50

<210> 5
<211> 19
<212> DNA
<213> Artificial Sequence

<220>
<223> Synthetic Oligonucleotide Probe

<400> 5
tcgtctaaca tagcaaatc 19

<210> 6
<211> 1353
<212> DNA
<213> Homo Sapien

<400> 6
cgatccctcg ggtcccggga tgggggggcg gtgaggcagg cacagccccc 50

cgcccccatg gccgcccgtc ggagccagag gcggaggggg cgccgggggg 100

agccgggcac cgccctgctg gtcccgctcg cgctgggcct gggcctggcg 150

ctggcctgcc tcggcctcct gctggccgtg gtcagtttgg ggagccgggc 200

atcgctgtcc gcccaggagc ctgcccagga ggagctggtg gcagaggagg 250

accaggaccc gtcggaactg aatccccaga cagaagaaag ccaggatcct 300

gcgcctttcc tgaaccgact agttcggcct cgcagaagtg cacctaaagg 350

ccggaaaaca cgggctcgaa gagcgatcgc agcccattat gaagttcatc 400

cacgacctgg acaggacgga gcgcaggcag gtgtggacgg gacagtgagt 450

ggctgggagg aagccagaat caacagctcc agccctctgc gctacaaccg 500

ccagatcggg gagtttatag tcacccgggc tgggctctac tacctgtact 550

gtcaggtgca ctttgatgag gggaaggctg tctacctgaa gctggacttg 600

ctggtggatg gtgtgctggc cctgcgctgc ctggaggaat tctcagccac 650

tgcggcgagt tccctcgggc cccagctccg cctctgccag gtgtctgggc 700

tgttggccct gcggccaggg tcctccctgc ggatccgcac cctcccctgg 750

gcccatctca aggctgcccc cttcctcacc tacttcggac tcttccaggt 800

tcactgaggg gccctggtct ccccgcagtc gtcccaggct gccggctccc 850

ctcgacagct ctctgggcac ccggtcccct ctgccccacc ctcagccgct 900

ctttgctcca gacctgcccc tccctctaga ggctgcctgg gcctgttcac 950

gtgttttcca tcccacataa atacagtatt cccactctta tcttacaact 1000

cccccaccgc ccactctcca cctcactagc tccccaatcc ctgacccttt 1050

gaggccccca gtgatctcga ctcccccctg gccacagacc cccaggtcat 1100
tgtgttcact gtactctgtg ggcaaggatg ggtccagaag accccacttc 1150

aggcactaag aggggctgga cctggcggca ggaagccaaa gagactgggc 1200

ctaggccagg agttcccaaa tgtgaggggc gagaaacaag acaagctcct 1250

cccttgagaa ttccctgtgg atttttaaaa cagatattat ttttattatt 1300

attgtgacaa aatgttgata aatggatatt aaatagaata agtcataaaa 1350

aaa 1353

<210> 7
<211> 249
<212> PRT
<213> Homo Sapien

<400> 7
Met Ala Ala Arg Arg Ser Gln Arg Arg Arg Gly Arg Arg Gly Glu
1               5                   10                  15

Pro Gly Thr Ala Leu Leu Val Pro Leu Ala Leu Gly Leu Gly Leu
                20                  25                  30

Ala Leu Ala Cys Leu Gly Leu Leu Leu Ala Val Val Ser Leu Gly
                35                  40                  45

Ser Arg Ala Ser Leu Ser Ala Gln Glu Pro Ala Gln Glu Glu Leu
                50                  55                  60

Val Ala Glu Glu Asp Gln Asp Pro Ser Glu Leu Asn Pro Gln Thr
                65                  70                  75

Glu Glu Ser Gln Asp Pro Ala Pro Phe Leu Asn Arg Leu Val Arg
                80                  85                  90

Pro Arg Arg Ser Ala Pro Lys Gly Arg Lys Thr Arg Ala Arg Arg
                95                  100                 105

Ala Ile Ala Ala His Tyr Glu Val His Pro Arg Pro Gly Gln Asp
                110                 115                 120

Gly Ala Gln Ala Gly Val Asp Gly Thr Val Ser Gly Trp Glu Glu
                125                 130                 135

Ala Arg Ile Asn Ser Ser Ser Pro Leu Arg Tyr Asn Arg Gln Ile
                140                 145                 150

Gly Glu Phe Ile Val Thr Arg Ala Gly Leu Tyr Tyr Leu Tyr Cys
                155                 160                 165

Gln Val His Phe Asp Glu Gly Lys Ala Val Tyr Leu Lys Leu Asp
                170                 175                 180

Leu Leu Val Asp Gly Val Leu Ala Leu Arg Cys Leu Glu Glu Phe
                185                 190                 195

Ser Ala Thr Ala Ala Ser Ser Leu Gly Pro Gln Leu Arg Leu Cys
                200                 205                 210

Gln Val Ser Gly Leu Leu Ala Leu Arg Pro Gly Ser Ser Leu Arg
                215                 220                 225

Ile Arg Thr Leu Pro Trp Ala His Leu Lys Ala Ala Pro Phe Leu
                230                 235                 240

Thr Tyr Phe Gly Leu Phe Gln Val His
                245             249

<210> 8
<211> 50
<212> DNA
<213> Artificial Sequence

<220>
<223> Synthetic Oligonucleotide Probe

<400> 8
ccagccctct gcgctacaac cgccagatcg gggagtttat agtcacccgg 50

<210> 9
<211> 2260
<212> DNA
<213> Homo Sapien

<220>
<221> unsure
<222> 2009, 2026, 2033, 2055, 2074, 2078, 2086
<223> unknown base

<400> 9
cggacgcgtg ggtgcgagtg gagcggagga cccgagcggc tgaggagaga 50

ggaggcggcg gcttagctgc tacggggtcc ggccggcgcc ctcccgaggg 100

gggctcagga ggaggaagga ggacccgtgc gagaatgcct ctgccctgga 150

gccttgcgct cccgctgctg ctctcctggg tggcaggtgg tttcgggaac 200

gcggccagtg caaggcatca cgggttgtta gcatcggcac gtcagcctgg 250

ggtctgtcac tatggaacta aactggcctg ctgctacggc tggagaagaa 300

acagcaaggg agtctgtgaa gctacatgcg aacctggatg taagtttggt 350

gagtgcgtgg gaccaaacaa atgcagatgc tttccaggat acaccgggaa 400

aacctgcagt caagatgtga atgagtgtgg aatgaaaccc cggccatgcc 450

aacacagatg tgtgaataca cacggaagct acaagtgctt ttgcctcagt 500

ggccacatgc tcatgccaga tgctacgtgt gtgaactcta ggacatgtgc 550

catgataaac tgtcagtaca gctgtgaaga cacagaagaa gggccacagt 600

gcctgtgtcc atcctcagga ctccgcctgg ccccaaatgg aagagactgt 650

ctagatattg atgaatgtgc ctctggtaaa gtcatctgtc cctacaatcg 700

aagatgtgtg aacacatttg gaagctacta ctgcaaatgt cacattggtt 750

tcgaactgca atatatcagt ggacgatatg actgtataga tataaatgaa 800

tgtactatgg atagccatac gtgcagccac catgccaatt gcttcaatac 850

ccaagggtcc ttcaagtgta aatgcaagca gggatataaa ggcaatggac 900
ttcggtgttc tgctatccct gaaaattctg tgaaggaagt cctcagagca 950

cctggtacca tcaaagacag aatcaagaag ttgcttgctc acaaaaacag 1000

catgaaaaag aaggcaaaaa ttaaaaatgt taccccagaa cccaccagga 1050

ctcctacccc taaggtgaac ttgcagccct tcaactatga agagatagtt 1100

tccagaggcg ggaactctca tggaggtaaa aaagggaatg aagagaaatg 1150

aaagaggggc ttgaggatga gaaaagagaa gagaaagccc tgaagaatga 1200

catagaggag cgaagcctgc gaggagatgt gtttttccct aaggtgaatg 1250

aagcaggtga attcggcctg attctggtcc aaaggaaagc gctaacttcc 1300

aaactggaac ataaagattt aaatatctcg gttgactgca gcttcaatca 1350

tgggatctgt gactggaaac aggatagaga agatgatttt gactggaatc 1400

ctgctgatcg agataatgct attggcttct atatggcagt tccggccttg 1450

gcaggtcaca agaaagacat tggccgattg aaacttctcc tacctgacct 1500

gcaaccccaa agcaacttct gtttgctctt tgattaccgg ctggccggag 1550

acaaagtcgg gaaacttcga gtgtttgtga aaaacagtaa caatgccctg 1600

gcatgggaga agaccacgag tgaggatgaa aagtggaaga cagggaaaat 1650

tcagttgtat caaggaactg atgctaccaa aagcatcatt tttgaagcag 1700

aacgtggcaa gggcaaaacc ggcgaaatcg cagtggatgg cgtcttgctt 1750

gtttcaggct tatgtccaga tagcctttta tctgtggatg actgaatgtt 1800

actatcttta tatttgactt tgtatgtcag ttccctggtt tttttgatat 1850

tgcatcatag gacctctggc attttagaat tactagctga aaaattgtaa 1900

tgtaccaaca gaaatattat tgtaagatgc ctttcttgta taagatatgc 1950

caatatttgc tttaaatatc atatcactgt atcttctcag tcatttctga 2000

atctttccnc attatattat aaaatntgga aangtcagtt tatctcccct 2050

cctcngtata tctgatttgt atangtangt tgatgngctt ctctctacaa 2100

catttctaga aaatagaaaa aaaagcacag agaaatgttt aactgtttga 2150

ctcttatgat acttcttgga aactatgaca tcaaagatag acttttgcct 2200

aagtggctta gctgggtctt tcatagccaa acttgtatat ttaattcttt 2250

gtaataataa 2260

<210> 10
<211> 338
<212> PRT
<213> Homo Sapien

<400> 10
Met Pro Leu Pro Trp Ser Leu Ala Leu Pro Leu Leu Leu Ser Trp
1               5                   10                  15

Val Ala Gly Gly Phe Gly Asn Ala Ala Ser Ala Arg His His Gly
                20                  25                  30

Leu Leu Ala Ser Ala Arg Gln Pro Gly Val Cys His Tyr Gly Thr
                35                  40                  45

Lys Leu Ala Cys Cys Tyr Gly Trp Arg Arg Asn Ser Lys Gly Val
                50                  55                  60

Cys Glu Ala Thr Cys Glu Pro Gly Cys Lys Phe Gly Glu Cys Val
                65                  70                  75

Gly Pro Asn Lys Cys Arg Cys Phe Pro Gly Tyr Thr Gly Lys Thr
                80                  85                  90

Cys Ser Gln Asp Val Asn Glu Cys Gly Met Lys Pro Arg Pro Cys
                95                  100                 105

Gln His Arg Cys Val Asn Thr His Gly Ser Tyr Lys Cys Phe Cys
                110                 115                 120

Leu Ser Gly His Met Leu Met Pro Asp Ala Thr Cys Val Asn Ser
                125                 130                 135

Arg Thr Cys Ala Met Ile Asn Cys Gln Tyr Ser Cys Glu Asp Thr
                140                 145                 150

Glu Glu Gly Pro Gln Cys Leu Cys Pro Ser Ser Gly Leu Arg Leu
                155                 160                 165

Ala Pro Asn Gly Arg Asp Cys Leu Asp Ile Asp Glu Cys Ala Ser
                170                 175                 180

Gly Lys Val Ile Cys Pro Tyr Asn Arg Arg Cys Val Asn Thr Phe
                185                 190                 195

Gly Ser Tyr Tyr Cys Lys Cys His Ile Gly Phe Glu Leu Gln Tyr
                200                 205                 210

Ile Ser Gly Arg Tyr Asp Cys Ile Asp Ile Asn Glu Cys Thr Met
                215                 220                 225

Asp Ser His Thr Cys Ser His His Ala Asn Cys Phe Asn Thr Gln
                230                 235                 240

Gly Ser Phe Lys Cys Lys Cys Lys Gln Gly Tyr Lys Gly Asn Gly

                245                 250                 255

Leu Arg Cys Ser Ala Ile Pro Glu Asn Ser Val Lys Glu Val Leu
                260                 265                 270

Arg Ala Pro Gly Thr Ile Lys Asp Arg Ile Lys Lys Leu Leu Ala
                275                 280                 285

His Lys Asn Ser Met Lys Lys Lys Ala Lys Ile Lys Asn Val Thr
                290                 295                 300

Pro Glu Pro Thr Arg Thr Pro Thr Pro Lys Val Asn Leu Gln Pro
                305                 310                 315
Phe Asn Tyr Glu Glu Ile Val Ser Arg Gly Gly Asn Ser His Gly
                320                 325                 330

Gly Lys Lys Gly Asn Glu Glu Lys
335 338

<210> 11
<211> 22
<212> DNA
<213> Artificial Sequence

<220>
<223> Synthetic Oligonucleotide Probe

<400> 11
cctcagtggc cacatgctca tg 22

<210> 12
<211> 24
<212> DNA
<213> Artificial Sequence

<220>
<223> Synthetic Oligonucleotide Probe

<400> 12
ggctgcacgt atggctatcc atag 24

<210> 13
<211> 50
<212> DNA
<213> Artificial Sequence

<220>
<223> Synthetic Oligonucleotide Probe

<400> 13
gataaactgt cagtacagct gtgaagacac agaagaaggg ccacagtgcc 50

<210> 14
<211> 3449
<212> DNA
<213> Homo Sapien

<400> 14
acttggagca agcggcggcg gcggagacag aggcagaggc agaagctggg 50

gctccgtcct cgcctcccac gagcgatccc cgaggagagc cgcggccctc 100

ggcgaggcga agaggccgac gaggaagacc cgggtggctg cgcccctgcc 150

tcgcttccca ggcgccggcg gctgcagcct tgcccctctt gctcgccttg 200

aaaatggaaa agatgctcgc aggctgcttt ctgctgatcc tcggacagat 250
cgtcctcctc cctgccgagg ccagggagcg gtcacgtggg aggtccatct 300
ctaggggcag acacgctcgg acccacccgc agacggccct tctggagagt 350
tcctgtgaga acaagcgggc agacctggtt ttcatcattg acagctctcg 400
cagtgtcaac acccatgact atgcaaaggt caaggagttc atcgtggaca 450
tcttgcaatt cttggacatt ggtcctgatg tcacccgagt gggcctgctc 500
caatatggca gcactgtcaa gaatgagttc tccctcaaga ccttcaagag 550
gaagtccgag gtggagcgtg ctgtcaagag gatgcggcat ctgtccacgg 600
gcaccatgac tgggctggcc atccagtatg ccctgaacat cgcattctca 650
gaagcagagg gggcccggcc cctgagggag aatgtgccac gggtcataat 700
gatcgtgaca gatgggagac ctcaggactc cgtggccgag gtggctgcta 750
aggcacggga cacgggcatc ctaatctttg ccattggtgt gggccaggta 800
gacttcaaca ccttgaagtc cattgggagt gagccccatg aggaccatgt 850
cttccttgtg gccaatttca gccagattga gacgctgacc tccgtgttcc 900
agaagaagtt gtgcacggcc cacatgtgca gcaccctgga gcataactgt 950
gcccacttct gcatcaacat ccctggctca tacgtctgca ggtgcaaaca 1000
aggctacatt ctcaactcgg atcagacgac ttgcagaatc caggatctgt 1050
gtgccatgga ggaccacaac tgtgagcagc tctgtgtgaa tgtgccgggc 1100
tccttcgtct gccagtgcta cagtggctac gccctggctg aggatgggaa 1150
gaggtgtgtg gctgtggact actgtgcctc agaaaaccac ggatgtgaac 1200
atgagtgtgt aaatgctgat ggctcctacc tttgccagtg ccatgaagga 1250
tttgctctta acccagatga aaaaacgtgc acaaggatca actactgtgc 1300
actgaacaaa ccgggctgtg agcatgagtg cgtcaacatg gaggagagct 1350
actactgccg ctgccaccgt ggctacactc tggaccccaa tggcaaaacc 1400
tgcagccgag tggaccactg tgcacagcag gaccatggct gtgagcagct 1450
gtgtctgaac acggaggatt ccttcgtctg ccagtgctca gaaggcttcc 1500
tcatcaacga ggacctcaag acctgctccc gggtggatta ctgcctgctg 1550
agtgaccatg gttgtgaata ctcctgtgtc aacatggaca gatcctttgc 1600
ctgtcagtgt cctgagggac acgtgctccg cagcgatggg aagacgtgtg 1650
caaaattgga ctcttgtgct ctgggggacc acggttgtga acattcgtgt 1700
gtaagcagtg aagattcgtt tgtgtgccag tgctttgaag gttatatact 1750
ccgtgaagat ggaaaaacct gcagaaggaa agatgtctgc caagctatag 1800

accatggctg tgaacacatt tgtgtgaaca gtgacgactc atacacgtgc 1850

gagtgcttgg agggattccg gctcgctgag gatgggaaac gctgccgaag 1900

gaaggatgtc tgcaaatcaa cccaccatgg ctgcgaacac atttgtgtta 1950

ataatgggaa ttcctacatc tgcaaatgct cagagggatt tgttctagct 2000

gaggacggaa gacggtgcaa gaaatgcact gaaggcccaa ttgacctggt 2050
ctttgtgatc gatggatcca agagtcttgg agaagagaat tttgaggtcg 2100

tgaagcagtt tgtcactgga attatagatt ccttgacaat ttcccccaaa 2150

gccgctcgag tggggctgct ccagtattcc acacaggtcc acacagagtt 2200

cactctgaga aacttcaact cagccaaaga catgaaaaaa gccgtggccc 2250

acatgaaata catgggaaag ggctctatga ctgggctggc cctgaaacac 2300

atgtttgaga gaagttttac ccaaggagaa ggggccaggc ccctttccac 2350

aagggtgccc agagcagcca ttgtgttcac cgacggacgg gctcaggatg 2400

acgtctccga gtgggccagt aaagccaagg ccaatggtat cactatgtat 2450

gctgttgggg taggaaaagc cattgaggag gaactacaag agattgcctc 2500

tgagcccaca aacaagcatc tcttctatgc cgaagacttc agcacaatgg 2550

atgagataag tgaaaaactc aagaaaggca tctgtgaagc tctagaagac 2600

tccgatggaa gacaggactc tccagcaggg gaactgccaa aaacggtcca 2650

acagccaaca gaatctgagc cagtcaccat aaatatccaa gacctacttt 2700

cctgttctaa ttttgcagtg caacacagat atctgtttga agaagacaat 2750

cttttacggt ctacacaaaa gctttcccat tcaacaaaac cttcaggaag 2800

ccctttggaa gaaaaacacg atcaatgcaa atgtgaaaac cttataatgt 2850

tccagaacct tgcaaacgaa gaagtaagaa aattaacaca gcgcttagaa 2900

gaaatgacac agagaatgga agccctggaa aatcgcctga gatacagatg 2950

aagattagaa atcgcgacac atttgtagtc attgtatcac ggattacaat 3000

gaacgcagtg cagagcccca aagctcaggc tattgttaaa tcaataatgt 3050

tgtgaagtaa aacaatcagt actgagaaac ctggtttgcc acagaacaaa 3100

gacaagaagt atacactaac ttgtataaat ttatctagga aaaaaatcct 3150

tcagaattct aagatgaatt taccaggtga gaatgaataa gctatgcaag 3200

gtattttgta atatactgtg gacacaactt gcttctgcct catcctgcct 3250

tagtgtgcaa tctcatttga ctatacgata aagtttgcac agtcttactt 3300

ctgtagaaca ctggccatag gaaatgctgt ttttttgtac tggactttac 3350

cttgatatat gtatatggat gtatgcataa aatcatagga catatgtact 3400

tgtggaacaa gttggatttt ttatacaata ttaaaattca ccacttcag 3449

<210> 15
<211> 915
<212> PRT
<213> Homo Sapien

<400> 15

<210> 16
<211> 23
<212> DNA
<213> Artificial Sequence

<220>
<223> Synthetic Oligonucleotide Probe

<400> 16
 gtgaccctgg ttgtgaatac tcc 23

<210> 17
<211> 22
<212> DNA
<213> Artificial Sequence

<220>
<223> Synthetic Oligonucleotide Probe

<400> 17
 acagccatgg tctatagctt gg 22

<210> 18
<211> 45
<212> DNA
<213> Artificial Sequence

<220>
<223> Synthetic Oligonucleotide Probe

<400> 18
 gcctgtcagt gtcctgaggg acacgtgctc cgcagcgatg ggaag 45

<210> 19
<211> 1305
<212> DNA
<213> Homo Sapien

<400> 19
 gcccgggact ggcgcaaggt gcccaagcaa ggaaagaaat aatgaagaga 50

 cacatgtgtt agctgcagcc ttttgaaaca cgcaagaagg aaatcaatag 100

 tgtggacagg gctggaacct ttaccacgct tgttggagta gatgaggaat 150

 gggctcgtga ttatgctgac attccagcat gaatctggta gacctgtggt 200

 taacccgttc cctctccatg tgtctcctcc tacaaagttt tgttcttatg 250

 atactgtgct ttcattctgc cagtatgtgt cccaagggct gtctttgttc 300

 ttcctctggg ggtttaaatg tcacctgtag caatgcaaat ctcaaggaaa 350

 tacctagaga tcttcctcct gaaacagtct tactgtatct ggactccaat 400

 cagatcacat ctattcccaa tgaaattttt aaggacctcc atcaactgag 450

 agttctcaac ctgtccaaaa atggcattga gtttatcgat gagcatgcct 500

 tcaaaggagt agctgaaacc ttgcagactc tggacttgtc cgacaatcgg 550

 attcaaagtg tgcacaaaaa tgccttcaat aacctgaagg ccagggccag 600

 aattgccaac aacccctggc actgcgactg tactctacag caagttctga 650

 ggagcatggc gtccaatcat gagacagccc acaacgtgat ctgtaaaacg 700

 tccgtgttgg atgaacatgc tggcagacca ttcctcaatg ctgccaacga 750

 cgctgacctt tgtaacctcc ctaaaaaaac taccgattat gccatgctgg 800

tcaccatgtt tggctggttc actatggtga tctcatatgt ggtatattat 850

gtgaggcaaa atcaggagga tgcccggaga cacctcgaat acttgaaatc 900

cctgccaagc aggcagaaga aagcagatga acctgatgat attagcactg 950

tggtatagtg tccaaactga ctgtcattga gaaagaaaga aagtagtttg 1000

cgattgcagt agaaataagt ggtttacttc tcccatccat tgtaaacatt 1050

tgaaactttg tatttcagtt ttttttgaat tatgccactg ctgaactttt 1100

aacaaacact acaacataaa taatttgagt ttaggtgatc caccccttaa 1150

ttgtaccccc gatggtatat ttctgagtaa gctactatct gaacattagt 1200

tagatccatc tcactattta ataatgaaat ttattttttt aatttaaaag 1250

caaataaaag cttaactttg aaccatggga aaaaaaaaaa aaaaaaaaaa 1300

aaaca 1305

<210> 20
<211> 259
<212> PRT
<213> Homo Sapien

<400> 20
Met Asn Leu Val Asp Leu Trp Leu Thr Arg Ser Leu Ser Met Cys
1               5                   10                  15

Leu Leu Leu Gln Ser Phe Val Leu Met Ile Leu Cys Phe His Ser
                20                  25                  30

Ala Ser Met Cys Pro Lys Gly Cys Leu Cys Ser Ser Ser Gly Gly
                35                  40                  45

Leu Asn Val Thr Cys Ser Asn Ala Asn Leu Lys Glu Ile Pro Arg
                50                  55                  60

Asp Leu Pro Pro Glu Thr Val Leu Leu Tyr Leu Asp Ser Asn Gln
                65                  70                  75

Ile Thr Ser Ile Pro Asn Glu Ile Phe Lys Asp Leu His Gln Leu
                80                  85                  90

Arg Val Leu Asn Leu Ser Lys Asn Gly Ile Glu Phe Ile Asp Glu
                95                  100                 105

His Ala Phe Lys Gly Val Ala Glu Thr Leu Gln Thr Leu Asp Leu
                110                 115                 120

Ser Asp Asn Arg Ile Gln Ser Val His Lys Asn Ala Phe Asn Asn
                125                 130                 135

Leu Lys Ala Arg Ala Arg Ile Ala Asn Asn Pro Trp His Cys Asp
                140                 145                 150

Cys Thr Leu Gln Gln Val Leu Arg Ser Met Ala Ser Asn His Glu
                155                 160                 165

Thr Ala His Asn Val Ile Cys Lys Thr Ser Val Leu Asp Glu His
                170                 175                 180

Ala Gly Arg Pro Phe Leu Asn Ala Ala Asn Asp Ala Asp Leu Cys
                185                 190                 195

Asn Leu Pro Lys Lys Thr Thr Asp Tyr Ala Met Leu Val Thr Met
                200                 205                 210

Phe Gly Trp Phe Thr Met Val Ile Ser Tyr Val Val Tyr Tyr Val
                215                 220                 225

Arg Gln Asn Gln Glu Asp Ala Arg Arg His Leu Glu Tyr Leu Lys
                230                 235                 240

Ser Leu Pro Ser Arg Gln Lys Lys Ala Asp Glu Pro Asp Asp Ile
                245                 250                 255

Ser Thr Val Val
            259

<210> 21
<211> 22
<212> DNA
<213> Artificial Sequence

<220>
<223> Synthetic Oligonucleotide Probe

<400> 21
ccatgtgtct cctcctacaa ag 22

<210> 22
<211> 23
<212> DNA
<213> Artificial Sequence

<220>
<223> Synthetic Oligonucleotide Probe

<400> 22
gggaatagat gtgatctgat tgg 23

<210> 23
<211> 50
<212> DNA
<213> Artificial Sequence

<220>
<223> Synthetic Oligonucleotide Probe

<400> 23
cacctgtagc aatgcaaatc tcaaggaaat acctagagat cttcctcctg 50

<210> 24
<211> 1210
<212> DNA
<213> Homo Sapien

<400> 24
cagcgcgtgg ccggcgccgc tgtggggaca gcatgagcgg cggttggatg 50

gcgcaggttg gagcgtggcg aacaggggct ctgggcctgg cgctgctgct 100
gctgctcggc ctcggactag gcctggaggc cgccgcgagc ccgctttcca 150

ccccgacctc tgcccaggcc gcaggcccca gctcaggctc gtgcccaccc 200

accaagttcc agtgccgcac cagtggctta tgcgtgcccc tcacctggcg 250

ctgcgacagg gacttggact gcagcgatgg cagcgatgag gaggagtgca 300

ggattgagcc atgtacccag aaagggcaat gcccaccgcc ccctggcctc 350

ccctgcccct gcaccggcgt cagtgactgc tctgggggaa ctgacaagaa 400

actgcgcaac tgcagccgcc tggcctgcct agcaggcgag ctccgttgca 450

cgctgagcga tgactgcatt ccactcacgt ggcgctgcga cggccaccca 500

gactgtcccg actccagcga cgagctcggc tgtggaacca atgagatcct 550

cccggaaggg gatgccacaa ccatggggcc ccctgtgacc ctggagagtg 600

tcacctctct caggaatgcc acaaccatgg ggccccctgt gaccctggag 650

agtgtcccct ctgtcgggaa tgccacatcc tcctctgccg gagaccagtc 700

tggaagccca actgcctatg gggttattgc agctgctgcg gtgctcagtg 750

caagcctggt caccgccacc ctcctccttt tgtcctggct ccgagcccag 800

gagcgcctcc gcccactggg gttactggtg gccatgaagg agtccctgct 850

gctgtcagaa cagaagacct cgctgccctg aggacaagca cttgccacca 900

ccgtcactca gccctgggcg tagccggaca ggaggagagc agtgatgcgg 950

atgggtaccc gggcacacca gccctcagag acctgagttc ttctggccac 1000

gtggaacctc gaacccgagc tcctgcagaa gtggccctgg agattgaggg 1050

tccctggaca ctccctatgg agatccgggg agctaggatg gggaacctgc 1100

cacagccaga actgaggggc tggccccagg cagctcccag ggggtagaac 1150

ggccctgtgc ttaagacact ccctgctgcc ccgtctgagg gtggcgatta 1200

aagttgcttc 1210

<210> 25
<211> 282
<212> PRT
<213> Homo Sapien

<400> 25
Met Ser Gly Gly Trp Met Ala Gln Val Gly Ala Trp Arg Thr Gly
1               5                   10                  15

Ala Leu Gly Leu Ala Leu Leu Leu Leu Leu Gly Leu Gly Leu Gly
                20                  25                  30

Leu Glu Ala Ala Ala Ser Pro Leu Ser Thr Pro Thr Ser Ala Gln
                35                  40                  45

Ala Ala Gly Pro Ser Ser Gly Ser Cys Pro Pro Thr Lys Phe Gln
                50                  55                  60
Cys Arg Thr Ser Gly Leu Cys Val Pro Leu Thr Trp Arg Cys Asp
                65                  70                  75

Arg Asp Leu Asp Cys Ser Asp Gly Ser Asp Glu Glu Glu Cys Arg
                80                  85                  90

Ile Glu Pro Cys Thr Gln Lys Gly Gln Cys Pro Pro Pro Pro Gly

95                 100                 105

Leu Pro Cys Pro Cys Thr Gly Val Ser Asp Cys Ser Gly Gly Thr
                110                 115                 120

Asp Lys Lys Leu Arg Asn Cys Ser Arg Leu Ala Cys Leu Ala Gly
                125                 130                 135

Glu Leu Arg Cys Thr Leu Ser Asp Asp Cys Ile Pro Leu Thr Trp
                140                 145                 150

Arg Cys Asp Gly His Pro Asp Cys Pro Asp Ser Ser Asp Glu Leu
                155                 160                 165

Gly Cys Gly Thr Asn Glu Ile Leu Pro Glu Gly Asp Ala Thr Thr
                170                 175                 180

Met Gly Pro Pro Val Thr Leu Glu Ser Val Thr Ser Leu Arg Asn
                185                 190                 195

Ala Thr Thr Met Gly Pro Pro Val Thr Leu Glu Ser Val Pro Ser
                200                 205                 210

Val Gly Asn Ala Thr Ser Ser Ser Ala Gly Asp Gln Ser Gly Ser
                215                 220                 225

Pro Thr Ala Tyr Gly Val Ile Ala Ala Ala Ala Val Leu Ser Ala
                230                 235                 240

Ser Leu Val Thr Ala Thr Leu Leu Leu Leu Ser Trp Leu Arg Ala
                245                 250                 255

Gln Glu Arg Leu Arg Pro Leu Gly Leu Leu Val Ala Met Lys Glu
                260                 265                 270

Ser Leu Leu Leu Ser Glu Gln Lys Thr Ser Leu Pro
                275                 280     282

<210> 26
<211> 24
<212> DNA
<213> Artificial Sequence

<220>
<223> Synthetic Oligonucleotide Probe

<400> 26
aagttccagt gccgcaccag tggc 24

<210> 27
<211> 24
<212> DNA
<213> Artificial Sequence

<220>
<223> Synthetic Oligonucleotide Probe
<400> 27
ttggttccac agccgagctc gtcg 24

<210> 28
<211> 50
<212> DNA
<213> Artificial Sequence

<220>

<223> Synthetic Oligonucleotide Probe

<400> 28
gaggaggagt gcaggattga gccatgtacc cagaaagggc aatgcccacc 50

<210> 29
<211> 1875
<212> DNA
<213> Homo Sapien

<400> 29
gacggctggc caccatgcac ggctcctgca gtttcctgat gcttctgctg 50

ccgctactgc tactgctggt ggccaccaca ggccccgttg gagccctcac 100

agatgaggag aaacgtttga tggtggagct gcacaacctc taccgggccc 150

aggtatcccc gacggcctca gacatgctgc acatgagatg ggacgaggag 200

ctggccgcct tcgccaaggc ctacgcacgg cagtgcgtgt ggggccacaa 250

caaggagcgc gggcgccgcg gcgagaatct gttcgccatc acagacgagg 300

gcatggacgt gccgctggcc atggaggagt ggcaccacga gcgtgagcac 350

tacaacctca gcgccgccac ctgcagccca ggccagatgt gcggccacta 400

cacgcaggtg gtatgggcca agacagagag gatcggctgt ggttcccact 450

tctgtgagaa gctccagggt gttgaggaga ccaacatcga attactggtg 500

tgcaactatg agcctccggg gaacgtgaag gggaaacggc cctaccagga 550

ggggactccg tgctcccaat gtccctctgg ctaccactgc aagaactccc 600

tctgtgaacc catcggaagc ccggaagatg ctcaggattt gccttacctg 650

gtaactgagg ccccatcctt ccgggcgact gaagcatcag actctaggaa 700

aatgggtact ccttcttccc tagcaacggg gattccggct ttcttggtaa 750

cagaggtctc aggctccctg gcaaccaagg ctctgcctgc tgtggaaacc 800

caggccccaa cttccttagc aacgaaagac ccgccctcca tggcaacaga 850

ggctccacct tgcgtaacaa ctgaggtccc ttccattttg gcagctcaca 900

gcctgccctc cttggatgag gagccagtta ccttccccaa atcgacccat 950

gttcctatcc caaaatcagc agacaaagtg acagacaaaa caaaagtgcc 1000

ctctaggagc ccagagaact ctctggaccc caagatgtcc ctgacagggg 1050

caagggaact cctaccccat gcccaggagg aggctgaggc tgaggctgag 1100
ttgcctcctt ccagtgaggt cttggcctca gtttttccag cccaggacaa 1150

gccaggtgag ctgcaggcca cactggacca cacggggcac acctcctcca 1200

agtccctgcc caatttcccc aatacctctg ccaccgctaa tgccacgggt 1250

gggcgtgccc tggctctgca gtcgtccttg ccaggtgcag agggccctga 1300

caagcctagc gttgtgtcag ggctgaactc gggccctggt catgtgtggg 1350

gccctctcct gggactactg ctcctgcctc ctctggtgtt ggctggaatc 1400

ttctgaatgg gataccactc aaagggtgaa gaggtcagct gtcctcctgt 1450

catcttcccc accctgtccc cagcccctaa acaagatact tcttggttaa 1500

ggccctccgg aagggaaagg ctacggggca tgtgcctcat cacaccatcc 1550

atcctggagg cacaaggcct ggctggctgc gagctcagga ggccgcctga 1600

ggactgcaca ccgggcccac acctctcctg cccctccctc ctgagtcctg 1650

ggggtgggag gatttgaggg agctcactgc ctacctggcc tggggctgtc 1700

tgcccacaca gcatgtgcgc tctccctgag tgcctgtgta gctggggatg 1750

gggattccta ggggcagatg aaggacaagc cccactggag tggggttctt 1800

tgagtggggg aggcagggac gagggaagga aagtaactcc tgactctcca 1850

ataaaaacct gtccaacctg tgaaa 1875

<210> 30
<211> 463
<212> PRT
<213> Homo Sapien

<400> 30
Met His Gly Ser Cys Ser Phe Leu Met Leu Leu Leu Pro Leu Leu
1               5                   10                  15

Leu Leu Leu Val Ala Thr Thr Gly Pro Val Gly Ala Leu Thr Asp
                20                  25                  30

Glu Glu Lys Arg Leu Met Val Glu Leu His Asn Leu Tyr Arg Ala
                35                  40                  45

Gln Val Ser Pro Thr Ala Ser Asp Met Leu His Met Arg Trp Asp
                50                  55                  60

Glu Glu Leu Ala Ala Phe Ala Lys Ala Tyr Ala Arg Gln Cys Val
                65                  70                  75

Trp Gly His Asn Lys Glu Arg Gly Arg Arg Gly Glu Asn Leu Phe
                80                  85                  90

Ala Ile Thr Asp Glu Gly Met Asp Val Pro Leu Ala Met Glu Glu
                95                  100                 105

Trp His His Glu Arg Glu His Tyr Asn Leu Ser Ala Ala Thr Cys
                110                 115                 120

Ser Pro Gly Gln Met Cys Gly His Tyr Thr Gln Val Val Trp Ala
                125                 130                 135

Lys Thr Glu Arg Ile Gly Cys Gly Ser His Phe Cys Glu Lys Leu
                140                 145                 150

Gln Gly Val Glu Glu Thr Asn Ile Glu Leu Leu Val Cys Asn Tyr
                155                 160                 165

Glu Pro Pro Gly Asn Val Lys Gly Lys Arg Pro Tyr Gln Glu Gly
                170                 175                 180

Thr Pro Cys Ser Gln Cys Pro Ser Gly Tyr His Cys Lys Asn Ser

                185                 190                 195

Leu Cys Glu Pro Ile Gly Ser Pro Glu Asp Ala Gln Asp Leu Pro
                200                 205                 210

Tyr Leu Val Thr Glu Ala Pro Ser Phe Arg Ala Thr Glu Ala Ser
                215                 220                 225

Asp Ser Arg Lys Met Gly Thr Pro Ser Ser Leu Ala Thr Gly Ile
                230                 235                 240

Pro Ala Phe Leu Val Thr Glu Val Ser Gly Ser Leu Ala Thr Lys
                245                 250                 255

Ala Leu Pro Ala Val Glu Thr Gln Ala Pro Thr Ser Leu Ala Thr
                260                 265                 270

Lys Asp Pro Pro Ser Met Ala Thr Glu Ala Pro Pro Cys Val Thr
                275                 280                 285

Thr Glu Val Pro Ser Ile Leu Ala Ala His Ser Leu Pro Ser Leu
                290                 295                 300

Asp Glu Glu Pro Val Thr Phe Pro Lys Ser Thr His Val Pro Ile
                305                 310                 315

Pro Lys Ser Ala Asp Lys Val Thr Asp Lys Thr Lys Val Pro Ser
                320                 325                 330

Arg Ser Pro Glu Asn Ser Leu Asp Pro Lys Met Ser Leu Thr Gly
                335                 340                 345

Ala Arg Glu Leu Leu Pro His Ala Gln Glu Glu Ala Glu Ala Glu
                350                 355                 360

Ala Glu Leu Pro Pro Ser Ser Glu Val Leu Ala Ser Val Phe Pro
                365                 370                 375

Ala Gln Asp Lys Pro Gly Glu Leu Gln Ala Thr Leu Asp His Thr
                380                 385                 390

Gly His Thr Ser Ser Lys Ser Leu Pro Asn Phe Pro Asn Thr Ser
                395                 400                 405

Ala Thr Ala Asn Ala Thr Gly Gly Arg Ala Leu Ala Leu Gln Ser
                410                 415                 420

Ser Leu Pro Gly Ala Glu Gly Pro Asp Lys Pro Ser Val Val Ser
                425                 430                 435

Gly Leu Asn Ser Gly Pro Gly His Val Trp Gly Pro Leu Leu Gly
                440                 445                 450

Leu Leu Leu Leu Pro Pro Leu Val Leu Ala Gly Ile Phe
455 460 463

<210> 31
<211> 19
<212> DNA
<213> Artificial Sequence

<220>
<223> Synthetic Oligonucleotide Probe

<400> 31

tcctgcagtt tcctgatgc 19

<210> 32
<211> 24
<212> DNA
<213> Artificial Sequence

<220>
<223> Synthetic Oligonucleotide Probe

<400> 32
ctcatattgc acaccagtaa ttcg 24

<210> 33
<211> 45
<212> DNA
<213> Artificial Sequence

<220>
<223> Synthetic Oligonucleotide Probe

<400> 33
atgaggagaa acgtttgatg gtggagctgc acaacctcta ccggg 45

<210> 34
<211> 1857
<212> DNA
<213> Homo Sapien

<400> 34
gtctgttccc aggagtcctt cggcggctgt tgtgtcagtg gcctgatcgc 50

gatggggaca aaggcgcaag tcgagaggaa actgttgtgc ctcttcatat 100

tggcgatcct gttgtgctcc ctggcattgg gcagtgttac agtgcactct 150

tctgaacctg aagtcagaat tcctgagaat aatcctgtga agttgtcctg 200

tgcctactcg ggcttttctt ctccccgtgt ggagtggaag tttgaccaag 250

gagacaccac cagactcgtt tgctataata acaagatcac agcttcctat 300

gaggaccggg tgaccttctt gccaactggt atcaccttca agtccgtgac 350

acgggaagac actgggacat acacttgtat ggtctctgag gaaggcggca 400

acagctatgg ggaggtcaag gtcaagctca tcgtgcttgt gcctccatcc 450

aagcctacag ttaacatccc ctcctctgcc accattggga accgggcagt 500

gctgacatgc tcagaacaag atggttcccc accttctgaa tacacctggt 550

tcaaagatgg gatagtgatg cctacgaatc ccaaaagcac ccgtgccttc 600

agcaactctt cctatgtcct gaatcccaca acaggagagc tggtctttga 650

tcccctgtca gcctctgata ctggagaata cagctgtgag gcacggaatg 700

ggtatgggac acccatgact tcaaatgctg tgcgcatgga agctgtggag 750

cggaatgtgg gggtcatcgt ggcagccgtc cttgtaaccc tgattctcct 800

gggaatcttg gtttttggca tctggtttgc ctatagccga ggccactttg 850

acagaacaaa gaaagggact tcgagtaaga aggtgattta cagccagcct 900

agtgcccgaa gtgaaggaga attcaaacag acctcgtcat tcctggtgtg 950

agcctggtcg gctcaccgcc tatcatctgc atttgcctta ctcaggtgct 1000

accggactct ggcccctgat gtctgtagtt tcacaggatg ccttatttgt 1050

cttctacacc ccacagggcc ccctacttct tcggatgtgt ttttaataat 1100

gtcagctatg tgccccatcc tccttcatgc cctccctccc tttcctacca 1150

ctgctgagtg gcctggaact tgtttaaagt gtttattccc catttctttg 1200

agggatcagg aaggaatcct gggtatgcca ttgacttccc ttctaagtag 1250

acagcaaaaa tggcgggggt cgcaggaatc tgcactcaac tgcccacctg 1300

gctggcaggg atctttgaat aggtatcttg agcttggttc tgggctcttt 1350

ccttgtgtac tgacgaccag ggccagctgt tctagagcgg gaattagagg 1400

ctagagcggc tgaaatggtt gtttggtgat gacactgggg tccttccatc 1450

tctggggccc actctcttct gtcttcccat gggaagtgcc actgggatcc 1500

ctctgccctg tcctcctgaa tacaagctga ctgacattga ctgtgtctgt 1550

ggaaaatggg agctcttgtt gtggagagca tagtaaattt tcagagaact 1600

tgaagccaaa aggatttaaa accgctgctc taaagaaaag aaaactggag 1650

gctgggcgca gtggctcacg cctgtaatcc cagaggctga ggcaggcgga 1700

tcacctgagg tcgggagttc gggatcagcc tgaccaacat ggagaaaccc 1750

tactggaaat acaaagttag ccaggcatgg tggtgcatgc ctgtagtccc 1800

agctgctcag gagcctggca acaagagcaa aactccagct caaaaaaaaa 1850

aaaaaaa 1857

<210> 35
<211> 299
<212> PRT
<213> Homo Sapien

<400> 35
Met Gly Thr Lys Ala Gln Val Glu Arg Lys Leu Leu Cys Leu Phe
1               5                   10                  15

Ile Leu Ala Ile Leu Leu Cys Ser Leu Ala Leu Gly Ser Val Thr
                20                  25                  30

Val His Ser Ser Glu Pro Glu Val Arg Ile Pro Glu Asn Asn Pro
                35                  40                  45

Val Lys Leu Ser Cys Ala Tyr Ser Gly Phe Ser Ser Pro Arg Val
                50                  55                  60

Glu Trp Lys Phe Asp Gln Gly Asp Thr Thr Arg Leu Val Cys Tyr
                65                  70                  75

Asn Asn Lys Ile Thr Ala Ser Tyr Glu Asp Arg Val Thr Phe Leu
                80                  85                  90

Pro Thr Gly Ile Thr Phe Lys Ser Val Thr Arg Glu Asp Thr Gly
                95                  100                 105

Thr Tyr Thr Cys Met Val Ser Glu Glu Gly Gly Asn Ser Tyr Gly
                110                 115                 120

Glu Val Lys Val Lys Leu Ile Val Leu Val Pro Pro Ser Lys Pro
                125                 130                 135

Thr Val Asn Ile Pro Ser Ser Ala Thr Ile Gly Asn Arg Ala Val
                140                 145                 150

Leu Thr Cys Ser Glu Gln Asp Gly Ser Pro Pro Ser Glu Tyr Thr
                155                 160                 165

Trp Phe Lys Asp Gly Ile Val Met Pro Thr Asn Pro Lys Ser Thr
                170                 175                 180

Arg Ala Phe Ser Asn Ser Ser Tyr Val Leu Asn Pro Thr Thr Gly
                185                 190                 195

Glu Leu Val Phe Asp Pro Leu Ser Ala Ser Asp Thr Gly Glu Tyr
                200                 205                 210

Ser Cys Glu Ala Arg Asn Gly Tyr Gly Thr Pro Met Thr Ser Asn
                215                 220                 225

Ala Val Arg Met Glu Ala Val Glu Arg Asn Val Gly Val Ile Val
                230                 235                 240

Ala Ala Val Leu Val Thr Leu Ile Leu Leu Gly Ile Leu Val Phe
                245                 250                 255

Gly Ile Trp Phe Ala Tyr Ser Arg Gly His Phe Asp Arg Thr Lys
                260                 265                 270

Lys Gly Thr Ser Ser Lys Lys Val Ile Tyr Ser Gln Pro Ser Ala
                275                 280                 285

Arg Ser Glu Gly Glu Phe Lys Gln Thr Ser Ser Phe Leu Val
                290                 295             299

<210> 36
<211> 24
<212> DNA
<213> Artificial Sequence

<220>
<223> Synthetic Oligonucleotide Probe

<400> 36
tcgcggagct gtgttctgtt tccc 24

<210> 37
<211> 20
<212> DNA
<213> Artificial Sequence

<220>
<223> Synthetic Oligonucleotide Probe

<400> 37
acacctggtt caaagatggg 20

<210> 38
<211> 20
<212> DNA
<213> Artificial Sequence

<220>
<223> Synthetic Oligonucleotide Probe

<400> 38
ttgccttact caggtgctac 20

<210> 39
<211> 24
<212> DNA
<213> Artificial Sequence

<220>
<223> Synthetic Oligonucleotide Probe

<400> 39
taggaagagt tgctgaaggc acgg 24

<210> 40
<211> 20
<212> DNA
<213> Artificial Sequence

<220>
<223> Synthetic Oligonucleotide Probe

<400> 40
actcagcagt ggtaggaaag 20

<210> 41
<211> 50
<212> DNA
<213> Artificial Sequence

<220>
<223> Synthetic Oligonucleotide Probe

<400> 41
tgatcgcgat ggggacaaag gcgcaagctc gagaggaaac tgttgtgcct 50

<210> 42
<211> 2236
<212> DNA
<213> Homo Sapien

<400> 42
ggcgccggtg caccgggcgg gctgagcgcc tcctgcggcc cggcctgcgc 50

gccccggccc gccgcgccgc ccacgcccca accccggccc gcgcccccta 100

gcccccgccc gggcccgcgc ccgcgcccgc gcccaggtga gcgctccgcc 150

cgccgcgagg ccccgccccg gcccgccccc gccccgcccc ggccggcggg 200

ggaaccgggc ggattcctcg cgcgtcaaac cacctgatcc cataaaacat 250

tcatcctccc ggcggcccgc gctgcgagcg ccccgccagt ccgcgccgcc 300

gccgccctcg ccctgtgcgc cctgcgcgcc ctgcgcaccc gcggcccgag 350

cccagccaga gccgggcgga gcggagcgcg ccgagcctcg tcccgcggcc 400
gggccggggc cgggccgtag cggcggcgcc tggatgcgga cccggccgcg 450
gggagacggg cgcccgcccc gaaacgactt tcagtccccg acgcgccccg 500
cccaacccct acgatgaaga gggcgtccgc tggagggagc cggctgctgg 550
catgggtgct gtggctgcag gcctggcagg tggcagcccc atgcccaggt 600
gcctgcgtat gctacaatga gcccaaggtg acgacaagct gcccccagca 650
gggcctgcag gctgtgcccg tgggcatccc tgctgccagc cagcgcatct 700
tcctgcacgg caaccgcatc tcgcatgtgc cagctgccag cttccgtgcc 750
tgccgcaacc tcaccatcct gtggctgcac tcgaatgtgc tggcccgaat 800
tgatgcggct gccttcactg gcctggccct cctggagcag ctggacctca 850
gcgataatgc acagctccgg tctgtggacc ctgccacatt ccacggcctg 900
ggccgcctac acacgctgca cctggaccgc tgcggcctgc aggagctggg 950
cccggggctg ttccgcggcc tggctgccct gcagtacctc tacctgcagg 1000
acaacgcgct gcaggcactg cctgatgaca ccttccgcga cctgggcaac 1050
ctcacacacc tcttcctgca cggcaaccgc atctccagcg tgcccgagcg 1100
cgccttccgt gggctgcaca gcctcgaccg tctcctactg caccagaacc 1150
gcgtggccca tgtgcacccg catgccttcc gtgaccttgg ccgcctcatg 1200
acactctatc tgtttgccaa caatctatca gcgctgccca ctgaggccct 1250
ggcccccctg cgtgccctgc agtacctgag gctcaacgac aacccctggg 1300
tgtgtgactg ccgggcacgc ccactctggg cctggctgca gaagttccgc 1350
ggctcctcct ccgaggtgcc ctgcagcctc ccgcaacgcc tggctggccg 1400
tgacctcaaa cgcctagctg ccaatgacct gcagggctgc gctgtggcca 1450
ccggccctta ccatcccatc tggaccggca gggccaccga tgaggagccg 1500
ctggggcttc ccaagtgctg ccagccagat gccgctgaca aggcctcagt 1550
actggagcct ggaagaccag cttcggcagg caatgcgctg aagggacgcg 1600
tgccgcccgg tgacagcccg ccgggcaacg gctctggccc acggcacatc 1650
aatgactcac cctttgggac tctgcctggc tctgctgagc ccccgctcac 1700
tgcagtgcgg cccgagggct ccgagccacc agggttcccc acctcgggcc 1750
ctcgccggag gccaggctgt tcacgcaaga accgcacccg cagccactgc 1800
cgtctgggcc aggcaggcag cgggggtggc gggactggtg actcagaagg 1850
ctcaggtgcc ctacccagcc tcacctgcag cctcaccccc ctgggcctgg 1900
cgctggtgct gtggacagtg cttgggccct gctgaccccc agcggacaca 1950
agagcgtgct cagcagccag gtgtgtgtac atacggggtc tctctccacg 2000

ccgccaagcc agccgggcgg ccgacccgtg gggcaggcca ggccaggtcc 2050

tccctgatgg acgcctgccg cccgccaccc ccatctccac cccatcatgt 2100

ttacagggtt cggcggcagc gtttgttcca gaacgccgcc tcccacccag 2150

atcgcggtat atagagatat gcattttatt ttacttgtgt aaaaatatcg 2200

gacgacgtgg aataaagagc tcttttctta aaaaaa 2236

<210> 43
<211> 473
<212> PRT
<213> Homo Sapien

<400> 43
Met Lys Arg Ala Ser Ala Gly Gly Ser Arg Leu Leu Ala Trp Val
1               5                   10                  15

Leu Trp Leu Gln Ala Trp Gln Val Ala Ala Pro Cys Pro Gly Ala
                20                  25                  30

Cys Val Cys Tyr Asn Glu Pro Lys Val Thr Thr Ser Cys Pro Gln
                35                  40                  45

Gln Gly Leu Gln Ala Val Pro Val Gly Ile Pro Ala Ala Ser Gln
                50                  55                  60

Arg Ile Phe Leu His Gly Asn Arg Ile Ser His Val Pro Ala Ala
                65                  70                  75

Ser Phe Arg Ala Cys Arg Asn Leu Thr Ile Leu Trp Leu His Ser
                80                  85                  90

Asn Val Leu Ala Arg Ile Asp Ala Ala Ala Phe Thr Gly Leu Ala
                95                  100                 105

Leu Leu Glu Gln Leu Asp Leu Ser Asp Asn Ala Gln Leu Arg Ser
                110                 115                 120

Val Asp Pro Ala Thr Phe His Gly Leu Gly Arg Leu His Thr Leu
                125                 130                 135

His Leu Asp Arg Cys Gly Leu Gln Glu Leu Gly Pro Gly Leu Phe
                140                 145                 150

Arg Gly Leu Ala Ala Leu Gln Tyr Leu Tyr Leu Gln Asp Asn Ala
                155                 160                 165

Leu Gln Ala Leu Pro Asp Asp Thr Phe Arg Asp Leu Gly Asn Leu
                170                 175                 180

Thr His Leu Phe Leu His Gly Asn Arg Ile Ser Ser Val Pro Glu
                185                 190                 195

Arg Ala Phe Arg Gly Leu His Ser Leu Asp Arg Leu Leu Leu His
                200                 205                 210

Gln Asn Arg Val Ala His Val His Pro His Ala Phe Arg Asp Leu
                215                 220                 225

Gly Arg Leu Met Thr Leu Tyr Leu Phe Ala Asn Asn Leu Ser Ala
                230                 235                 240

Leu Pro Thr Glu Ala Leu Ala Pro Leu Arg Ala Leu Gln Tyr Leu
                245                 250                 255

Arg Leu Asn Asp Asn Pro Trp Val Cys Asp Cys Arg Ala Arg Pro
                260                 265                 270

Leu Trp Ala Trp Leu Gln Lys Phe Arg Gly Ser Ser Ser Glu Val
                275                 280                 285

Pro Cys Ser Leu Pro Gln Arg Leu Ala Gly Arg Asp Leu Lys Arg
                290                 295                 300

Leu Ala Ala Asn Asp Leu Gln Gly Cys Ala Val Ala Thr Gly Pro
                305                 310                 315

Tyr His Pro Ile Trp Thr Gly Arg Ala Thr Asp Glu Glu Pro Leu
                320                 325                 330

Gly Leu Pro Lys Cys Cys Gln Pro Asp Ala Ala Asp Lys Ala Ser
                335                 340                 345

Val Leu Glu Pro Gly Arg Pro Ala Ser Ala Gly Asn Ala Leu Lys
                350                 355                 360

Gly Arg Val Pro Pro Gly Asp Ser Pro Pro Gly Asn Gly Ser Gly
                365                 370                 375

Pro Arg His Ile Asn Asp Ser Pro Phe Gly Thr Leu Pro Gly Ser
                380                 385                 390

Ala Glu Pro Pro Leu Thr Ala Val Arg Pro Glu Gly Ser Glu Pro
                395                 400                 405

Pro Gly Phe Pro Thr Ser Gly Pro Arg Arg Arg Pro Gly Cys Ser
                410                 415                 420

Arg Lys Asn Arg Thr Arg Ser His Cys Arg Leu Gly Gln Ala Gly
                425                 430                 435

Ser Gly Gly Gly Gly Thr Gly Asp Ser Glu Gly Ser Gly Ala Leu
                440                 445                 450

Pro Ser Leu Thr Cys Ser Leu Thr Pro Leu Gly Leu Ala Leu Val
                455                 460                 465

Leu Trp Thr Val Leu Gly Pro Cys
470 473

<210> 44
<211> 24
<212> DNA
<213> Artificial Sequence

<220>
<223> Synthetic Oligonucleotide Probe

<400> 44
tggctgccct gcagtacctc tacc 24

<210> 45
<211> 24
<212> DNA
<213> Artificial Sequence

<220>

<223> Synthetic Oligonucleotide Probe

<400> 45
 ccctgcaggt cattggcagc tagg 24

<210> 46
<211> 45
<212> DNA
<213> Artificial Sequence

<220>
<223> Synthetic Oligonucleotide Probe

<400> 46
 aggcactgcc tgatgacacc ttccgcgacc tgggcaacct cacac 45

<210> 47
<211> 2181
<212> DNA
<213> Homo Sapien

<400> 47
 cccacgcgtc cgcccacgcg tccgcccacg ggtccgccca cgcgtccggg 50

 ccaccagaag tttgagcctc tttggtagca ggaggctgga agaaaggaca 100

 gaagtagctc tggctgtgat ggggatctta ctgggcctgc tactcctggg 150

 gcacctaaca gtggacactt atggccgtcc catcctggaa gtgccagaga 200

 gtgtaacagg accttggaaa ggggatgtga atcttccctg cacctatgac 250

 cccctgcaag gctacaccca agtcttggtg aagtggctgg tacaacgtgg 300

 ctcagaccct gtcaccatct ttctacgtga ctcttctgga gaccatatcc 350

 agcaggcaaa gtaccagggc cgcctgcatg tgagccacaa ggttccagga 400

 gatgtatccc tccaattgag caccctggag atggatgacc ggagccacta 450

 cacgtgtgaa gtcacctggc agactcctga tggcaaccaa gtcgtgagag 500

 ataagattac tgagctccgt gtccagaaac tctctgtctc caagcccaca 550

 gtgacaactg gcagcggtta tggcttcacg gtgccccagg gaatgaggat 600

 tagccttcaa tgccaggctc ggggttctcc tcccatcagt tatatttggt 650

 ataagcaaca gactaataac caggaaccca tcaaagtagc aaccctaagt 700

 accttactct tcaagcctgc ggtgatagcc gactcaggct cctatttctg 750

 cactgccaag ggccaggttg gctctgagca gcacagcgac attgtgaagt 800

 ttgtggtcaa agactcctca aagctactca agaccaagac tgaggcacct 850

 acaaccatga catacccctt gaaagcaaca tctacagtga agcagtcctg 900

 ggactggacc actgacatgg atggctacct tggagagacc agtgctgggc 950

 caggaaagag cctgcctgtc tttgccatca tcctcatcat ctccttgtgc 1000

 tgtatggtgg tttttaccat ggcctatatc atgctctgtc ggaagacatc 1050

 ccaacaagag catgtctacg aagcagccag gtaagaaagt ctctcctctt 1100

ccatttttga ccccgtccct gccctcaatt ttgattactg gcaggaaatg 1150

tggaggaagg ggggtgtggc acagacccaa tcctaaggcc ggaggccttc 1200

agggtcagga catagctgcc ttccctctct caggcacctt ctgaggttgt 1250

tttggccctc tgaacacaaa ggataattta gatccatctg ccttctgctt 1300

ccagaatccc tgggtggtag gatcctgata attaattggc aagaattgag 1350

gcagaagggt gggaaaccag gaccacagcc ccaagtccct tcttatgggt 1400

ggtgggctct tgggccatag ggcacatgcc agagaggcca acgactctgg 1450

agaaaccatg agggtggcca tcttcgcaag tggctgctcc agtgatgagc 1500

caacttccca gaatctgggc aacaactact ctgatgagcc ctgcatagga 1550

caggagtacc agatcatcgc ccagatcaat ggcaactacg cccgcctgct 1600

ggacacagtt cctctggatt atgagtttct ggccactgag ggcaaaagtg 1650

tctgttaaaa atgccccatt aggccaggat ctgctgacat aattgcctag 1700

tcagtccttg ccttctgcat ggccttcttc cctgctacct ctcttcctgg 1750

atagcccaaa gtgtccgcct accaacactg gagccgctgg gagtcactgg 1800

ctttgccctg gaatttgcca gatgcatctc aagtaagcca gctgctggat 1850

ttggctctgg gcccttctag tatctctgcc gggggcttct ggtactcctc 1900
tctaaatacc agagggaaga tgcccatagc actaggactt ggtcatcatg 1950

cctacagaca ctattcaact ttggcatctt gccaccagaa gacccgaggg 2000

aggctcagct ctgccagctc agaggaccag ctatatccag gatcatttct 2050

ctttcttcag ggccagacag cttttaattg aaattgttat ttcacaggcc 2100

agggttcagt tctgctcctc cactataagt ctaatgttct gactctctcc 2150

tggtgctcaa taaatatcta atcataacag c 2181

<210> 48
<211> 321
<212> PRT
<213> Homo Sapien

<400> 48
Met Gly Ile Leu Leu Gly Leu Leu Leu Leu Gly His Leu Thr Val
1               5                   10                  15

Asp Thr Tyr Gly Arg Pro Ile Leu Glu Val Pro Glu Ser Val Thr
                20                  25                  30

Gly Pro Trp Lys Gly Asp Val Asn Leu Pro Cys Thr Tyr Asp Pro
                35                  40                  45

Leu Gln Gly Tyr Thr Gln Val Leu Val Lys Trp Leu Val Gln Arg
                50                  55                  60

Gly Ser Asp Pro Val Thr Ile Phe Leu Arg Asp Ser Ser Gly Asp
                65                  70                  75

His Ile Gln Gln Ala Lys Tyr Gln Gly Arg Leu His Val Ser His
                80                  85                  90

Lys Val Pro Gly Asp Val Ser Leu Gln Leu Ser Thr Leu Glu Met
                95                  100                 105

Asp Asp Arg Ser His Tyr Thr Cys Glu Val Thr Trp Gln Thr Pro
                110                 115                 120

Asp Gly Asn Gln Val Val Arg Asp Lys Ile Thr Glu Leu Arg Val
                125                 130                 135

Gln Lys Leu Ser Val Ser Lys Pro Thr Val Thr Thr Gly Ser Gly
                140                 145                 150

Tyr Gly Phe Thr Val Pro Gln Gly Met Arg Ile Ser Leu Gln Cys
                155                 160                 165

Gln Ala Arg Gly Ser Pro Pro Ile Ser Tyr Ile Trp Tyr Lys Gln
                170                 175                 180

Gln Thr Asn Asn Gln Glu Pro Ile Lys Val Ala Thr Leu Ser Thr
                185                 190                 195

Leu Leu Phe Lys Pro Ala Val Ile Ala Asp Ser Gly Ser Tyr Phe
                200                 205                 210

Cys Thr Ala Lys Gly Gln Val Gly Ser Glu Gln His Ser Asp Ile
                215                 220                 225

Val Lys Phe Val Val Lys Asp Ser Ser Lys Leu Leu Lys Thr Lys
                230                 235                 240

Thr Glu Ala Pro Thr Thr Met Thr Tyr Pro Leu Lys Ala Thr Ser
                245                 250                 255

Thr Val Lys Gln Ser Trp Asp Trp Thr Thr Asp Met Asp Gly Tyr
                260                 265                 270

Leu Gly Glu Thr Ser Ala Gly Pro Gly Lys Ser Leu Pro Val Phe
                275                 280                 285

Ala Ile Ile Leu Ile Ile Ser Leu Cys Cys Met Val Val Phe Thr
                290                 295                 300

Met Ala Tyr Ile Met Leu Cys Arg Lys Thr Ser Gln Gln Glu His
                305                 310                 315

Val Tyr Glu Ala Ala Arg
320 321

<210> 49
<211> 24
<212> DNA
<213> Artificial Sequence

<220>
<223> Synthetic Oligonucleotide Probe

<400> 49
tatccctcca attgagcacc ctgg 24

<210> 50
<211> 21
<212> DNA

<213> Artificial Sequence

<220>
<223> Synthetic Oligonucleotide Probe

<400> 50
gtcggaagac atcccaacaa g 21

<210> 51
<211> 24
<212> DNA
<213> Artificial Sequence

<220>
<223> Synthetic Oligonucleotide Probe

<400> 51
cttcacaatg tcgctgtgct gctc 24

<210> 52
<211> 24
<212> DNA
<213> Artificial Sequence

<220>
<223> Synthetic Oligonucleotide Probe

<400> 52
agccaaatcc agcagctggc ttac 24

<210> 53
<211> 50
<212> DNA
<213> Artificial Sequence

<220>
<223> Synthetic Oligonucleotide Probe

<400> 53
tggatgaccg gagccactac acgtgtgaag tcacctggca gactcctgat 50

<210> 54
<211> 2212
<212> DNA
<213> Homo Sapien

<400> 54
gaaagctata ggctacccat tcagctcccc tgtcagagac tcaagctttg 50

agaaaggcta gcaaagagca aggaaagaga gaaaacaaca aagtggcgag 100

gccctcagag tgaaagcgta aggttcagtc agcctgctgc agctttgcag 150

acctcagctg ggcatctcca gactcccctg aaggaagagc cttcctcacc 200

caaacccaca aaagatgctg aaaaagcctc tctcagctgt gacctggctc 250

tgcattttca tcgtggcctt tgtcagccac ccagcgtggc tgcagaagct 300

ctctaagcac aagacaccag cacagccaca gctcaaagcg gccaactgct 350

gtgaggaggt gaaggagctc aaggcccaag ttgccaacct tagcagcctg 400

ctgagtgaac tgaacaagaa gcaggagagg gactgggtca gcgtggtcat 450

gcaggtgatg gagctggaga gcaacagcaa gcgcatggag tcgcggctca 500
cagatgctga gagcaagtac tccgagatga acaaccaaat tgacatcatg 550
cagctgcagg cagcacagac ggtcactcag acctccgcag atgccatcta 600
cgactgctct tccctctacc agaagaacta ccgcatctct ggagtgtata 650
agcttcctcc tgatgacttc ctgggcagcc ctgaactgga ggtgttctgt 700
gacatggaga cttcaggcgg aggctggacc atcatccaga gacgaaaaag 750
tggccttgtc tccttctacc gggactggaa gcagtacaag cagggctttg 800
gcagcatccg tggggacttc tggctgggga acgaacacat ccaccggctc 850
tccagacagc caacccggct gcgtgtagag atggaggact gggagggcaa 900
cctgcgctac gctgagtata gccactttgt tttgggcaat gaactcaaca 950
gctatcgcct cttcctgggg aactacactg gcaatgtggg gaacgacgcc 1000
ctccagtatc ataacaacac agccttcagc accaaggaca aggacaatga 1050
caactgcttg gacaagtgtg cacagctccg caaaggtggc tactggtaca 1100
actgctgcac agactccaac ctcaatggag tgtactaccg cctgggtgag 1150
cacaataagc acctggatgg catcacctgg tatggctggc atggatctac 1200
ctactccctc aaacgggtgg agatgaaaat ccgcccagaa gacttcaagc 1250
cttaaaagga ggctgccgtg gagcacggat acagaaactg agacacgtgg 1300
agactggatg agggcagatg aggacaggaa gagagtgtta gaaagggtag 1350
gactgagaaa cagcctataa tctccaaaga aagaataagt ctccaaggag 1400
cacaaaaaaa tcatatgtac caaggatgtt acagtaaaca ggatgaacta 1450
tttaaaccca ctgggtcctg ccacatcctt ctcaaggtgg tagactgagt 1500
ggggtctctc tgcccaagat ccctgacata gcagtagctt gtcttttcca 1550
catgatttgt ctgtgaaaga aaataatttt gagatcgttt tatctatttt 1600
ctctacggct taggctatgt gagggcaaaa cacaaatccc tttgctaaaa 1650
agaaccatat tattttgatt ctcaaaggat aggcctttga gtgttagaga 1700
aaggagtgaa ggaggcaggt gggaaatggt atttctattt ttaaatccag 1750
tgaaattatc ttgagtctac acattatttt taaaacacaa aaattgttcg 1800
gctggaactg acccaggctg gacttgcggg gaggaaactc cagggcactg 1850
catctggcga tcagactctg agcactgccc ctgctcgcct tggtcatgta 1900
cagcactgaa aggaatgaag caccagcagg aggtggacag agtctctcat 1950
ggatgccggc acaaaactgc cttaaaatat tcatagttaa tacaggtata 2000
tctattttta tttactttgt aagaaacaag ctcaaggagc ttccttttaa 2050
attttgtctg taggaaatgg ttgaaaactg aaggtagatg gtgttatagt 2100

taataataaa tgctgtaaat aagcatctca ctttgtaaaa ataaaatatt 2150

gtggttttgt tttaaacatt caacgtttct tttccttcta caataaacac 2200

tttcaaaatg tg 2212

<210> 55
<211> 346
<212> PRT
<213> Homo Sapien

<400> 55
Met Leu Lys Lys Pro Leu Ser Ala Val Thr Trp Leu Cys Ile Phe
1               5                   10                  15

Ile Val Ala Phe Val Ser His Pro Ala Trp Leu Gln Lys Leu Ser
                20                  25                  30

Lys His Lys Thr Pro Ala Gln Pro Gln Leu Lys Ala Ala Asn Cys
                35                  40                  45

Cys Glu Glu Val Lys Glu Leu Lys Ala Gln Val Ala Asn Leu Ser
                50                  55                  60
Ser Leu Leu Ser Glu Leu Asn Lys Lys Gln Glu Arg Asp Trp Val
                65                  70                  75

Ser Val Val Met Gln Val Met Glu Leu Glu Ser Asn Ser Lys Arg
                80                  85                  90

Met Glu Ser Arg Leu Thr Asp Ala Glu Ser Lys Tyr Ser Glu Met
                95                  100                 105

Asn Asn Gln Ile Asp Ile Met Gln Leu Gln Ala Ala Gln Thr Val
                110                 115                 120

Thr Gln Thr Ser Ala Asp Ala Ile Tyr Asp Cys Ser Ser Leu Tyr
                125                 130                 135

Gln Lys Asn Tyr Arg Ile Ser Gly Val Tyr Lys Leu Pro Pro Asp
                140                 145                 150

Asp Phe Leu Gly Ser Pro Glu Leu Glu Val Phe Cys Asp Met Glu
                155                 160                 165

Thr Ser Gly Gly Gly Trp Thr Ile Ile Gln Arg Arg Lys Ser Gly
                170                 175                 180

Leu Val Ser Phe Tyr Arg Asp Trp Lys Gln Tyr Lys Gln Gly Phe
                185                 190                 195

Gly Ser Ile Arg Gly Asp Phe Trp Leu Gly Asn Glu His Ile His
                200                 205                 210

Arg Leu Ser Arg Gln Pro Thr Arg Leu Arg Val Glu Met Glu Asp
                215                 220                 225

Trp Glu Gly Asn Leu Arg Tyr Ala Glu Tyr Ser His Phe Val Leu
                230                 235                 240

Gly Asn Glu Leu Asn Ser Tyr Arg Leu Phe Leu Gly Asn Tyr Thr
                245                 250                 255

Gly Asn Val Gly Asn Asp Ala Leu Gln Tyr His Asn Asn Thr Ala
                260                 265                 270

Phe Ser Thr Lys Asp Lys Asp Asn Asp Asn Cys Leu Asp Lys Cys
                275                 280                 285

Ala Gln Leu Arg Lys Gly Gly Tyr Trp Tyr Asn Cys Cys Thr Asp
                290                 295                 300

Ser Asn Leu Asn Gly Val Tyr Tyr Arg Leu Gly Glu His Asn Lys
                305                 310                 315

His Leu Asp Gly Ile Thr Trp Tyr Gly Trp His Gly Ser Thr Tyr
                320                 325                 330

Ser Leu Lys Arg Val Glu Met Lys Ile Arg Pro Glu Asp Phe Lys
                335                 340                 345

Pro
346

<210> 56
<211> 31
<212> DNA
<213> Artificial Sequence

<220>
<223> Synthetic Oligonucleotide Probe

<400> 56
ttcagcacca aggacaagga caatgacaac t 31

<210> 57
<211> 32
<212> DNA
<213> Artificial Sequence

<220>
<223> Synthetic Oligonucleotide Probe

<400> 57
tgtgcacact tgtccaagca gttgtcattg tc 32

<210> 58
<211> 23
<212> DNA
<213> Artificial Sequence

<220>
<223> Synthetic Oligonucleotide Probe

<400> 58
gtagtacact ccattgaggt tgg 23

<210> 59
<211> 1049
<212> DNA
<213> Homo Sapien

<400> 59
gccgcagcaa tggcgctgag ttcctctgct ggagttcatc ctgctagctg 50

ggttcccgag ctgccggtct gagcctgagg catggagcct cctggagact 100

gggggcctcc tccctggaga tccaccccca gaaccgacgt cttgaggctg 150

gtgctgtatc tcaccttcct gggagccccc tgctacgccc cagctctgcc 200

gtcctgcaag gaggacgagt acccagtggg ctccgagtgc tgccccaagt 250

gcagtccagg ttatcgtgtg aaggaggcct gcggggagct gacgggcaca 300

gtgtgtgaac cctgccctcc aggcacctac attgcccacc tcaatggcct 350

aagcaagtgt ctgcagtgcc aaatgtgtga cccagccatg ggcctgcgcg 400

cgagccggaa ctgctccagg acagagaacg ccgtgtgtgg ctgcagccca 450

ggccacttct gcatcgtcca ggacggggac cactgcgccg cgtgccgcgc 500

ttacgccacc tccagcccgg gccagagggt gcagaaggga ggcaccgaga 550

gtcaggacac cctgtgtcag aactgccccc cggggacctt ctctcccaat 600

gggaccctgg aggaatgtca gcaccagacc aagtgcagct ggctggtgac 650

gaaggccgga gctgggacca gcagctccca ctgggtatgg tggtttctct 700
cagggagcct cgtcatcgtc attgtttgct ccacagttgg cctaatcata 750

tgtgtgaaaa gaagaaagcc aaggggtgat gtagtcaagg tgatcgtctc 800

cgtccagcgg aaaagacagg aggcagaagg tgaggccaca gtcattgagg 850

ccctgcaggc ccctccggac gtcaccacgg tggccgtgga ggagacaata 900

ccctcattca cggggaggag cccaaaccac tgacccacag actctgcacc 950

ccgacgccag agatacctgg agcgacggct gctgaaagag gctgtccacc 1000

tggcgaaacc accggagccc ggaggcttgg gggctccgcc ctgggctgg 1049

<210> 60
<211> 283
<212> PRT
<213> Homo Sapien

<400> 60

<210> 61
<211> 1847
<212> DNA
<213> Homo Sapien

<400> 61
gccaggggaa gagggtgatc cgacccgggg aaggtcgctg ggcagggcga 50

gttgggaaag cggcagcccc cgccgccccc gcagcccctt ctcctccttt 100

ctcccacgtc ctatctgcct ctcgctggag gccaggccgt gcagcatcga 150

agacaggagg aactggagcc tcattggccg gcccggggcg ccggcctcgg 200

gcttaaatag gagctccggg ctctggctgg gacccgaccg ctgccggccg 250

cgctcccgct gctcctgccg ggtgatggaa aaccccagcc cggccgccgc 300

cctgggcaag gccctctgcg ctctcctcct ggccactctc ggcgccgccg 350

gccagcctct tgggggagag tccatctgtt ccgccagagc cccggccaaa 400

tacagcatca ccttcacggg caagtggagc cagacggcct tccccaagca 450

gtaccccctg ttccgccccc ctgcgcagtg gtcttcgctg ctgggggccg 500

cgcatagctc cgactacagc atgtggagga agaaccagta cgtcagtaac 550

gggctgcgcg actttgcgga gcgcggcgag gcctgggcgc tgatgaagga 600

gatcgaggcg gcgggggagg cgctgcagag cgtgcacgag gtgttttcgg 650

cgcccgccgt ccccagcggc accgggcaga cgtcggcgga gctggaggtg 700

cagcgcaggc actcgctggt ctcgtttgtg gtgcgcatcg tgcccagccc 750

cgactggttc gtgggcgtgg acagcctgga cctgtgcgac ggggaccgtt 800

ggcgggaaca ggcggcgctg gacctgtacc cctacgacgc cgggacggac 850

agcggcttca ccttctcctc ccccaacttc gccaccatcc cgcaggacac 900

ggtgaccgag ataacgtcct cctctcccag ccacccggcc aactccttct 950

actacccgcg gctgaaggcc ctgcctccca tcgccagggt gacactgctg 1000

cggctgcgac agagccccag ggccttcatc cctcccgccc cagtcctgcc 1050

cagcagggac aatgagattg tagacagcgc ctcagttcca gaaacgccgc 1100

tggactgcga ggtctccctg tggtcgtcct ggggactgtg cggaggccac 1150

tgtgggaggc tcgggaccaa gagcaggact cgctacgtcc gggtccagcc 1200

cgccaacaac gggagcccct gccccgagct cgaagaagag gctgagtgcg 1250

tccctgataa ctgcgtctaa gaccagagcc ccgcagcccc tggggccccc 1300

cggagccatg gggtgtcggg ggctcctgtg caggctcatg ctgcaggcgg 1350

ccgagggcac agggggtttc gcgctgctcc tgaccgcggt gaggccgcgc 1400

cgaccatctc tgcactgaag ggccctctgg tggccggcac gggcattggg 1450

aaacagcctc ctcctttccc aaccttgctt cttaggggcc cccgtgtccc 1500

gtctgctctc agcctcctcc tcctgcagga taaagtcatc cccaaggctc 1550

cagctactct aaattatgtc tccttataag ttattgctgc tccaggagat 1600

tgtccttcat cgtccagggg cctggctccc acgtggttgc agatacctca 1650

gacctggtgc tctaggctgt gctgagccca ctctcccgag ggcgcatcca 1700

agcgggggcc acttgagaag tgaataaatg gggcggtttc ggaagcgtca 1750

gtgtttccat gttatggatc tctctgcgtt tgaataaaga ctatctctgt 1800

tgctcacaaa aaaaaaaaaa aaaaaaaaaa aaaaaaaaaa aaaaaaa 1847

<210> 62
<211> 331
<212> PRT
<213> Homo Sapien

<400> 62

<210> 63
<211> 22
<212> DNA
<213> Artificial Sequence

<220>

<223> Synthetic Oligonucleotide Probe

<400> 63
cagcactgcc aggggaagag gg 22

<210> 64
<211> 18
<212> DNA
<213> Artificial Sequence

<220>
<223> Synthetic Oligonucleotide Probe
<400> 64
caggactcgc tacgtccg 18

<210> 65
<211> 24
<212> DNA
<213> Artificial Sequence

<220>
<223> Synthetic Oligonucleotide Probe

<400> 65
cagccccttc tcctcctttc tccc 24

<210> 66
<211> 25
<212> DNA
<213> Artificial Sequence

<220>
<223> Synthetic Oligonucleotide Probe

<400> 66
gcagttatca gggacgcact cagcc 25

<210> 67
<211> 18
<212> DNA
<213> Artificial Sequence

<220>
<223> Synthetic Oligonucleotide Probe

<400> 67
ccagcgagag gcagatag 18

<210> 68
<211> 23
<212> DNA
<213> Artificial Sequence

<220>
<223> Synthetic Oligonucleotide Probe

<400> 68
cggtcaccgt gtcctgcggg atg 23

<210> 69
<211> 42
<212> DNA
<213> Artificial Sequence

<220>

<223> Synthetic Oligonucleotide Probe

<400> 69
 cagccccttc tcctcctttc tcccacgtcc tatctgcctc tc 42

<210> 70
<211> 47
<212> DNA
<213> Artificial Sequence

<220>
<223> Synthetic Oligonucleotide Probe

<400> 70
 ggattctaat acgactcact atagggctcc tgcgcctttc ctgaacc 47

<210> 71
<211> 48
<212> DNA
<213> Artificial Sequence

<220>
<223> Synthetic Oligonucleotide Probe

<400> 71
 ctatgaaatt aaccctcact aaagggagac ccatccttgc ccacagag 48

<210> 72
<211> 48
<212> DNA
<213> Artificial Sequence

<220>
<223> Synthetic Oligonucleotide Probe

<400> 72
 ggattctaat acgactcact atagggctgt gctttcattc tgccagta 48

<210> 73
<211> 48
<212> DNA
<213> Artificial Sequence

<220>
<223> Synthetic Oligonucleotide Probe

<400> 73
   ctatgaaatt aaccctcact aaagggaggg tacaattaag gggtggat 48

<210> 74
<211> 48
<212> DNA
<213> Artificial Sequence

<220>
<223> Synthetic Oligonucleotide Probe

<400> 74
 ggattctaat acgactcact atagggcgca gcgatggcag cgatgagg 48

<210> 75
<211> 48
<212> DNA
<213> Artificial Sequence

## Claims

1. Use of an agent for the preparation of a medicament for the treatment of tumour in a mammal, wherein the agent is:
a polypeptide having at least 80% amino acid sequence identity as determined by the ALIGN-2 computer program either (i) to the amino acid sequence of residues 1 or X to 331 shown in Figure 26 (SEQ ID NO : 62) , wherein X is any amino acid residue from 22 to 31; or (ii) to a fragment of the amino acid sequence shown in Figure 26, wherein the polypeptide is at least 10 amino acids in length; or
an agonist antibody of the polypeptide having the amino acid sequence shown in Figure 26 (SEQ ID NO:62).

2. The use according to claim 1, wherein the tumour is a cancer.

3. The use according to claim 2, wherein the cancer is selected from the group consisting of breast cancer, ovarian cancer, renal cancer, colorectal cancer, uterine cancer, prostate cancer, lung cancer, bladder cancer, central nervous system cancer, melanoma and leukaemia.

4. The use according to any preceding claim, wherein the level of identity is at least 90%.

5. The use according to claim 4, wherein the level of identity is at least 95%.

6. The use according to claim 5, wherein the polypeptide has the amino acid sequence of residues 1 or X to 331 shown in Figure 26 (SEQ ID NO:62) , wherein X is any amino acid residue from 22 to 31.

7. The use according to any preceding claim, wherein X is 27.

8. The use according to any preceding claim, wherein the polypeptide is at least 50 amino acids in length.

9. The use according to claim 8, wherein the polypeptide is at least 100 amino acids in length.

10. The use according to any preceding claim, wherein the polypeptide is at least 150 amino acids in length.

11. The use according to claim 10, wherein the polypeptide is at least 200 amino acids in length.

12. The use according to any preceding claim, wherein the medicament additionally comprises a further growth inhibitory agent, cytotoxic agent or chemotherapeutic agent.

13. The use according to any preceding claim, wherein the mammal is a human.

14. An *in vitro* method for inhibiting the growth of a tumour cell, the method comprising exposing said tumour cell to an effective amount of an agent as defined in any one of claims 1 to 11.

15. The method of claim 14, wherein the tumour is as defined in claim 2 or claim 3.
